# EUROPEAN PATENT APPLICATION

(11) **EP 4 806 899 A1**
(43) Date of publication of application: **16.09.2026**
(21) Application number: 24888076.7
(22) Date of filing: 08.11.2024
(51) Int. Cl.: C07D 491/052, C07D 401/04, C07D 401/14, C07D 413/04, C07D 471/04, A61K 31/4725, A61K 31/536, A61K 31/538, A61K 31/4709, A61P 5/40, A61P 9/12

(54) **FUSED RING DERIVATIVE, PREPARATION METHOD THEREFOR AND USE THEREOF IN MEDICINE**

(30) Priority: 10.11.2023 CN 202311501230; 19.12.2023 CN 202311755774; 19.03.2024 CN 202410316983; 24.04.2024 CN 202410502260; 25.04.2024 CN 202410510376; 18.07.2024 CN 202410970662; 01.11.2024 CN 202411558530
(71) Applicant: Shanghai Qilu Pharmaceutical Research and Development Centre Ltd., Shanghai 201203 (CN)
(72) Inventor: WANG, Jianfei, Shanghai 201203 (CN); QIAN, Wenyuan, Shanghai 201203 (CN); CHI, Bo, Shanghai 201203 (CN); WANG, Zheng, Shanghai 201203 (CN); GUO, Xin, Shanghai 201203 (CN); XU, Guiliang, Shanghai 201203 (CN); QIAN, Su, Shanghai 201203 (CN); SUN, Daqing, Shanghai 201203 (CN); TAO, Weikang, Shanghai 201203 (CN)
(74) Representative: Staeger & Sperling Partnerschaftsgesellschaft mbB
(86) International application number: PCT/CN2024/130756
(87) International publication number: WO 2025/098465

(57) **Abstract**

The present disclosure relates to a fused ring derivative, a preparation method therefor and a use thereof in medicine. Specifically, the present disclosure relates to a fused ring derivative represented by formula (IA), a preparation method therefor, a pharmaceutical composition containing the derivative and a use thereof as a therapeutic agent, in particular, a use thereof as a CYP11B2 inhibitor and a use thereof in preparation of drugs for treating and/or relieving CYP11B2-mediated diseases. (FIG. 1)

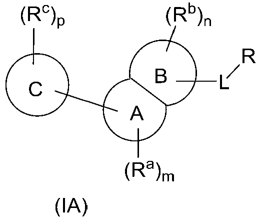

## Description

The present disclosure claims the priority to Chinese Patent Application No. CN202311501230.1, titled "FUSED RING DERIVATIVE, PREPARATION METHOD THEREFOR AND USE THEREOF IN MEDICINE", filed before the China National Intellectual Property Administration on November 10, 2023; Chinese Patent Application No. CN202311755774.0, titled "FUSED RING DERIVATIVE, PREPARATION METHOD THEREFOR AND USE THEREOF IN MEDICINE", filed before the China National Intellectual Property Administration on December 19, 2023; Chinese Patent Application No. CN202410316983.3, titled "FUSED RING DERIVATIVE, PREPARATION METHOD THEREFOR AND USE THEREOF IN MEDICINE", filed before the China National Intellectual Property Administration on March 19, 2024; Chinese Patent Application No. CN202410502260.2, titled "FUSED RING DERIVATIVE, PREPARATION METHOD THEREFOR AND USE THEREOF IN MEDICINE", filed before the China National Intellectual Property Administration on April 24, 2024; Chinese Patent Application No. CN202410510376.0, titled "FUSED RING DERIVATIVE, PREPARATION METHOD THEREFOR AND USE THEREOF IN MEDICINE", filed before the China National Intellectual Property Administration on April 25, 2024; Chinese Patent Application No. CN202410970662.5, titled "FUSED RING DERIVATIVE, PREPARATION METHOD THEREFOR AND USE THEREOF IN MEDICINE", filed before the China National Intellectual Property Administration on July 18, 2024; and Chinese Patent Application No. CN202411558530.8, titled "FUSED RING DERIVATIVE, PREPARATION METHOD THEREFOR AND USE THEREOF IN MEDICINE", filed before the China National Intellectual Property Administration on November 01, 2024, each of which is incorporated herein by reference in its entirety.

### TECHNICAL FIELD

The present disclosure belongs to the field of pharmaceuticals and relates to a fused ring derivative, a preparation method therefor and use thereof in medicine. Specifically, the present disclosure relates to a fused ring derivative of formula (IA), a preparation method therefor, a pharmaceutical composition containing the derivative, and use thereof as a therapeutic agent, in particular, use thereof as a CYP11B2 inhibitor and use thereof in preparation of medicaments for treating and/or preventing CYP11B2-mediated diseases.

### BACKGROUND

Hypertension is a significant risk factor for cardiovascular disease worldwide, affecting approximately 30% of the global population. A comprehensive report of worldwide trends in hypertension prevalence in *The Lancet* in 2021 stated that there were about 1.3 billion patients with hypertension worldwide (DOI: 10.1016/S0140-6736(21)01330-1). Inadequately controlled blood pressure over the long term may lead to vascular and organ damage, inducing heart disease, kidney injury, and retinopathy. Hypertension is closely related to diseases such as diabetes mellitus, chronic kidney disease, and obesity.

For the vast majority of hypertensive patients, blood pressure can be controlled to normal levels, i.e., below 140/90 millimeters of mercury, after using three different types of full-dose anti-hypertensive medicaments. Despite the availability of various anti-hypertensive medicaments, approximately 10% of patients with resistant hypertension still cannot achieve effective control of blood pressure even when using at least three different types of full-dose antihypertensive medicaments (the combination must include a diuretic), such as angiotensin II receptor blocker (ARB), angiotensin-converting enzyme inhibitor (ACE-I), diuretic, calcium channel blocker (CCB), and α-receptor/β-receptor blocker. Resistant hypertension is often caused by excessive sodium retention. A clinical experiment funded by the British Heart Foundation and initiated by the University of Cambridge in May 2009 (PATHWAY-2, ClinicalTrials.gov No. NCT02369081) confirmed: compared to adding an ACE inhibitor, ARB or renin inhibitor, CCB or diuretic, adding the mineralocorticoid receptor antagonist spironolactone enabled 60% of patients with resistant hypertension to achieve effective control of blood pressure (systolic blood pressure <135 mm Hg) within 3 months of treatment. Due to off-target blockade of multiple steroid hormone receptors by spironolactone, patients with resistant hypertension may experience side effects such as gynecomastia in males, menstrual disorder and postmenopausal bleeding in females after long-term medication. Additionally, spironolactone carries the risk of inducing hyperkalemia, greatly limiting its application in the treatment of resistant hypertension (DOI: 10.1016/S0140-6736(15)00257-3).

Aldosterone is synthesized by aldosterone synthase (AS, encoded by the CYP11B2 gene) and regulates salt-water balance. Aldosterone binds to and activates the mineralocorticoid receptor (MR) in the epithelial cells of the renal distal tubules and collecting ducts, promoting sodium and water retention while inducing the excretion of potassium and hydrogen ions. Excessive aldosterone can overactivate the mineralocorticoid receptor, causing significant sodium and water retention and potassium excretion, leading to hypokalemia, tissue edema, hypertension, and other symptoms. Furthermore, excessively high aldosterone levels can lead to inflammation associated with cardiometabolic disease, myocardial and vascular interstitial fibrosis, ventricular and vascular remodeling, induce leukocyte infiltration, and cause coronary and myocardial injury, arrhythmia, etc.

Aldosterone synthase (AS) is expressed in cells of the outer region of the adrenal zona glomerulosa (ZG). Aldosterone synthase has two isoenzymes, cortisol synthase (CYP11B1) and aldosterone synthase (CYP11B2), which share 93% homology. CYP11B1 converts 11-deoxycortisol to cortisol. Both CYP11B2 and CYP11B1 can 11-β-hydroxylate 11-deoxycorticosterone (11-DOC) to corticosterone. Subsequently, corticosterone is hydroxylated to 18-hydroxycorticosterone under the action of CYP11B2. Finally, CYP11B2 oxidizes 18-hydroxycorticosterone to generate aldosterone.

LCI699, developed by Novartis, was among the first aldosterone synthase inhibitors (ASIs) to enter clinical development. Preclinical data showed it was a dual inhibitor of CYP11B2 and CYP11B1, lowering aldosterone while further strongly inhibiting CYP11B1 to reduce blood cortisol. Phase I clinical data indicated that this compound could lower plasma aldosterone levels in healthy subjects and patients with primary aldosteronism, and could be used to treat patients with primary aldosteronism, primary hypertension, and resistant hypertension. However, at clinically effective therapeutic doses, LCI699 lacked sufficient selectivity for CYP11B2 (*in vitro* selectivity factor of 3 to 15), resulting in inhibition of cortisol synthesis. Novartis ultimately terminated the development plan for the hypertension indication, but its strategy of lowering plasma aldosterone as a human treatment for hypertension provided new directions for subsequent medicament development.

Cortisol synthesis is catalyzed by another enzyme that shares 93% sequence similarity with aldosterone synthase and plays an important physiological role. Selective inhibition of aldosterone synthase (AS) while avoiding inhibition of cortisol synthesis is necessary. Following the discovery of FAD286 and LCI699, novel next-generation aldosterone synthase inhibitors (ASIs) have been successively developed, with significantly improved effectiveness and selectivity. Aldosterone synthase inhibitors (ASIs) directly attenuate aldosterone production and have been proposed as upgraded alternatives for mineralocorticoid receptor antagonists for treating diseases caused by excessive aldosterone. Currently, the number of medicaments targeting this site that have entered the clinical medicament development stage is still relatively small. Therefore, developing highly specific and selective medicaments for CYP11B2 is expected to address the urgent needs of domestic patients with resistant hypertension.

### SUMMARY

The object of the present disclosure is to provide a compound of formula (IA), a stereoisomer thereof, an isotopic derivative thereof, a prodrug thereof, an N-oxide thereof, a solvate thereof, or a pharmaceutically acceptable salt thereof; 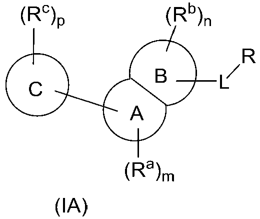 wherein:
ring A is 5- to 6-membered heteroaryl;
ring B is selected from the group consisting of 3- to 8-membered heterocyclyl, 3- to 8-membered monocyclic cycloalkyl, 3- to 8-membered monocyclic cycloalkenyl, 5- to 10-membered fused ring group, 5- to 10-membered spiro ring group, and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -(CR^{d}R^{e})ᵣ-, -(CR^{d}=CR^{e})ᵣ-, -O-(CR^{d}R^{e})ᵣ-, -(CR^{d}R^{e})r-O-, -NRⁿ¹-(CR^{d}R^{e})ᵣ-, -(CR^{d}R^{e})ᵣ-NRⁿ¹-, -(CR^{d}R^{e})ᵣ-C(O)-, -C(O)-(CR^{d}R^{e})ᵣ-, -O-C(O)-, -C(O)-O-, -O-C(O)-NRⁿ¹-, -NRⁿ¹-C(O)-O-, -NRⁿ¹-C(O)-NRⁿ¹-, -(CR^{d}R^{e})ᵣ-NRⁿ¹-C(O)-, -C(O)-NRⁿ¹-(CR^{d}R^{e})ᵣ-, -NRⁿ¹-C(O)-(CR^{d}R^{e})ᵣ-, -NRⁿ¹-S(O)₂-, and -S(O)₂-NRⁿ¹-;
R is selected from the group consisting of hydrogen atom, C₁₋₆ alkyl, C₂₋₆ alkenyl, C₂₋₆ alkynyl, halogen, cyano, -OR^{o}, -NRⁿ²Rⁿ³, 3- to 8-membered heterocyclyl, 3- to 8-membered cycloalkyl, 3- to 8-membered cycloalkenyl, 6- to 10-membered aryl and 5- to 10-membered heteroaryl, wherein the C₁₋₆ alkyl, C₂₋₆ alkenyl, C₂₋₆ alkynyl, 3- to 8-membered heterocyclyl, 3-to 8-membered cycloalkyl, 3- to 8-membered cycloalkenyl, 6- to 10-membered aryl and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, hydroxy, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or Rⁿ¹ and R together with the atom to which they are attached form 3- to 8-membered heterocyclyl or 5- to 10-membered heteroaryl; wherein the 3- to 8-membered heterocyclyl or 5-to 10-membered heteroaryl is each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
each R^{a}, R^{b} and R^{c} is identical or different, and is independently selected from the group consisting of hydrogen atom, deuterium atom, C₁₋₆ alkyl, -OR^{o}, -NRⁿ²Rⁿ³, halogen, cyano, oxo, -C(O)NRⁿ²Rⁿ³, -C(O)R^{o}, -C(O)OR^{o}, -C(O)NRⁿ¹OR^{o}, 3- to 8-membered heterocyclyl, 3- to 8-membered cycloalkyl, 3- to 8-membered cycloalkenyl, 6- to 10-membered aryl and 5- to 10-membered heteroaryl, wherein the C₁₋₆ alkyl, 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR°, -C(O)NRⁿ⁴OR^{o}, hydroxy, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or two R^{b} together with the atom to which they are attached form 3- to 8-membered heterocyclyl or 3- to 8-membered cycloalkyl, wherein the 3- to 8-membered heterocyclyl and 3-to 8-membered cycloalkyl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or two R^{c} together with the atom to which they are attached form 3- to 8-membered heterocyclyl or 3- to 8-membered cycloalkyl, wherein the 3- to 8-membered heterocyclyl and 3-to 8-membered cycloalkyl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
R^{d} and R^{e} are identical or different at each occurrence, and are each independently selected from the group consisting of hydrogen atom, deuterium atom, C₁₋₆ alkyl, halogen, deuterated C₁₋₆ alkyl, C₁₋₆ haloalkyl, C₁₋₆ hydroxyalkyl, C₂₋₆ alkenyl, C₂₋₆ alkynyl, C₂₋₆ haloalkenyl, C₂₋₆ haloalkynyl, -OR^{o} and C₁₋₆ haloalkoxy;
R^{o} is identical or different at each occurrence, and is independently selected from the group consisting of hydrogen atom, C₁₋₆ alkyl, C₁₋₆ haloalkyl, C₁₋₆ hydroxyalkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl and 5- to 10-membered heteroaryl; wherein the C₁₋₆ alkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
Rⁿ¹, Rⁿ², Rⁿ³, Rⁿ⁴ and Rⁿ⁵ are identical or different at each occurrence, and are independently selected from the group consisting of hydrogen atom, C₁₋₆ alkyl, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl; wherein the C₁₋₆ alkyl, 3- to 8-membered heterocyclyl, 3- to 8-membered cycloalkyl, 3- to 8-membered cycloalkenyl, 6-to 10-membered aryl and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or Rⁿ² and Rⁿ³, Rⁿ⁴ and Rⁿ⁵ together with the nitrogen atom to which they are attached respectively form 3- to 8-membered heterocyclyl; wherein the 3- to 8-membered heterocyclyl is optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
n is 0, 1, 2, 3, 4, 5, 6, 7, 8, 9, 10, 11 or 12;
m is 0, 1, 2, 3, 4, 5 or 6;
p is 0, 1, 2, 3, 4, 5, 6, 7, 8, 9, 10, 11 or 12; and
r is 0, 1, 2, 3 or 4.

The object of the present disclosure is to provide a compound of formula (IA), a stereoisomer thereof, an isotopic derivative thereof, a prodrug thereof, an N-oxide thereof, a solvate thereof, or a pharmaceutically acceptable salt thereof; 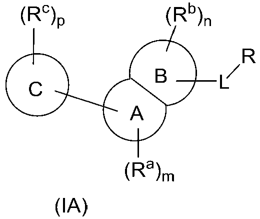 wherein:
ring A is 5- to 6-membered heteroaryl;
ring B is selected from the group consisting of 3- to 8-membered heterocyclyl, 3- to 8-membered monocyclic cycloalkyl, 3- to 8-membered monocyclic cycloalkenyl, 5- to 10-membered fused ring group, 5- to 10-membered spiro ring group, and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -(CR^{d}R^{e})ᵣ-, -(CR^{d}=CR^{e})ᵣ-, -O-(CR^{d}R^{e})ᵣ-, -(CR^{d}R^{e})ᵣ-O-, -NRⁿ¹-(CR^{d}R^{e})ᵣ-, -(CR^{d}R^{e})ᵣ-NRⁿ¹-, -(CR^{d}R^{e})ᵣ-C(O)-, -C(O)-(CR^{d}R^{e})ᵣ-, -O-C(O)-, -C(O)-O-, -O-C(O)-NRⁿ¹-, -NRⁿ¹-C(O)-O-, -NRⁿ¹-C(O)-NRⁿ¹-, -(CR^{d}R^{e})ᵣ-NRⁿ¹-C(O)-, -C(O)-NRⁿ¹-(CR^{d}R^{e})ᵣ-, -NRⁿ¹-C(O)-(CR^{d}R^{e})ᵣ-, -NRⁿ¹-S(O)₂-, and -S(O)₂-NRⁿ¹-;
R is selected from the group consisting of hydrogen atom, C₁₋₆ alkyl, C₂₋₆ alkenyl, C₂₋₆ alkynyl, halogen, cyano, -OR^{o}, -NRⁿ²Rⁿ³, 3- to 8-membered heterocyclyl, 3- to 8-membered cycloalkyl, 3- to 8-membered cycloalkenyl, 6- to 10-membered aryl and 5- to 10-membered heteroaryl, wherein the C₁₋₆ alkyl, C₂₋₆ alkenyl, C₂₋₆ alkynyl, 3- to 8-membered heterocyclyl, 3-to 8-membered cycloalkyl, 3- to 8-membered cycloalkenyl, 6- to 10-membered aryl and 5- to 10-membered heteroaryl are each independently optionally substituted with one or more substituents selected from the group consisting of deuterium atom, halogen, oxo, C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, hydroxy, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or Rⁿ¹ and R together with the atom to which they are attached form 3- to 8-membered heterocyclyl or 5- to 10-membered heteroaryl; wherein the 3- to 8-membered heterocyclyl or 5-to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
each R^{a}, R^{b} and R^{c} is identical or different, and is independently selected from the group consisting of hydrogen atom, C₁₋₆ alkyl, -OR^{o}, -NRⁿ²Rⁿ³, halogen, cyano, oxo, -C(O)NRⁿ²Rⁿ³, -C(O)R°, -C(O)OR°, -C(O)NRⁿ¹OR^{o}, 3- to 8-membered heterocyclyl, 3- to 8-membered cycloalkyl, 3- to 8-membered cycloalkenyl, 6- to 10-membered aryl and 5- to 10-membered heteroaryl, wherein the C₁₋₆ alkyl, 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, hydroxy, C₁₋₆ hydroxyalkyl, 3-to 8-membered heterocyclyl, 3- to 8-membered cycloalkyl, 3- to 8-membered cycloalkenyl, 6- to 10-membered aryl and 5- to 10-membered heteroaryl;
or two R^{b} together with the atom to which they are attached form 3- to 8-membered heterocyclyl or 3- to 8-membered cycloalkyl, wherein the 3- to 8-membered heterocyclyl and 3-to 8-membered cycloalkyl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or two R^{c} together with the atom to which they are attached form 3- to 8-membered heterocyclyl or 3- to 8-membered cycloalkyl, wherein the 3- to 8-membered heterocyclyl and 3-to 8-membered cycloalkyl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
R^{d} and R^{e} are identical or different at each occurrence, and are each independently selected from the group consisting of hydrogen atom, C₁₋₆ alkyl, halogen, C₁₋₆ haloalkyl, C₁₋₆ hydroxyalkyl, C₂₋₆ alkenyl, C₂₋₆ alkynyl, C₂₋₆ haloalkenyl, C₂₋₆ haloalkynyl, -OR^{o} and C₁₋₆ haloalkoxy;
R^{o} is identical or different at each occurrence, and is independently selected from the group consisting of hydrogen atom, C₁₋₆ alkyl, C₁₋₆ haloalkyl, C₁₋₆ hydroxyalkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl and 5- to 10-membered heteroaryl; wherein the C₁₋₆ alkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
Rⁿ¹, Rⁿ², Rⁿ³, Rⁿ⁴ and Rⁿ⁵ are identical or different at each occurrence, and are independently selected from the group consisting of hydrogen atom, C₁₋₆ alkyl, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl; wherein the C₁₋₆ alkyl, 3- to 8-membered heterocyclyl, 3- to 8-membered cycloalkyl, 3- to 8-membered cycloalkenyl, 6-to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or Rⁿ² and Rⁿ³, Rⁿ⁴ and Rⁿ⁵ together with the nitrogen atom to which they are attached respectively form 3- to 8-membered heterocyclyl; wherein the 3- to 8-membered heterocyclyl is optionally substituted with one or more substituents selected from the group consisting of halogen, oxo, C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
n is 0, 1, 2, 3, 4, 5, 6, 7, 8, 9, 10, **11** or 12;
m is 0, 1, 2, 3, 4, 5 or 6;
p is 0, 1, 2, 3, 4, 5, 6, 7, 8, 9, 10, **11** or 12; and
r is 0, 1, 2, 3 or 4.

The object of the present disclosure is to provide a compound of formula (IA), a stereoisomer thereof, an isotopic derivative thereof, a prodrug thereof, an N-oxide thereof, a solvate thereof, or a pharmaceutically acceptable salt thereof; 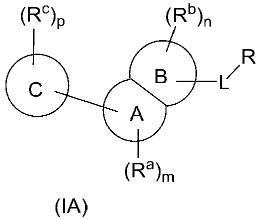 wherein:
ring A is 5- to 6-membered heteroaryl;
ring B is selected from the group consisting of 3- to 8-membered heterocyclyl, 3- to 8-membered monocyclic cycloalkyl, 3- to 8-membered monocyclic cycloalkenyl, 5- to 10-membered fused ring group, 5- to 10-membered spiro ring group, and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -(CR^{d}R^{e})ᵣ-, -(CR^{d}=CR^{e})ᵣ-, -O-(CR^{d}R^{e})ᵣ-, -(CR^{d}R^{e})ᵣ-O-, -NRⁿ¹-(CR^{d}R^{e})ᵣ-, -(CR^{d}R^{e})ᵣ-NRⁿ¹-, -(CR^{d}R^{e})ᵣ-C(O)-, -C(O)-(CR^{d}R^{e})ᵣ-, -O-C(O)-, -C(O)-O-, -O-C(O)-NRⁿ¹-, -NRⁿ¹-C(O)-O-, -NRⁿ¹-C(O)-NRⁿ¹-, -(CR^{d}R^{e})ᵣ-NRⁿ¹-C(O)-, -C(O)-NRⁿ¹-(CR^{d}R^{e})ᵣ-, -NRⁿ¹-C(O)-(CR^{d}R^{e})ᵣ-, -NRⁿ¹-S(O)₂-, and -S(O)₂-NRⁿ¹-;
R is selected from the group consisting of hydrogen atom, C₁₋₆ alkyl, C₂₋₆ alkenyl, C₂₋₆ alkynyl, halogen, cyano, -OR^{o}, -NRⁿ²Rⁿ³, 3- to 8-membered heterocyclyl, 3- to 8-membered cycloalkyl, 3- to 8-membered cycloalkenyl, 6- to 10-membered aryl and 5- to 10-membered heteroaryl, wherein the C₁₋₆ alkyl, C₂₋₆ alkenyl, C₂₋₆ alkynyl, 3- to 8-membered heterocyclyl, 3-to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, hydroxy, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or Rⁿ¹ and R together with the atom to which they are attached form 3- to 8-membered heterocyclyl; wherein the 3- to 8-membered heterocyclyl is optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
each R^{a}, R^{b} and R^{c} is identical or different, and is independently selected from the group consisting of hydrogen atom, C₁₋₆ alkyl, -OR^{o}, -NRⁿ²Rⁿ³, halogen, cyano, oxo, -C(O)NRⁿ²Rⁿ³, -C(O)R°, -C(O)OR^{o}, -C(O)NRⁿ¹OR^{o}, 3- to 8-membered heterocyclyl, 3- to 8-membered cycloalkyl, 3- to 8-membered cycloalkenyl, 6- to 10-membered aryl and 5- to 10-membered heteroaryl, wherein the C₁₋₆ alkyl, 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, hydroxy, C₁₋₆ hydroxyalkyl, 3-to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
R^{d} and R^{e} are identical or different at each occurrence, and are independently selected from the group consisting of hydrogen atom, C₁₋₆ alkyl, halogen, C₁₋₆ haloalkyl, C₁₋₆ hydroxyalkyl, C₂₋₆ alkenyl, C₂₋₆ alkynyl, C₂₋₆ haloalkenyl, C₂₋₆ haloalkynyl, -OR^{o} and C₁₋₆ haloalkoxy;
R^{o} is identical or different at each occurrence, and is independently selected from the group consisting of hydrogen atom, C₁₋₆ alkyl, C₁₋₆ haloalkyl, C₁₋₆ hydroxyalkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl and 5- to 10-membered heteroaryl; wherein the C₁₋₆ alkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
Rⁿ¹, Rⁿ², Rⁿ³, Rⁿ⁴ and Rⁿ⁵ are identical or different at each occurrence, and are independently selected from the group consisting of hydrogen atom, C₁₋₆ alkyl, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl; wherein the C₁₋₆ alkyl, 3- to 8-membered heterocyclyl, 3- to 8-membered cycloalkyl, 3- to 8-membered cycloalkenyl, 6-to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or Rⁿ² and Rⁿ³, Rⁿ⁴ and Rⁿ⁵ together with the nitrogen atom to which they are attached respectively form 3- to 8-membered heterocyclyl; wherein the 3- to 8-membered heterocyclyl is optionally substituted with one or more substituents selected from the group consisting of halogen, oxo, C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
n is 0, 1, 2, 3, 4, 5, 6, 7, 8, 9, 10, 11 or 12;
m is 0, 1, 2, 3, 4, 5 or 6;
p is 0, 1, 2, 3, 4, 5, 6, 7, 8, 9, 10, 11 or 12; and
r is 0, 1, 2, 3 or 4.

In some embodiments of the present disclosure, in the compound of formula (IA), the stereoisomer thereof, the isotopic derivative thereof, the prodrug thereof, the N-oxide thereof, its solvate thereof, or the pharmaceutically acceptable salt thereof, ring A is 5- to 6-membered heteroaryl containing 1 to 3 nitrogen atoms; preferably, ring A is selected from the group consisting of pyridinyl, pyrimidinyl, pyridazinyl and 5-membered heteroaryl containing 1 to 3 nitrogen atoms.

In some embodiments of the present disclosure, the compound of formula (IA), the stereoisomer thereof, the isotopic derivative thereof, the prodrug thereof, the N-oxide thereof, the solvate thereof, or the pharmaceutically acceptable salt thereof is a compound of formula (I), a stereoisomer thereof, an isotopic derivative thereof, a prodrug thereof, an N-oxide thereof, a solvate thereof, or a pharmaceutically acceptable salt thereof; 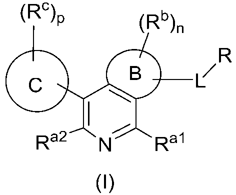 wherein:
R^{a1} and R^{a2} are identical or different, and are each independently as defined for R^{a} in formula (IA);
ring B is selected from the group consisting of 3- to 8-membered heterocyclyl, 5- to 6-membered cycloalkenyl, 7- to 8-membered monocyclic cycloalkyl, 5- to 10-membered fused ring group, 5- to 10-membered spiro ring group, and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
provided that the compound of formula (I) is not the compound with CAS number 1428652-88-3;
R, R^{a}, R^{b}, R^{c}, Rⁿ¹, Rⁿ⁴, Rⁿ⁵, R^{o}, R^{d}, R^{e}, n and p are as defined in formula (IA).

The structural formula of the compound with CAS number 1428652-88-3 as described in this application is:

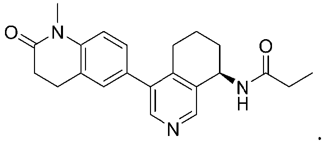

In some embodiments of the present disclosure, in the compound of formula (I), the stereoisomer thereof, the isotopic derivative thereof, the prodrug thereof, the N-oxide thereof, the solvate thereof, or the pharmaceutically acceptable salt thereof, in the structural moiety 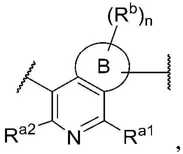 ring B is selected from the group consisting of 5- to 6-membered heterocyclyl, 5- to 6-membered cycloalkenyl, 6- to 10-membered fused ring group and 6- to 10-membered bridged ring group, R^{a1} and R^{a2} are identical or different, and are each independently as defined for R^{a} in formula (IA), n and R^{b} are as defined in formula (IA); preferably, in the structural moiety 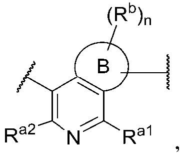 ring B is selected from the group consisting of 5- to 6-membered heterocyclyl, 5- to 6-membered cycloalkenyl, 6- to 10-membered fused ring group and 6- to 10-membered bridged ring group; each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; R^{a1} and R^{a2} are identical or different, and are independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; more preferably, 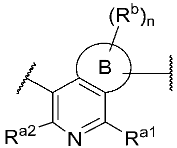 is selected from the group consisting of 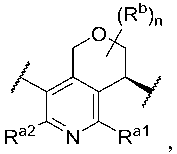 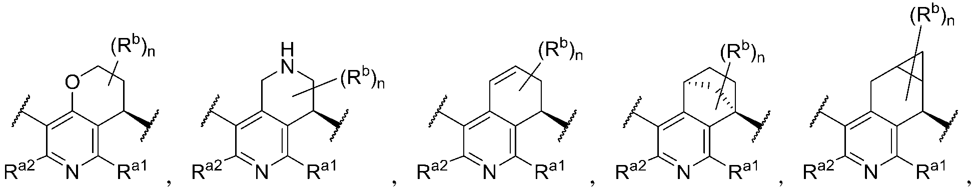 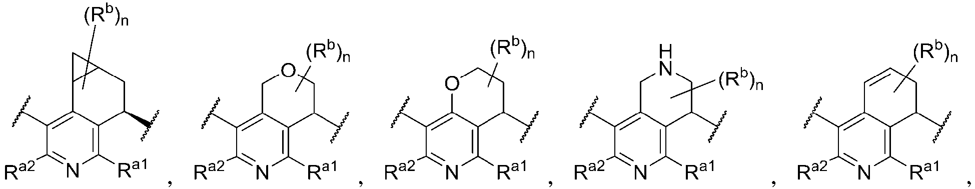 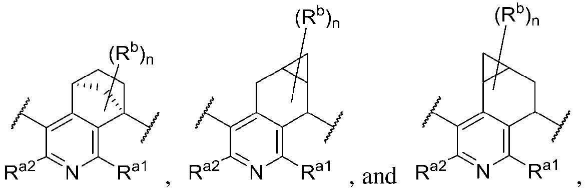 each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; R^{a1} and R^{a2} are identical or different, and are independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; further preferably, 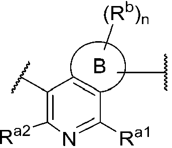 is selected from the group consisting of 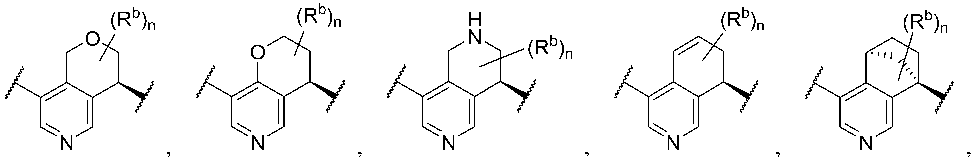 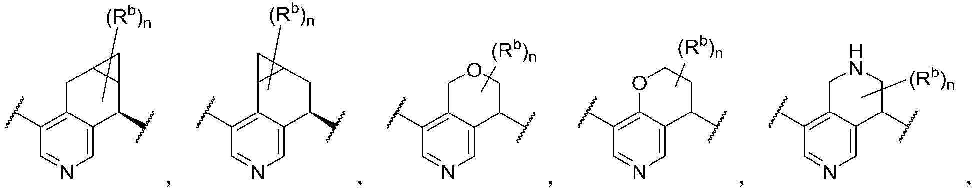 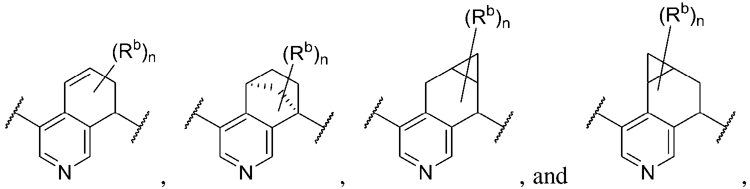 each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; even more preferably, 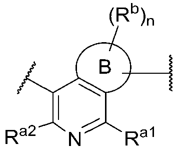 is selected from the group consisting of 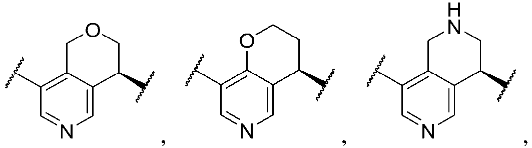 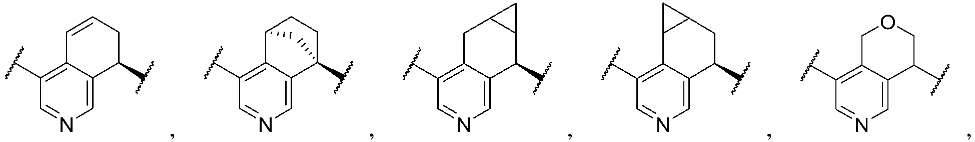 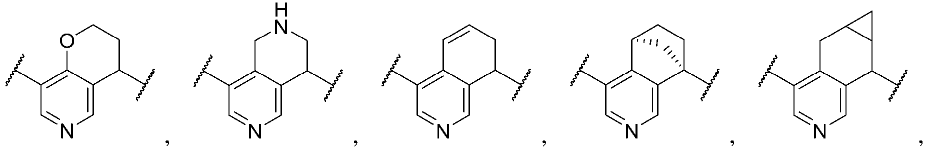 and

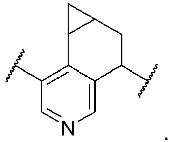

In some embodiments of the present disclosure, in the compound of formula (I), the stereoisomer thereof, the isotopic derivative thereof, the prodrug thereof, the N-oxide thereof, the solvate thereof, or the pharmaceutically acceptable salt thereof, ring B is selected from the group consisting of 5- to 6-membered heterocyclyl, 5- to 6-membered cycloalkenyl, 6- to 10-membered fused ring group and 6- to 10-membered bridged ring group; preferably, ring B is selected from the group consisting of 6-membered heterocyclyl, cyclohexenyl, 7-membered fused ring group and 7-membered bridged ring group; more preferably, ring B is selected from the group consisting of 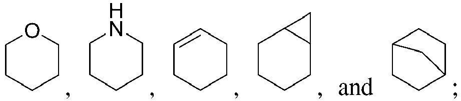 even more preferably, ring B is selected from the group consisting of 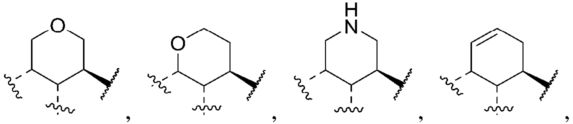 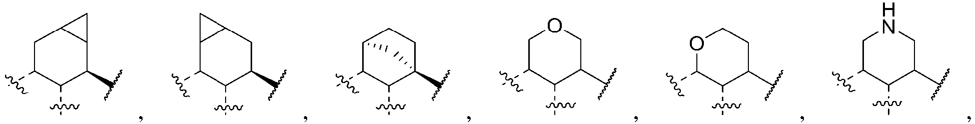 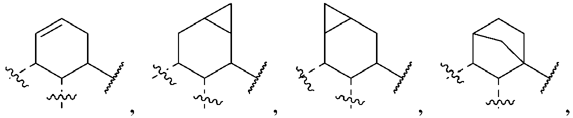 wherein 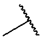 , 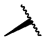 are connected to L.

In some embodiments of the present disclosure, in the compound of formula (I), the stereoisomer thereof, the isotopic derivative thereof, the prodrug thereof, the N-oxide thereof, the solvate thereof, or the pharmaceutically acceptable salt thereof, ring B is 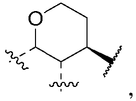 wherein 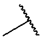 , 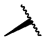 are connected to L.

In some embodiments of the present disclosure, the compound of formula (IA) or formula (I), the stereoisomer thereof, the isotopic derivative thereof, the prodrug thereof, the N-oxide thereof, the solvate thereof, or the pharmaceutically acceptable salt thereof is a compound of formula (I-1) or formula (I-2), a stereoisomer thereof, an isotopic derivative thereof, a prodrug thereof, an N-oxide thereof, a solvate thereof, or a pharmaceutically acceptable salt thereof; 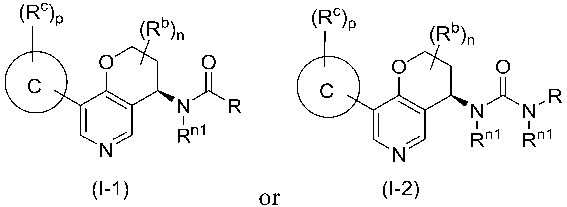 wherein:
ring C, R, Rⁿ¹, R^{b}, R^{c}, n and p are as defined in formula (IA) or formula (I).

In some embodiments of the present disclosure, the compound of formula (IA), the stereoisomer thereof, the isotopic derivative thereof, the prodrug thereof, the N-oxide thereof, the solvate thereof, or the pharmaceutically acceptable salt thereof is a compound of formula (II), the stereoisomer thereof, the isotopic derivative thereof, the prodrug thereof, the N-oxide thereof, the solvate thereof, or the pharmaceutically acceptable salt thereof; 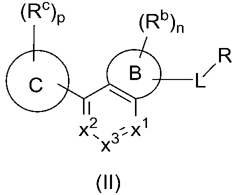 wherein:
x¹ is selected from N or CR^{a1};
x² is selected from N or CR^{a2};
x³ is selected from N or CR^{a3}; R^{a1}, R^{a2}, R^{a3} are identical or different, and are each independently as defined for R^{a} in formula (IA);
at least one of x¹, x², x³ is N, and when x¹ is selected from CR^{a1} and x² is selected from CR^{a2}, x³ is not selected from N;
ring B is selected from the group consisting of 3- to 8-membered heterocyclyl, 3- to 8-membered monocyclic cycloalkyl, 3- to 8-membered monocyclic cycloalkenyl, 5- to 10-membered fused ring group, 5- to 10-membered spiro ring group and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
R, R^{a}, R^{b}, R^{c}, Rⁿ¹, Rⁿ⁴, Rⁿ⁵, R^{d}, R^{e}, n and p are as defined in formula (IA).

In some embodiments of the present disclosure, in the compound of formula (II), the stereoisomer thereof, the isotopic derivative thereof, the prodrug thereof, the N-oxide thereof, the solvate thereof, or the pharmaceutically acceptable salt thereof, the structural moiety 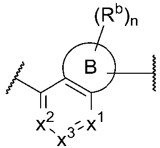 is selected from 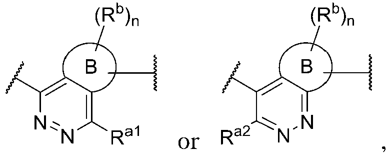 wherein ring B is selected from the group consisting of 5- to 6-membered heterocyclyl, 5- to 6-membered cycloalkyl, 5- to 6-membered cycloalkenyl, 6- to 10-membered fused ring group and 6- to 10-membered bridged ring group; R^{a1} and R^{a2} are identical or different, and are each independently as defined for R^{a} in formula (IA); n and R^{b} are as defined in formula (IA); preferably, 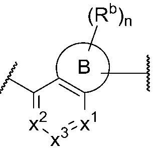 is selected from 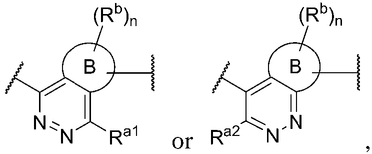 wherein ring B is selected from the group consisting of 5- to 6-membered heterocyclyl or 5- to 6-membered cycloalkyl; each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; R^{a1} and R^{a2} are identical or different, and are independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; more preferably, 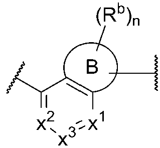 is selected from the group consisting of 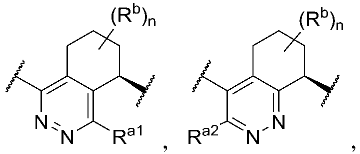 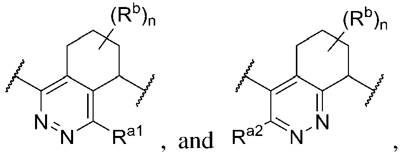 each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; R^{a1} and R^{a2} are identical or different, and are independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; further preferably, 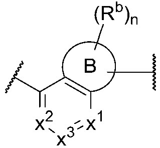 is selected from the group consisting of 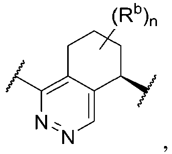 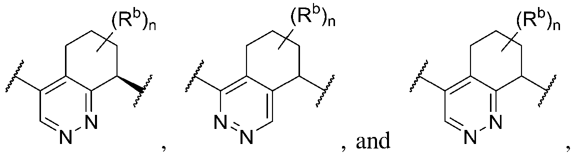 each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; even more preferably, 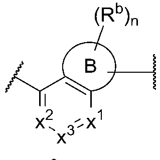 is selected from the group consisting of

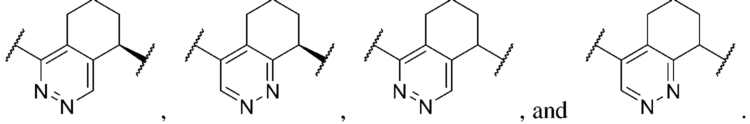

In some embodiments of the present disclosure, in the compound of formula (II), the stereoisomer thereof, the isotopic derivative thereof, the prodrug thereof, the N-oxide thereof, the solvate thereof, or the pharmaceutically acceptable salt thereof, ring B is selected from the group consisting of 5- to 6-membered heterocyclyl, 5- to 6-membered cycloalkyl, 5- to 6-membered cycloalkenyl, 6- to 10-membered fused ring group and 6- to 10-membered bridged ring group; preferably, ring B is selected from the group consisting of 5- to 6-membered heterocyclyl and 5-to 6-membered cycloalkyl; more preferably, ring B is selected from 5- to 6-membered cycloalkyl; even more preferably, ring B is selected from the group consisting of 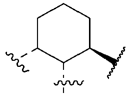 and 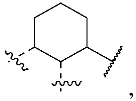 wherein 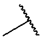 , 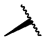 are connected to L.

In some embodiments of the present disclosure, the compound of formula (IA), the stereoisomer thereof, the isotopic derivative thereof, the prodrug thereof, the N-oxide thereof, the solvate thereof, or the pharmaceutically acceptable salt thereof is a compound of formula (III), the stereoisomer thereof, the isotopic derivative thereof, the prodrug thereof, the N-oxide thereof, the solvate thereof, or the pharmaceutically acceptable salt thereof; 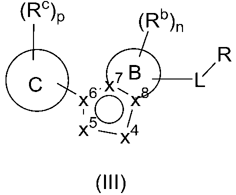 wherein:
x⁴ is selected from the group consisting of N, NR^{a4}, O, S, S(O), S(O)₂ and CR^{a4};
x⁵ is selected from the group consisting of N, NR^{a5}, O, S, S(O), S(O)₂ and CR^{a5};
x⁶ is selected from the group consisting of N and C;
x⁷ is selected from the group consisting of N and C;
x⁸ is selected from the group consisting of N and C; R^{a4} and R^{a5} are as defined for R^{a} in formula (IA);
ring B is selected from the group consisting of 3- to 8-membered heterocyclyl, 3- to 8-membered monocyclic cycloalkyl, 3- to 8-membered monocyclic cycloalkenyl, 5- to 10-membered fused ring group, 5- to 10-membered spiro ring group and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
R, R^{a}, R^{b}, R^{c}, Rⁿ¹, Rⁿ⁴, Rⁿ⁵, R^{o}, R^{d}, R^{e}, n and p are as defined in formula (IA).

In some embodiments of the present disclosure, in the compound of formula (III), the stereoisomer thereof, the isotopic derivative thereof, the prodrug thereof, the N-oxide thereof, the solvate thereof, or the pharmaceutically acceptable salt thereof, the structural moiety 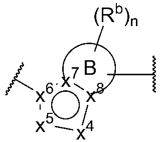 is selected from the group consisting of 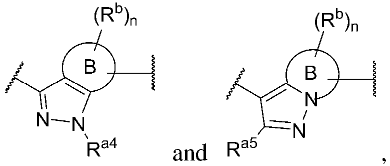 wherein ring B is selected from the group consisting of 5- to 6-membered heterocyclyl, 5- to 6-membered cycloalkyl, 5- to 6-membered cycloalkenyl, 6- to 10-membered fused ring group and 6- to 10-membered bridged ring group; R^{a4} and R^{a5} are identical or different, and are each independently as defined for R^{a} in formula (IA); n and R^{b} are as defined in formula (IA); preferably, 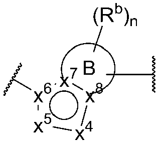 is selected from the group consisting of 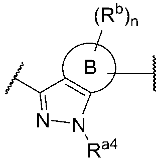 and 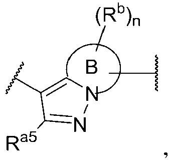 wherein ring B is selected from the group consisting of 5- to 6-membered heterocyclyl and 5- to 6-membered cycloalkyl; each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; R^{a4} and R^{a5} are identical or different, and are independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; more preferably, 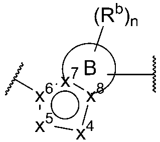 is selected from the group consisting of 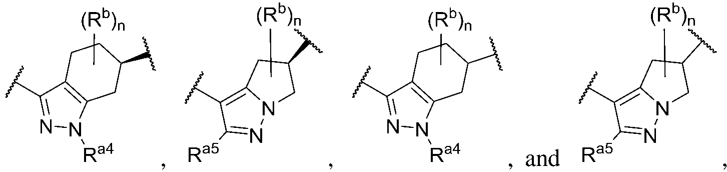 each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; R^{a4} and R^{a5} are identical or different, and are independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; further preferably, 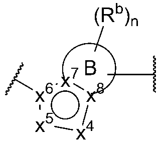 is selected from the group consisting of 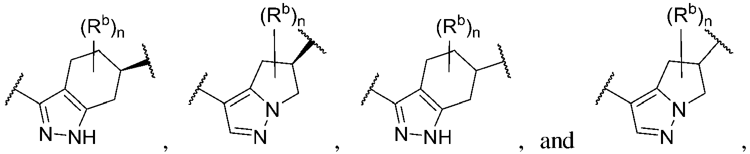 each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3 or 4; even more preferably, 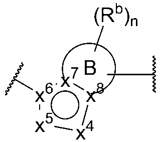 is selected from the group consisting of

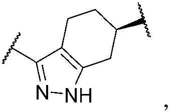

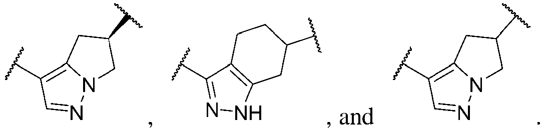

In some embodiments of the present disclosure, in the compound of formula (III), the stereoisomer thereof, the isotopic derivative thereof, the prodrug thereof, the N-oxide thereof, the solvate thereof, or the pharmaceutically acceptable salt thereof, ring B is selected from the group consisting of 5- to 6-membered heterocyclyl, 5- to 6-membered cycloalkyl, 5- to 6-membered cycloalkenyl, 6- to 10-membered fused ring group and 6- to 10-membered bridged ring group; preferably, ring B is selected from the group consisting of 5- to 6-membered heterocyclyl or 5- to 6-membered cycloalkyl; more preferably, ring B is selected from the group consisting of 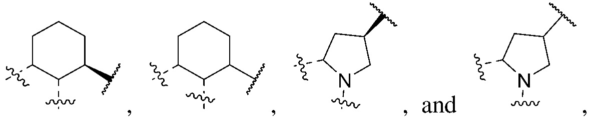 wherein 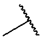 , 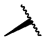 are connected to L.

In some embodiments of the present disclosure, the compound of formula (IA), the stereoisomer thereof, the isotopic derivative thereof, the prodrug thereof, the N-oxide thereof, the solvate thereof, or the pharmaceutically acceptable salt thereof is a compound of formula (IV) or formula (IV-1), the stereoisomer thereof, the isotopic derivative thereof, the prodrug thereof, the N-oxide thereof, the solvate thereof, or the pharmaceutically acceptable salt thereof; 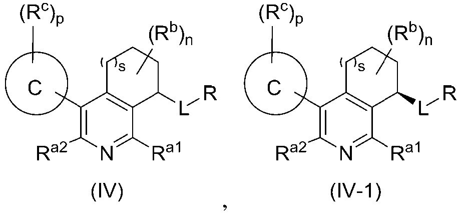 wherein:
R^{a1} and R^{a2} are as defined for R^{a} in formula (IA);
s is 0, 1, 2, 3 or 4;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group; and 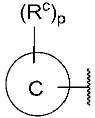 is not 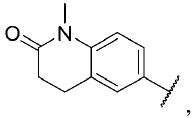 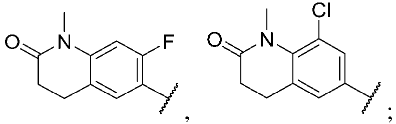
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
R, R^{a}, R^{b}, R^{c}, Rⁿ¹, Rⁿ⁴, Rⁿ⁵, R^{o}, R^{d}, R^{e}, n and p are as defined in formula (IA).

In some embodiments of the present disclosure, the compound of formula (IA), the stereoisomer thereof, the isotopic derivative thereof, the prodrug thereof, the N-oxide thereof, the solvate thereof, or the pharmaceutically acceptable salt thereof is a compound of formula (V) or formula (V-1), the stereoisomer thereof, the isotopic derivative thereof, the prodrug thereof, the N-oxide thereof, the solvate thereof, or the pharmaceutically acceptable salt thereof; 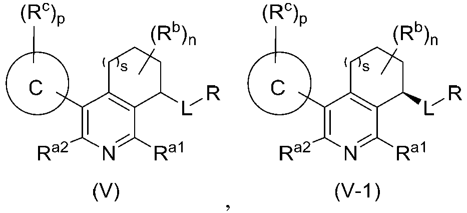 wherein:
R^{a1} and R^{a2} are as defined for R^{a} in formula (IA);
s is 0, 1, 2, 3 or 4;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl or 5- to 10-membered heteroaryl; wherein the 3- to 8-membered heterocyclyl, 3- to 8-membered cycloalkyl, 3- to 8-membered cycloalkenyl, 6- to 10-membered aryl and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl and 5- to 10-membered heteroaryl;
and when L is -NHC(O)-, R is not methyl, ethyl, cyclopropyl, CH₃CH(OH)-, and 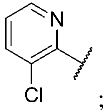
R, R^{a}, R^{b}, R^{c}, Rⁿ¹, Rⁿ⁴, Rⁿ⁵, R^{o}, R^{d}, R^{e}, n and p are as defined in formula (IA).

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, in the structural moiety 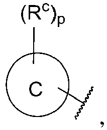 ring C is selected from the group consisting of phenyl, 5- to 6-membered heteroaryl, 7- to 14-membered fused aryl, 6- to 14-membered fused heterocyclyl, and 6- to 14-membered fused heteroaryl; each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4; preferably, in the structural moiety 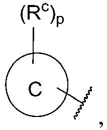 ring C is selected from the group consisting of phenyl, 5- to 6-membered heteroaryl, 5- to 8-membered monocyclic heterocyclyl fused with phenyl, 7- to 10-membered fused heterocyclyl fused with phenyl, 5- to 8-membered monocyclic heterocyclyl fused with 5- to 6-membered heteroaryl, 7- to 10-membered fused heterocyclyl fused with 5- to 6-membered heteroaryl, 5- to 6-membered heteroaryl fused with phenyl; each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4; more preferably, the structural moiety 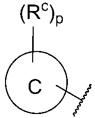 is selected from the group consisting of 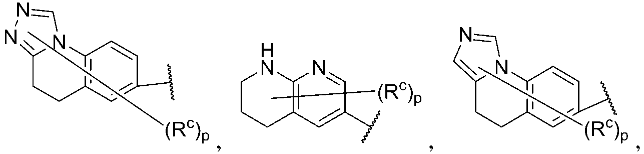 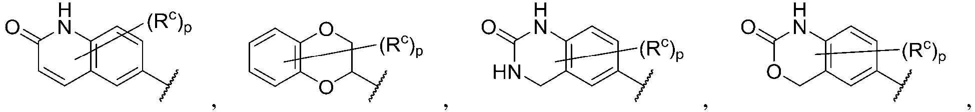 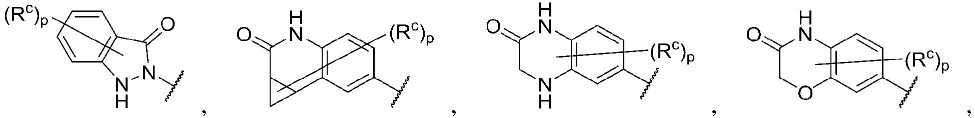 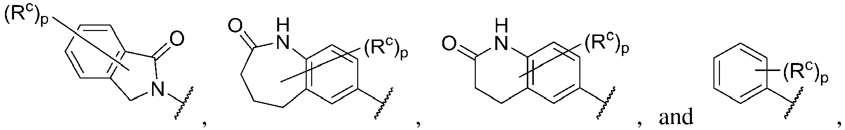 each R^{c} is identical or different, and is independently selected from the group consisting of hydrogen atom, halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, C₁₋₆ alkoxy, and 5- to 6-membered heteroaryl, wherein the 5- to 6-membered heteroaryl is optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4; even more preferably, the structural moiety 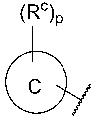 is selected from the group consisting of 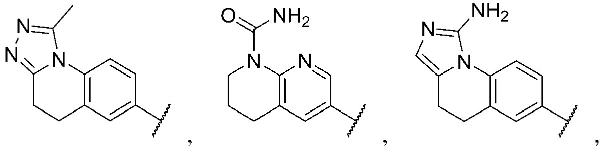 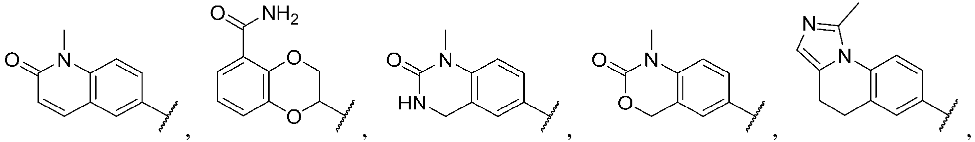 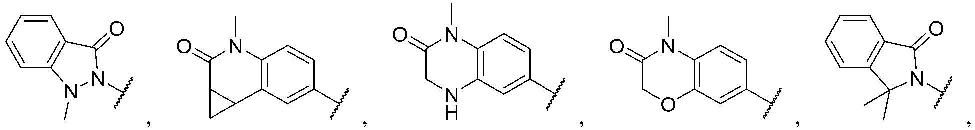 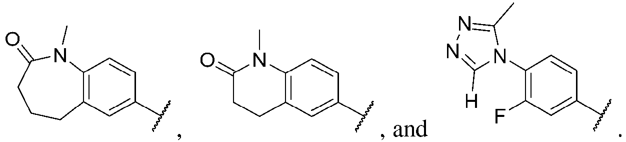 In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, in the structural moiety 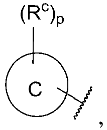 ring C is selected from the group consisting of 7- to 10-membered spiro heterocyclyl fused with phenyl; each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 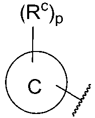 is selected from the group consisting of 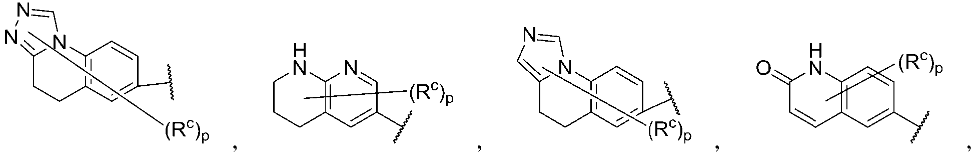 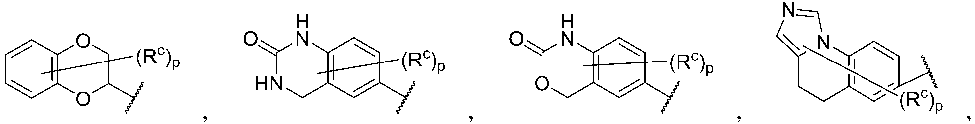 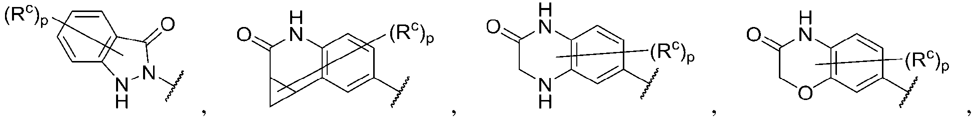 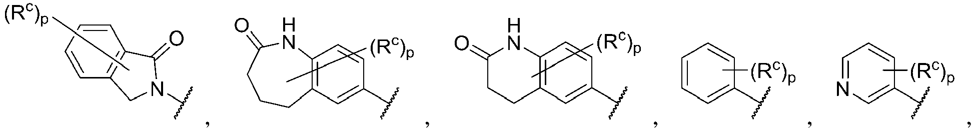 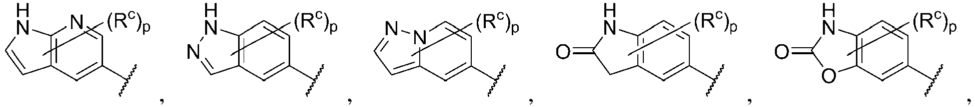 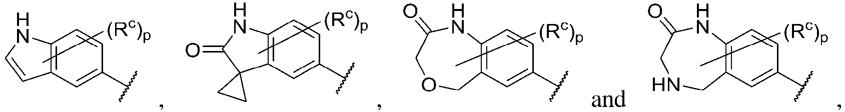 each R^{c} is identical or different, and is independently selected from the group consisting of hydrogen atom, halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, C₁₋₆ alkoxy, 3- to 6-membered cycloalkyl, and 5- to 6-membered heteroaryl, wherein the 3- to 6-membered cycloalkyl and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 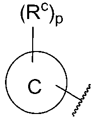 is selected from the group consisting of 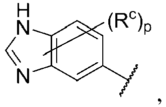 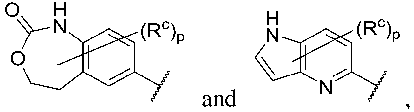 each R^{c} is identical or different, and is independently selected from the group consisting of hydrogen atom, halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, C₁₋₆ alkoxy, 3- to 6-membered cycloalkyl, and 5- to 6-membered heteroaryl, wherein the 3- to 6-membered cycloalkyl and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, in the structural moiety 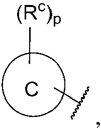 ring C is selected from the group consisting of phenyl, 5- to 6-membered heteroaryl, 7- to 14-membered fused aryl, 6- to 14-membered fused heterocyclyl, and 6- to 14-membered fused heteroaryl; each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, deuterated C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4;
preferably, in the structural moiety 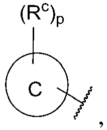 ring C is selected from the group consisting of phenyl, 5- to 6-membered heteroaryl, 5- to 8-membered monocyclic heterocyclyl fused with phenyl, 7- to 10-membered fused heterocyclyl fused with phenyl, 7- to 10-membered spiro heterocyclyl fused with phenyl, 5- to 8-membered monocyclic heterocyclyl fused with 5- to 6-membered heteroaryl, 7- to 10-membered fused heterocyclyl fused with 5- to 6-membered heteroaryl, and 5- to 6-membered heteroaryl fused with phenyl; each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, deuterated C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4;
more preferably, the structural moiety 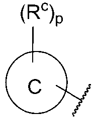 is selected from the group consisting of 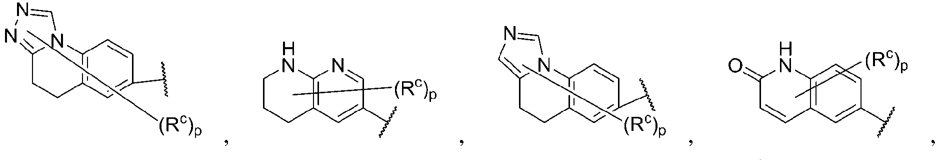 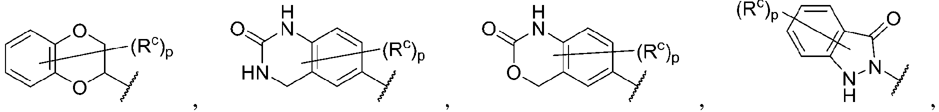 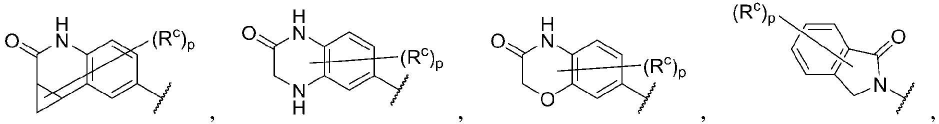 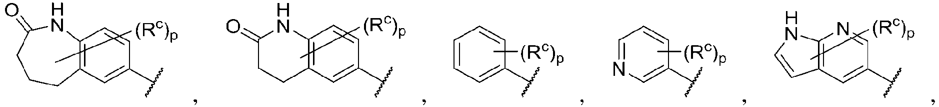 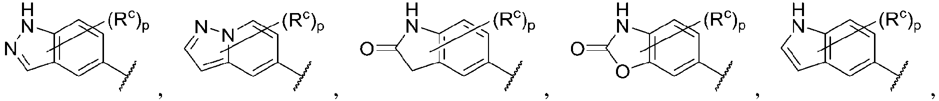 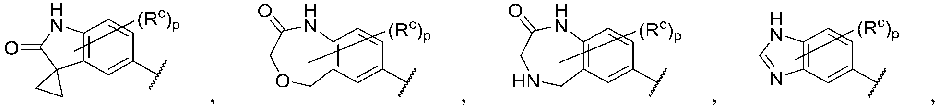 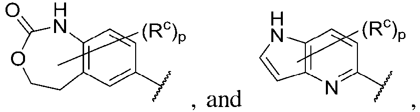 each R^{c} is identical or different, and is independently selected from the group consisting of hydrogen atom, halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, deuterated C₁₋₆ alkyl, C₁₋₆ alkoxy, 3- to 6-membered cycloalkyl, and 5- to 6-membered heteroaryl, wherein the 3- to 6-membered cycloalkyl and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3 or 4.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 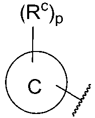 is selected from the group consisting of 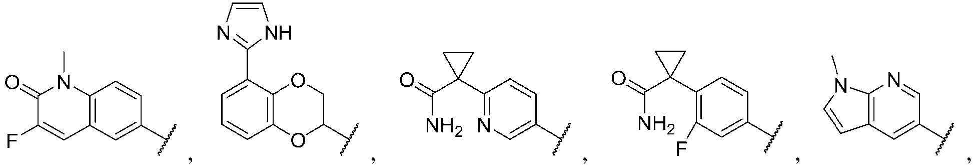 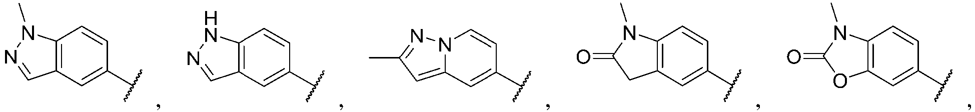 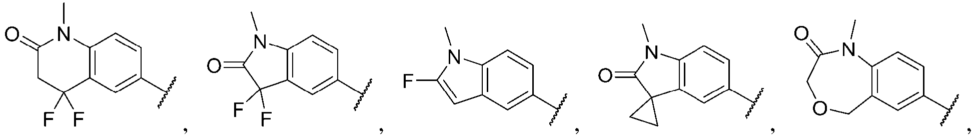 and

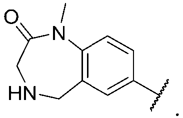

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 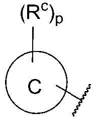 is selected from the group consisting of

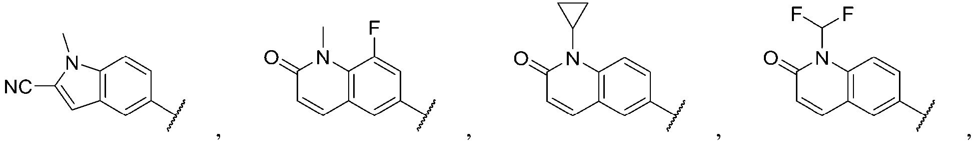

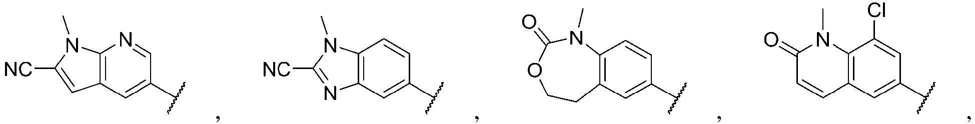

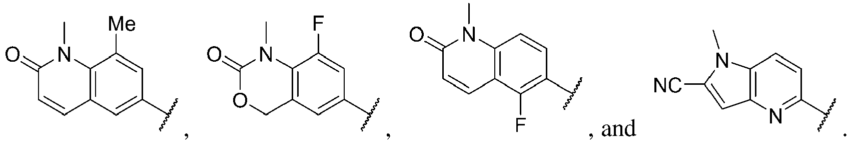

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 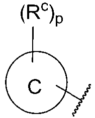 is selected from the group consisting of

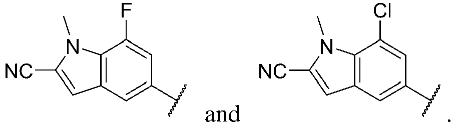

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 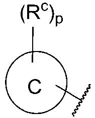 is selected from

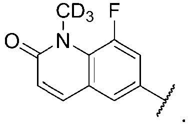

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 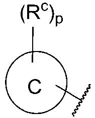 is selected from

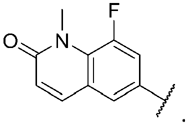

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 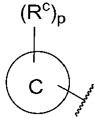 is selected from

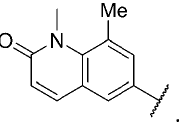

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, the structural moiety 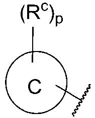 is selected from

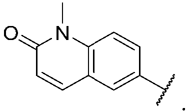

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from the group consisting of phenyl, 5- to 6-membered heteroaryl, 7-to 14-membered fused aryl, 6- to 14-membered fused heterocyclyl, and 6- to 14-membered fused heteroaryl; preferably, ring C is selected from the group consisting of phenyl, 5- to 6-membered heteroaryl, 5- to 8-membered monocyclic heterocyclyl fused with phenyl, 7- to 10-membered fused heterocyclyl fused with phenyl, 5- to 8-membered monocyclic heterocyclyl fused with 5-to 6-membered heteroaryl, 7- to 10-membered fused heterocyclyl fused with 5- to 6-membered heteroaryl, and 5- to 6-membered heteroaryl fused with phenyl; more preferably, ring C is selected from the group consisting of 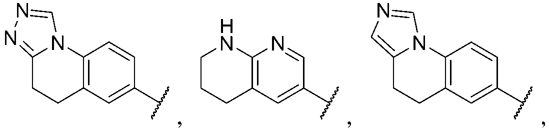 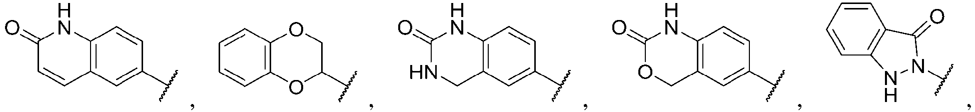 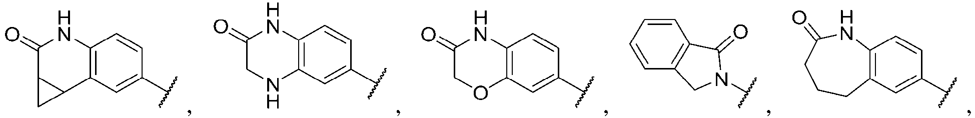 and

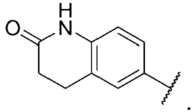

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from 5- to 6-membered heteroaryl fused with 5- to 6-membered heteroaryl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from the group consisting of

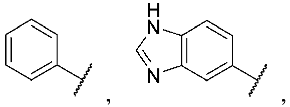

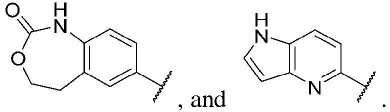

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from 7- to 10-membered spiro heterocyclyl fused with phenyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from the group consisting of

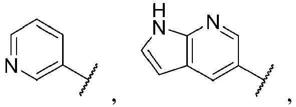

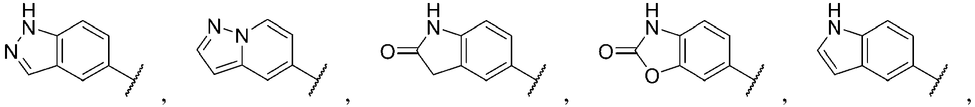

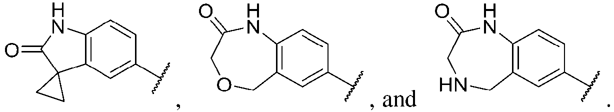

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from the group consisting of 5- to 6-membered heterocyclyl fused with phenyl and 5- to 6-membered heterocyclyl fused with 6-membered heteroaryl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, ring C is selected from

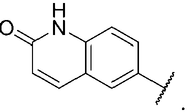

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, oxo, C₁₋₆ haloalkyl, cyano, -CONH₂, amino, C₁₋₆ alkyl, 3- to 8-membered cycloalkyl optionally substituted with -CONH₂, and 5- to 6-membered heteroaryl optionally substituted with C₁₋₆ alkyl; or two R^{c} attached to the same carbon atom or to adjacent carbon atoms together with the carbon atom to which they are attached form 3- to 6-membered cycloalkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, oxo, C₁₋₄ haloalkyl, cyano, -CONH₂, amino, C₁₋₄ alkyl, 3- to 6-membered cycloalkyl optionally substituted with -CONH₂, and 5- to 6-membered heteroaryl optionally substituted with C₁₋₄ alkyl; or two R^{c} attached to the same carbon atom or to adjacent carbon atoms together with the carbon atom to which they are attached form 3- to 6-membered cycloalkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, hydroxy, oxo, C₁₋₆ hydroxyalkyl, C₁₋₆ haloalkyl, C₁₋₆ haloalkoxy, cyano, amino, -CONRⁿ²Rⁿ³, -NRⁿ²Rⁿ³, C₁₋₆ alkyl, C₁₋₆ alkoxy, 3- to 8-membered cycloalkyl, 3-to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, C₁₋₆ hydroxyalkyl, C₁₋₆ haloalkyl, C₁₋₆ haloalkoxy, cyano, amino, -C(O)NRⁿ⁴Rⁿ⁵, -NRⁿ⁴Rⁿ⁵, C₁₋₆ alkyl, and C₁₋₆ alkoxy, wherein Rⁿ², Rⁿ³, Rⁿ⁴ and Rⁿ⁵ are as defined in formula (IA); preferably, each R^{c} is identical or different, and is independently selected from the group consisting of hydrogen atom, halogen, C₁₋₆ alkyl, oxo, amino, NH(C₁₋₆ alkyl), N(C₁₋₆ alkyl)₂, -CONH₂, 3- to 8-membered cycloalkyl, 3-to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, amino, NH(C₁₋₆ alkyl), N(C₁₋₆ alkyl)2, -CONH₂ and C₁₋₆ alkyl; more preferably, each R^{c} is identical or different, and is independently selected from the group consisting of hydrogen atom, halogen, C₁₋₆ alkyl, oxo, amino, NH(C₁₋₆ alkyl), N(C₁₋₆ alkyl)₂, -CONH₂, 3- to 8-membered cycloalkyl, 3- to 8-membered cycloalkenyl, and 5- to 6-membered heteroaryl, wherein the 3- to 8-membered cycloalkyl, 3- to 8-membered cycloalkenyl, and 5- to 6-membered heteroaryl are optionally substituted with one or more substituents selected from the group consisting of halogen, amino, NH(C₁₋₆ alkyl), N(C₁₋₆ alkyl)₂, -CONH₂ and C₁₋₆ alkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, hydroxy, oxo, C₁₋₄ hydroxyalkyl, C₁₋₄ haloalkyl, C₁₋₄ haloalkoxy, cyano, amino, -CONRⁿ²Rⁿ³, -NRⁿ²Rⁿ³, C₁₋₄ alkyl, deuterated C₁₋₄ alkyl, C₁₋₄ alkoxy, 3- to 8-membered cycloalkyl, 3- to 8-membered cycloalkenyl, 3- to 8-membered heterocyclyl, and 5-to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, C₁₋₄ hydroxyalkyl, C₁₋₄ haloalkyl, genated C₁₋₄ haloalkoxy, cyano, amino, -C(O)NRⁿ⁴Rⁿ⁵, -NRⁿ⁴Rⁿ⁵, C₁₋₄ alkyl, and C₁₋₄ alkoxy, wherein Rⁿ², Rⁿ³, Rⁿ⁴ and Rⁿ⁵ are as defined in formula (IA).

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, C₁₋₄ alkyl, deuterated C₁₋₄ alkyl, oxo, amino, NH(C₁₋₄ alkyl), N(C₁₋₄ alkyl)₂, -CONH₂, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, amino, NH(C₁₋₄ alkyl), N(C₁₋₄ alkyl)₂, -CONH₂, and C₁₋₄ alkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, C₁₋₄ alkyl, deuterated C₁₋₄ alkyl, oxo, amino, NH(C₁₋₄ alkyl), N(C₁₋₄ alkyl)₂, -CONH₂, 3- to 6-membered cycloalkyl, 3- to 6-membered cycloalkenyl, and 5- to 6-membered heteroaryl, wherein the 3- to 6-membered cycloalkyl, 3- to 6-membered cycloalkenyl, and 5- to 6-membered heteroaryl are optionally substituted with one or more substituents selected from the group consisting of halogen, amino, NH(C₁₋₄ alkyl), N(C₁₋₄ alkyl)₂, -CONH₂, and C₁₋₄ alkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, two R^{c} together with the atom to which they are attached form 3- to 6-membered heterocyclyl or 3- to 6-membered cycloalkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, two R^{c} attached to the same carbon atom or to adjacent carbon atoms together with the carbon atom to which they are attached form 3- to 6-membered cycloalkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, two R^{c} attached to the same carbon atom together with the carbon atom to which they are attached form cyclopropyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, F, methyl, amino, -CONH₂,

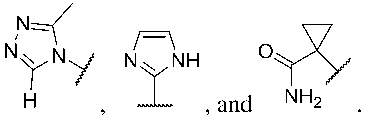

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of cyano, -CHF₂, cyclopropyl, and Cl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the prodrug thereof, the N-oxide thereof, the solvate thereof, or the pharmaceutically acceptable salt thereof, each R^{c} is identical or different, and is independently selected from deuterated C₁₋₄ alkyl, preferably deuterated methyl, more preferably -CD₃.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{c} is identical or different, and is independently selected from the group consisting of hydrogen atom, halogen, C₁₋₆ alkyl, preferably hydrogen atom, F, and methyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, p is 0, 1, 2, 3 or 4; preferably, p is 0, 1, 2 or 3; more preferably, p is 0, 1 or 2.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{b} is identical or different, and is independently selected from the group consisting of hydrogen atom, halogen, hydroxy, C₁₋₆ hydroxyalkyl, C₁₋₆ haloalkyl, C₁₋₆ haloalkoxy, cyano, -CONRⁿ²Rⁿ³, -NRⁿ²Rⁿ³, C₁₋₆ alkyl, and C₁₋₆ alkoxy, wherein Rⁿ² and Rⁿ³ are as defined in formula (IA); preferably, each R^{b} is identical or different, and is independently selected from the group consisting of hydrogen atom, halogen, and C₁₋₆ alkyl; more preferably, each R^{b} is hydrogen atom.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, each R^{b} is identical or different, and is independently selected from the group consisting of hydrogen atom, halogen, hydroxy, C₁₋₄ hydroxyalkyl, C₁₋₄ haloalkyl, C₁₋₄ haloalkoxy, cyano, -CONRⁿ²Rⁿ³, -NRⁿ²Rⁿ³, C₁₋₄ alkyl, and C₁₋₄ alkoxy, wherein Rⁿ² and Rⁿ³ are as defined in formula (IA); preferably, each R^{b} is identical or different, and is independently selected from the group consisting of hydrogen atom, halogen, and C₁₋₄ alkyl.

In some embodiments of the present disclosure, in the compound of formula (IA), formula (I), formula (I-1), formula (I-2), formula (II), formula (III), formula (IV), formula (IV-1), formula (V), or formula (V-1), the stereoisomer thereof, the isotopic derivative thereof, the prodrug thereof, the N-oxide thereof, the solvate thereof, or the pharmaceutically acceptable salt thereof, n is 0, 1, 2, 3 or 4; preferably, n is 0 or 1.

In some embodiments of the present disclosure, the compound of formula (IA), the stereoisomer thereof, the isotopic derivative thereof, the prodrug thereof, the N-oxide thereof, the solvate thereof, or the pharmaceutically acceptable salt thereof is a compound of formula (I-a), the stereoisomer thereof, the isotopic derivative thereof, the prodrug thereof, the N-oxide thereof, the solvate thereof, or the pharmaceutically acceptable salt thereof; 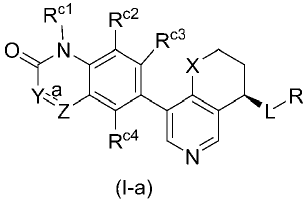 wherein:
X is selected from the group consisting of -CH₂-, -O-, -NH-, and -S-;
L is selected from the group consisting of -NRⁿ¹C(O)- and -NRⁿ¹-C(O)-NRⁿ¹-;
bond a is a single bond or a double bond;
when bond a is a single bond, Y and Z are identical or different, and are each independently selected from the group consisting of -O-, -NH-, and -CR^{c5}R^{c6}-;
when bond a is a double bond, Y and Z are identical, and are both selected from CH;
R^{c1} is selected from the group consisting of hydrogen atom, C₁₋₆ alkyl, deuterated C₁₋₆ alkyl, C₁₋₆ haloalkyl, 3- to 6-membered heterocyclyl, and 3- to 6-membered cycloalkyl;
R^{c2}, R^{c3}, R^{c4}, R^{c5}, R^{c6} are identical or different, and are each independently selected from the group consisting of hydrogen atom, halogen, C₁₋₆ alkyl, and C₁₋₆ haloalkyl;
Rⁿ¹ and R are as defined in formula (IA);
provided that: when X is -CH₂-, 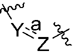 is not -CH₂-CH₂- at the same time, and -L-R is not -NHC(O)-CH₂CH₃ at the same time.

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, X is selected from the group consisting of -CH₂- and -O-.

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, when bond a is a single bond, one of Y and Z is -O-, and the other is -CR^{c5}R^{c6}-; R^{c5} and R^{c6} are as defined in formula (I-a).

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, R^{c2}, R^{c3}, R^{c4} , R^{c5}, R^{c6} are identical or different, and are each independently selected from the group consisting of hydrogen atom, halogen, C₁₋₄ alkyl, and C₁₋₄ haloalkyl; preferably, R^{c2}, R^{c3}, R^{c4}, R^{c5}, R^{c6} are identical or different, and are each independently selected from the group consisting of hydrogen atom, F, Cl, -CHF₂, and methyl.

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, R^{c1} is selected from the group consisting of hydrogen atom, C₁₋₄ alkyl, deuterated C₁₋₄ alkyl, C₁₋₄ haloalkyl, 3- to 6-membered heterocyclyl, and 3- to 6-membered cycloalkyl; preferably, R^{c1} is selected from the group consisting of hydrogen atom, cyclopropyl, methyl, and deuterated methyl; more preferably, R^{c1} is selected from the group consisting of methyl and deuterated methyl; further preferably, R^{c1} is selected from the group consisting of methyl and -CD₃; even more preferably, R^{c1} is selected from methyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹-, -NRⁿ¹C(O)-, -NRⁿ¹-C(O)-NRⁿ¹-, and -CR^{d}=CR^{e}-; Rⁿ¹, R^{d}, R^{e} are as defined in formula (IA); R is selected from the group consisting of C₁₋₆ alkyl, C₁₋₆ alkoxy, C₁₋₆ hydroxyalkyl, cyclopropyl, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 3- to 8-membered heterocyclyl or 5- to 10-membered heteroaryl; or R^{b} and R together with the atom to which they are attached form 3- to 6-membered heterocyclyl;
wherein the 3- to 8-membered heterocyclyl, 3- to 6-membered heterocyclyl, and 5- to 10-membered heteroaryl are each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom, C₁₋₆ alkyl, and deuterated C₁₋₆ alkyl; R^{d} and R^{e} are identical or different, and are each independently selected from the group consisting of hydrogen atom, C₁₋₆ alkyl, and halogen; R is selected from the group consisting of C₁₋₆ alkyl, C₁₋₆ alkoxy, C₁₋₆ hydroxyalkyl, cyclopropyl, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 5- to 8-membered heterocyclyl; or R^{b} and R together with the atom to which they are attached form 5- to 6-membered heterocyclyl; wherein the 5- to 8-membered heterocyclyl and 5- to 6-membered heterocyclyl are each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
more preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom, C₁₋₆ alkyl, and deuterated C₁₋₆ alkyl; R^{d} and R^{e} are identical or different, and are each independently selected from the group consisting of hydrogen atom, C₁₋₆ alkyl, and halogen; R is selected from the group consisting of C₁₋₆ alkyl, C₁₋₆ alkoxy, C₁₋₆ hydroxyalkyl, cyclopropyl, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 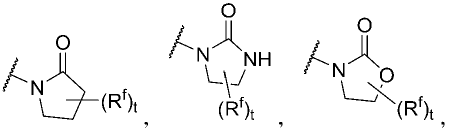 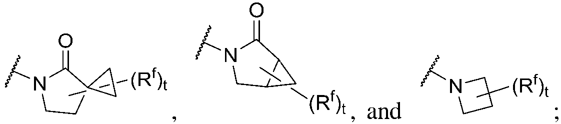 or R^{b} and R together with the atom to which they are attached form 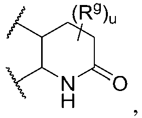 each R^{f} and R^{g} is identical or different, and is independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; t is 0, 1, 2, 3 or 4; u is 0, 1, 2, 3, 4 or 5;
more preferably, L is selected from the group consisting of -NHC(O)-, -NH-C(O)-NH-, -NH-C(O)-N(CH₃)-, -NH-C(O)-N(CD₃)-, and -CH=CF-; R is selected from the group consisting of C₁₋₆ alkyl, C₁₋₆ alkoxy, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 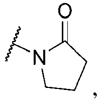 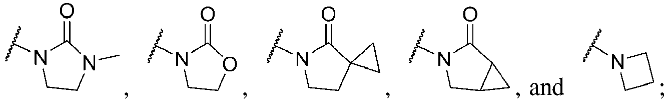 or R^{b} and R together with the atom to which they are attached, form

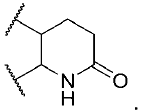

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹-, -NRⁿ¹C(O)-, -NRⁿ¹-C(O)-NRⁿ¹-, and -CR^{d}=CR^{e}-; Rⁿ¹, R^{d}, R^{e} are as defined in formula (IA); R is selected from the group consisting of C₁₋₆ alkyl, C₁₋₆ alkoxy, C₁₋₆ hydroxyalkyl, and cyclopropyl; or Rⁿ¹ and R together with the atom to which they are attached form 3- to 6-membered heterocyclyl or 5- to 10-membered heteroaryl; or R^{b} and R together with the atom to which they are attached form 3- to 6-membered heterocyclyl;
wherein the 3- to 6-membered heterocyclyl or 5- to 10-membered heteroaryl is each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom and C₁₋₆ alkyl; R^{d} and R^{e} are identical or different, and are each independently selected from the group consisting of hydrogen atom, C₁₋₆ alkyl, and halogen; R is selected from the group consisting of C₁₋₆ alkyl, C₁₋₆ alkoxy, C₁₋₆ hydroxyalkyl, and cyclopropyl; or Rⁿ¹ and R together with the atom to which they are attached form 5- to 6-membered heterocyclyl; or R^{b} and R together with the atom to which they are attached form 5- to 6-membered heterocyclyl; wherein the 5- to 6-membered heterocyclyl is each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
more preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom and C₁₋₆ alkyl; R^{d} and R^{e} are identical or different, and are each independently selected from the group consisting of hydrogen atom, C₁₋₆ alkyl, and halogen; R is selected from the group consisting of C₁₋₆ alkyl, C₁₋₆ alkoxy, C₁₋₆ hydroxyalkyl, and cyclopropyl; or Rⁿ¹ and R together with the atom to which they are attached form 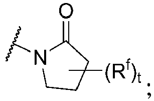 or R^{b} and R together with the atom to which they are attached form 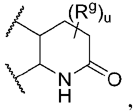 each R^{f} and R^{g} is identical or different, and is independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; t is 0, 1, 2, 3 or 4; u is 0, 1, 2, 3, 4 or 5; more preferably, L is selected from the group consisting of -NHC(O)-, -NH-C(O)-NH-, and -CH=CF-; R is selected from the group consisting of C₁₋₆ alkyl and C₁₋₆ alkoxy; or Rⁿ¹ and R together with the atom to which they are attached form 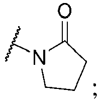 or R^{b} and R together with the atom to which they are attached form

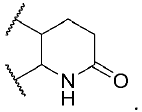

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹-, -NRⁿ¹C(O)-, -NRⁿ¹-C(O)-NRⁿ¹-, and -CR^{d}=CR^{e}-; Rⁿ¹, R^{d}, R^{e} are as defined in formula (IA); R is selected from the group consisting of C₁₋₆ haloalkyl, deuterated C₁₋₆ alkyl, and C₁₋₆ alkoxy C₁₋₆ alkyl; preferably, L is selected from the group consisting of -NRⁿ¹C(O)-, -NRⁿ¹-C(O)-NRⁿ¹- and -CR^{d}=CR^{e}-; Rⁿ¹ is selected from the group consisting of hydrogen atom and C₁₋₆ alkyl; R^{d} and R^{e} are identical or different, and are each independently selected from the group consisting of hydrogen atom, C₁₋₆ alkyl, and halogen; R is selected from the group consisting of C₁₋₆ haloalkyl, deuterated C₁₋₆ alkyl, and C₁₋₆ alkoxy C₁₋₆ alkyl; more preferably, L is selected from the group consisting of -NHC(O)-, -NH-C(O)-NH-, and -CH=CF-; R is selected from the group consisting of C₁₋₆ haloalkyl, deuterated C₁₋₆ alkyl, and C₁₋₆ alkoxy C₁₋₆ alkyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹-, -NRⁿ¹C(O)-, -NRⁿ¹-C(O)-NRⁿ¹-, and -CR^{d}=CR^{e}-; Rⁿ¹, R^{d}, R^{e} are as defined in formula (IA); preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom and C₁₋₆ alkyl; R^{d} and R^{e} are identical or different, and are each independently selected from the group consisting of hydrogen atom, halogen, and C₁₋₆ alkyl; more preferably, L is selected from the group consisting of -NHC(O)-, -NH-C(O)-NH-, and -CH=CF-.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom, C₁₋₆ alkyl, and deuterated C₁₋₆ alkyl; R^{d} and R^{e} are identical or different, and are each independently selected from the group consisting of hydrogen atom, C₁₋₆ alkyl, and halogen.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is -NH-C(O)-N(CH₃)- or -NH-C(O)-N(CD₃)-.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹C(O)- and -NRⁿ¹-C(O)-NRⁿ¹-; Rⁿ¹ is selected from the group consisting of hydrogen atom, C₁₋₆ alkyl, and deuterated C₁₋₆ alkyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NRⁿ¹C(O)- and -NRⁿ¹-C(O)-NRⁿ¹-; Rⁿ¹ is selected from the group consisting of hydrogen atom, C₁₋₄ alkyl and deuterated C₁₋₄ alkyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from the group consisting of -NHC(O)-, -NH-C(O)-NH-, -NH-C(O)-N(CH₃)-, and -NH-C(O)-N(CD₃)-.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, L is selected from -NHC(O)-.

In some embodiments of the present disclosure, in the compound of formula (IA), formula (I), formula (I-1), formula (I-2), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₆ alkyl, C₁₋₆ alkoxy, C₁₋₆ hydroxyalkyl, and cyclopropyl; preferably, R is selected from the group consisting of C₁₋₆ alkyl and C₁₋₆ alkoxy; more preferably, R is selected from the group consisting of methyl, ethyl, and methoxy.

In some embodiments of the present disclosure, in the compound of formula (IA), formula (I), formula (I-1), formula (I-2), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₆ haloalkyl, deuterated C₁₋₆ alkyl, C₁₋₆ alkoxy C₁₋₆ alkyl, C₁₋₆ alkyl, C₁₋₆ alkoxy, and deuterated C₁₋₆ alkoxy.

In some embodiments of the present disclosure, in the compound of formula (IA), formula (I), formula (I-1), formula (I-2), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₆ haloalkyl, deuterated C₁₋₆ alkyl, and C₁₋₆ alkoxy C₁₋₆ alkyl; preferably, R is selected from the group consisting of

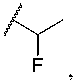

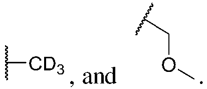

In some embodiments of the present disclosure, in the compound of formula (IA), formula (I), formula (I-1), formula (I-2), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of ethyl, methyl, and methoxy.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₆ haloalkyl, deuterated C₁₋₆ alkyl, C₁₋₆ alkoxy C₁₋₆ alkyl, C₁₋₆ alkyl, C₁₋₆ alkoxy, and deuterated C₁₋₆ alkoxy.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, Rⁿ¹ is selected from the group consisting of hydrogen atom, C₁₋₆ alkyl, and deuterated C₁₋₆ alkyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₄ alkyl, C₁₋₄ alkoxy, C₁₋₄ hydroxyalkyl, cyclopropyl, C₁₋₄ haloalkyl, deuterated C₁₋₄ alkyl, deuterated C₁₋₄ alkoxy, and C₁₋₄ alkoxy C₁₋₄ alkyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₄ haloalkyl, deuterated C₁₋₄ alkyl, C₁₋₄ alkoxy C₁₋₄ alkyl, C₁₋₄ alkyl, C₁₋₄ alkoxy, and deuterated C₁₋₄ alkoxy; preferably, R is selected from the group consisting of 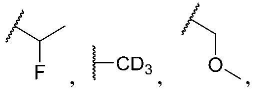 ethyl, methyl, methoxy, and

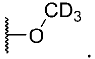

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from C₁₋₄ alkyl; preferably, R is selected from ethyl.

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from deuterated C₁₋₄ alkoxy; preferably, R is selected from

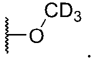

In some embodiments of the present disclosure, in the compound of formula (IA), formula (I), formula (1-1), formula (I-2), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, R is selected from the group consisting of C₁₋₄ alkyl, C₁₋₄ alkoxy, and deuterated C₁₋₄ alkoxy, preferably ethyl, methoxy, and deuterated methoxy, more preferably ethyl and

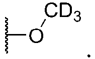

In some embodiments of the present disclosure, in the compound of formula (IA), formula (I), formula (I-1), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, Rⁿ¹ and R together with the atom to which they are attached form 3- to 8-membered heterocyclyl optionally substituted with C₁₋₆ alkyl; wherein the 3- to 8-membered heterocyclyl is selected from the group consisting of 5- to 6-membered monocyclic heterocyclyl, 5- to 8-membered spiro heterocyclyl, and 5- to 8-membered fused heterocyclyl.

In some embodiments of the present disclosure, in the compound of formula (IA), formula (I), formula (I-1), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, Rⁿ¹ and R together with the atom to which they are attached form

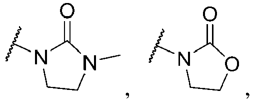

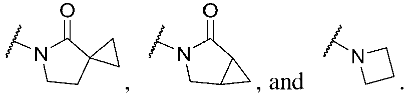

In some embodiments of the present disclosure, in the compound of formula (IA), formula (I), formula (I-1), formula (I-2), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, Rⁿ¹ and R together with the atom to which they are attached form 3- to 6-membered heterocyclyl or 5- to 10-membered heteroaryl; or R^{b} and R together with the atom to which they are attached form 3- to 6-membered heterocyclyl, wherein the 3- to 6-membered heterocyclyl or 5- to 10-membered heteroaryl is each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; more preferably, Rⁿ¹ and R together with the atom to which they are attached form 5- to 6-membered heterocyclyl; or R^{b} and R together with the atom to which they are attached form 5- to 6-membered heterocyclyl, wherein the 5- to 6-membered heterocyclyl is each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; further preferably, Rⁿ¹ and R together with the atom to which they are attached form 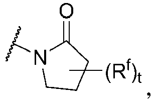 or R^{b} and R together with the atom to which they are attached form 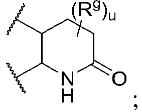 wherein each R^{b} and R^{g} is identical or different, and is independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; t is 0, 1, 2, 3 or 4; u is 0, 1, 2, 3, 4 or 5; even more preferably, Rⁿ¹ and R together with the atom to which they are attached form 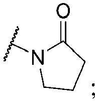 or R^{b} and R together with the atom to which they are attached form

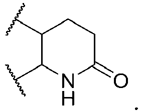

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, the structural moiety 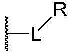 is selected from the group consisting of

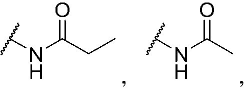

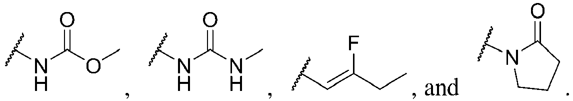

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, the structural moiety 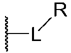 is selected from the group consisting of 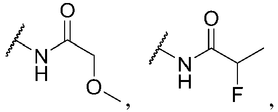 and

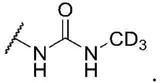

In some embodiments of the present disclosure, in the compound of formula (IA), formula (I), formula (II), formula (III), formula (IV), formula (IV-1), formula (V), formula (V-1), or formula (I-a), the stereoisomer thereof, the isotopic derivative thereof, the prodrug thereof, the N-oxide thereof, the solvate thereof, or the pharmaceutically acceptable salt thereof, the structural moiety 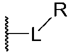 is selected from the group consisting of 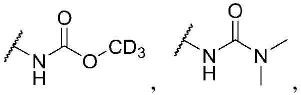 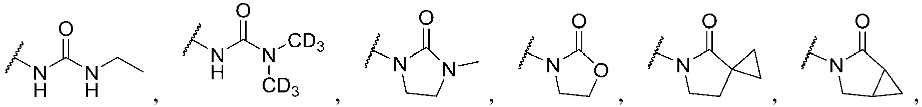 and

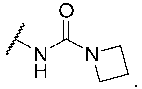

In some embodiments of the present disclosure, in the compound of formula (IA), formula (I), formula (II), formula (III), formula (IV), or formula (V), the stereoisomer thereof, the isotopic derivative thereof, the prodrug thereof, the N-oxide thereof, the solvate thereof, or the pharmaceutically acceptable salt thereof, the structural moiety 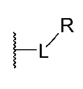 is selected from

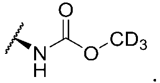

In some embodiments of the present disclosure, in the compound of formula (IV-1), formula (V-1), or formula (I-a), the stereoisomer thereof, the isotopic derivative thereof, the prodrug thereof, the N-oxide thereof, the solvate thereof, or the pharmaceutically acceptable salt thereof, the structural moiety 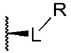 is selected from

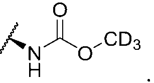

In some embodiments of the present disclosure, in the compound of formula (IA), formula (I), formula (II), formula (III), formula (IV), or formula (V), the stereoisomer thereof, the isotopic derivative thereof, the prodrug thereof, the N-oxide thereof, the solvate thereof, or the pharmaceutically acceptable salt thereof, the structural moiety 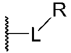 is selected from

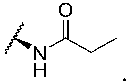

In some embodiments of the present disclosure, in the compound of formula (IV-1), formula (V-1), or formula (I-a), the stereoisomer thereof, the isotopic derivative thereof, the prodrug thereof, the N-oxide thereof, the solvate thereof, or the pharmaceutically acceptable salt thereof, the structural moiety 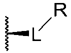 is selected from

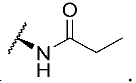

In some embodiments of the present disclosure, in the compound of formula (I-1) or formula (I-2), the stereoisomer thereof, the isotopic derivative thereof, the prodrug thereof, the N-oxide thereof, the solvate thereof, or the pharmaceutically acceptable salt thereof, ring C is selected from the group consisting of 5- to 8-membered monocyclic heterocyclyl fused with phenyl, 7- to 10-membered fused heterocyclyl fused with phenyl, 7- to 10-membered spiro heterocyclyl fused with phenyl, 5- to 8-membered monocyclic heterocyclyl fused with 5- to 6-membered heteroaryl, 7- to 10-membered fused heterocyclyl fused with 5- to 6-membered heteroaryl, and 5- to 6-membered heteroaryl fused with phenyl; each R^{c} is identical or different, and is independently selected from the group consisting of hydrogen atom, halogen, oxo, C₁₋₆ haloalkyl, cyano, amino, C₁₋₆ alkyl, 3- to 6-membered cycloalkyl; p is 0, 1, 2, 3 or 4; R^{b} is selected from the group consisting of hydrogen atom, halogen, and C₁₋₆ alkyl; n is 0 or 1; R is selected from the group consisting of C₁₋₆ haloalkyl, deuterated C₁₋₆ alkyl, C₁₋₆ alkoxy C₁₋₆ alkyl, C₁₋₆ alkyl, C₁₋₆ alkoxy, and deuterated C₁₋₆ alkoxy; Rⁿ¹ is selected from the group consisting of hydrogen atom, C₁₋₆ alkyl, and deuterated C₁₋₆ alkyl.

In some embodiments of the present disclosure, in the compound of formula (I-1) or formula (I-2), the stereoisomer thereof, the isotopic derivative thereof, the prodrug thereof, the N-oxide thereof, the solvate thereof, or the pharmaceutically acceptable salt thereof, ring C is selected from 5- to 8-membered monocyclic heterocyclyl fused with phenyl; each R^{c} is identical or different, and is independently selected from the group consisting of hydrogen atom, halogen, oxo, and C₁₋₆ alkyl; p is 0, 1, 2, 3 or 4; R^{b} is selected from the group consisting of hydrogen atom; R is selected from the group consisting of C₁₋₆ alkyl and deuterated C₁₋₆ alkoxy; Rⁿ¹ is selected from hydrogen atom.

In some embodiments of the present disclosure, in the compound of formula (I-1) or formula (I-2), the stereoisomer thereof, the isotopic derivative thereof, the prodrug thereof, the N-oxide thereof, the solvate thereof, or the pharmaceutically acceptable salt thereof, ring C is selected from 5- to 8-membered monocyclic heterocyclyl fused with phenyl; each R^{c} is identical or different, and is independently selected from the group consisting of hydrogen atom, halogen, oxo, and C₁₋₆ alkyl; p is 0, 1, 2 or 3; R^{b} is selected from hydrogen atom; R is selected from the group consisting of ethyl and -OCD₃; Rⁿ¹ is selected from hydrogen atom.

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, X is selected from the group consisting of -CH₂- and -O-; L is selected from the group consisting of -NHC(O)- and -NH-C(O)-NH-; bond a is a single bond or a double bond; when bond a is a single bond, Y and Z are identical or different, and are each independently selected from the group consisting of -O-and -CH₂-; when bond a is a double bond, Y and Z are identical, and are both selected from CH; R^{c1} is selected from hydrogen atom, C₁₋₆ alkyl, C₁₋₆ haloalkyl, 3- to 6-membered heterocyclyl, and 3- to 6-membered cycloalkyl; R^{c2}, R^{c3}, R^{c4} are identical or different, and are each independently selected from the group consisting of hydrogen atom, halogen, C₁₋₆ alkyl, and C₁₋₆ haloalkyl; R is selected from the group consisting of 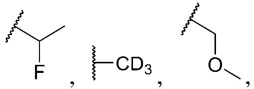 ethyl, methyl, methoxy, and 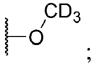 provided that: when X is -CH₂-, 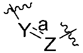 is not -CH₂-CH₂- at the same time, and -L-R is not -NHC(O)-CH₂CH₃ at the same time.

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, X is selected from the group consisting of -CH₂- and -O-; L is selected from -NHC(O)-; bond a is a double bond, Y and Z are identical, and are both selected from CH; R^{c1} is selected from hydrogen atom and C₁₋₆ alkyl; R^{c2}, R^{c3}, R^{c4} are identical or different, and are each independently selected from the group consisting of hydrogen atom, halogen, and C₁₋₆ alkyl; R is selected from the group consisting of 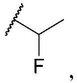 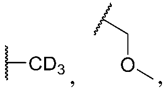 ethyl, methyl, methoxy, and

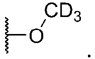

In some embodiments of the present disclosure, in the compound of formula (I-a), the stereoisomer thereof, the isotopic derivative thereof, the prodrug thereof, the N-oxide thereof, the solvate thereof, or the pharmaceutically acceptable salt thereof, X is selected from the group consisting of -CH₂- and -O-; L is selected from -NHC(O)-; bond a is a double bond, Y and Z are identical, and are both selected from CH; R^{c1} is selected from the group consisting of methyl; R^{c2} is selected from the group consisting of hydrogen atom, halogen, and C₁₋₆ alkyl; R^{c3} and R^{c4} are identical, and are selected from hydrogen atom; R is selected from the group consisting of 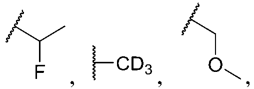 ethyl, methyl, methoxy, and

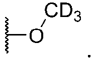

The present disclosure further provides the following compounds, stereoisomers thereof, isotopic derivatives thereof, prodrugs thereof, N-oxides thereof, solvates thereof, or pharmaceutically acceptable salts thereof, wherein the representative compounds of the present disclosure include, but are not limited to, any one of the structures listed in Table 1 below, prodrugs thereof, N-oxides thereof, solvates thereof, or pharmaceutically acceptable salts thereof:

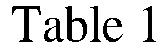

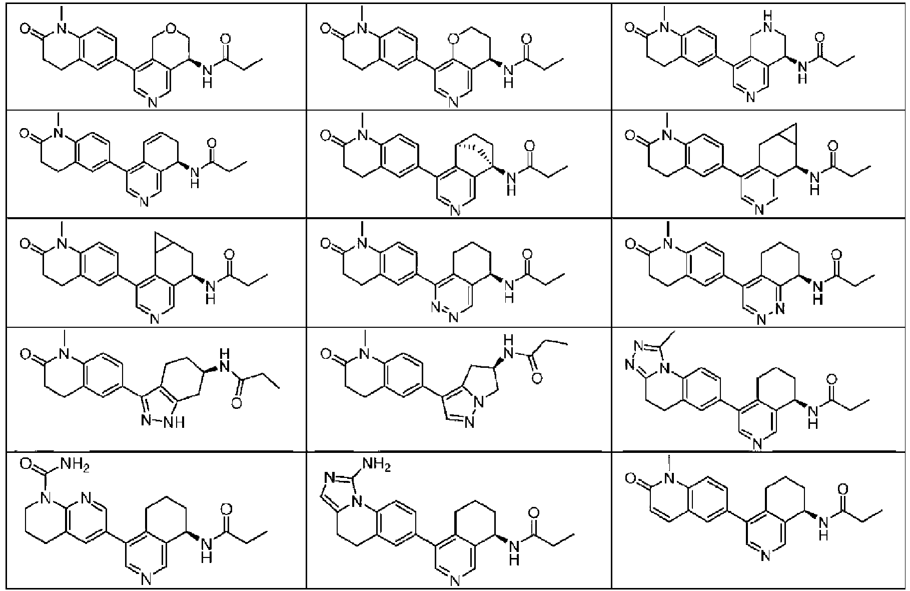

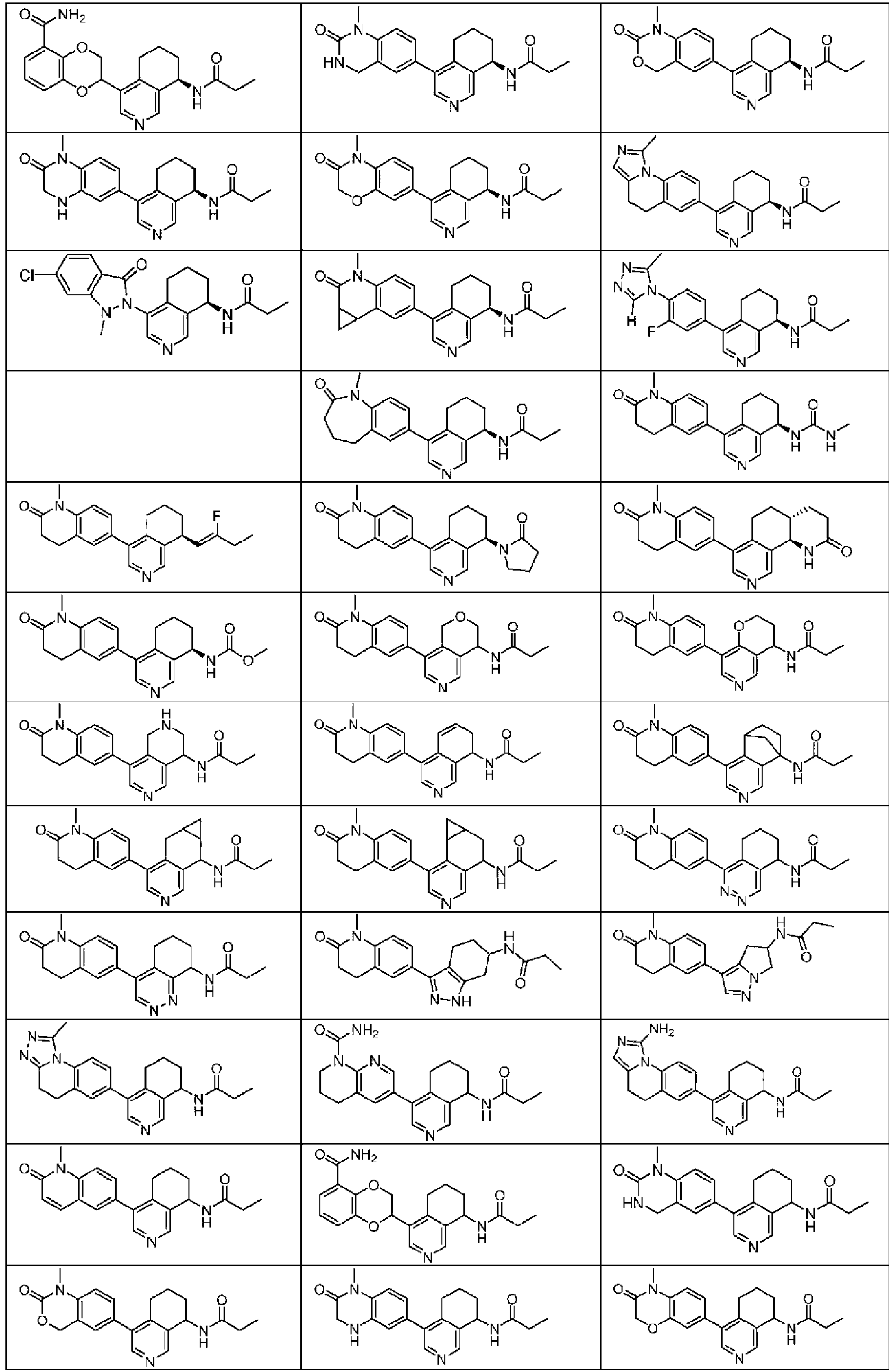

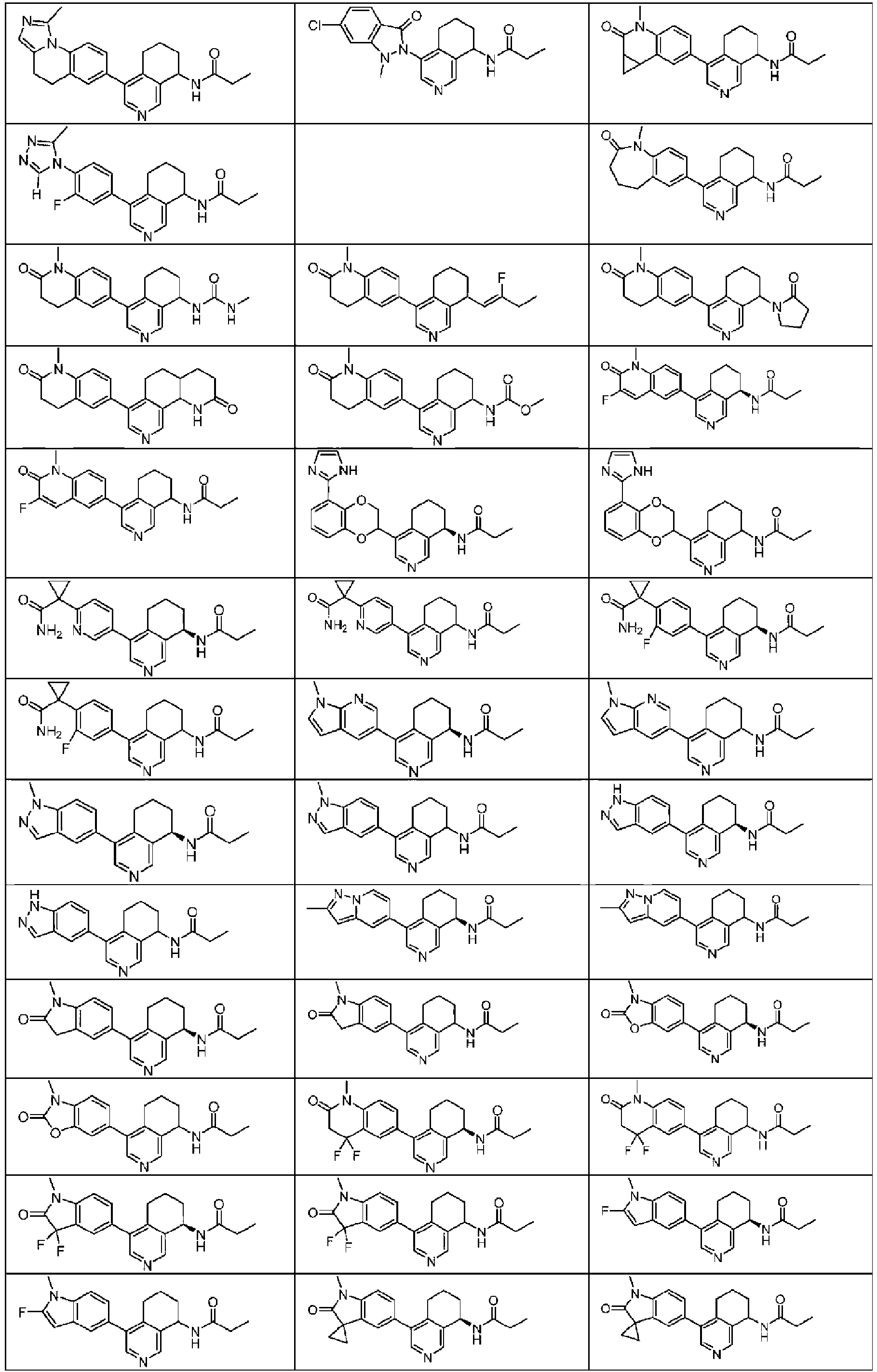

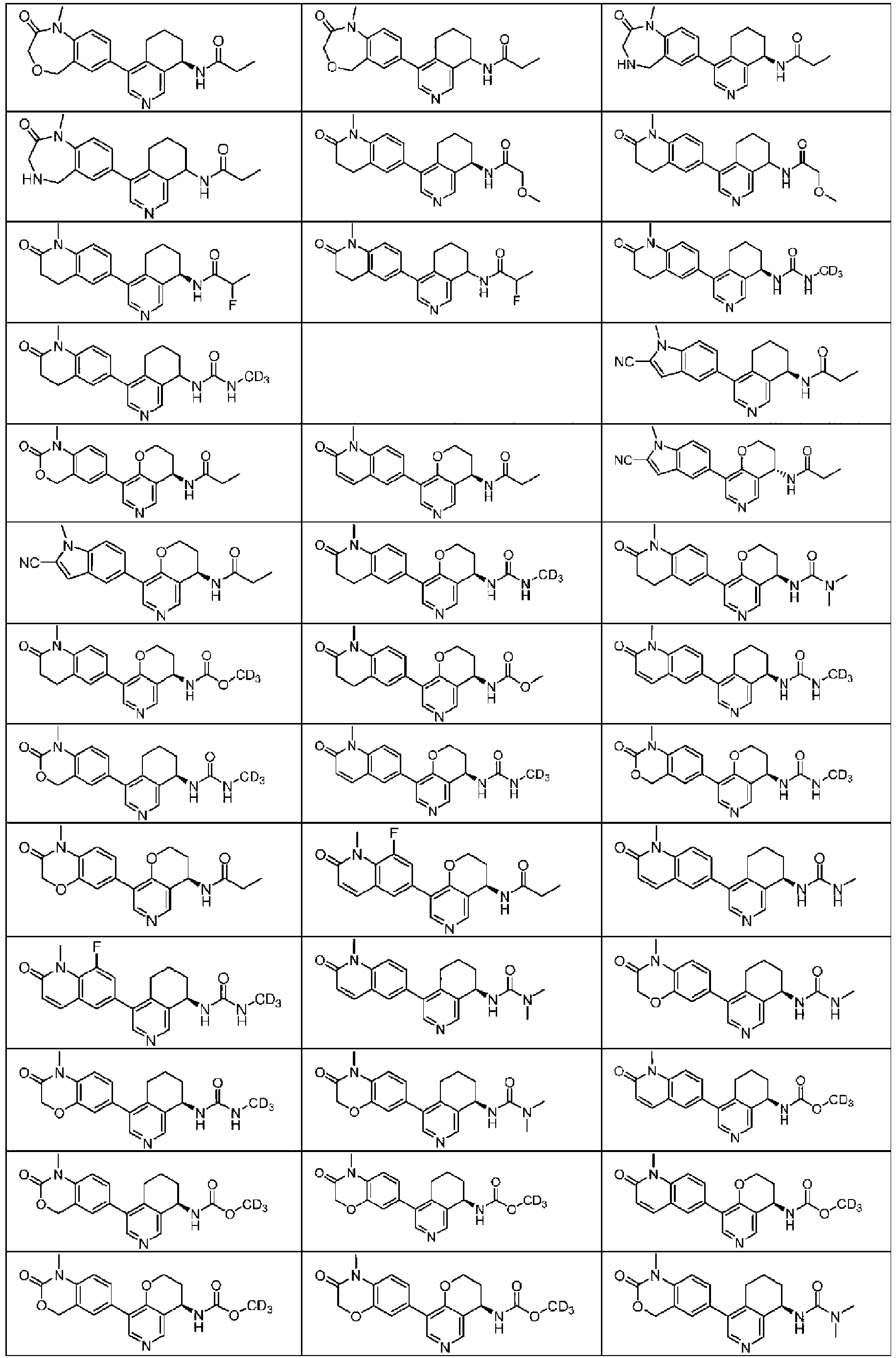

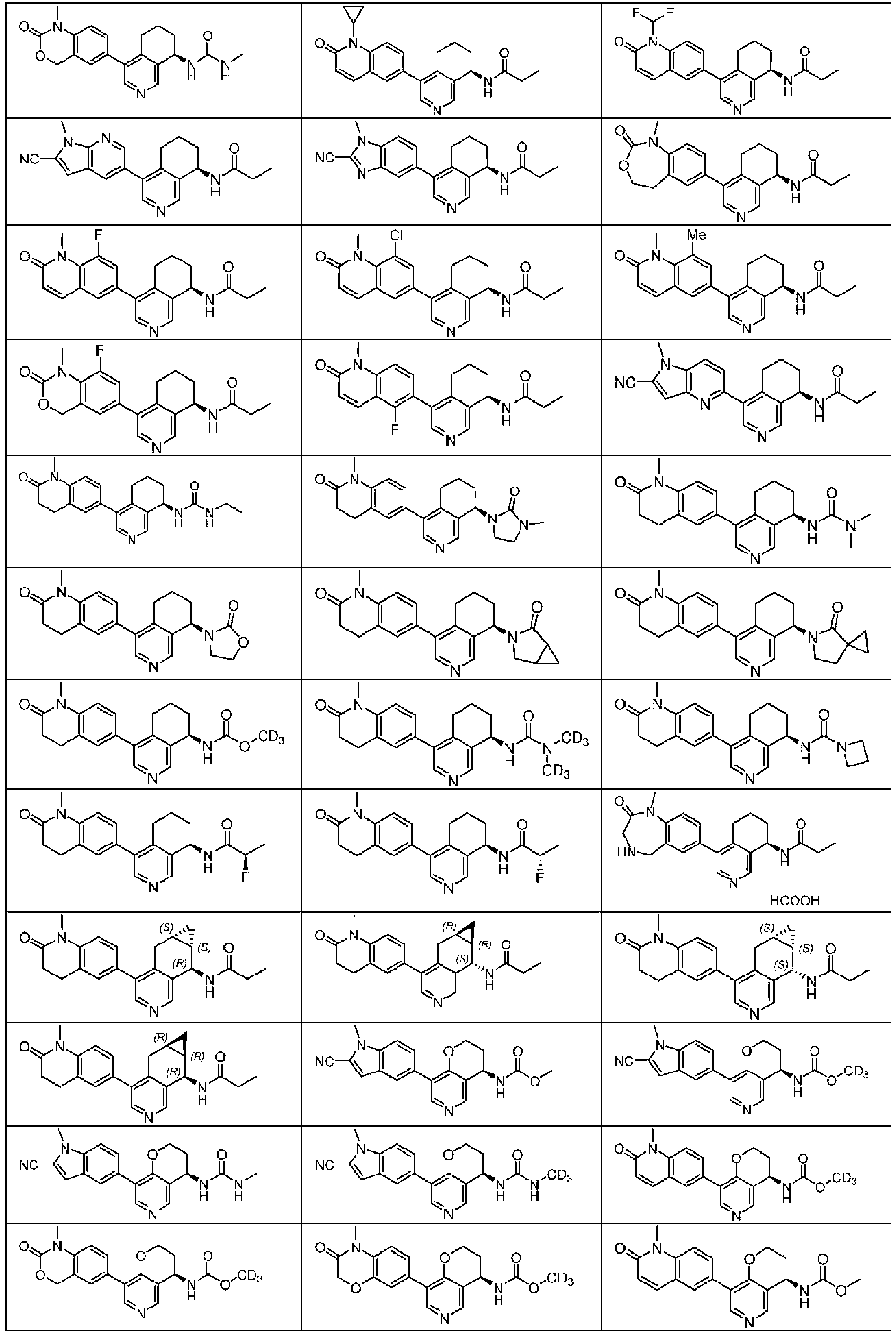

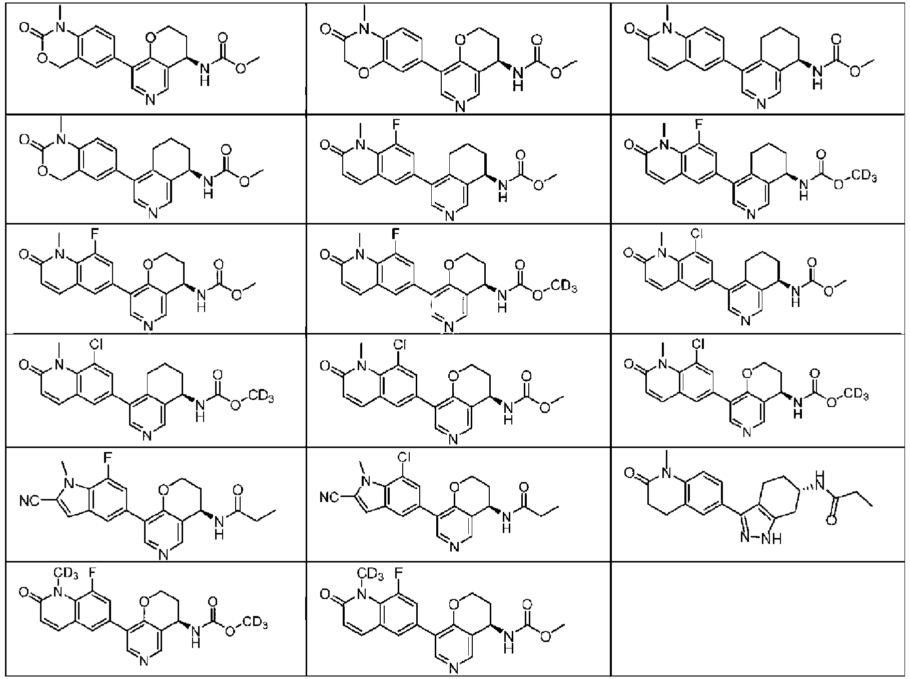

Another aspect of the present disclosure provides a pharmaceutical composition comprising the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, and one or more pharmaceutically acceptable carriers.

The present disclosure further provides the use of the compound of formula (IA), formula (I), formula (1-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same, in the manufacture of a medicament for inhibiting CYP11B2, preferably in the manufacture of a medicament for selectively inhibiting CYP11B2.

The present disclosure further provides the use of the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same, in the manufacture of a medicament for treating and/or preventing a CYP11B2-mediated disease.

The present disclosure further provides a method for inhibiting CYP11B2, comprising administering to a patient in need thereof a therapeutically effective amount of the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same.

The present disclosure further provides a method for treating and/or preventing a CYP11B2-mediated disease, comprising administering to a patient in need thereof a therapeutically effective amount of the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same.

The present disclosure further provides the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same, for use as a medicament.

The present disclosure further provides the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same, for use as a CYP11B2 inhibitor, preferably for use as a selective CYP11B2 inhibitor.

The present disclosure further provides the compound of formula (IA), formula (I), formula (I-1), formula (I-2), formula (II), formula (III), formula (IV), formula (IV-1), formula (V), formula (V-1), formula (I-a), and the compound shown in Table 1 of the present disclosure, the stereoisomer thereof, the isotopic derivative thereof, the prodrug thereof, the N-oxide thereof, the solvate thereof, or the pharmaceutically acceptable salt thereof, or the pharmaceutical composition comprising the same, for use in treating and/or preventing a CYP11B2-mediated disease.

Preferably, the disease as described above in the present disclosure is selected from the group consisting of hypertension, chronic kidney disease, diabetic nephropathy, primary aldosteronism, chronic kidney disease, and diabetic nephropathy; the hypertension is preferably selected from the group consisting of resistant hypertension, uncontrolled hypertension, low-renin hypertension, and primary hypertension.

The active compound can be formulated into a form suitable for administration by any appropriate route, preferably in a moiety dose, or in a form that allows the patient to self-administer a single dose. The moiety dose of the compound or composition of the present disclosure may be presented as a tablet, capsule, cachet, vial, powder, granule, lozenge, suppository, reconstitutable powder, or liquid formulation.

In addition to the active compound, the pharmaceutical composition of the present disclosure may contain one or more adjuvants selected from the group consisting of the following components: fillers (diluents), binders, wetting agents, disintegrating agents and excipients, etc. Depending on the route of administration, the composition may contain 0.1 to 99 wt.% of the active compound; preferably, the composition may contain 1 to 95 wt.% of the active compound.

The pharmaceutical composition containing the active ingredient may be in a form suitable for oral administration, such as tablets, troches, lozenges, aqueous or oil suspensions, dispersible powders or granules, emulsions, hard or soft capsules, or syrups or elixirs. Oral compositions may be prepared by any known methods in the art for preparing pharmaceutical compositions, and such compositions may contain one or more components selected from the group consisting of sweeteners, flavoring agents, coloring agents and preservatives to provide a pleasing and palatable pharmaceutical preparation. Tablets contain the active ingredient and suitable, non-toxic pharmaceutically acceptable excipients for mixing to prepare the tablets. These excipients may be inert excipients, granulating agents, disintegrating agents, binders and lubricants. These tablets may be uncoated or may be coated using known techniques to mask the taste of the drug or to delay disintegration and absorption in the gastrointestinal tract, thereby providing a sustained-release effect over a longer period.

As well known to those skilled in the art, the dosage of a drug depends on various factors, including but not limited to: the activity of the specific compound used, the age of the patient, the weight of the patient, the health status of the patient, the behavior of the patient, the diet of the patient, the time of administration, the route of administration, the rate of excretion, drug combinations, the severity of the disease, etc.; furthermore, the optimal treatment regimen, such as the mode of treatment, the daily dose of the compound, or the type of pharmaceutically acceptable salt, may be validated according to conventional treatment protocols.

### Terms and definitions

Unless otherwise specified, the following terms and phrases used herein are intended to have the following meanings. A specific term or phrase without a particular definition should not be considered indefinite or unclear, but should be understood according to its ordinary meaning.

"Optionally" or "optional" means that the subsequently described event or circumstance may but need not necessarily occur, and includes both the situation where the event or circumstance occurs and the situation where it does not occur. For example, "alkyl optionally substituted with halogen or cyano" includes the situation where the alkyl is substituted with halogen or cyano and the situation where the alkyl is not substituted with halogen and cyano.

Unless otherwise specified, the term "alkyl" refers to a branched or linear chain saturated aliphatic hydrocarbon group derived by removing one hydrogen from an alkane with the specified number of carbon atoms. For example, "C₁₋₁₀ alkyl" includes C₁, C₂, C₃, C₄, C₅, C₆, C₇, C₈, C₉, C₁₀ alkyl, "C₁₋₆ alkyl", "C₁₋₄ alkyl", "C₁₋₃ alkyl"; specific examples include but are not limited to: methyl, ethyl, n-propyl, isopropyl, sec-butyl, 2-methylbutyl, 1,1-dimethylbutyl, etc. Alkyl may be substituted or unsubstituted. When substituted, it may be substituted at any available connecting point, and the substituent is preferably selected from the group consisting of one or more of D atom, halogen, alkoxy, haloalkyl, haloalkoxy, cycloalkyloxy, heterocyclyloxy, hydroxy, hydroxyalkyl, cyano, amino, nitro, cycloalkyl, cycloalkenyl, heterocyclyl, aryl and heteroaryl.

Unless otherwise specified, the term "haloalkyl" refers to an alkyl in which one or more hydrogen atoms are replaced by halogen atoms, wherein alkyl is as defined above. Preferably C₁₋₆ haloalkyl. Examples of haloalkyl include but are not limited to monofluoromethyl, difluoromethyl, trifluoromethyl, trichloromethyl, tribromomethyl, 2,2,2-trifluoroethyl, 2,2,2-trichloroethyl, etc. Alkyl is as defined above.

Unless otherwise specified, the term "hydroxyalkyl" refers to a group derived by replacing one or more hydrogen atoms in an alkyl with a hydroxy group, wherein alkyl is as defined above. The "hydroxyalkyl" described in the present disclosure includes "C₁₋₆ hydroxyalkyl", "C₁₋₄ hydroxyalkyl"; specific examples include but are not limited to -CH₂OH, -CH₂CH₂OH, -CH(OH)CH₃, -CH₂CH₂CH₂OH, 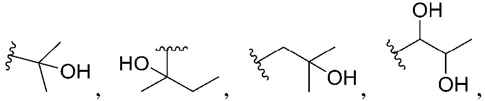 etc.

Unless otherwise specified, the term "alkoxy" refers to an alkyl as defined herein connected to another group via an oxygen atom, i.e., "-O-(alkyl)", wherein alkyl is as defined above. It includes "C₁₋₆ alkoxy" (structure: C₁₋₆ alkyl-O-), "C₁₋₄ alkoxy", specific examples include but are not limited to methoxy, ethoxy, propoxy, 1-methylethoxy, butoxy, 2-methylbutoxy, 3-methylbutoxy, 1,1-dimethylpropoxy, 1,2-dimethylpropoxy, etc.; preferably, the "alkoxy" described in the present disclosure is preferably C₁₋₆ alkoxy, more preferably C₁₋₄ alkoxy. Alkoxy may be substituted or unsubstituted. When substituted, it may be substituted at any available connecting point, and the substituent is preferably selected from the group consisting of one or more of D atom, halogen, alkoxy, haloalkyl, haloalkoxy, cycloalkyloxy, heterocyclyloxy, hydroxy, hydroxyalkyl, cyano, amino, nitro, cycloalkyl, cycloalkenyl, heterocyclyl, aryl and heteroaryl.

Unless otherwise specified, the term "haloalkoxy" refers to an alkoxy group in which one or more hydrogens are replaced by halogen, wherein alkoxy is as defined above. Preferably, the "haloalkoxy" described in the present disclosure is preferably "C₁₋₆ haloalkoxy", "C₁₋₄ haloalkoxy". Specific examples described in the present disclosure include: fluoromethoxy (including monofluoromethoxy, difluoromethoxy, trifluoromethoxy), -OCH₂CF₃, -OCHFCH₃, etc.

Unless otherwise specified, the term "alkenyl" refers to a group derived from a linear or branched alkene (or an alkyl containing at least one carbon-carbon double bond, wherein alkyl is as defined above) by removal of one hydrogen atom, including "C₂₋₆ alkenyl", "C₂₋₅ alkenyl", "C₂₋₄ alkenyl", "C₂₋₃ alkenyl", specific examples include but are not limited to: -CH=CH₂, -CH=CHCH₃, -C (CH₂)=CH₂, -CH=CHCH₂CH₃, -CH₂CH=CHCH₃, etc. Alkenyl may be substituted or unsubstituted. When substituted, it may be substituted at any available connecting point, and the substituent is preferably selected from the group consisting of one or more of D atom, alkoxy, halogen, haloalkyl, haloalkoxy, cycloalkyloxy, heterocyclyloxy, hydroxy, hydroxyalkyl, cyano, amino, nitro, cycloalkyl, cycloalkenyl, heterocyclyl, aryl and heteroaryl.

Unless otherwise specified, the term "alkynyl" refers to a group derived from a linear or branched alkyne (or an alkyl containing at least one carbon-carbon triple bond, wherein alkyl is as defined above) by removal of one hydrogen atom, including "C₂₋₅ alkynyl", "C₂₋₄ alkynyl", "C₂₋₃ alkynyl", specific examples include but are not limited to: -C≡CH, -C≡CCH₃, CH≡CCH₂-, CH≡C-C≡C-, etc. Alkynyl may be substituted or unsubstituted. When substituted, it may be substituted at any available connecting point, and the substituent is preferably selected from the group consisting of one or more of D atom, alkoxy, halogen, haloalkyl, haloalkoxy, cycloalkyloxy, heterocyclyloxy, hydroxy, hydroxyalkyl, cyano, amino, nitro, cycloalkyl, cycloalkenyl, heterocyclyl, aryl and heteroaryl.

Unless otherwise specified, the term "hetero" indicates a substituted or unsubstituted heteroatom and oxidized forms of the heteroatom (further called heteroatom groups), the heteroatoms generally selected from the group consisting of N, O, S, and P, and oxidized forms generally include NO, SO, S(O)₂, P(O); nitrogen atoms may be substituted, i.e., NR (R is H or other substituents defined herein); the number of ring atoms is usually defined as the ring size, e.g., "3- to 6-membered heterocyclyl" refers to a ring formed by 3 to 6 atoms arranged around, each ring optionally containing 1 to 3 heteroatoms and/or heteroatom groups, i.e., N, O, S, NO, SO, S(O)₂, P(O), or NR, each ring is optionally substituted with an R group, where R is a group defined herein.

Unless otherwise specified, the term "cycloalkyl" refers to a saturated cyclic alkyl derived by removing one hydrogen atom from a cycloalkane, including monocyclic or polycyclic saturated hydrocarbon groups; the polycyclic saturated hydrocarbon group refers to a polycyclic group formed by connecting two or more cyclic alkyl structures via spiro, bridged, or fused linkages. Carbon atoms in the cycloalkyl may be further oxidized, i.e., forming C(O). Unless specifically stated, the "X-membered cycloalkyl" described herein can be understood as a monocyclic cycloalkyl; when polycyclic, it will be specifically indicated as a spiro, fused, or bridged ring group. The cycloalkyl includes "3- to 8-membered cycloalkyl", "3- to 8-membered monocyclic cycloalkyl", "3- to 6-membered cycloalkyl", "3- to 5-membered cycloalkyl". Preferably, the cycloalkyl is monocyclic and saturated; specific examples include but are not limited to: cyclopropyl, cyclobutyl, cyclopentyl, cyclohexyl, cycloheptyl, cyclooctyl.

Unless otherwise specified, "cycloalkenyl" refers to a "cycloalkyl" in which one or more ring bonds are double bonds and the cycloalkenyl is not aromatic. Carbon atoms in the cycloalkenyl may be further oxidized, i.e., forming C(O). The cycloalkenyl includes "3- to 8-membered cycloalkenyl", "3- to 8-membered monocyclic cycloalkenyl", "3- to 6-membered cycloalkenyl", "3- to 5-membered cycloalkenyl", "5- to 6-membered cycloalkenyl". Specific examples include but are not limited to:

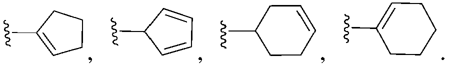

The term "spiro ring group" refers to a polycyclic system in which rings share one carbon atom (called the spiro atom), which may contain one or more double bonds within the ring, or may contain one or more heteroatoms selected from the group consisting of nitrogen, oxygen, and sulfur within the ring (the nitrogen may optionally be oxidized, i.e., forming a nitrogen oxide; the sulfur may optionally be oxidized, i.e., forming a sulfoxide or sulfone, but excluding -O-O-, -O-S-, or -S-S-), provided that it contains at least one all-carbon non-aromatic ring and the attachment point is on that all-carbon non-aromatic ring, and it has 5 to 10 (e.g., 5, 6, 7, 8, 9, 10) ring atoms (i.e., 5- to 10-membered spiro ring group). The spiro ring group preferably has 6 to 10 ring atoms (i.e., 6- to 10-membered spiro ring group), more preferably 7 to 10 ring atoms (i.e., 7-to 10-membered spiro ring group). The spiro ring group includes monospiro ring groups and polyspiro ring groups (such as dispiro ring groups, etc.), preferably monospiro ring groups or dispiro ring groups, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiro ring groups. Non-limiting examples include: 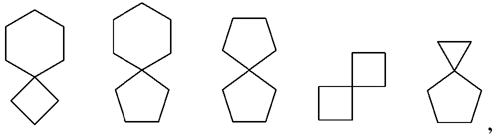 wherein the attachment point may be at any position; 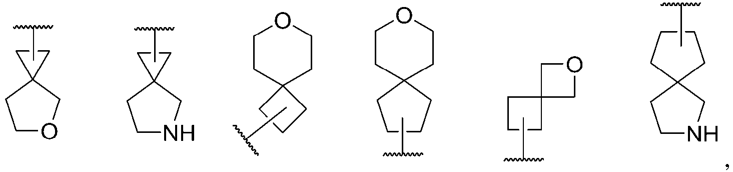 etc.

The term "fused ring group" refers to a polycyclic system in which rings share two adjacent carbon atoms, which is monocyclic cycloalkyl fused with one or more monocyclic cycloalkyls, or a monocyclic cycloalkyl fused with one or more of cycloalkenyl, heterocyclyl, aryl, or heteroaryl, wherein the attachment point is on the all-carbon non-aromatic ring, which may contain one or more double bonds within the ring, and has 5 to 14 (e.g., 5, 6, 7, 8, 9, 10, 11, 12, 13, 14) ring atoms (i.e., 5- to 14-membered fused ring group). The fused ring group preferably has 6 to 14 ring atoms (i.e., 6- to 14-membered fused ring group), more preferably 6 to 10 ring atoms (i.e., 6- to 10-membered fused ring group), and even more preferably 7 to 10 ring atoms (i.e., 7- to 10-membered fused ring group, including 7-membered fused ring group). The fused ring group includes bicyclic fused ring groups and polycyclic fused ring groups (such as tricyclic fused ring groups, tetracyclic fused ring groups, etc.), preferably bicyclic fused ring groups or tricyclic fused ring groups, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused ring groups. Non-limiting examples include: 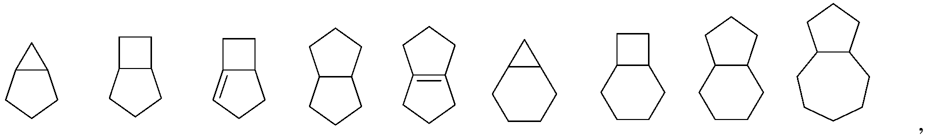 wherein the attachment point may be at any position; 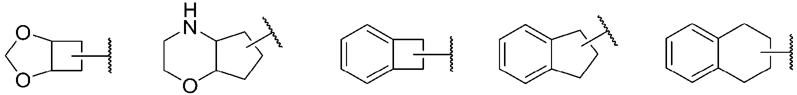 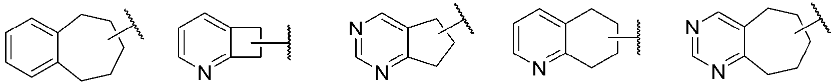 etc.

The term "bridged ring group" refers to an all-carbon polycyclic system in which rings share two carbon atoms that are not directly connected, which may contain one or more double bonds within the ring, and has 5 to 10 (e.g., 5, 6, 7, 8, 9, 10) carbon atoms (i.e., 5- to 10-membered bridged ring group). The bridged ring group preferably has 6 to 10 carbon atoms (i.e., 6- to 10-membered bridged ring group), more preferably 7 to 10 carbon atoms (i.e., 7- to 10-membered bridged ring group, including 7-membered bridged ring group). The bridged ring group includes bicyclic bridged ring groups and polycyclic bridged ring groups (such as tricyclic bridged ring groups, tetracyclic bridged ring groups, etc.), preferably bicyclic bridged ring groups or tricyclic bridged ring groups. Non-limiting examples include: 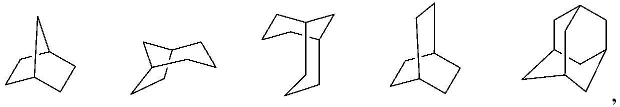 wherein the attachment point may be at any position.

Unless otherwise specified, the term "heterocyclyl" refers to a saturated or unsaturated cyclic group derived by replacing one or more ring carbon atoms in a cycloalkyl with heteroatoms and/or heteroatom groups (heterocyclyl includes heterocycloalkenyl, the same below). The heteroatoms and/or heteroatom groups are generally selected from the group consisting of N, O, S, NO, SO, S(O)₂, P(O), and NR, wherein carbon atoms in the heterocycle are optionally oxidized, i.e., forming -C(O); preferably, the heteroatoms are independently selected from 1-3 N and/or O. The heterocyclyl may be monocyclic heterocyclyl or polycyclic heterocyclyl, the polycyclic heterocyclyl including spiro heterocyclyl, fused heterocyclyl, and bridged heterocyclyl. Non-limiting examples include "3- to 8-membered heterocyclyl", "3- to 8-membered monocyclic heterocyclyl", "3- to 6-membered heterocyclyl", "3- to 5-membered heterocyclyl", "4- to 6-membered heterocyclyl", "5- to 6-membered heterocyclyl", "6-membered heterocyclyl", "5- to 6-membered monocyclic heterocyclyl", 5- to 8-membered spiro heterocyclyl, 5- to 8-membered fused heterocyclyl, 5- to 8-membered monocyclic heterocyclyl. Specific examples include but are not limited to azetidinyl, pyrrolidinyl, tetrahydrofuranyl, piperidinyl, piperazinyl, tetrahydropyranyl, morpholinyl, etc. When the heterocyclyl contains unsaturated structures, the heterocyclyl is further called "heterocycloalkenyl", which refers to a "heterocyclyl" in which one or more ring bonds are double bonds and the heterocycle is not aromatic. Preferably, the heteroatoms are independently selected from 1-3 N and/or O. The heterocyclyl includes "3- to 8-membered heterocycloalkenyl", "3- to 6-membered heterocycloalkenyl", "3- to 5-membered heterocycloalkenyl", "5- to 6-membered heterocycloalkenyl". Specific examples include but are not limited to: 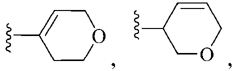 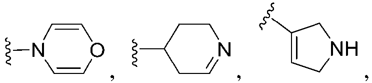 etc.

The term "fused heterocyclyl" refers to a polycyclic heterocyclic system in which rings share two adjacent atoms, which may contain one or more double bonds within the ring, and contains at least one (e.g., 1, 2, 3, or 4) heteroatom selected from the group consisting of nitrogen, oxygen, and sulfur within the ring (the nitrogen may optionally be oxidized, i.e., forming a nitrogen oxide; the sulfur may optionally be oxidized, i.e., forming a sulfoxide or sulfone, but excluding -O-O-, -O-S-, or -S-S-), formed by a monocyclic heterocyclyl fused with one or more monocyclic heterocyclyls, or a monocyclic heterocyclyl fused with one or more of cycloalkyl, cycloalkenyl, aryl, or heteroaryl, wherein the attachment point is on the monocyclic heterocyclyl, and having 6 to 14 (e.g., 6, 7, 8, 9, 10, 11, 12, 13, 14) ring atoms (i.e., 6- to 14-membered fused heterocyclyl). The fused heterocyclyl preferably has 7 to 10 ring atoms (i.e., 7- to 10-membered fused heterocyclyl) or preferably has 5 to 8 ring atoms (i.e., 5- to 8-membered fused heterocyclyl). The fused heterocyclyl includes bicyclic and polycyclic fused heterocyclyls (such as tricyclic fused heterocyclyls, tetracyclic fused heterocyclyls, etc.), preferably bicyclic fused heterocyclyls or tricyclic fused heterocyclyls, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyls. Non-limiting examples include: 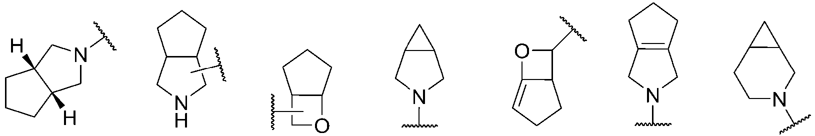 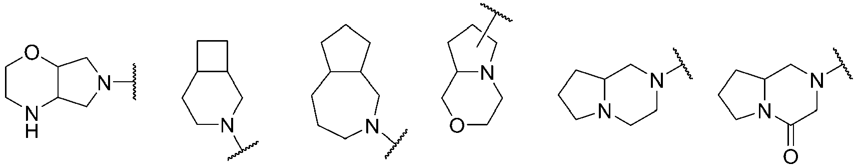 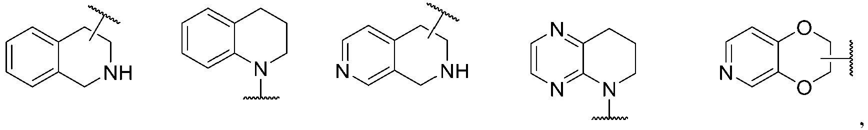 etc.

The term "spiro heterocyclyl" refers to a polycyclic heterocyclic system in which rings share one atom (called the spiro atom), which may contain one or more double bonds within the ring, and contains at least one (e.g., 1, 2, 3, or 4) heteroatom selected from the group consisting of nitrogen, oxygen, and sulfur within the ring (the nitrogen may optionally be oxidized, i.e., forming a nitrogen oxide; the sulfur may optionally be oxidized, i.e., forming a sulfoxide or sulfone, but excluding -O-O-, -O-S-, or -S-S-), provided that it contains at least one monocyclic heterocyclyl and the attachment point is on that monocyclic heterocyclyl. It preferably has 6 to 14 ring atoms (i.e., 6- to 14-membered spiro heterocyclyl), more preferably 7 to 10 ring atoms (i.e., 7- to 10-membered spiro heterocyclyl), or preferably 5 to 8 ring atoms (i.e., 5- to 8-membered spiro heterocyclyl). Non-limiting examples include: 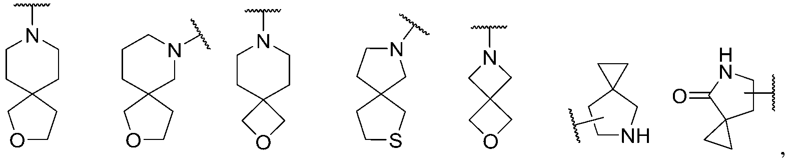 etc.

The term "aryl" refers to a monocyclic all-carbon aromatic ring (i.e., monocyclic aryl) or a polycyclic aromatic ring system (i.e., polycyclic aryl) having a conjugated π-electron system, which has 6 to 14 (e.g., 6, 7, 8, 9, 10, 11, 12, 13, or 14) ring atoms (i.e., 6- to 14-membered aryl). The aryl preferably has 6 to 10 ring atoms (i.e., 6- to 10-membered aryl). The monocyclic aryl, for example, phenyl. The polycyclic aryl, non-limiting examples include: naphthyl, anthryl, phenanthryl, etc. The polycyclic aryl further includes phenyl fused with one or more of heterocyclyl or cycloalkyl, or naphthyl fused with one or more of heterocyclyl or cycloalkyl, wherein the attachment point is on the phenyl or naphthyl, and in this case, the number of ring atoms continues to represent the number of ring atoms in the polycyclic aromatic ring system and preferably has 7 to 14 (e.g., 7, 8, 9, 10, 11, 12, 13, or 14) ring atoms (i.e., 7- to 14-membered fused aryl). Non-limiting examples include: 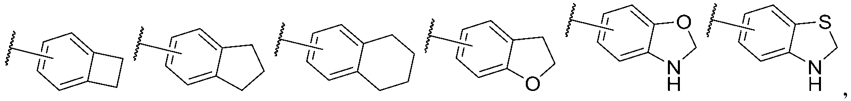 etc.

The term "heteroaryl" refers to a monocyclic heteroaromatic ring (i.e., monocyclic heteroaryl) or a polycyclic heteroaromatic ring system (i.e., polycyclic heteroaryl) having a conjugated π-electron system, containing at least one (e.g., 1, 2, 3, or 4) heteroatom selected from the group consisting of nitrogen, oxygen, and sulfur within the ring (the nitrogen may optionally be oxidized, i.e., forming a nitrogen oxide; the sulfur may optionally be oxidized, i.e., forming a sulfoxide or sulfone, but excluding -O-O-, -O-S-, or -S-S-), having 5 to 14 (e.g., 5, 6, 7, 8, 9, 10, 11, 12, 13, or 14) ring atoms (i.e., 5- to 14-membered heteroaryl). The heteroaryl preferably has 5 to 10 ring atoms (i.e., 5- to 10-membered heteroaryl), more preferably 5 or 6 ring atoms (i.e., 5- to 6-membered heteroaryl). The polycyclic heteroaryl preferably has 6 to 14 (e.g., 6, 7, 8, 9, 10, 11, 12, 13, or 14) ring atoms (i.e., 6- to 14-membered fused heteroaryl).

The monocyclic heteroaryl, non-limiting examples include: furyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridyl, pyrimidinyl, pyridonyl, N-alkylpyridone (such as 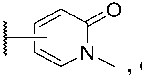 etc.), pyrazinyl, pyridazinyl, etc.

The polycyclic heteroaryl, non-limiting examples include: indolyl, indazolyl, quinolinyl, isoquinolinyl, quinoxalinyl, phthalazinyl, benzimidazolyl, benzothienyl, quinazolinyl, benzothiazolyl, carbazolyl, etc. The polycyclic heteroaryl further includes a monocyclic heteroaryl fused with one or more aryls, wherein the attachment point is on the aromatic ring, and in this case, the number of ring atoms continues to represent the number of ring atoms in the polycyclic heteroaromatic ring system. The polycyclic heteroaryl further includes a monocyclic heteroaryl fused with one or more of cycloalkyl or heterocyclyl, wherein the attachment point is on the monocyclic heteroaromatic ring, and in this case, the number of ring atoms continues to represent the number of ring atoms in the polycyclic heteroaromatic ring system and preferably has 6 to 14 (e.g., 6, 7, 8, 9, 10, 11, 12, 13, or 14) ring atoms (i.e., 6- to 14-membered fused heteroaryl). Non-limiting examples include: 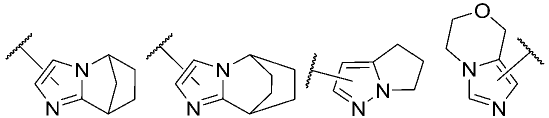 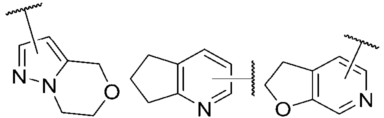 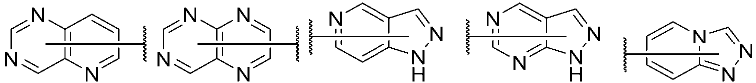 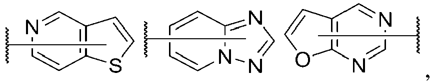 etc.

The term "halogen" refers to fluorine, chlorine, bromine or iodine.

The term "hydroxy" refers to -OH.

The term "amino" refers to -NH₂.

The term "cyano" refers to -CN.

The term "oxo" or "oxo group" refers to "=O".

The compounds of the present disclosure may exist in specific stereoisomeric forms. The term "stereoisomer" refers to isomers that have the idential structure but differ in the spatial arrangement of atoms. This includes cis and trans (or Z and E) isomers, (-)- and (+)- isomers, (R)- and *(S)-* enantiomers, diastereomers, *(D)-* and *(L)-* isomers, tautomers, atropisomers, conformers, and mixtures thereof (such as racemates, mixtures of diastereomers). All these stereoisomers and mixtures thereof are included within the scope of the present disclosure. Optically active (-)- and (+)- isomers, (R)- and (S)- enantiomers, and *(D)-* and *(L)-* isomers can be prepared by chiral synthesis, chiral reagents, or other conventional techniques. An isomer of a compound of the present disclosure can be prepared by asymmetric synthesis or using chiral auxiliaries, alternatively, when the molecule contains a basic functional group (such as amino) or an acidic functional group (such as carboxyl), by forming a diastereomeric salt with a suitable optically active acid or base, followed by conventional methods known in the art for diastereomer resolution to obtain a pure isomer. Furthermore, the separation of enantiomers and diastereomers is typically accomplished by chromatography.

The absolute stereochemistry of the compounds can be confirmed by conventional techniques in the art. For example, single-crystal X-ray diffraction, or by the chiral structure of the starting material and the reaction mechanism of asymmetric synthesis to determine the absolute configuration of the compound. Alternatively, after resolution, its stereoconfiguration can be determined by comparison with a product whose absolute configuration is known. For example, by comparison with the compound of Reference Example 1 with an absolute configuration (refer to patent WO2013041591A1), its stereo configuration can be determined by differences in activity. Compounds labeled as "absolute configuration unknown/undetermined" herein are typically obtained by resolving racemic compounds into single isomers via chiral preparative SFC, followed by characterization and testing.

The compounds of the present disclosure include all suitable isotopic derivatives of the compounds. The term "isotopic derivative" refers to a compound in which at least one atom is replaced by an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T), ¹¹C, ¹³C, ¹⁴C, ¹⁵N, ¹⁷O, ¹⁸O, ³²P, ³³P, ³³S, ³⁴S, ³⁵S, ³⁶S, ¹⁸F , ³⁶Cl , ⁸²Br, ¹²³I, ¹²⁴I, ¹²⁵I, ¹²⁹I, and ¹³¹I, etc., preferably deuterium. Compared to non-deuterated drugs, deuterated drugs have advantages such as reduced toxicity and side effects, increased drug stability, enhanced efficacy, and prolonged biological half-life. All isotopic variations of the compounds of the present disclosure, whether radioactive or not, are encompassed within the scope of the present disclosure. Each available hydrogen atom attached to a carbon atom may be independently replaced by a deuterium atom, wherein deuterium substitution can be partial or complete. Partial deuterium substitution means that at least one hydrogen is replaced by at least one deuterium.

The term "prodrug" refers to certain derivatives of the compounds of the present disclosure that themselves have little or no pharmacological activity, which contain cleavable groups and decompose by solvolysis or under physiological conditions to become the compounds of the present disclosure. Types of the prodrug include but are not limited to amides, esters, anhydrides, salts, etc. The term "ester" refers to a derivative formed when a compound of the present disclosure contains an acidic group (such as carboxylic acid) with a suitable alcohol; or when a compound of the present disclosure contains a hydroxy group, with a suitable acid (including organic or inorganic acid). Methods for preparing prodrugs are well known to those skilled in the art.

The term "solvate" refers to an associate or complex of one or more solvent molecules with a compound of the present disclosure. The term "hydrate" may be used when the solvent is water. The solvent molecules may be stoichiometric or non-stoichiometric.

The term "nitrogen oxide" or "N-oxide" refers to a derivative formed by further oxidation of a nitrogen atom in a nitrogen-containing group. Common N-oxides include N-oxides of tertiary amines or N-oxides of nitrogen atoms in nitrogen-containing heterocycles. Methods for synthesizing N-oxides are well known to those skilled in the art, including oxidation of heterocycles and tertiary amines with peroxy acids such as peroxyacetic acid and m-chloroperbenzoic acid, hydrogen peroxide, alkyl hydroperoxides such as tert-butyl hydroperoxide, sodium perborate, and dioxiranes such as dimethyldioxirane.

In the chemical structures of the compounds described in the present disclosure, the bond " 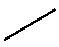 " indicates an unspecified configuration. That is, if chiral isomers exist in the chemical structure, the bond " 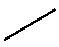 " can be " 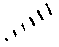 " or " 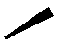 ", or include both " 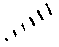 " and " 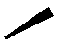 " configurations. For all carbon-carbon double bonds, both Z and E configurations are included, even if only one configuration is named.

When the bond of a substituent can cross-connect to a ring, it indicates that the substituent can bond to any atom on that ring. For example, the structural moiety 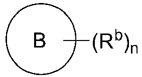 indicates that the substituent R^{b} can substitute at any position on ring B (including NH), and the number of R^{b} is n; when the ring contains NH, it means that NH, like other ring atoms, can be substituted with R^{b}, i.e., R^{b} may or may not substitute H. For example, the structural moiety 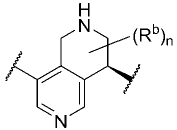 includes both cases where R^{b} substitutes the H on N and where it does not substitute the H on N.

When any variable (e.g., R^{b}) appears more than once in the composition or structure of a compound, its definition in each case is independent. For example, the structural moiety 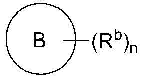 indicates that ring B is substituted with n R^{b} groups, and each R^{b} has independent options.

When a listed substituent does not specify through which atom it is connected to a given group or a given structural formula, then the substituent can be connected via any of its bondable atoms. For example, pyrimidine as a substituent means that any carbon atom or nitrogen atom on the pyrimidine ring is attached to the substituted group. Another example is that in the present disclosure, ring B is selected from 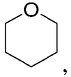 the structural moiety does not specify the connection site, meaning that any carbon atom on the tetrahydropyran ring can be connected to the given group or given structural formula.

When the term "fused with" appears between the names of two chemical structures, it means the two chemical structures are connected in a fused manner, but does not specify the direction of connection to the given group or given structural formula. For example, as recorded in the present disclosure: in the structural moiety 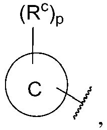 "Ring C is selected from 5- to 8-membered monocyclic heterocyclyl fused with phenyl" means that ring C is selected from a fused structure of 5- to 8-membered monocyclic heterocyclyl and phenyl, but the structural fragment directly connected to 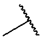 may be either the 5- to 8-membered monocyclic heterocyclyl or phenyl.

When 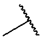 appears in a structure, it indicates that the atom connected to this 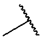 is the bonding atom, for example 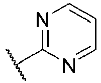 indicates that the carbon atom on the pyrimidine ring is the bonding atom.

When 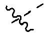 appears in a structure, it indicates that the atom connected to this 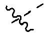 is the bonding atom, and 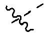 may be a single bond or a double bond; For example 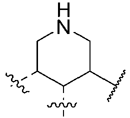 contains two bonding atoms, 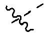 and the bond between the two bonding atoms may be either a single bond or a double bond, depending on the specific structure fused with

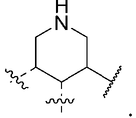

In a substituent structure or any structural moiety mentioned in the present disclosure, a dash "-" indicates the attachment point for the substituent. For example, -CH₃ is connected via the C atom. When two dashes "-" appear in a substituent structure, such as the L group in the present disclosure, unless otherwise specified, the dash "-" on the right end of the substituent connects to the right side in the corresponding formula. For example, if L is selected from -NHC(O)-, the dash "-" on its right end indicates the position connected to R, and similarly for the left side.

It is specifically noted that combinations of all substituents and/or their variants herein are permitted only if such combinations yield stable compounds.

"Substituted" or "substituted by" means that one or more hydrogen atoms, preferably 1-6, more preferably 1-3 hydrogen atoms, of a group are independently replaced by a corresponding number of substituents. Those skilled in the art can determine possible or impossible substitutions without undue effort (through experiment or theory). For example, an amino or hydroxyl group with a free hydrogen bound to a carbon atom with an unsaturated (e.g., olefinic) bond may be unstable.

"Pharmaceutical composition" means a mixture containing one or more of the compounds described herein, or their physiologically/pharmaceutically acceptable salts or prodrugs, with other chemical components, such as physiological/pharmaceutically acceptable carriers and excipients. The purpose of the pharmaceutical composition is to facilitate administration to an organism, aiding the absorption of the active ingredient to exert biological activity.

The term "pharmaceutically acceptable salt" refers to a derivative of a compound of the present disclosure prepared with a relatively non-toxic acid or base. These salts can be prepared during the synthesis, isolation, or purification of the compound, or separately by reacting the purified compound in its free form with a suitable acid or base. When the compound contains a relatively acidic functional group, it reacts with alkali metal, alkaline earth metal hydroxides, or organic amines to form basic addition salts, including cations based on alkali metals and alkaline earth metals, as well as non-toxic ammonium, quaternary ammonium, and amine cations, and further includes salts of amino acids, etc. When the compound contains a relatively basic functional group, it reacts with organic or inorganic acids to form acid addition salts.

The term "pharmaceutically acceptable" refers to compounds, materials, compositions, and/or dosage forms that, within the scope of sound medical judgment, are suitable for contact with human and animal tissues without excessive toxicity, irritation, allergic reactions, or other problems or complications, commensurate with a reasonable benefit/risk ratio.

The term "pharmaceutically acceptable carrier" refers to a medium generally accepted in the art for delivering a biologically active agent to an animal, particularly a mammal. Depending on the route of administration and the nature of the dosage form, it includes, for example, adjuvants, excipients, or vehicles, such as diluents, preservatives, fillers, flow regulators, disintegrating agents, wetting agents, emulsifiers, suspending agents, sweeteners, flavoring agents, aromatic agents, antibacterial agents, antifungal agents, lubricants, and dispersants. Pharmaceutically acceptable carriers are formulated within the purview of one of ordinary skill in the art based on numerous factors. These include, but are not limited to: the type and nature of the formulated active agent, the subject to be administered the composition containing the agent, the intended route of administration for the composition, and the target therapeutic indication. Pharmaceutically acceptable carriers include both aqueous and non-aqueous media, as well as various solid and semi-solid dosage forms. In addition to the active agent, such carriers include many different ingredients and additives. The inclusion of such additional ingredients in the formulation for various reasons (e.g., stabilizing the active agent, binders, etc.) is well known to one of ordinary skill in the art.

The term "therapeutically effective amount" refers to a sufficient amount of a compound of the present disclosure, the pharmaceutically acceptable salt thereof, the stereoisomer thereof, the isotopic derivative thereof, etc., to treat a disorder at a reasonable benefit/risk ratio applicable to any medical treatment and/or prevention. However, it should be recognized that the total daily dosage of all formulas recorded in the present disclosure, including but not limited to formulas (IA), (I), (II), etc., and the compounds shown in Table 1, the stereoisomer thereof, the isotopic derivative thereof, the prodrug thereof, the N-oxide thereof, the solvate thereof, or the pharmaceutically acceptable salt thereof and compositions, must be determined by the attending physician within the scope of sound medical judgment. For any specific patient, the specific therapeutically effective dosage level will depend on various factors, including the disorder being treated and its severity; the activity of the specific compound employed; the specific composition employed; the patient's age, body weight, general health, sex, and diet; the time of administration, route of administration, and excretion rate of the specific compound employed; the duration of treatment; drugs used in combination or concurrently with the specific compound employed; and similar factors well known in the medical arts. For a drug or pharmacologically active agent, the term "therapeutically effective amount" means a sufficient amount that is non-toxic but achieves the desired effect. The effective amount varies from person to person, depending on the age and general condition of the recipient, as well as the specific active substance. A suitable effective amount in individual cases can be determined by those skilled in the art through routine experimentation.

As used herein, the singular forms "a", "an", and "the" include plural references, and vice versa, unless the context clearly indicates otherwise.

When the term "about" is applied to a parameter such as pH, concentration, temperature, etc., it indicates that the parameter may vary by ±10%, and sometimes more preferably within ±5%. As will be understood by those skilled in the art, when the parameter is not critical, numbers are often given for illustrative purposes only and not as limitations.

### TECHNICAL EFFECTS

The compounds of the present disclosure exhibit significant inhibitory activity against CYP11B2, with markedly superior *in vitro* activity, selectivity, and pharmacokinetic properties compared to the Control Compound (the Control Compound is Compound 3-1 on page 95 of patent WO2013041591A1). The structure of the Control Compound is as follows:

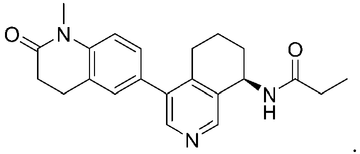

Additionally, the compounds of the present disclosure demonstrated significant inhibition of aldosterone in monkeys at multiple doses (such as 0.03 mpk, 0.1 mpk, and 0.3 mpk) without causing notable changes in other hormones like cortisol (e.g., 11-deoxycorticosterone, 11-deoxycortisol, 17-hydroxyprogesterone, corticosterone, etc.).

### BRIEF DESCRIPTION OF THE DRAWINGS

The drawings provided here are intended to further aid in the understanding of the present disclosure and constitute a part of the present disclosure. The illustrative embodiments and their descriptions of the present disclosure are for explaining the present disclosure and do not constitute an undue limitation of the present disclosure.
Fig. 1 is a pharmacodynamic result graph of Example Compound C44 in monkeys;
Fig. 2 is a pharmacodynamic result graph of Example Compound A09 in monkeys;
Fig. 3 is a pharmacodynamic result graph of Example Compound A50 in monkeys;
Fig. 4 is a pharmacodynamic result graph of Example Compound C43 in monkeys;
Fig. 5 is a pharmacodynamic result graph of Example Compound A02-P2 in monkeys.

### DETAILED DESCRIPTION

The present disclosure is described in detail below through examples, but this does not imply any adverse limitation to the present disclosure. The present disclosure has been described in detail herein, and specific embodiments are further disclosed. For those skilled in the art, various changes and modifications to the specific embodiments of the present disclosure will be apparent without departing from the spirit and scope of the application.

In the examples of the present disclosure, compound names were generated from the compound structures using Chemdraw. If there is any inconsistency between the compound name and the structure, comprehensive information and reaction pathways can be used to assist in determination. The preparation methods for some compounds in the present disclosure refer to the preparation methods of similar compounds mentioned above. Those skilled in the art should be aware that when using or referring to these preparation methods, the feed ratio of reactants, reaction solvent, reaction temperature, etc., can be appropriately adjusted according to the different reactants. The compounds of the present disclosure can be prepared by various synthesis methods well-known to those skilled in the art, including the specific embodiments listed below, embodiments formed by combining them with other chemical synthesis methods, and equivalent alternatives well-known to those skilled in the art. Preferred embodiments include, but are not limited to, the examples of the present disclosure.

### 1. Summary of Experimental Instruments

The structures of the compounds in the present disclosure were determined by nuclear magnetic resonance (NMR) and/or liquid chromatography-mass spectrometry (LC-MS), or ultra-performance liquid chromatography-mass spectrometry (UPLC-MS).

NMR was measured using a Bruker AVANCE III HD 400MHz or Bruker AVANCE NEO 400MHz nuclear magnetic resonance spectrometer. The determination solvents used were deuterated dimethyl sulfoxide (DMSO-*d*₆), deuterated methanol (CD₃OD), deuterated chloroform (CDCl₃), and heavy water (D₂O), with tetramethylsilane (TMS) as the internal standard. NMR chemical shifts (δ) are given in parts per million (ppm).

Liquid chromatography-mass spectrometry LC-MS measurements were performed using Shimadzu LC-20AD & MS 2020, Shimadzu LC-20AD XR & MS 2020, or Agilent 1260 & MS 6125B, and the mass spectrometer (ion source was electrospray ionization).

HPLC measurements were performed using Agilent 1200, Agilent 1100, Shimadzu LC-20AD, or Shimadzu LC-20AD high-performance liquid chromatography.

Preparative HPLC used Gilson (GX281), Waters (2767), or Waters (2767/QDA).

Chiral HPLC measurements used waters Arc with 2998 with QDA.

Supercritical fluid chromatography (SFC) used waters SFC150 MGM.

The starting materials and intermediates directly used in the present disclosure can be synthesized according to methods known in the art or purchased commercially. For experimental methods not specifying specific conditions in the examples, they were generally carried out under conventional conditions or according to the conditions recommended by the raw material or commodity manufacturer. Reagents not specifying specific sources are conventional reagents purchased commercially.

Reaction progress in the examples can be monitored using conventional methods such as thin-layer chromatography (TLC), LC-MS, etc. The eluant systems for column chromatography (which can be conventional column chromatography or flash column chromatography) and the developing solvent systems for thin-layer chromatography can consist of one or more of the following solvents: dichloromethane, methanol, n-hexane, ethyl acetate, petroleum ether, ethyl acetate, acetone, dichloromethane, etc. The volume ratio of solvents is adjusted according to the polarity of the compounds, and small amounts of basic or acidic reagents such as triethylamine, acetic acid, trifluoroacetic acid, etc., may further be added.

Percentages (%) mentioned in the present disclosure, unless otherwise specified, refer to mass percentage for solid-liquid mixtures and solid-solid mixtures, and refer to volume percentage for liquid-liquid mixtures.

### 2. Synthesis Examples

### Reference Example 1 (Control Compound):

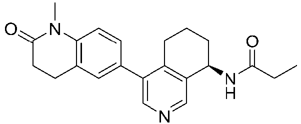

The preparation method is referred to patent WO2013041591A1.

The synthetic routes for some compounds of the present disclosure are described as follows:

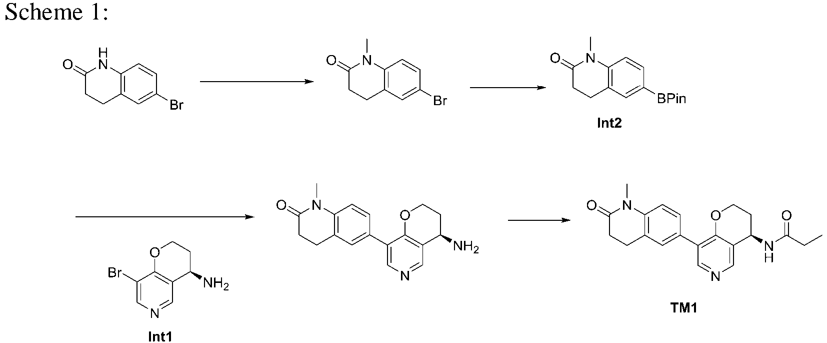

### Synthesis scheme for Int1:

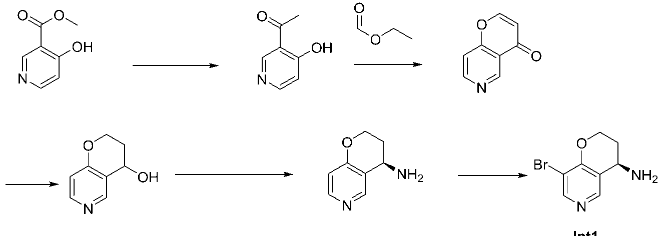

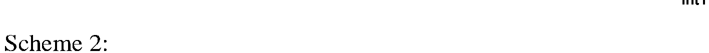

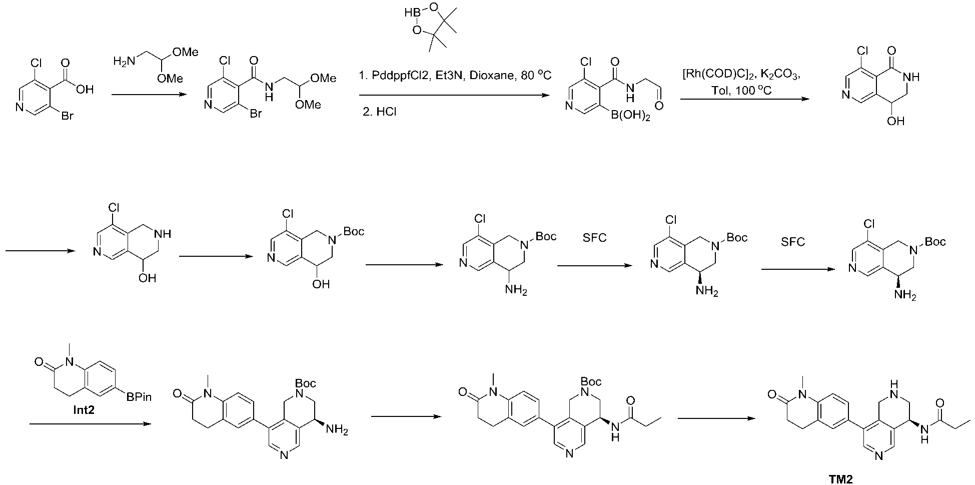

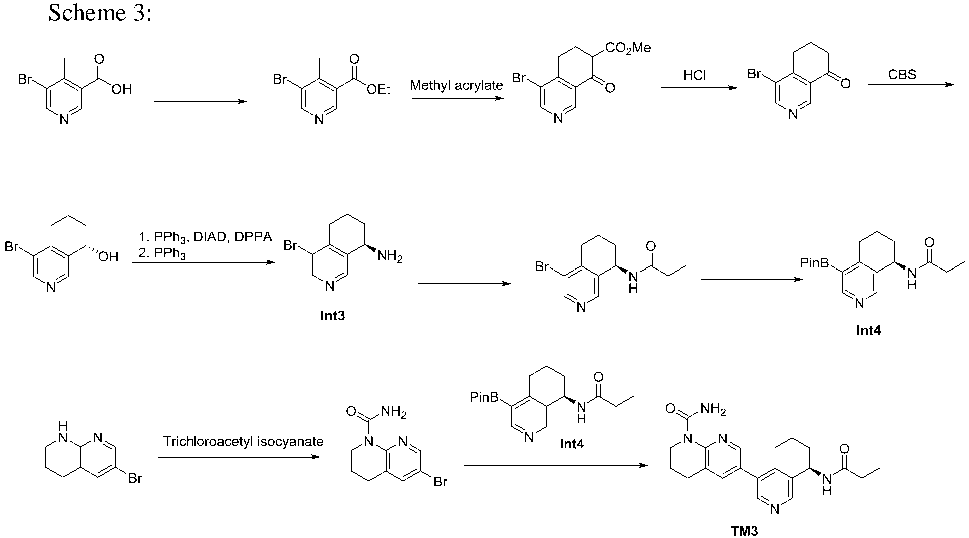

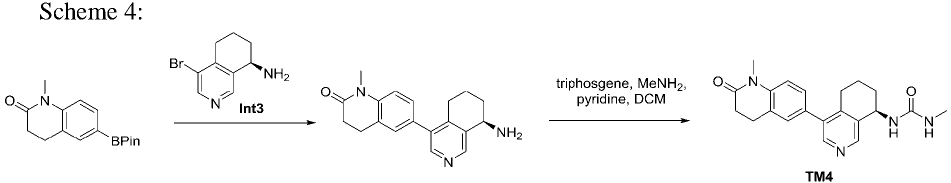

The synthesis of other compounds in Table 1 of the present disclosure, excluding TM1 to TM4, refers to any one of Scheme 1 to Scheme 4 above. The structures of the target compounds obtained according to the above synthetic routes were determined by nuclear magnetic resonance (NMR) and/or mass spectrometry (MS).

The compounds shown in Table 2 below can be synthesized according to the synthetic schemes of Scheme 1 to Scheme 4 above, or according to the synthetic schemes of specific compounds recorded in the present disclosure, or according to methods from existing literature.

**Table 2**

| **Example** | **Structural Formula** | **Structural Characterization** |
|---|---|---|
| A01 | 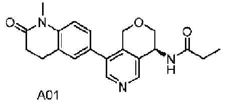 | **¹H NMR** (400 MHz, CD₃OD) δ 8.48 (s, 1H), 8.28 (s, 1H), 7.28 - 7.22 (m, 3H), 5.15 (t,*J* = 3.2 Hz, 1H), 4.70 (s, 2H), 3.99 - 3.91 (m, 2H), 3.40 (s, 3H), 3.01 - 2.97 (m, 2H), 2.69 - 2.65 (m, 2H), 2.26 (q, *J* = 7.6 Hz, 2H), 1.16 (t, *J* = 7.6 Hz, 3H). |
| | | **MS(ESI)M/Z:** 366.2 [M+H]⁺. |
| C01 | 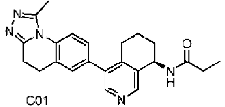 | **¹H NMR** (400 MHz, DMSO-*d*₆) δ 8.36 (s, 1H), 8.29 (d, *J* = 8.4 Hz), 8.25 (s, 1H), 7.75 (d, *J =* 8.3 Hz, 1H), 7.49 (d, *J =* 2.1 Hz, 1H), 7.40 (dd, *J* = 8.3, 2.1 Hz, 1H), 5.11 (q, *J* = 6.5 Hz, 1H), 3.04 (s, 4H), 2.71 (s, 3H), 2.65 - 2.60 (m, 2H), 2.16 (qd, *J* = 7.4, 4.8 Hz, 2H), 1.94 - 1.66 (m, 4H), 1.08 - 1.05 (t, *J* = 7.6 Hz, 3H). |
| | | **MS(ESI)M/Z:** 388.3 [M+H]⁺. |
| C04 | 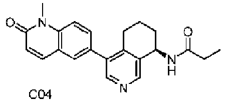 | **¹H NMR** (400 MHz, DMSO-*d₆*) δ 8.36 (s, 1H), 8.30 - 8.28 (m, 2H), 7.96 (d, *J* = 9.5 Hz, 1H), 7.75 (s, 1H), 7.63 (s, 2H), 6.68 (d, *J* = 9.4 Hz, 1H), 5.11 (q, *J* = 6.5 Hz, 1H), 3.67 (s, 3H), 2.68 - 2.59 (m, 2H), 2.16 (qd, J = 7.4. 4.7 Hz, 2H), 1.92 - 1.65 (m, 4H), 1.06 (t, *J* = 7.6 Hz, 3H). |
| | | **MS(ESI)M/Z:** 362.2 [M+H]⁺. |
| C06 | 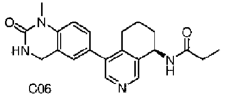 | **¹H NMR** (400 MHz, DMSO-*d₆*) δ 8.32 (s, 1H), 8.28 (d, *J* = 8.5 Hz, 1H), 8.20 (s, 1H), 7.24 (dd, *J* = 8.2, 2.1 Hz, 1H), 7.17 (d, *J* = 2.1 Hz, 1H), 7.13 (s, 1H), 7.02 (d, *J* = 8.3 Hz, 1H), 5.09 (q, *J* = 6.3 Hz, 1H), 4.33 (d, *J* = 2.0 Hz, 2H), 3.20 (s, 3H), 2.64 - 2.57 (m, 2H), 2.16 (qd, *J =* 7.4, 4.6 Hz, 2H), 1.93 - 1.64 (m, 4H), 1.06 (t, *J* - 7.6 Hz, 3H). |
| | | **MS(ESI)M/Z:** 365.2 [M+H]⁺. |
| C07 | 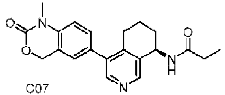 | **¹H NMR** (400 MHz, DMSO-*d₆*) δ 8.33 (s, 1H), 8.27 (d, *J* = 8.4 Hz, 1H), 8.21 (s, 1H), 7.36 (dd, *J* = 8.3, 2.1 Hz, 1H), 7.29 (d, *J* = 2.0 Hz, 1H), 7.19 (d, J= 8.3 Hz, 1H), 5.30 (s, 2H), 5.13 - 5.06 (m, 1H), 3.32 (s, 3H), 2.61 - 2.58 (m, 2H), 2.15 (qd, *J* = 7.5, 4.7 Hz, 2H), 1.93 - 1.64 (m, 4H), 1.05 (t, *J* = *7.6* Hz, 3H). |
| | | **MS(ESI)M/Z:** 366.2 [M+H]⁺. |
| C08 | 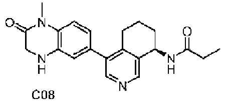 | ¹H NMR (400 MHz, DMSO-*d*₆) δ 8.33 (s, 1H), 8.28 (d, *J* = 8.5 Hz, 1H), 8.24 (s, 1H), 7.57 (d, *J* = 8.2 Hz, 1H), 7.46 (d, *J =* 1.6 Hz, 1H), 7.16 (dd, *J =* 8.2, 1.6 Hz, 1H), 5.11 (q, *J* = 6.5 Hz, 1H), 3.78 (s, 3H), 2.58 (t, *J* = 8.0 Hz, 2H), 2.56 (s, 2H), 2.21 - 2.10 (m, 2H), 1.92 - 1.82 (m, 1H), 1.79 - 1.66 (m, 3H), 1.06 (t, *J* = 7.6 Hz, 3H). |
| | | **MS(ESI)M/Z:** 349.2 [M-Me]⁺. |
| C09 | 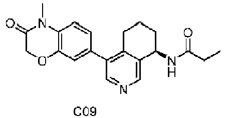 | ¹H NMR (400 MHz, DMSO-*d*₆) δ 8.33 (s, 1H), 8.26 (d, *J* = 8.4 Hz, 1H), 8.21 (s, 1H), 7.25 (d, *J* - 8.3 Hz, 1H), 7.05 (dd, *J* - 8.2, 2.0 Hz, 1H), 7.01 (d, *J* - 2.0 Hz, 1H), 5.09 (q, *J* = 6.5 Hz, IH), 4.71 (s, 2H), 2.61 (t, *J* = 5.8 Hz, 2H), 2.20 - 2.08 (m, 2H), 1.93 - 1.81 (m, 1H), 1.81 - 1.64 (m, 3H), 1.05 (t, *J* = 7.6 Hz, 3H). |
| | | **MS(ESI)M/Z:** 366.1 [M+H]⁺. |
| C12 | 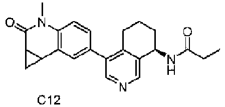 | **¹H NMR** (400 MHz, DMSO-*d₆*) δ 8.33 (s, 1H), 8.27 (d, *J* = 8.4 Hz, 1H), 8.22 (s, 1H), 7.44 (t, *J* = 2.4 Hz, 1H), 7.21 (dt, *J =* 8.4, 2.1 Hz, 1H), 7.13 (d, *J* = 8.5 Hz, 1H), 5.12 - 5.07 (m, 1H), 3.26 (s, 3H), 2.71 - 2.58 (m, 3H), 2.25 (ddd, *J* = 9.3, 7.5, 4.6 Hz, 1H), 2.15 (qd, *J* = 7.4, 4.7 Hz, 2H), 1.88 - 1.86(m, 1H), 1.82 - 1.65 (m, 3H), 1.64 - 1.56 (m, 1H), 1.06 (t, *J* = 7.6 Hz, 3H), 0.61 (q, *J* = 4.4 Hz, 1H). |
| | | **MS(ESI)M/Z:** 376.2 [M+H]⁺. |
| C13 | 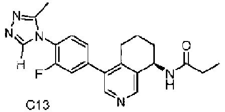 | **¹H NMR** (400 MHz, DMSO-*d₆*) δ 8.74 (s, 1H), 8.40 (s, 1H), 8.32 - 8.28 (m, 2H), 7.76 (t, J = 8.1 Hz, 1H), 7.64 (dd, *J* = 10.9, 1.9 Hz, 1H), 7.42 (dd, *J* = 8.3, 2.0 Hz, 1H), 5.14 - 5.07 (m, 1H), 2.68 - 2.62 (m, 2H), 2.33 (s, 3H), 2.22 - 2.11 (m, 2H), 1.92 - 1.68 (m, 4H), 1.06 (t, *J* = 7.6 Hz, 3H). |
| | | **MS(ESI)M/Z:** 380.2 [M+H]⁺. |
| C15 | 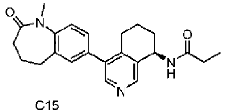 | ¹H NMR (400 MHz, DMSO-*d*₆) δ 8.34 (s, 1H), 8.27 (d, *J* = 8.4 Hz, 1H), 8.24 (s, 1H), 7.41 (d, *J* = 8.2 Hz, 1H), 7.31 (dd, *J* = 8.1, 2.1 Hz, 1H), 7.27 (d, *J* = 2.1 Hz, 1H), 5.10 (q, *J* = 6.5 Hz, 1H), 3.27 (s, 3H), 2.71 (t, J= 7.0 Hz, 2H), 2.65 - 2.58 (m, 2H), 2.22 - 2.05 (m, 6H), 1.93 - 1.83 (m, 1H), 1.81 - 1.64 (m, 3H), 1.06 (t, *J* = 7.6 Hz, 3H). |
| | | **MS(ESI)M/Z:** 378.2 [M+H]⁺. |
| C16 | 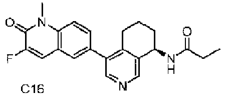 | **¹HNMR** (400 MHz, CD₃OD) δ 8.61 (s, 1H), 8.52 (brs, 1H), 7.80 - 7.75 (m, **3H),** 7.70 - 7.67 (m, 1H), 5.28 - 5.25 (m, 1H), 3.86 (s, 3H), 2.90 - 2.84 (m, 2H), 2.32 (q, *J* = 7.6, 2H), 2.13 - 2.09 (m, 1H), 1.98 - 1.86 (m, 3H), 1.20 (t, *J* = 10.0, 3H) |
| | | **MS (ESI)M/Z :** 380.2 [M+H]⁺. |
| C18 | 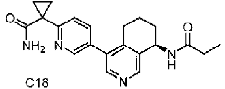 | **¹H NMR** (400 MHz, CD₃OD) 8.49 (d, *J* = 1.6 Hz, 1H), 8.41 (s, 1H), 8.24 (s, 1H), 7.82 (dd, *J* = 8.0, 2.4 Hz, 1H), 7.56 (d, *J* = 8.0 Hz, 1H), 5.22 (t, *J* = 5.2 Hz, 1H), 2.76 - 2.64 (m, 2H), 2.31 - 2.25 (m, 2H), 2.08 - 1.74 (m, 4H), 1.63 - 1.60 (m, 2H), 1.36 - 1.30 (m, 2H), 1.19 (t, *J* = 7.6 Hz, 3H). |
| | | **MS(ESI)M/Z:** 365.2 [M+H]⁺. |
| C19 | 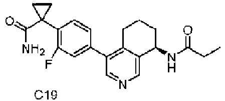 | **¹HNMR** (400 MHz, DMSO-*d₆*) δ 8.35 (s, 1H), 8.28 (d, *J* = 8.4 Hz, 1H), 8.22 (s, 1H), 7.44 (t, *J* = 8.0 Hz, 1H), 7.20 (dd, *J* = 10.8, 9.2 Hz, 1H), 7.14 (dd, *J* = 8.0, 2.0 Hz, 1H), 7.04 (s, 1H), 6.50 (s, 1H), 5.12 - 5.07 (m, 1H), 2.67 - 2.65 (m, 2H), 2.19 - 2.12 (m, 2H), 1.91 - 1.87 (m, 1H), 1.78 - 1.69 (m, 3H), 1.44 - 1.41 (m, 2H), 1.05 (t, *J* = 7.6 Hz, 3H), 1.03 - 1.01 (m, 2H). |
| | | **MS (ESI)M/Z:** 382.1 [M+H]⁺. |
| C20 | 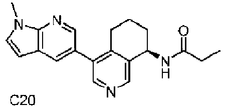 | ¹H NMR (400 MHz, DMSO-*d*₆) δ 8.36 (s, 1H), 8.29 (d, *J =* 9.0 Hz, 1H), 8.27 (s, 1H), 8.21 (d, *J* = 2.1 Hz, 1H), 7.96 (d, *J* = 2.1 Hz, 1H), 7.60 (d, *J* = 3.4 Hz, 1H), 6.51 (d, *J* = 3.4 Hz, 1H), 5.12 (q, *J* = 6.5 Hz, 1H), 3.87 (s, 3H), 2.60 (t, *J =* 6.0 Hz, 2H), 2.25 - 2.06 (m, 2H), 1.94 - 1.84 (m, 1H), 1.83 - 1.63 (m, 3H), 1.06 (t, *J* = 7.6 Hz, 3H). |
| | | **MS(ESI)M/Z:** 335.1 [M+H]⁺. |
| C21 | 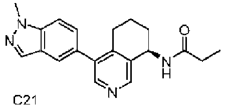 | **¹H NMR** (400 MHz, DMSO-*d*₆) δ 8.34 (s, 1H), 8.29 (d, *J* = 8.4 Hz, 1H), 8.25 (s, 1H), 8.08 (d, *J* = 0.9 Hz, 1H), 7.75 - 7.70 (m, 2H), 7.37 (dd, *J* = 8.5, 1.7 Hz, 1H), 5.14 - 5.06 (m, 1H), 4.09 (s, 3H), 2.59 (t, *J* = 6.0 Hz, 2H), 2.16 (qd, *J* = 7.4, 5.1 Hz, 2H), 1.93 - 1.62 (m, 4H), 1.06 (t, *J* = 7.6 Hz, 3H). |
| | | **MS(ESI)M/Z:** 335.2 [M+H]⁺. |
| C22 | 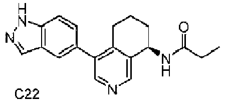 | **¹H NMR** (400 MHz, DMSO-*d₆)* δ 13.19 (s, 1H), 8.34 (s, 1H), 8.29 (d, *J* = 8.4 Hz, 1H), 8.26 (s, 1H), 8.12 (s, 1H), 7.72 (s, 1H), 7.62 (d, *J* = 8.4 Hz, 1H), 7.32 (dd, *J =* 8.5, 1.6Hz, 1H), 5.11 (q, *J* = 6.3 Hz, 1H), 2.59 (t, *J* = 6.1 Hz, 2H), 2.16 (qd, *J* = 7.4, 5.1 Hz, 2H), 1.93 - 1.63 (m, 4H), 1.06 (t, *J* = 7.6 Hz, 3H). |
| | | **MS(ESI)M/Z:** 321.2 [M+H]⁺. |
| C23 | 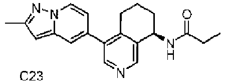 | ¹H NMR (400 MHz, DMSO-*d*₆) δ 8.59 (s, 1H), 8.39 (s, 1H), 8.34 (s, 1H), 8.29 (d, *J =* 8.4 Hz, 1H), 7.64 (d, *J* = 9.1 Hz, 1H), 7.17 (dd, *J* = 9.0, 1.6 Hz, 1H), 6.45 (s, 1H), 5.11 (q, *J* = 6.5 Hz, 1H), 2.71 - 2.64 (m. 2H), 2.41 (s, 3H), 2.26 - 2.07 (m, 2H), 1.95 - 1.84 (m, 1H), 1.85 - 1.66 (m, 3H), 1.06 (t, *J* = 7.6 Hz, 3H). |
| | | **MS(ESI)M/Z:** 335.2 [M+H]⁺. |
| C24 | 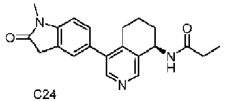 | ¹H NMR (400 MHz, DMSO-*d₆*) δ 8.34 (s, 1H), 8.28 (d, *J* = 8.4 Hz, 1H), 8.22 (s, 1H), 7.28 - 7.23 (m, 2H), 7.07 (d, *J* = 7.9 Hz, 1H), 5.21 - 5.03 (m, 1 H), 3.61 (s, 2H), 2.67 - 2.57 (m, 2H), 2.21 - 2.10 (m, 2H), 1.95 - 1.82 (m, 1H), 1.80 - 1.64 (m, 3H), 1.05 (t, *J* = 7.6 Hz, 3H). |
| | | **MS(ESI)M/Z:** 350.1 [M+H]⁺. |
| C25 | 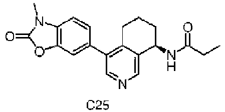 | ¹H NMR (400 MHz, DMSO- *d*₆) δ 8.34 (s, 1H), 8.27 (d, *J* = *8.4* Hz, 1H), 8.22 (s, 1H), 7.39 (d, J = 1.6 Hz, 1H), 7.35 (d, *J =* 8.0 Hz, 1H), 7.20 (dd, *J =* 8.0, 1.6 Hz, 1H), 5.14 - 5.05 (m, 1H), 3.39 (s, 3H), 2.60 (t, *J* = 6.0 Hz, 2H), 2.15 (qd, *J* = 7.4, 4.6 Hz, 2H), 1.93 - 1.64 (m, 4H), 1.05 (t, *J* = 7.6 Hz, 3H). |
| | | **MS(ESI)M/Z:** 352.2 [M+H]⁺. |
| D01 | 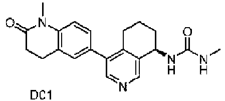 | **¹H NMR** (400 MHz, DMSO-*d₆*) δ 8.40 (s, 1H), 8.18 (s, 1H), 7.26 - 7.23 (m, 2H), 7.17 (d, *J* = 8.0 Hz, 1H), 6.44 (d, *J* = 8.8 Hz, 1H), 5.71 - 5.68 (m, 1H), 4.93 - 4.88 (m, 1H), 3.29 (s, 3H), 2.92 (t, *J* = 7.2 Hz, 1H), 2.65 - 2.55 (m, 7H), 1.90 - 1.84 (m, 1H), 1.74 - 1.69 (m, 3H). |
| | | **MS(ESI)M/Z:** 365.1 [M+H]⁺. |
| D03 | 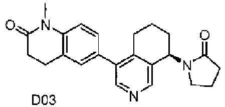 | **¹HNMR** (400 MHz, CD₃OD) δ 8.20 (s, 1H), 8.16 (s, 1H), 7.29 - 7.22 (m, 3H), 5.42 (m, 1H), 3.51 - 3.44 (m, 1H), 3.42 (s, 3H), 3.19 - 3.14 (m, 1H), 2.98 (t, *J* = 6.8, 2H), 2.83 - 2.75 (m, 1H), 2.69 -2.64 (m, 3H), 2.55 - 2.50 (m, 2H), 2.13 - 1.75 (m, 6H). |
| | | **MS (ESI)M/Z :** 376.2 [M+H]⁺. |
| D05 | 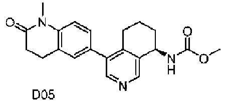 | ¹H NMR (400 MHz, CDCl₃) δ 8.57 (s, 1H), 8.29 (s, 1H), 7.19 - 7.16 (m, 1H), 7.09 (s, 1H), 705 (d, *J* = 8.4 Hz, 1H), 5.03 - 4.97 (m, 1H), 3.74 (s, 3H), 3.40 (s, 3H), 2.95 (t, J = 6.8 Hz, 2H), 2.71 (t, *J* = 8 Hz, 2H), 2.67 - 2.55 (m, 2H), 2.09 - 2.05 (m, 1H), 1.93 - 1.84 (m, 1H), 1.82 - 1.76 (m, 2H). |
| | | **MS(ESI)M/Z:** 366.2 [M-H]⁺. |
| A04-P1 | 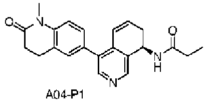 | **¹H NMR** (400 MHz, DMSO-*d*₆) δ 8.38 (s, 1 H), 8.34 (s, 1 H), 8.27 (d, J=8.0 Hz, 1 H), 7.27-7.20 (m, 3 H), 6.53 (d, J=10.0 Hz, 1 H), 6.34-6.30 (m, 1 H), 5.14-5.08 (m, 1 H), 3.30 (s, 3 H), 2.94 (t, J=7.2 Hz, 2 H), 2.60 (t, J=7.6 Hz, 2 H), 2.20-2.13 (m, 2 H), 1.28-1.22 (m, 2 H), 1.04 (t, J=7.6 Hz, 3 H). |
| | | **MS(ESI)M/Z:** 362.2 [M+H]⁺. |
| A04-P2 | 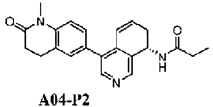 | **¹H NMR** (400 MHz, DMSO-*d₆*) δ 8.38 (s, 1 H), 8.34 (s, 1 H), 8.27 (d, J=8.0 Hz, 1 H), 7.27-7.20 (m, 3 H), 6.53 (d, J=10.0 Hz, 1 H), 6.34-6.30 (m, 1 H), 5.14-5.08 (m, 1 H), 3.30 (s, 3 H), 2.93 (t, J=7.2 Hz, 2 H), 2.60 (t, J=7.6 Hz, 2 H), 2.20-2.13 (m, 2 H), 1.28-1.23 (m, 2 H), 1.04 (t, J=7.6 Hz, 3 H). |
| | | **MS(ESI)M/Z:** 362.2 [M+H⁻]. |
| A06-P1 | 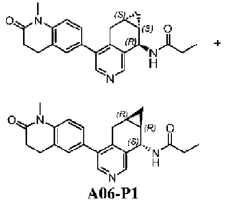 | **¹HNMR** (400 MHz, CDCl₃) δ 8.56 (s, 1 H), 8.40 (s, 1 H), 7.20 (d, J=8.4 Hz, 1 H), 7.12 (s, 1 H), 7.07 (d, J=8.4 Hz, 1 H), 6.08 (s, 1 H), 5.51 (d, J =5.6 Hz, 1 H), 3.41 (s, 3 H), 3.20-3.07 (m, 2 H), 2.99-2.95 (m, 2 H), 2.72 (t, J=8.0 Hz, 2 H), 2.28-2.20 (m, 2 H), 1.37 (s, 1 H), 1.25 (s, 1 H), 1.16 (t, J=7.6 Hz, 3 H), 0.57-0.54 (m, 1 H), 0.24-0.08 (m, 1 H). |
| | | **MS (ESI)M/Z** : 376.2 [M+H]⁺. |
| A06-P2 | 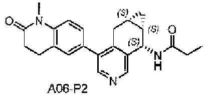 | **¹HNMR** (400 MHz, CDCl₃) δ 8.40 (s, 1 H), 8.36 (s, 1 H), 7.17 (d, J=8.0 Hz, 1 H), 7.08 (s, 1 H), 7.06 (d, J=8.4 Hz, 1 H), 6.05 (d, J=8.0 Hz, 1 H), 5.67 (s, 1 H), 3.41 (s, 3 H), 3.12-3.07 (m, 1 H), 3.01-3.00 (m, 1 H), 2.97-2.95 (m, 2 H), 2.71 (t, J=7.6 Hz, 2 H), 2.44 (q, J=7.2 Hz, 2 H), 1.43-1.40 (m, 1 H), 1.31 (t, J=7.6 Hz, 3 H), 1.26 (s, 1 H), 0.47-0.42 (m, 1 H), 0.16-0.12 (m, 1 H). |
| | | **MS (ESI)M/Z** : 376.2 [M+H]⁺. |
| A06-P3 | 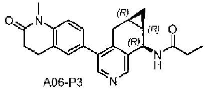 | **¹HNMR** (400 MHz, CDCl₃) δ 8.43 (s, 1 H), 8.37 (s, 1 H), 7.17 (d, J=8.0 Hz, 1 H), 7.09 (s, 1 H), 7.07 (d, J=8.8 Hz,1 H), 6.20 (d, J =8.4 Hz, 1 H), 5.70 (s, 1 H), 3.41 (s, 3 II), 3.16-3.12 (m, 1 11), 3.04-3.03 (m, 1 II), 2.99-2.95 (m, 2 II), 2.72 (t, J=7.6 Hz, 2 H), 2.45 (q, J=6.8 Hz, 2 H), 1.44-1.41 (m, 1 H), 1.31 (t, J=7.6 Hz, 3 H), 1.26 (s, 1 H), 0.50-0.44 (m, 1 H), 0.17-0.13 (m, 1 H). |
| | | **MS (ESI)M/Z :** 376.2 [M+H]⁺. |
| A11 | 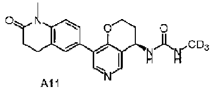 | **¹HNMR** (400 MHz, DMSO-*d₆)* δ 8.27 (s, 1 H), 8.24 (s, 1 H), 7.40 (dd, J=8.4 Hz, 1.6 Hz, 1 H), 7.36 (s, 1 H), 7.15 (d, J=8.4 Hz, 1 H), 6.59 (d, J=7.6 Hz, 1 H), 5.72 (s, 1 H), 4.95-4.87 (m, 1 H), 4.38-4.30 (m, 1 H), 4.28-4.19 (m, 1 H), 3.28 (s, 3 H), 2.91 (t, J=7.2 Hz, 2 H), 2.57 (t, J=7.6 Hz, 2 H), 2.11-2.01 (m, 1 H), 2.00-1.89 (m, 1 H). |
| | | **MS (ESI)M/Z** : 370.1 [M+H]⁺. |
| A12 | 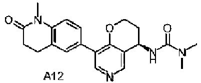 | **¹HNMR** (400 MHz, CDCl₃) δ 8.43 (s, 1 H), 8.31 (s, 1 H), 7.40 (dd, J=8.4 Hz, 2.0 Hz, 1 H), 7.31 (d, J=1.6 Hz, 1 H), 7.05 (d, J=8.4 Hz, 1 H), 5.20-5.16 (m, 1 H), 4.82 (d, J=7.2 Hz, 1 H), 4.44-4.32 (m, 2 H), 3.39 (s, 3 H), 2.96 (s, 6 H), 2.96-2.94 (m, 2 H), 2.71-2.68 (m, 2 H), 2.29-2.18 (m, 2 H). |
| | | **MS (ESI)M/Z :** 381.1 [M+H]⁺. |
| A14 | 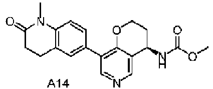 | **¹HNMR** (400 MHz, DMSO-*d₆)* δ 8.26 (s, 1 H), 8.25 (s, 1 H), 7.80 (d, J=8.0 Hz, 1 H), 7.39 (d, J=8.4 Hz, 1 H), 7.36 (s, 1 H), 7.15 (d, J=8.4 Hz, 1 H), 4.90-4.81 (m, 1 H), 4.38-4.23 (m, 2 H), 3.61 (s, 3 H), 3.28 (s, 3 H), 2.91 (t, J=6.8 Hz, 2 H), 2.57 (t, J=8.0 Hz. 2 H), 2.15-2.04 (m, 1 H), 2.02-1.91 (m, 1 H). |
| | | **MS (ESI)M/Z** : 368.1 [M+H]⁺ |
| C31 | 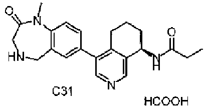 | **¹HNMR** (400 MHz, DMSO-d₆) δ 8.34 (s, 1 H), 8.28 (d, J=8.4 Hz, 1 H), 8.23 (s, 1 H), 8.19 (s, 1 H), 7.44 (d, J=8.0 Hz, 1 H), 7.38 (d, J=8.0 Hz, 1 H), 7.34 (s, 1 H), 5.12-5.09 (m, 1 H), 3.78 (s, 2 H), 3.31 (s, 3 H), 3.11 (s, 2 H), 2.68-2.57 (m, 2 H), 2.22-2.08 (m, 2 H), 1.94-1.88 (m, 1 H), 1.84-1.61 (m, 3 H), 1.06 (t, J=7.6 Hz, 3 H). |
| | | **MS (ESI)M/Z** : 379.1 [M+H]⁺ |
| C32 | 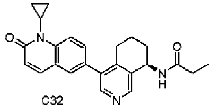 | **¹H NMR** (400 MHz, DMSO-*d₆)* δ 8.33 (s, 1H), 8.31 - 8.25 (m, 2 H), 7.93 (d, J = 8.7 Hz, 1 H), 7.89 (d, J = 9.6 Hz, 1 H), 7.70 (d, J = 2.1 Hz, 1 H), 7.62 (dd, J = 8.7, 2.1 Hz, 1 H), 6.57 (d, J = 9.5 Hz, 1 H), 5.14 - 5.06 (m, 1 H), 2.99 (tt, J = 7.1, 4.0 Hz, 1 H), 2.66 - 2.58 (m, 2 H), 2.16 (qd, J = 7.4, 4.8 Hz, 2 H), 1.95 - 1.85 (m, 1 H), 1.82 - 1.65 (m, 3H), 1.34 - 1.26 (m, 2H), 1.06 (t, J = 7.6 Hz, 3 H), 0.84 - 0.73 (m, 2 H). |
| | | **MS (ESI)M/Z** : 388.2 [M+H]⁺ |
| C33 | 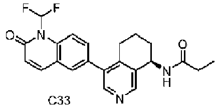 | **¹H NMR** (400 MHz, DMSO-*d₆*) δ 8.46 - 8.11 (m, 4 H), 8.08 (d, J = 9.7 Hz, 1 H), 7.88 - 7.78 (m, 2 H), 7.68 (dd, J = 8.8, 2.2 Hz, 1 H), 6.70 (d, J = 9.7 Hz, 1 H), 5.15 - 5.06 (m, 1 H), 2.71 - 2.58 (m, 2 H), 2.16 (qd, J = 7.4, 4.8 Hz, 2 H), 1.95 - 1.85 (m, 1 H), 1.85 - 1.65 (m, 3 H), 1.06 (t, J = 7.6 Hz, 3 H). |
| | | **MS (ESI)M/Z** : 398.2 [M+H]⁺. |
| C37 | 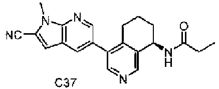 | **¹HNMR** (400 MHz, DMSO-*d₆*) δ 8.53 (d, J=2.0 Hz, 1 H), 8.39 (s, 1 H), 8.30 (s, 1 H), 8.29 (d, *J*=6.4 Hz, 1 H), 8.20 (d, *J=2.0* Hz, 1 H), 7.52 (s, 1 H), 5.14-5.09 (m, 1 H), 3.97 (s, 3 H), 2.60 (t, *J=6.0* Hz, 2 H), 2.21-2.11 (m, 2 H), 193-1.85 (m, 1 H), 1.80-1.63 (m, 3 H), 1.06 (t, J=7.6 Hz, 3 H). |
| | | **MS (ESI)M/Z** : 360.2 [M+H]⁺ |
| C38 | 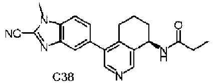 | **¹HNMR** (400 MHz, CD₃OD) δ 8.39 (s, 1 H), 8.23 (s, 1 H), 7.77 (d, *J*=8.8 Hz, 1 H), 7.71 (s, 1 H), 7.49 (dd, *J* = 8.4 Hz, 1.2 Hz, 1 H), 5.29-5.17 (m, 1 H), 4.10 (s, 3 H), 2.75-2.55 (m, 2 H), 2.34-2.22 (m, 2 H), 2.09-1.97 (m, 1 H), 1.91-1.70 (m, 3 H), 1.19 (t, J=7.6 Hz, 3 H). |
| | | **MS (ESI)M/Z** : 360.1 [M+H]⁺ |
| C40 | 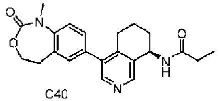 | **¹HNMR** (400 MHz, DMSO-*d₆*) δ 8.34 (s, 1 H), 8.27 (d, J=8.4 Hz, 1 H), 8.23 (s, 1 H), 7.40 (d, J=8.4 Hz, 1 H), 7.33-7.31 (m, 2 H), 5.11-5.07 (m, 1 H), 4.41 (t, J=6.4 Hz, 2 H), 3.33 (s, 3 H), 3.03 (t, J=6.8 Hz, 2 H), 2.62-2.61 (m, 2 H), 2.19-2.10 (m, 2 H), 1.89-1.86 (m, 1 H), 1.81-1.69 (m, 3 **H**), 1.06 (d, *J*=7.6 Hz, 3 H). |
| | | **MS (ESI)M/Z** : 380.1 [M+H]⁺ |
| C41 | 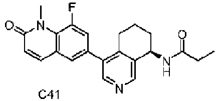 | **¹HNMR** (400 MHz, DMSO-*d₆*) δ 8.38 (s, 1 H), 8.29 (s, 1 H), 8.29 (d, J=7.2 Hz, 1 H), 7.97 (dd, J=9.6 Hz, 1.6 Hz, 1 H), 7.60-7.55 (m, 2 H), 6.73 (d, J=9.6 Hz, 1 H), 5.13-5.08 (m, 1 H), 3.82 (d, J=8.4 Hz, 3 H), 2.66 (t, J=10.0 Hz, 2 H), 2.20-2.13 (m, 2 H), 1.91-1.87 (m, 1 H), 1.82-1.70 (m, 3 H), 1.06 (t, J=7.6 Hz, 3 H). |
| | | **MS (ESI)M/Z** : 380.2 [M+H]⁺ |
| C42 | 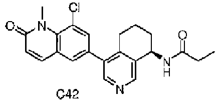 | **¹H NMR** (400 MHz, DMSO-*d₆*) δ 8.37 (d, J=6.8 Hz, 1 H), 8.30-8.28 (m, 2 H), 7.96 (t, J=8.8 Hz, 1 H), 7.72 (dd, *J*=14.4 Hz, 5.6 Hz, 2 H), 6.73 (t, J=8.4 Hz, 1 H), 5.14-5.05 (m, 1 H), 3.87 (d, *J =* 6.8 Hz, 3 H), 2.63 (d, *J* = 40 Hz, 2 H), 2.18-2.13 (m, 2 H). 1.92-1.65 (m, 4 H), 1.06 (t, J=7.6 Hz, 3 H) |
| | | **MS(ESI)M/Z**: 396.1 [M+H⁻] |
| C43 | 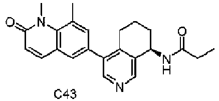 | **¹HNMR** (400 MHz, DMSO-*d₆*) δ 8.35 (s, 1 H), 8.28 (d, *J=*8.4 Hz, 1 H), 8.27 (s, 1 H), 7.90 (d, *J=*9.2 Hz, 1 H), 7.56 (d, *J*=2.0 Hz, 1 H), 7.42 (d, *J=*2.0 Hz, 1 H), 6.64 (d, *J*=9.6 Hz, 1 H), 5.13-5.08 (m, 1 H), 3.79 (s, 3 H), 2.77 (s, 3 H), 2.65-2.62 (m, 2 H), 2.18-2.10 (m, 2 H), 2.19-2.13 (m, 2 H), 1.92-1.87 (m, 1 H), 1.79-1.68 (m, 3 H), 1.06 (t, *J*=7.6 Hz, 3 H). |
| | | **MS (ESI)M/Z** : 376.1 [M+H]⁺ |
| C44 | 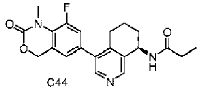 | **¹HNMR** (400 MHz, DMSO-*d₆*) δ 8.35 (s, 1 H), 8.27 (d, J=8.4 Hz, 1 H), 8.23 (s, 1 H), 7.33 (dd, *J=13.6* Hz, 1.6 Hz. 1 H), 7.17 (d, *J*=1.2 Hz, 1 H), 5.28 (s, 2 H), 5.14-5.05 (m, 1 H), 3.43 (d, *J*=6.0 Hz, 3 H), 2.68-2.59 (m, 2 H), 2.22-2.09 (m, 2 H), 1.94-1.83 (m, 1 H), 1.80-1.63 (m, 3 H), 1.05 (t, *J*=7.6 Hz, 3 H). |
| | | **MS (ESI)M/Z** : 384.1 [M+H]⁺ |
| D06 | 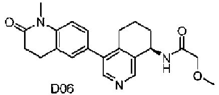 | **¹HNMR** (400 MHz, DMSO-*d₆*) δ 8.31 (s, 1 H), 8.26 (d, J=8.4 Hz, 1 H), 8.20 (s, 1 H), 7.25 (d, J=8.4 Hz, 1 H), 7.24 (s, 1 H), 7.18 (d, J=8.0 Hz, 1 H), 5.19-5.14 (m, 1 H), 3.90 (s, 2 H), 3.32 (s, 3 H), 3.29 (s, 3 H), 2.92 (t, J=7.2 Hz, 2 H), 2.63-2.57 (m, 4 H), 1.87-1.79 (m, 3 H), 1.72-1.65 (m, 1 H). |
| | | **MS (ESI)M/Z** : 380.2 [M+H]⁺ |
| D07-P1 | 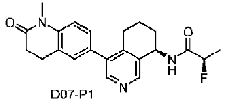 | **¹HNMR** (400 MHz, DMSO-*d₆*) δ 8.63 (d, J=8.4 Hz, 1 H), 8.30 (s, 1 H), 8.22 (s, 1 H), 7.26-7.24 (m, 2 H), 7.18 (d, J=8.4 Hz, 1 H), 5.17-4.98 (m, 2 H), 3.29 (s, 3 H), 2.93 (t, J=6.8 Hz, 2 H), 2.69-2.55 (m, 4 H), 1.95-1.61 (m, 4 H), 1.50 (dd, J=24.4 Hz, 6.8 Hz, 3 H). |
| | | **¹⁹FNMR** (377 MHz, DMSO-*d₆*) δ 180.89. |
| | | **MS (ESI)M/Z** : 382.2 [M+H]⁺ |
| D07-P2 | 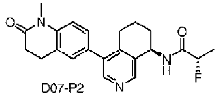 | **¹HNMR** (400 MHz, DMSO-*d₆*) δ 8.63 (d, J=8.4 Hz, 1 H), 8.30 (s, 1 H), 8.22 (s, 1 H), 7.26-7.24 (m, 2 H), 7.18 (d, J=8.0 Hz, 1 H), 5.18-5.00 (m, 2 H), 3.29 (s, 3 H), 2.93 (t, J=6.8 Hz, 2 H), 2.68-2.55 (m, 4 H), 1.95-1.61 (m, 4 H), 1.47 (dd, J=24.4 Hz, 6.4 Hz, 3 H). |
| | | **¹⁹FNMR** (377 MHz, DMSO-*d₆*) δ 180.89. |
| | | **MS (ESI)M/Z** : 382.2 [M+H]⁺ |
| D09 | 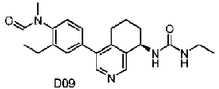 | **¹HNMR** (400 MHz, DMSO-*d₆*) δ 8.40 (s, 1 H), 8.18 (s, 1 H), 7.26-7.24 (m, 2 H), 7.17 (d, J=8.0 Hz, 1 H), 6.36 (d, J=8.4 Hz, 1 H), 5.77 (t, J=4.4 Hz, 1 H), 4.93-4.88 (m, 1 H), 3.29 (s, 3 H), 3.11-3.04 (m, 2 H), 2.92 (t, J=7.6 Hz, 2 H), 2.66-2.54 (m, 4 H), 1.92-1.81 (m, 1 H), 1.74-1.66 (m, 3 H), 1.02 (t, J=6.8 Hz, 3 H). |
| | | **MS (ESI)M/Z** : 379.2 [M+H]⁺ |
| D10 | 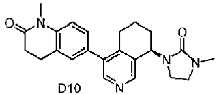 | **¹HNMR** (400 MHz, DMSO-*d₆*) δ 8.21 (s, 1 H), 8.20 (s, 1 H), 7.31-7.24 (m, 2 H), 7.19 (d, J=7.6 Hz, 1 H), 5.12-5.01 (m, 1 H), 3.29-3.21 (m, 5 H), 2.99-2.88 (m, 3 H), 2.74 (s, 3 H), 2.71-2.64 (m, 1 H), 2.60-2.55 (m, 4 H), 1.95-1.78 (m, 3 H), 1.75-1.62 (m, 1 H). |
| | | **MS (ESI)M/Z** : 391.1 [M+H]⁺. |
| D12 | 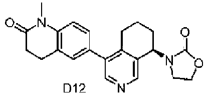 | **¹H NMR** (400 MHz, CD₃OD) δ 8.48 (s, 1 H), 8.33 (s, 1 H), 8.22 (s, 1 H), 7.28-7.22 (m, 3 H), 5.21-5.17 (m, 1 H), 4.42 (t, J=8.4 Hz, 1 H), 3.69-3.62 (m, 1 H), 3.40 (s, 3 H), 3.38-3.34 (m, 1 H), 2.98 (t, J=6.8 Hz, 2 H), 2.79-2.75 (m, 1 H), 2.69-2.64 (m, 3 H), 2.12-2.00 (m, 1 H), 1.98-1.95 (m, 2 H), 1.80-1.77 (m, 1 H). |
| | | **MS(ESI)M/Z:** 378.1 [M+H]⁺ |
| D16 | 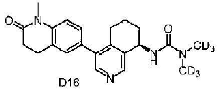 | **¹ HNMR** (400 MHz, DMSO-*d₆*) δ 8.38 (s, 1 H), 8.18 (s, 1 H), 7.29-7.12(m, 3 H), 6.64 (d, *J=*8.4 Hz, 1 H), 5.03-4.92 (m, 1 H), 3.29 (s, 3 H), 2.92 (t, *J*=6.8 Hz, 2 H), 2.69-2.55 (m, 4 H), 1.95-1.70 (m, 3 H), 1.69-1.55 (m, 1 H). |
| | | **MS (ESI)M/Z** : 385.2 [M+H]⁺ |
| D17 | 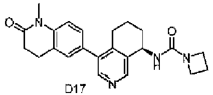 | **¹HNMR** (400 MHz, DMSO-*d₆*) δ 8.38 (s, 1 H), 8.18 (s, 1 H), 7.26-7.20 (m, 2 H), 7.17 (d, *J*=8.0 Hz, 1 H), 6.69 (d, *J*=8.4 Hz, 1 H), 4.95-4.86 (m, 1 H), 3.82 (t, *J*=8.0 Hz, 4 H), 3.29 (s, 3 H), 2.92 (d, *J*=6.8 Hz, 2 H), 2.69-2.54 (m, 4 H), 2.19-2.08 (m, 2 H), 1.92-1.77 (m, 2 H), 1.71-1.56 (m, 2 H). |
| | | **MS (ESI)M/Z** : 391.1 [M+H]⁺. |
| B03-P1 | 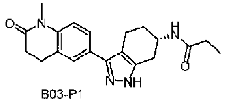 | **¹HNMR** (400 MHz, DMSO-*d₆)* δ 12.73-12.41 (m, 1 H), 7.86 (d, J=6.8 Hz, 1 H), 7.53 (s, 2 H), 7.14 (s, 1 H), 4.03-3.98 (m, 1 H), 3.27 (s, 3 H), 2.93-2.86 (m, 3 H), 2.79-2.68 (m, 2 H), 2.57 (t, J=7.2 Hz, 2 H), 2.47-2.44 (m, 1 H), 2.10 (q, J=7.2 Hz, 2 H), 1.93-1.90 (m, 1 H), 1.67-1.64 (m, 1 H), 1.01 (t, J=7.6 Hz, 3 H). |
| | | **MS (ESI)M/Z:** 353.1 [M+H]⁺ |
| B03-P2 | 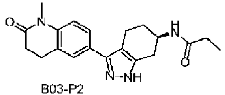 | **¹HNMR** (400 MHz, DMSO-*d₆)* δ 12.72-12.40 (m, 1 H), 7.85 (d, J=7.2 Hz, 1 H), 7.53 (s, 2 H), 7.14 (s, 1 H), 4.04-3.97 (m, 1 H), 3.27 (s, 3 H), 2.92-2.86 (m, 3 H), 2.79-2.66 (m, 2 H), 2.56 (t, J=8.0 Hz, 2 H), 2.48-2.44 (m, 1 H), 2.10 (q, J=7.6 Hz, 2 H), 1.93-1.87 (m, 1 H), 1.71-1.62 (m, 1 H), 1.01 (t, J=7.2 Hz, 3 H). |
| | | **MS (ESI)M/Z:** 353.1 [M+H]⁺ |
| D14 | 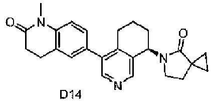 | **¹H NMR** (400 MHz, DMSO-d6) δ 8.17 (s, 1 H), 8.13 (s, 1 H), 7.25 (d, J=9.6 Hz, 1 H), 7.24 (s, 1 H), 7.16 (d, J=8.4 Hz, 1 H), 4.82-4.79 (m, 1 H), 4.42-4.37 (m, 2 H), 3.29 (s, 3 H), 2.91 (t, J= 8.0 Hz, 2 H), 2.67-2.55 (m, 4 H), 2.22 (t, J= 7.2 Hz, 2 H), 1.92-1.79 (m, 2 H), 1.66-1.48 (m, 2 H), 0.94-0.92 (m, 2 H), 0.90-0.89 (m, 2 H). |
| | | **MS (ESI)M/Z:** 402.3 [M+H]⁺ |
| C02 | 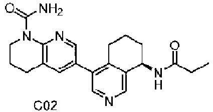 | ¹H NMR (400 MHz, DMSO-d6) δ 9.65 (s, 1 H), 8.35 (s, 1H), 8.27 (d, J = 8.4 Hz, 1 H), 8.25 (s, 1 H), 8.12 (d, J = 2.4 Hz, 1 H), 7.64 (d, J = 2.4 Hz, 1 H), 7.01 (s, 1 H), 5.12 - 4.99 (m, 1 H), 3.99 - 3.75 (m, 2 H), 2.85 (t, J = 6.3 Hz, 2 H), 2.64 (d, J = 6.5 Hz, 2 H), 2.15 (qd, J = 7.4, 4.6 Hz, 2 H), 1.92 - 1.64 (m, 6 H), 1.05 (t, J = 7.6 Hz, 3 H). |
| | | MS (ESI)M/Z: 380.2 [M-H]⁻ |
| C27 | 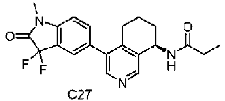 | ¹H NMR (400 MHz, DMSO-d6) δ 8.36 (s, 1 H), 8.27 (d, J = 8.4 Hz, 1 H), 8.23 (s, 1 H), 7.75 (s, J = 1.9 Hz, 1 H), 7.61 (d, J = 8.2 Hz, 1 H), 7.33 (d, J = 8.2 Hz, 1 H), 5.09 (m, 1 H), 3.23 (s, 3 H), 2.60 (t, J = 5.8 Hz, 2 H), 2.16 (m, 2 H), 1.92 - 1.67 (m, 4 H), 1.06 (t, J = 7.6 Hz, 3 H). |
| | | MS(ESI)M/Z: 386.1 [M+H]⁻ |
| C29 | 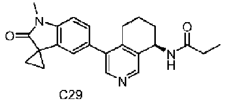 | ¹H NMR (400 MHz, DMSO-d6) δ 8.31 (s, 1 H), 8.25 (d, J = 8.4 Hz, 1 H), 8.20 (s, 1 H), 7.23 (dd, J = 8.0, 1.7 Hz, 1 H), 7.15 (d, J = 8.0 Hz, 1 H), 7.04 (d, J = 1.7 Hz, 1 H), 5.08 (m, 1 H), 3.26 (s, 3 H), 2.60 (t, J = 6.0 Hz, 2 H), 2.15 (m, 2 H), 1.92 - 1.64 (m, 6 H), 1.54 (m, 2 H), 1.05 (t, J = 7.6 Hz, 3 H). |
| | | MS(ESI)M/Z: 376.2 [M+H]⁻ |
| C30 | 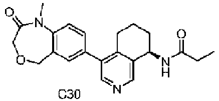 | ¹H NMR (400 MHz, DMSO-d6) δ 8.36 (s, 1 H), 8.28 (d, J = 8.4 Hz, 1 H), 8.25 (s, 1 H), 7.57 - 7.46 (m, 3 H), 5.10 (q, J = 6.6 Hz, 1 H), 4.64 (s, 2 H), 3.91 (s, 2 H), 3.37 (s, 3 H), 2.67 - 2.57 (m, 2 H), 2.16 (qd, J = 7.4, 4.9 Hz, 2 H), 1.94 - 1.84 (m, 1 H), 1.83 - 1 65 (m, 3 H), 1.06 (t, J = 7.6 Hz, 3 H). |
| | | **MS (ESI)M/Z,** 380.2 [M+H]⁺ |
| C45 | 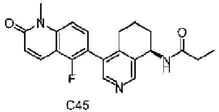 | **¹HNMR** (400 MHz, DMSO-*d₆*) δ 8.41 (s, 1 H), 8.32 (d, J=8.4 Hz, 1 H), 8.28 (s, 1 H), 8.06 (d, J=9.6 Hz, 1 H), 7.59 (t, J=8.0 Hz, 1H), 7.49 (d, J=8.8 Hz, 1H), 6.75 (d, J=10.0 Hz, 1 H), 5.17-5.07 (m, 1 H), 3.67 (s, 3 H), 2.48-2.43 (m, 2 H), 2.23-2.11 (m, 2 H), 1.94-1.84 (m, 1 H), 1.77-1.61 (m, 3 H), 1.06 (t, J=7.6 Hz, 3H). |
| | | **¹⁹FNMR** (376 MHz, DMSO-*d₆*) δ -122.04. |
| | | **MS (ESI)M/Z:** 379.9 [M+H]⁺ |
| C46 | 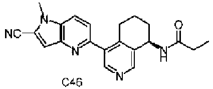 | **¹HNMR** (400 MHz, CD₃OD) δ 8.44 (s, 1 H), 8.36 (s, 1 H), 8.16 (d, J=8.8 Hz, 1 H), 7.55 (d, J=8.4 Hz, 1 H), 7.40 (s, 1 H), 5.24 (t, J=6.0 Hz, 1 H), 4.03 (s, 3 H), 2.86-2.78 (m, 1 H), 2.74-2.67 (m, 1 H), 2.33-2.26 (m, 2 H), 2.09-2.01 (m, 1 H), 1.93-1.76 (m, 3 H), 1.20 (t, J=7.6 Hz, 3 H). |
| | | **MS (ESI)M/Z;** 360.2 [M+H]⁺ |
| A19 | 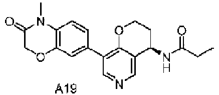 | **¹HNMR** (400 MHz, DMSO-*d*₆) δ 8.36 (d, J=7.6 Hz, 1 H), 8.28 (s, 1 H), 8.23 (s, 1 H), 7.25-7.20 (m, 2 H), 7.17 (s, 1 H), 5.12-5.08 (m, 1 H), 4.70 (s, 2 H), 4.39-4.34 (m, 1 H), 4.31-4.25 (m, 1 H), 3.32 (s, 3 H), 2.19-2.14 (m, 2 H), 2.13-2.06 (m, 1 H), 1.97-1.92 (m, 1 H), 1.06 (t, J=7.6 Hz, 3 H). |
| | | MS**(ESI)M/Z:** 368.1 [M+H]⁺ |
| A20 | 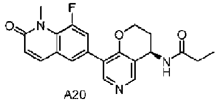 | **¹HNMR** (400 MHz, DMSO-*d₆*) δ 8.39 (s, 1 H), 8.36 (d, J=8.0 Hz, 1 H), 8.27 (s, 1 H), 7.97 (dd, J=9.6 Hz, 1.6 Hz, 1 H), 7.76 (d, J=2.0 Hz, 1 H), 7.68 (dd, J=16.0 Hz, 2.0 Hz, 1 H), 6.71 (d, J=9.6 Hz, 1 H), 5.14-5.09 (m, 1 H), 4.43-4.38 (m, 1 H), 4.34-4.29 (m, 1 H), 3.81 (d, J=8.4 Hz, 3 H), 2.22-2.13 (m, 2 H), 212-2.07 (m, 1 H), 2.00-1.93 (m, 1 H), 1.06 (t, J=7.6 Hz, 3 H). |
| | | **MS (ESI)M/Z:** 382.1 [M+H]⁺ |
| A21 | 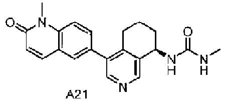 | **¹HNMR** (400 MHz, DMSO-*d₆)* δ 8.44 (s, 1 H), 8.26 (s, 1 H), 7.95 (d, J=9.6 Hz, 1 H), 7.75 (s, 1 H), 7.65-7.60 (m, 2 H), 6.68 (d, J-9.6 Hz, 1 H), 6.44 (d, J=8.4 Hz, 1 H), 5.68 (q, J=4.4 Hz, 1 H), 4.94-4.90 (m, 1 H), 3.66 (s, 3 H), 2.62 (d, J=4.4 Hz, 3 H), 2.61-2.58 (m, 2 H), 1.92-1.86 (m, 1 H), 1.74-1.70 (m, 3 H). |
| | | **MS (ESI)M/Z:** 363.2 [M+H]⁺ |
| A22 | 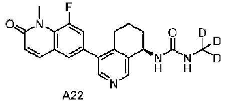 | **¹HNMR** (400 MHz, DMSO-*d₆*) δ 8.46 (s, 1 H), 8.27 (s, 1 H), 7.96 (dd, J=9.6 Hz, 1.2 Hz, 1 H), 7.60 (d, J=1.6 Hz, 1 H), 7.57 (dd, J=18.0 Hz, 2.4 Hz, 1 H), 6.73 (d, J=9.6 Hz, 1 H), 6.44 (d, J=8.4 Hz, 1 H), 5.68 (s, 1 H), 4.95-4.90 (m, 1 H), 3.82 (d, J=8.4 Hz, 3 H), 2.68-2.61 (m, 2 H), 1.92-1.86 (m, 1 H), 1.77-1.68 (m, 3 H). |
| | | **¹⁹FNMR** (376 MHz, DMSO-*d₆*) δ -121.21. |
| | | **MS (ESI)M/Z:** 384.1 [M+H]⁺ |
| A23 | 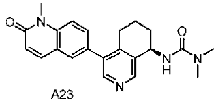 | **¹HNMR** (400 MHz, DMSO-*d₆*) δ 8.42 (s, 1 H), 8.25 (s, 1 H), 7.97 (d, J=9.6 Hz, 1 H), 7.74 (s, 1 H), 7.62 (s, 2 H), 6.68 (d, J=9.2 Hz, 2 H), 5.02-4.97 (m, 1 H), 3.67 (s, 3 H), 2.84 (s, 6 H), 2.67-2.61 (m, 2 H), 1.87-1.76 (m, 3 H), 1.67-1.61 (m, 1 H). |
| | | **MS (ESI)M/Z:** 377.2 [M+H]⁺ |
| A24 | 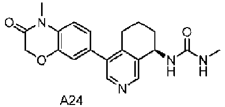 | **¹HNMR** (400 MHz, DMSO-*d₆*) δ 8.41 (s, 1 H), 8.18 (s, 1 H), 7.24 (d, J=8.4 Hz, 1 H), 7.05 (dd, J=8.4 Hz, 2.0 Hz, 1 H), 7.01 (d, J=2.0 Hz, 1 H), 6.42 (d, J=8.8 Hz, 1 H), 5.68 (q, J=4.4 Hz, 1 H), 4.91-4.88 (m, 1 H), 4.71 (s, 2 H), 3.32 (s, 3 H), 2.61 (d, J=4.8 Hz, 3 H), 2.60-2.59 (m, 2 H), 1.88-1.82 (m, 1 H), 1.74-1.69 (m, 3 H). |
| | | **MS (ESI)M/Z:** 367.1 [M+H]⁺ |
| A25 | 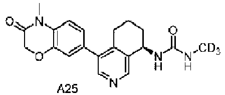 | **¹HNMR** (400 MHz, DMSO-*d₆*) δ 8.41 (s, 1 H), 8.18 (s, 1 H), 7.24 (d, J=8.4 Hz, 1 H), 7.05 (dd, J=8.0 Hz, 1.6 Hz, 1 H), 7.01 (d, J=1.6 Hz, 1 H), 6.41 (d, J=8.4 Hz, 1 H), 5.63 (s, 1 H), 4.92-4.88 (m, 1 H), 4.71 (s, 2 H), 3.32 (s, 3 H), 2.62-2.57 (m, 2 H), 1.90-1.84 (m, 1 H), 1.75-1.68 (m, 3 H). |
| | | **MS (ESI)M/Z:** 370.1 [M+H]⁺ |
| A26 | 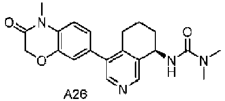 | **¹H NMR** (400 MHz, DMSO-*d₆*) δ 8.39 (s, 1 H), 8.18 (s, 1 H), 7.25 (d, J=8.0 Hz, 1 H), 7.04 (dd, J=8.0 Hz, 2.0 Hz, 1 H), 6.99 (d, J=1.6 Hz, 1 H), 6.65 (d, J=8.4 Hz, 1 H), 5.00-4.94 (m, 1 H), 4.71 (s, 2 H), 3.32 (s, 3 H), 2.83 (s, 6 H), 261-257 (m, 2 H), 1.91-1.82 (m, 2 H), 1.76-1.63 (m, 2 H) |
| | | **MS(ESI)M/Z:** 381.2 [M+H]⁺ |
| A27 | 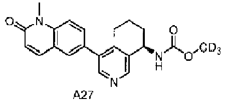 | **¹HNMR** (400 MHz, DMSO-*d₆*) δ 8.42 (s, 1 H), 8.27 (s, 1 H), 7.96 (d, J=9.6 Hz, 1 H), 7.75 (s, 1 H), 7.72 (d, J=8.8 Hz, 1 H), 7.66-7.59 (m, 2 H), 6.68 (d, J=9.6 Hz, 1 H), 4.89-4.78 (m, 1 H). 3.66 (s, 3 H), 2.69-2.57 (m, 2 H), 1.99-1.86 (m, 1 H), 1.78-1.60 (m, 3 H). |
| | | MS **(ESI)M/Z:** 367.1 [M+H]⁺ |
| A28 | 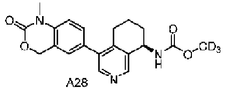 | **¹HNMR** (400 MHz, DMSO-*d₆*) δ 8.39 (s, 1 H), 8.20 (s, 1 H), 7.70 (d, J=8.8 Hz, 1 H), 7.36 (dd, J=8.4 Hz, 1.6 Hz, 1 H), 7.29 (s, 1 H), 7.18 (d, J=8.4 Hz, 1 H), 5.30 (s, 2 H), 4.88-4.76 (m, 1 H), 3.32 (s, 3 H), 2.63-2.54 (m, 2 H), 1.98-1.85 (m, 1 H), 1.76-1.59 (m, 3 H). |
| | | **MS (ESI)M/Z:** 371.1 [M+H]⁺ |
| A29 | 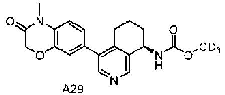 | **¹HNMR** (400 MHz, DMSO-*d₆*) δ 8.38 (s, 1 H), 8.20 (s, 1 H), 7.68 (d, J=8.4 Hz, 1 H), 7.25 (d, J=8.4 Hz, 1 H), 7.04 (dd, J=8.0 Hz, 1.6 Hz, 1 H), 7.00 (d, J=1.6 Hz, 1 H), 4.86-4.77 (m, 1 H), 4.71 (s, 2 H), 3.32 (s, 3 H), 2.63-2.55 (m, 2 H), 1.96-1.85 (m, 1 H), 1.76-1.60 (m, 3 H). |
| | | **MS (ESI)M/Z:** 371.1 [M+H]⁺ |
| A32 | 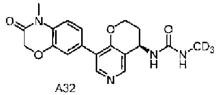 | **¹HNMR** (400 MHz, DMSO-*d₆*) δ 8.27 (s, 1 H), 8.25 (s, 1 H), 7.24-7.19 (m, 2 H), 7.16 (s, 1 H), 6.56 (d, J=8.0 Hz, 1 H), 5.69 (s, 1 H), 4.93-4.89 (m, 1 H), 4.69 (s, 2 H), 4.38-4.32 (m, 1 H), 4.28-4.22 (m, 1 H), 3.31 (s, 3 H), 2.09-2.03 (m, 1 H), 1.98-1.93 (m, 1 H). |
| | | MS **(ESI)M/Z:** 372.1 [M+H]⁺ |
| A33 | 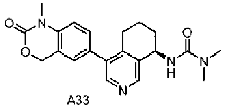 | **¹HNMR** (400 MHz, DMSO-*d₆*) δ 8.39 (s, 1 H), 8.18 (s, 1 H), 7.36 (dd, J=8.4 Hz, 2.0Hz, 1 H), 7.28 (d, J=1.6 Hz, 1 H), 7.18 (d, J=8.4 Hz, 1 H), 6.66 (d, J=8.4 Hz, 1 H), 5.30 (s, 2 H), 5.00-4.95 (m, 1 H), 3.32 (s, 3 H), 2.83 (s, 6 H), 2.66-2.54 (m, 2 H), 1.92-1.62 (m, 4 H). |
| | | **MS (ESI)M/Z:** 381.1 [M+H]⁺ |
| A34 | 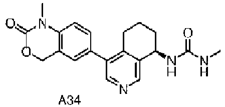 | **¹HNMR** (400 MHz, DMSO-*d₆*) δ 8.41 (s, 1 H), 8.19 (s, 1 H), 7.36 (dd, J=8.4 Hz, 1.6 Hz, 1 H), 7.29 (s, 1 H), 7.18 (d, J=8.4 Hz, 1 H), 6.42 (d, J=8.4 Hz, 1 H), 5.70-5.62 (m, 1 H), 5.30 (s, 2 H), 4.95-4.87 (m, 1 H), 332 (s, 3 H), 2.61 (d, J=4.8 Hz, 3 H), 2.59-2.54 (m, 2 H), 1.92-1.81 (m, 1 H), 1.78-1.62 (m, 3 H). |
| | | **MS (ESI)M/Z:** 366.9 [M+H]⁺ |
| A35 | 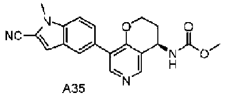 | **¹HNMR** (400 MHz, DMSO-*d₆*) δ 8.30 (s, 1 H), 8.29 (s, 1 H), 7.81 (d, J=8.8 Hz, 1 H), 7.80 (s, 1 H), 7.68 (d, J=8.8 Hz, 1 H), 7.55 (dd, J=8.8 Hz, 1.6 Hz, 1 H), 7.48 (s, 1 II), 4.93-4.84 (m, 1 II), 4.39-4.24 (m, 2 H), 3.93 (s, 3 H), 3.62 (s, 3 H), 2.17-2.06 (m, 1 H), 2.04-1.92 (m, 1 H). |
| | | **MS (ESI)M/Z:** 362.9 [M+H]⁺ |
| A36 | 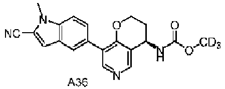 | **¹HNMR** (400 MHz, DMSO-*d₆*) δ 8.29 (s, 1 H), 8.28 (s, 1 H), 7.81 (d, J=6.4 Hz, 1 H), 7.80 (d, J=1.2 Hz, 1 H), 7.67 (dd, J=8.8 Hz, 1 H), 7.54 (dd, J=8.8 Hz, 1.6 Hz, 1 H), 7.47 (s, 1 H), 4.92-4.84 (m, 1 H), 4.38-4.24 (m, 2 H), 3.93 (s, 3 H), 2.16-2.06 (m, 1 H), 2.03-1.93 (m, 1 H). |
| | | **MS (ESI)M/Z:** 366.1 [M+H]⁺ |
| A37 | 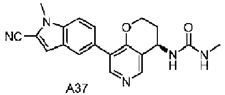 | **¹HNMR** (400 MHz, DMSO-*d₆*) δ 8.29 (s, 1 H), 8.28 (s, 1 H), 7.80 (d, J=0.8 Hz, 1 H), 7.67 (d, J=8.8 Hz, 1 H), 7.54 (dd, J=8.8 Hz, 1.6 Hz, 1 H), 7.47 (s, 1 H), 6.58 (d, J=7.6 Hz, 1 H), 5.73 (q, J=4.8 Hz, 1 H), 4.98-4.89 (m, 1 H), 4.39-4.30 (m, 1 H), 4.29-4.19 (m, 1 H), 3.93 (s, 3 H), 2.61 (d, J=4.8 Hz, 3 H), 2.13-2.02 (m, 1 H), 2.01-1.90 (m, 1 H). |
| | | **MS (ESI)M/Z:** 362.1 [M+H]⁺ |
| A38 | 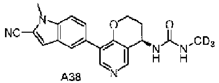 | **¹HNMR** (400 MHz, DMSO-*d₆*) δ 8.29 (s, 1 H), 8.28 (s, 1 H), 7.79 (d, J=0.8 Hz, 1 H), 7.67 (d, J=8.8 Hz, 1 H), 7.54 (dd, J=8.8 Hz, 1.6 Hz, 1 H), 7.47 (s, 1 H), 6.61 (d, J=8.0 Hz, 1 H), 5.73 (s, 1 H), 4.95-4.91 (m, 1 H), 4.36-4.31 (m, 1 H), 4.26-4.21 (m, 1 H), 3.93 (s, 3 H), 2.10-2.04 (m, 1 H), 1.98-1.93 (m, 1 H). |
| | | **MS (ESI)M/Z:** 365.1 [M+H]⁺ |
| A39 | 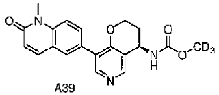 | **¹HNMR** (400 MHz, DMSO-*d₆*) δ 8.35 (s, 1 H), 8.31 (s, 1 H), 7.96 (d, J=9.6 Hz, 1 H), 7.88 (d, J=2.0 Hz, 1 H), 7.81 (d, J=10.8 Hz, 1 H), 7.78 (dd, J=8.8 Hz, 2.4 Hz, 1 H), 7.60 (d, J=8.8 Hz, 1 H), 6.65 (d, J=9.2 Hz, 1 H), 4.90-4.86 (m, 1 H), 4.38-4.28 (m, 2 H), 3.65 (s, 3H), 2.15-2.08 (m, 1 H), 2.03-1.96 (m, 1 H). |
| | | **MS (ESI)M/Z:** 369.1 [M+H]⁺ |
| A40 | 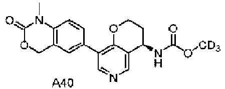 | **¹HNMR** (400 MHz, DMSO-*d₆*) δ 8.27 (s, 1 H), 8.26 (s, 1 H), 7.79 (d, J=8.0 Hz, 1 H), 7.51 (dd, J=8.4 Hz, 2.0 Hz, 1 H), 7.42 (d, J=2.0 Hz, 1 H), 7.16 (d, J=8.8 Hz, 1 H), 5.29 (s, 2 H), 4.88-4.84 (m, 1 H), 4.36-4.26 (m, 2 H), 3.31 (s, 3H), 2.13-2.07 (m, 1 H), 2.01-1.95 (m, 1 H). |
| | | **MS (ESI)M/Z:** 373.1 [M+H]⁺ |
| A41 | 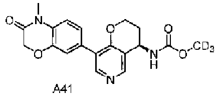 | **¹HNMR** (400 MHz, DMSO-*d₆*) δ 8.27 (s, 2 H), 7.78 (d, J=8.0 Hz, 1 H), 7.24-7.19 (m, 2 H), 7.15 (s, 1 H), 4.88-4.83 (m, 1 H), 4.69 (s, 2 H), 4.38-4.26 (m, 2 H), 3.31 (s, 3 H), 2.13-2.06 (m, 1 H), 2.00-1.94 (m, 1 H). |
| | | **MS (ESI)M/Z:** 373.1 [M+H]⁺ |
| A42 | 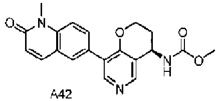 | **¹H NMR** (400 MHz, DMSO-*d₆*) δ 8.34 (s, 1 H), 8.30 (s, 1 H), 7.96 (d, J=9.6 Hz, 1 H), 7.88 (d, J=1.6 Hz, 1 H), 7.82 (d, J=8.0 Hz, 1 H), 7.78 (dd, J=8.8 Hz, J=2.0 Hz, 1 H), 7.60 (d, J=8.8 Hz, 1 H), 6.65 (dd, J=9.2 Hz, 1 H), 4.90-4.86 (m, 1 H), 4.38-4.28 (m, 2 H), 3.65 (s, 3 H), 3.61 (s, 3 H), 2.16-2.09 (m, 1 H), 2.03-1.94 (m, 1 H). |
| | | **MS (ESI)M/Z:** 366.2 [M+H]⁺ |
| A43 | 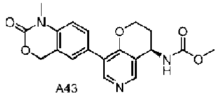 | **¹H NMR** (400 MHz, DMSO-*d₆*) δ 8.27 (s, 1 H), 8.26 (s, 1 H), 7.80 (d, J=8.0 Hz, 1 H), 7.51 (dd, J=8.4 Hz, J=2.0 Hz, 1 H), 7.42 (d, J=1.2 Hz, 1 H), 7.16 (d, J=8.4 Hz, 1 H), 5.29 (s, 2 H), 4.88-4.84 (m, 1 H), 4.37-4.25 (m, 2 H), 3.61 (s, 3 H), 3.31 (s, 3 H), 2.14-2.06 (m, 1 H), 2.00-1.93 (m, 1 H). |
| | | **MS (ESI)M/Z:** 370.2 [M+H]⁺ |
| A44 | 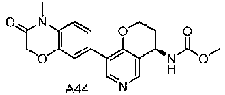 | **¹H NMR** (400 MHz, DMSO-*d6*) δ 8.27 (s, 2 H), 7.79 (d, J=8.0 Hz, 1 H), 7.24-7.19 (m, 2 H), 7.15 (s, 1 H), 4.88-4.83 (m, 1 H), 4.69 (s. 2 H), 4.37-4.26 (m, 2 H), 3.61 (s, 3 H), 3.31 (s, 3 H), 2.14-2.07 (m, 1 H), 2.00-1.93 (m, 1 H). |
| | | **MS (ESI)M/Z:** 370.2 [M+H]⁺ |
| A45 | 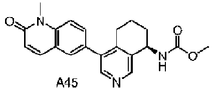 | **¹HNMR** (400 MHz, DMSO-*d₆*) δ 8.42 (s, 1 H), 8.27 (s, 1 H), 7.96 (d, J=9.6 Hz, 1 H), 7.75 (s, 1 H), 7.72 (d, J=8.4 Hz, 1 H), 7.65-7.60 (m, 2 H), 6.68 (d, J=9.6 Hz, 1 H), 4.86-4.81(m, 1 H), 3.66 (s, 3H), 3.61 (s, 3 H), 2.63-2.59 (m, 2 H), 1.95-1.89 (m, 1 H), 1.82-1.72 (m, 2 H), 1.70-1.64 (m, 1 H). |
| | | **MS (ESI)M/Z:** 364.1 [M+H]⁺ |
| A46 | 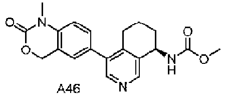 | **¹HNMR** (400 MHz, DMSO-*d₆*) δ 8.39 (s, 1 H), 8.20 (s, 1 H), 7.70 (d, J=8.4 Hz, 1 H), 7.36 (dd, J=8.4 Hz, 2.0 Hz, 1 H), 7.29 (d, J=1.6 Hz, 1 H), 7.18 (d, J=8.4 Hz, 1 H), 5.30 (s, 2 H), 4.84-4.79 (m, 1 H), 3.60 (s, 3 H), 3.31 (s, 3 H), 2.60-2.55 (m, 2 H), 1.92-1.87 (m, 1 H), 1.80-1.73 (m, 2 H), 1.69-1.64 (m, 1 H). |
| | | **MS (ESI)M/Z:** 368.1 [M+H]⁺ |
| A47 | 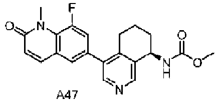 | **¹HNMR** (400 MHz, DMSO-*d₆*) δ 8.43 (s, 1 H), 8.29 (s, 1 H), 7.97 (dd, J=10.0 Hz, 1.6 Hz, 1 H), 7.73 (d, J=8.8 Hz, 1 H), 7.60 (d, J=1.6 Hz, 1 H), 7.56 (dd, J=15.6 Hz, 1.6 Hz, 1 H), 6.73 (d, J=9.2 Hz, 1 H), 4.85-4.80 (m, 1 H), 3.81 (d, J=8.4 Hz, 3 H), 3.61 (s, 3 H), 2.64 (t, J=5.6 Hz, 2 H), 1.95-1.89 (m, 1 H), 1.84-1.75 (m, 2 H), 1.72-1.67 (m, 1 H). |
| | | **¹⁹FNMR** (376 MHz, DMSO-*d₆*) δ -121.16. |
| | | **MS (ESI)M/Z:** 382.2 [M+H]⁺ |
| A48 | 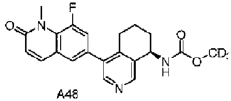 | **¹HNMR** (400 MHz, DMSO-*d₆*) δ 8.43 (s, 1 H), 8.29 (s, 1 H), 7.97 (dd, J=9.6 Hz, 1.6 Hz, 1 H), 7.72 (d, J=8.8 Hz, 1 H), 7.60 (d, J=2.0 Hz, 1 H), 7.56 (dd, J=15.6 Hz, 2.0 Hz, 1 H), 6.73 (d, J=9.6 Hz, 1 H), 4.85-4.80 (m, 1 H), 3.81 (d, J=8.4 Hz, 3 H), 2.67-2.61 (m, 2 H), 1.94-1.65 (m, 4 H). |
| | | **¹⁹FNMR** (376 MHz, DMSO-*d₆*) δ -121.16. |
| | | **MS (ESI)M/Z,** 385.1 [M+H]⁺ |
| A49 | 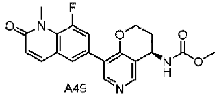 | **¹HNMR** (400 MHz, DMSO-*d₆*) δ 8.39 (s, 1 H), 8.32 (s, 1 H), 7.97 (dd, J=9.2 Hz, 1.2 Hz, 1 H), 7.81 (d, J=7.6 Hz, 1 H), 7.75 (d, J=1.6 Hz, 1 H), 7.67 (dd, J=16.0 Hz, 2.0 Hz, 1 H), 6.71 (d, J=9.6 Hz, 1 H), 4.93-4.83 (m, 1 H), 4.44-4.29 (m, 2 H), 3.80 (d, J=8.4 Hz, 3 H), 3.61 (s, 3 H), 2.18-2.07 (m, 1 H), 2.05-1.93 (m, 1 H). |
| | | **¹⁹FNMR** (376 MHz, DMSO-*d₆*) δ -121.58. |
| | | **MS (ESI)M/Z:** 384.0 [M+H]⁺ |
| A50 | 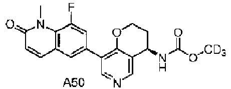 | **¹HNMR** (400 MHz, DMSO-*d₆*) δ 8.39 (s, 1 H), 8.32 (s, 1 H), 7.97 (dd, J=9.6 Hz, 1.6 Hz, 1 H), 7.80 (d, J=8.4 Hz, 1 H), 7.75 (d, J=2.0 Hz, 1 H), 7.67 (dd, J=16.0 Hz, 2.0 Hz, 1 H), 6.71 (d, J=9.6 Hz, 1 H), 4.90-4.85 (m, 1 H), 4.41-4.31 (m, 2 H), 3.81 (d, J=8.4 Hz, 3 H), 2.14-2.09 (m, 1 H), 2.02-1.97 (m, 1 H). |
| | | **¹⁹FNMR** (376 MHz, DMSO-*d₆*) δ -121.58. |
| | | **MS (ESI)M/Z:** 387.1 [M+H]⁺ |
| A51 | 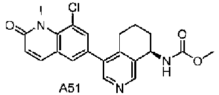 | **¹HNMR** (400 MHz, DMSO-*d₆*) δ 8.44 (s, 1 H), 8.30 (s, 1 H), 7.80 (d, J=9.2 Hz, 1 H), 7.75 (d, J=2.0 Hz, 1 H), 7.74 (d, J=7.6 Hz, 1 H), 7.71 (d, J=2.0 Hz, 1 H), 6.74 (d, J=9.6 Hz, 1 H), 4.85-4.81 (m, 1 H), 3.88 (s, 3 H), 3.61 (s, 3 H), 2.67-2.57 (m, 2 H), 1.95-1.89 (m, 1 H), 1.84-1.67 (m, 3 H). |
| | | **MS (ESI)M/Z:** 398.2 [M+H]⁺ |
| A52 | 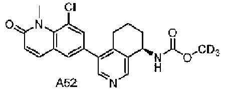 | **¹HNMR** (400 MHz, DMSO-*d₆*) δ 8.44 (s, 1 H), 8.30 (s, 1 H), 7.97 (d, J=9.6 Hz, 1 H), 7.75 (d, J=2.0 Hz, 1 H), 7.72 (d, J=7.6 Hz, 1 H), 7.71 (d, J=2.4 Hz, 1 H), 6.74 (d, J=9.2 Hz, 1 H), 4.85-4.80 (m, 1 H), 3.88 (s, 3 H), 2.63-2.61 (m, 2 H), 1.95-1.65 (m, 4 H). |
| | | **MS (ESI)M/Z:** 401.0 [M+H]⁺ |
| A53 | 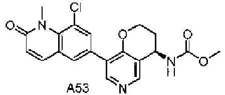 | **¹HNMR** (400 MHz, DMSO-*d₆*) δ 8.38 (s, 1 H), 8.32 (s, 1 H), 7.98 (d, J=9.6 Hz, 1 H), 7.89 (d, J=2.0 Hz, 1 H), 7.85 (d, J=2.0 Hz, 1 H), 7.81 (d, J=8.0 Hz, 1 H), 6.71 (d, J=9.2 Hz, 1 H), 4.92-4.83 (m, 1 H), 4.43-4.28 (m, 2 H), 3.87 (s, 3 H), 3.61 (s, 3 H), 2.18-2.07 (m, 1 H), 2.04-1.94 (m, 1 H). |
| | | **MS (ESI)M/Z:** 400.0 [M+H]⁺ |
| A54 | 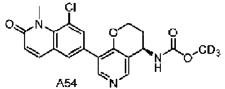 | **¹HNMR** (400 MHz, DMSO-*d₆*) δ 8.38 (s, 1 H), 8.32 (s, 1 H), 7.97 (d, J=9.6 Hz, 1 H), 7.89 (d, J=2.0 Hz, 1 H), 7.84 (d, J=2.0 Hz, 1 H), 7.80 (d, J=8.0 Hz, 1 H), 6.71 (d, J=9.2 Hz, 1 H), 4.90-4.85 (m, 1 H), 4.42-4.30 (m, 2 H), 3.87 (s, 3 H), 2.16-2.08 (m, 1 H), 2.03-1.95 (m, 1 H). |
| | | **MS (ESI)M/Z:** 403.0 [M+H]⁺ |
| A55 | 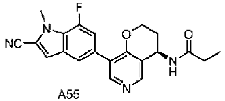 | **¹HNMR** (400 MHz, DMSO-d6) δ 8.36 (d, J=7.6 Hz, 1 H), 8.33 (s, 1 H), 8.25 (s, 1 H), 7.65 (d, J=1.2 Hz, 1 H), 7.55 (d, J=2.0 Hz, 1 H), 7.39 (dd, J=14.0 Hz, 1.2 Hz, 1 H), 5.14-5.09 (m, 1 H), 4.41-4.31 (m, 1 H), 4.29-4.25 (m, 1 H), 4.06 (d, J=1.6 Hz, 3 H), 2.23-2.13 (m, 2 H), 2.11-2.05 (m, 1 H), 2.00-1.90 (m, 1 H), 1.06 (t, J=7.6 Hz, 3 H). |
| | | **¹⁹FNMR** (376 MHz, DMSO-d6) δ -134.85. |
| | | **MS (ESI)M/Z:** 379.1 [M+H]⁺ |
| A56 | 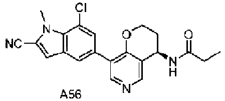 | **¹HNMR** (400 MHz, DMSO-d6) δ 8.36 (d, J=8.0 Hz, 1 H), 8.32 (s, 1 H), 8.25 (s, 1 H), 7.79 (d, J=1.2 Hz, 1 H), 7.60-7.55 (m, 2 H), 5.14-5.09 (m, 1 H), 4.41-4.34 (m, 1 H), 4.33-4.25 (m, 1 H), 4.21 (s, 3 H), 2.22-2.13 (m, 2 H), 2.11-2.05 (m, 1 H), 1.99-1.89 (m, 1 H), 1.06 (t, J=7.6 Hz, 3 H). |
| | | **MS (ESI)M/Z:** 394.9 [M+H]⁺ |

### Synthesis of Specific Compounds:

### Synthesis of Intermediates:

### Intermediates INT-1A, INT-1B, INT-1C, INT-1D, INT-1E, INT-1F

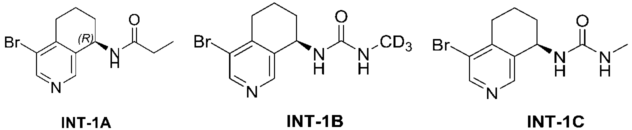

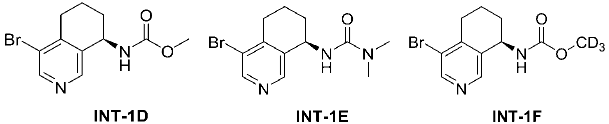

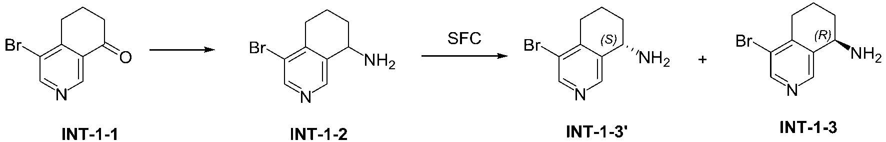

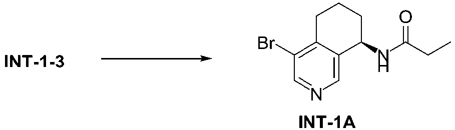

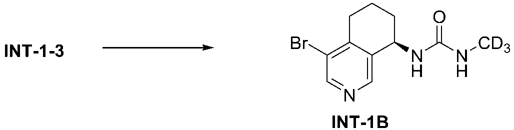

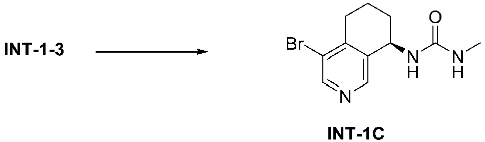

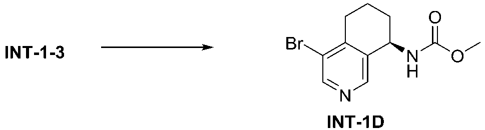

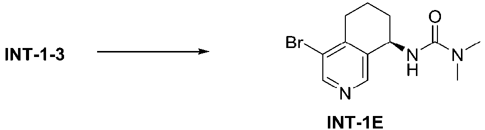

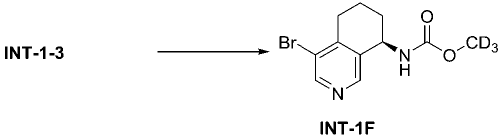

### Step 1: Synthesis of Compound INT-1-2

At 25 °C, ammonia-methanol solution (7 M, 6.32 mL, 44.25 mmol) and titanium isopropoxide (5.03 g, 17.70 mmol) were added to a solution of compound **INT-1-1** (2.0 g, 8.85 mmol) in methanol (13 mL). The resulting mixture was stirred at 25 °C under nitrogen protection for 16 hours. At 0 °C, sodium borohydride (502 mg, 13.27 mmol) was added to the above reaction mixture, and the resulting mixture was stirred at 25 C under nitrogen protection for 2 hours. The mixture was concentrated under reduced pressure. The residue was purified by silica gel column chromatography to obtain compound **INT-1-2** (1.45 g).

MS (ESI) m/z: 210.0, 212.0 [M+H-17]⁺.

### Step 2: Synthesis of Compounds INT-1-3 and INT-1-3'

Compound **INT-1-2** (1.45 g) was purified by supercritical fluid chromatography (chromatography conditions: system: Waters SFC 150; column name: DAICELCHIRALCEL^{®}AD; column dimensions: 250 × 25 mm, 10 µm; mobile phase A: supercritical CO₂; mobile phase B: methanol (+0.1% 7.0 mol/L ammonia in methanol); A:B = 75:25; wavelength: 214 nm; flow rate: 120 mL/min; column temperature: room temperature; column pressure: 100 bar; injection volume: 8.0 mL; cycle time: 6.58 min) to obtain compound **INT-1-3'** (peak 1, ee = 100%, 675 mg) and compound **INT-1-3** (peak 2, ee = 99.4%, 665 mg).

**Compound INT-1-3':** Peak 1, analyzed by supercritical fluid chromatography (chromatography conditions: system: Waters UPCC (CA352); column name: DAICELCHIRALCEL^{®}AD; column dimensions: 100 × 3.0 mm, 3.0 µm; mobile phase A: supercritical CO₂; mobile phase B: methanol (0.1% DEA (diethanolamine)); A:B = 80:20; wavelength: 214 nm; flow rate: 1.5 mL/min; column temperature: 35 °C; column pressure: 1800 psi; injection volume: 1.0 µL; cycle time: 8.0 min). Retention time RT: 2.020 min, ee = 100%.

¹H NMR (400 MHz, DMSO-*d₆*) δ 8.59 (s, 1H), 8.47 (s, 1H), 3.89 (t, J = 6.0 Hz, 1H), 2.68-2.56 (m, 2H), 1.99-1.90 (m, 3H), 1.87-1.80 (m, 1H), 1.73-1.67 (m, 1H), 1.62-1.55 (m, 1H).

MS (ESI) m/z: 210.0, 212.0 [M+H-17]⁺.

**Compound INT-1-3:** Peak 2, analyzed by supercritical fluid chromatography (chromatography conditions: system: Waters UPCC (CA352); column name: DAICELCHIRALCEL^{®}AD; column dimensions: 100 × 3.0 mm, 3.0 µm; mobile phase A: supercritical CO₂; mobile phase B: methanol (0.1% DEA); A:B = 80:20; wavelength: 214 nm; flow rate: 1.5 mL/min; column temperature: 35 °C; column pressure: 1800 psi; injection volume: 1.0 µL; cycle time: 8.0 min). Retention time RT: 2.826 min, ee = 99.4%.

¹H NMR (400 MHz, DMSO-*d₆*) δ 8.60 (s, 1H), 8.48 (s, 1H), 3.93 (t, J = 6.0 Hz, 1H), 2.69-2.55 (m, 2H), 1.97-1.91 (m, 1H), 1.88-1.81 (m, 1H), 1.74-1.68 (m, 1H), 1.64-1.58 (m, 1H).

MS (ESI) m/z: 210.0, 212.0 [M+H-17]⁺.

### Step 3: Synthesis of Compound INT-1A

Under nitrogen protection at 0 °C, propionyl chloride (271 mg, 2.93 mmol) was added dropwise to a solution of compound **INT-1-3** (665 mg, 2.93 mmol) and triethylamine (1.48 g, 14.65 mmol) in dichloromethane (20 mL). The mixture was stirred at 0 °C under nitrogen protection for 1 hour. The reaction mixture was quenched with water (20 mL) and extracted with ethyl acetate (20 mL × 2). The combined organic phases were concentrated under reduced pressure. The residue was purified by silica gel column chromatography to obtain compound **INT-1A** (650 mg).

**Compound INT-1A:** Analyzed by supercritical fluid chromatography (chromatography conditions: system: Waters UPCC (CA352); column name: DAICELCHIRALCEL^{®}AD; column dimensions: 100 × 3.0 mm, 3.0 µm; mobile phase A: supercritical CO₂; mobile phase B: methanol (0.1% DEA); gradient: 5% B to 40% B; wavelength: 214 nm; flow rate: 1.5 mL/min; column temperature: 35 °C; column pressure: 1800 psi; injection volume: 0.5 µL; cycle time: 8.0 min). Retention time RT: 3.961 min.

¹H NMR (400 MHz, DMSO-*d₆*) δ 8.54 (s, 1H), 8.29 (s, 1H), 8.23 (d, J = 8.4 Hz, 1H), 5.04-5.01 (m, 1H), 2.69-2.64 (m, 2H), 2.17-2.10 (m, 2H), 1.91-1.68 (m, 4H), 1.04 (d, J = 7.2 Hz, 3H).

MS (ESI) m/z: 283.0, 285.0 [M+H]⁺.

### Step 4: Synthesis of Compound INT-1B

At room temperature, N,N'-carbonyldiimidazole (115 mg, 0.71 mmol) and N,N-diisopropylethylamine (454 mg, 3.5 mmol) were added to a solution of compound **INT-1-3** (80 mg, 0.35 mmol) in anhydrous tetrahydrofuran (5 mL). The mixture was stirred at room temperature for 2 hours, then deuterated methylamine hydrochloride (100 mg, 1.41 mmol) was added. The mixture was stirred at room temperature for 16 hours. After the reaction was completed, the mixture was diluted with water (5 mL) and concentrated. The residue was separated and purified by reverse-phase column chromatography (0.1% formic acid-acetonitrile, 15% acetonitrile) to obtain compound **INT-1B** (50 mg).

MS (ESI) m/z: 287.0, 289.0 [M+H]⁺.

### Step 5: Synthesis of Compound INT-1C

Under nitrogen protection at 25 °C, N,N'-carbonyldiimidazole (129.72 mg, 0.80 mmol) and ethyldiisopropylamine (1033.92 mg, 8 mmol) were added to a solution of compound **INT-1-3** (90 mg, 0.40 mmol) in tetrahydrofuran (5 mL). The mixture was stirred at 25 °C under nitrogen protection for 2 hours. Methylamine hydrochloride (270.08 mg, 4 mmol) was added to the above mixture, and stirring continued at 25 °C under nitrogen protection for 16 hours. The reaction mixture was quenched with saturated ammonium chloride solution (10 mL) and extracted with ethyl acetate (10 mL × 2). The combined organic phases were washed with saturated brine (10 mL × 3) and concentrated under reduced pressure. The residue was purified by silica gel column chromatography to obtain compound **INT-1C** (50 mg).

¹H NMR (400 MHz, DMSO-*d₆*) δ 8.52 (s, 1H), 8.37 (s, 1H), 6.41 (d, J = 8.8 Hz, 1H), 5.70-5.69 (m, 1H), 4.88-4.83 (m, 1H), 2.68-2.63 (m, 2H), 2.59 (d, J = 4.4 Hz, 3H), 1.87-1.68 (m, 4H).

MS (ESI) m/z: 284.0, 286.0 [M+H]⁺.

### Step 6: Synthesis of Compound INT-1D

Compound **INT-1-3** (50 mg, 0.22 mmol) was dissolved in dichloromethane (5 mL). At room temperature, triethylamine (67 mg, 0.66 mmol) and methyl chloroformate (21 mg, 0.22 mmol) were added. The reaction was stirred at room temperature for 1 hour. Water (100 mL) was added, and the mixture was extracted twice with ethyl acetate (30 mL). The combined organic phases were dried over anhydrous sodium sulfate and concentrated under reduced pressure. The residue was purified by silica gel column chromatography to obtain compound **INT-1D** (30 mg).

MS (ESI) m/z: 285.1, 287.1 [M+H]⁺.

### Step 7: Synthesis of Compound INT-1E

Under nitrogen protection at 25 °C, N,N'-carbonyldiimidazole (84.32 mg, 0.52 mmol) and ethyldiisopropylamine (336.02 mg, 2.60 mmol) were added to a solution of compound **INT-1-3** (60 mg, 0.26 mmol) in tetrahydrofuran (2 mL). The reaction mixture was stirred at 25 °C under nitrogen protection for 2 hours. A solution of dimethylamine in tetrahydrofuran (2.0 M in THF, 0.65 mL, 1.30 mmol) was added, and stirring continued at 25 °C under nitrogen protection for 16 hours. The reaction mixture was quenched with saturated ammonium chloride solution (10 mL) and extracted with ethyl acetate (10 mL × 2). The combined organic phases were washed with saturated brine (10 mL × 3) and concentrated under reduced pressure. The residue was purified by silica gel column chromatography to obtain compound **INT-1E** (50 mg).

¹H NMR (400 MHz, DMSO-*d₆*) δ 8.51 (s, 1H), 8.34 (s, 1H), 6.63 (d, J = 8.4 Hz, 1H), 4.95-4.89 (m, 1H), 2.81 (s, 6H), 2.65 (d, t = 6.0 Hz, 2H), 1.97-1.91 (m, 1H), 1.85-1.72 (m, 3H).

MS (ESI) m/z: 298.0, 300.0 [M+H]⁺.

### Step 8: Synthesis of Compound INT-1F

Under nitrogen protection at 25 °C, N,N'-carbonyldiimidazole (28.54 mg, 0.18 mmol) and ethyldiisopropylamine (227.46 mg, 1.76 mmol) were added to a solution of compound **INT-1-3** (20 mg, 0.088 mmol) in tetrahydrofuran (4 mL). The reaction mixture was stirred at 25 °C under nitrogen protection for 2 hours. Deuterated methanol (3 mL) was added, and the mixture was stirred at 60 C under nitrogen protection for 16 hours. The reaction mixture was quenched with saturated ammonium chloride solution (10 mL) and extracted with ethyl acetate (10 mL × 2). The combined organic phases were washed with saturated brine (10 mL × 3), dried, filtered, and concentrated under reduced pressure. The residue was purified by silica gel column chromatography to obtain compound **INT-1F** (26 mg).

MS (ESI) m/z: 288.0, 290.0 [M+H]⁺.

### Intermediate INT-2

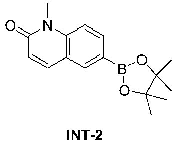

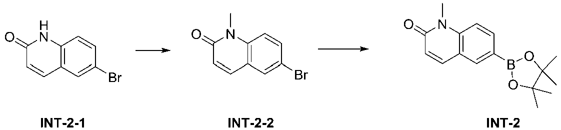

### Step 1: Synthesis of Compound INT-2-2

First, compound **INT-2-1** (2 g, 8.93 mmol) was dissolved in N,N-dimethylformamide (20 mL). The reaction mixture was placed in an ice bath at 0 °C, then potassium tert-butoxide (2 g, 17.86 mmol) was added and stirred under the ice bath for 15 minutes. Iodomethane (1.65 g, 11.61 mmol) was added, and the system was substituted with nitrogen three times. The mixture was allowed to warm to room temperature naturally and stirred for 16 hours. The mixture was extracted with ethyl acetate (200 mL) and water (100 mL). The organic phase was washed with saturated sodium chloride solution (100 mL), dried over anhydrous sodium sulfate, filtered, and the filtrate was concentrated under vacuum to obtain a crude product. The crude product was purified by column chromatography to obtain compound **INT-2-2** (1.5 g).

MS (ESI) m/z: 238.0, 240.0 [M+H]⁺.

### Step 2: Synthesis of Compound INT-2

Compound **INT-2-2** (0.2 g, 0.84 mmol) and bis(pinacolato)diboron (0.32 g, 1.26 mmol) were dissolved in 1,4-dioxane (10 mL). Then potassium acetate (0.25 g, 2.52 mmol) and [1,1'-bis(diphenylphosphino)ferrocene]palladium(II) dichloride (0.061 g, 0.084 mmol) were added. The system was thoroughly substituted with nitrogen and reacted at 80 °C for 2 hours. The reaction mixture was cooled to room temperature, diluted with water (60 mL), and extracted with ethyl acetate (80 mL × 2). The combined organic phases were dried over anhydrous sodium sulfate, filtered, and concentrated to obtain a crude product. The crude product was purified by column chromatography to obtain compound **INT-2** (0.24 g).

¹H NMR (400 MHz, CDCl₃) δ 8.02 (d, J = 1.2 Hz, 1H), 7.97 (dd, J = 8.4 Hz, 1.2 Hz, 1H), 7.70 (d, J = 9.6 Hz, 1H), 7.35 (d, J = 8.4 Hz, 1H), 6.71 (d, J = 9.2 Hz, 1H), 3.73 (s, 3H), 1.37 (s, 12H).

MS (ESI) m/z: 286.10 [M+H]⁺.

### Intermediate INT-3

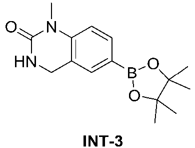

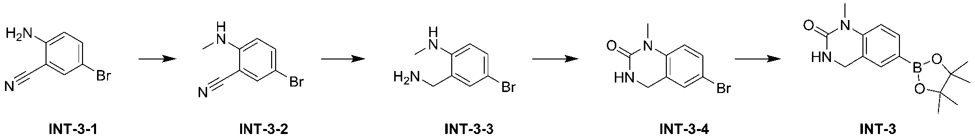

### Step 1: Synthesis of Compound INT-3-2

Compound **INT-3-1** (7.0 g, 35.53 mmol) was dissolved in N,N-dimethylacetamide (70 mL). Potassium tert-butoxide solution (4.98 g, 44.41 mmol, 1 mol/L) and dimethyl oxalate (6.29 g, 53.30 mmol) were added separately. The system was thoroughly substituted with nitrogen and reacted at 130 C for 16 hours. The mixture was diluted with water (150 mL) and extracted with ethyl acetate (100 mL × 3). The combined organic phases were dried over anhydrous sodium sulfate, filtered, and concentrated to obtain a crude product. The crude product was purified by column chromatography to obtain compound **INT-3-2** (1.26 g).

MS (ESI) m/z: 211.1 [M+H]⁺.

### Step 2: Synthesis of Compound INT-3-3

Compound **INT-3-2** (0.2 g, 0.95 mmol) in anhydrous tetrahydrofuran (5 mL) was thoroughly substituted with nitrogen. The reaction mixture was placed in an ice bath at 0 °C, and lithium aluminum hydride (0.18 g, 4.75 mmol, 2.5 mol/L) was added dropwise slowly. The system was reacted at room temperature for 2 hours. The reaction mixture was placed in an ice bath, and water (10 mL), 15% sodium hydroxide aqueous solution (10 mL), and water (30 mL) were added sequentially. The mixture was filtered through celite and washed with ethyl acetate (20 mL × 3). The filtrate was concentrated under vacuum to obtain a crude product. The combined organic phases were dried over anhydrous sodium sulfate, filtered, and concentrated. The residue was purified by column chromatography to obtain compound **INT-3-3** (0.16 g).

MS (ESI) m/z: 215.1 [M+H]⁺.

### Step 3: Synthesis of Compound INT-3-4

Compound **INT-3-3** (0.16 g, 0.74 mmol) was dissolved in tetrahydrofuran (5 mL). N,N'-Carbonyldiimidazole (0.06 g, 0.37 mmol) was added, and the system was thoroughly substituted with nitrogen. The mixture was reacted at 60 °C for 2 hours. The reaction mixture was cooled to room temperature, diluted with water (80 mL), and extracted with ethyl acetate (80 mL × 3). The combined organic phases were dried over anhydrous sodium sulfate, filtered, and concentrated. The residue was purified by column chromatography to obtain compound **INT-3-4** (0.1 g).

MS (ESI) m/z: 241.0 [M+H]⁺.

### Step 4: Synthesis of Compound INT-3

Compound **INT-3-4** (0.1 g, 0.41 mmol) and bis(pinacolato)diboron (120 mg, 0.49 mmol) were dissolved in 1,4-dioxane (10 mL). DPPF-PdCl₂ (1,1'-bis(diphenylphosphino)ferrocene palladium(II) dichloride, 33 mg, 0.041 mmol) and potassium acetate (120 mg, 1.23 mmol) were added. The system was thoroughly substituted with nitrogen and reacted at 80 °C under nitrogen protection for 12 hours. The reaction mixture was cooled to room temperature, filtered through celite, and washed with ethyl acetate (20 mL × 3). The filtrate was extracted with water (40 mL). The combined organic phases were washed with saturated sodium chloride (30 mL), dried over anhydrous sodium sulfate, filtered, and concentrated under vacuum to remove the solvent, yielding a crude product. The crude product was purified by column chromatography to obtain compound **INT-3** (80 mg).

MS (ESI) m/z: 289.2 [M+H]⁺.

### Intermediate INT-4

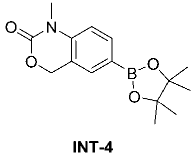

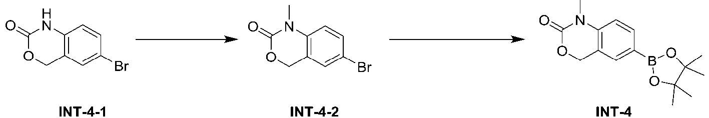

### Step 1: Synthesis of Compound INT-4-2

Compound **INT-4-1** (0.8 g, 3.51 mmol) was dissolved in N,N-dimethylformamide (10 mL). Potassium carbonate (0.97 g, 7.02 mmol) and iodomethane (0.75 g, 5.26 mmol) were added separately. The system was substituted with nitrogen and reacted at 50 °C under nitrogen protection for 16 hours. The reaction mixture was cooled to room temperature, diluted with water (80 mL), and extracted with ethyl acetate (80 mL × 3). The organic phase was washed with saturated sodium chloride (100 mL), dried over anhydrous sodium sulfate, filtered, and the filtrate was concentrated under vacuum to obtain a crude product. The crude product was separated and purified by column chromatography to obtain compound **INT-4-2** (0.7 g).

MS (ESI) m/z: 242.0, 244.0 [M+H]⁺.

### Step 2: Synthesis of Compound INT-4

Compound **INT-4-2** (0.2 g, 0.83 mmol) and bis(pinacolato)diboron (0.25 g, 1.00 mmol) were dissolved in 1,4-dioxane (10 mL). DPPF-PdCl₂ (0.0068 g, 0.083 mmol) and potassium acetate (0.24 g, 2.49 mmol) were added. The system was thoroughly substituted with nitrogen and reacted at 80 °C for 12 hours. The reaction mixture was cooled to room temperature, filtered through celite, and extracted with ethyl acetate (50 mL × 2) and water (60 mL). The organic phase was washed with saturated sodium chloride (60 mL), dried over anhydrous sodium sulfate, filtered, and the filtrate was concentrated under vacuum to obtain a crude product. The crude product was separated and purified by column chromatography to obtain compound **INT-4** (0.2 g).

¹H NMR (400 MHz, CDCl₃): δ 7.79 (d, J = 7.6 Hz, 1H), 7.57 (s, 1H), 6.93 (d, J = 8.0 Hz, 1H), 5.20 (s, 2H), 3.39 (s, 3H), 1.35 (s, 12H).

MS (ESI) m/z: 290.2 [M+H]⁺.

### Intermediate INT-5A

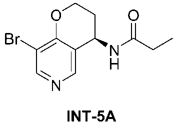

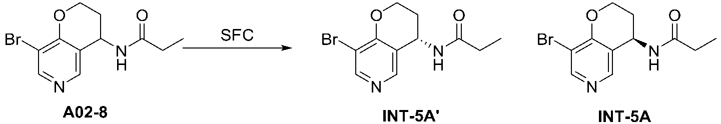

### Step 1: Synthesis of Compounds INT-5A and INT-5A'

Compound **A02-8** (168 mg) was purified by supercritical fluid chromatography (chromatography conditions: system: Waters SFC 150; column name: DAICELCHIRALPAK^{®}AD; column dimensions: 250 × 30 mm, 10 µm; mobile phase A: supercritical CO₂; mobile phase B: ethanol (+0.1% 7.0 mol/L ammonia in methanol); A:B = 75:25; wavelength: 214 nm; flow rate: 120 mL/min; column temperature: room temperature; column pressure: 100 bar; injection volume: 7.0 mL; cycle time: 5.21 min) to obtain compound **INT-SA'** (peak 1, 66 mg) and compound **INT-5A** (peak 2, 62 mg).

**Compound INT-5A':** Peak 1, analyzed by supercritical fluid chromatography (chromatography conditions: system: Waters UPCC (CA-060); column name: DAICELCHIRALCEL^{®}AD; column dimensions: 100 × 3.0 mm, 3.0 µm; mobile phase A: supercritical CO₂; mobile phase B: methanol (0.1% DEA); A:B = 85:15; wavelength: 214 nm; flow rate: 1.5 mL/min; column temperature: 35 °C; column pressure: 1800 psi; injection volume: 0.2 µL; cycle time: 8.0 min). Retention time RT: 1.799 min, ee > 99%.

**Compound INT-5A:** Peak 2, analyzed by supercritical fluid chromatography (chromatography conditions: system: Waters UPCC (CA-060); column name: DAICELCHIRALCEL^{®}AD; column dimensions: 100 × 3.0 mm, 3.0 µm; mobile phase A: supercritical CO₂; mobile phase B: methanol (0.1% DEA); A:B = 85:15; wavelength: 214 nm; flow rate: 1.5 mL/min; column temperature: 35 °C; column pressure: 1800 psi; injection volume: 0.2 µL; cycle time: 8.0 min). Retention time RT: 3.949 min, ee > 99%.

### Intermediates INT-5B, INT-5C, INT-5D, INT-5E

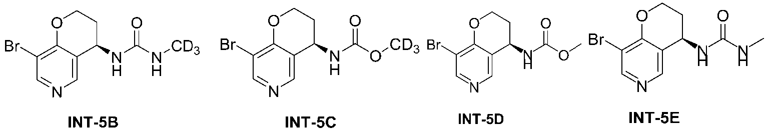

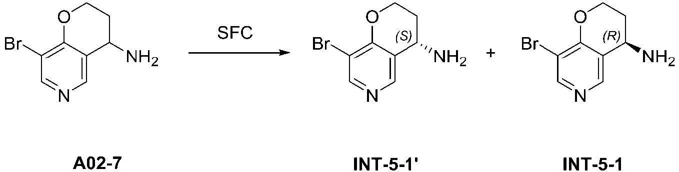

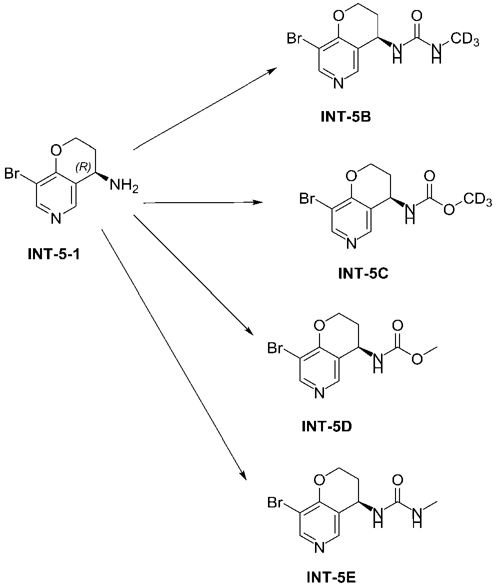

### Step 1: Synthesis of Compounds INT-5-1 and INT-5-1'

Compound **A02-7** (2.3 g) was purified by supercritical fluid chromatography (chromatography conditions: system: Waters SFC 150; column name: DAICELCHIRALPAK^{®}AY; column dimensions: 250 × 30 mm, 10 µm; mobile phase A: supercritical CO₂; mobile phase B: methanol (+0.1% 7.0 mol/L ammonia in methanol); A:B = 70:30; wavelength: 214 nm; flow rate: 120 mL/min; column temperature: room temperature; column pressure: 100 bar; injection volume: 2.0 mL; cycle time: 11.00 min) to obtain compound **INT-5-1'** (peak 1, 490 mg) and compound **INT-5-1** (peak 2, 568 mg).

**Compound INT-5-1':** Peak 1, analyzed by supercritical fluid chromatography (chromatography conditions: system: Waters UPCC (CA060); column name: DAICELCHIRALCEL^{®}AD; column dimensions: 100 × 3.0 mm, 3.0 µm; mobile phase A: supercritical CO₂; mobile phase B: methanol (0.1% DEA); A:B = 75:25; wavelength: 214 nm; flow rate: 1.5 mL/min; column temperature: 35 °C; column pressure: 1800 psi; injection volume: 0.10 µL; cycle time: 8.0 min). Retention time RT: 1.376 min; ee = 98.4%.

¹H NMR (400 MHz, DMSO-*d₆*) δ 8.41 (s, 1H), 8.38 (s, 1H), 4.50-4.33 (m, 2H), 3.99 (t, J = 5.2 Hz, 1H), 2.41-2.14 (m, 2H), 2.09-1.94 (m, 1H), 1.88-1.75 (m, 1H).

MS (ESI) m/z: 229.0, 231.0 [M+H]⁺.

Compound INT-5-1: Peak 2, analyzed by supercritical fluid chromatography (chromatography conditions: system: Waters UPCC (CA060); column name: DAICELCHIRALCEL^{®}AD; column dimensions: 100 × 3.0 mm, 3.0 µm; mobile phase A: supercritical CO₂; mobile phase B: methanol (0.1% DEA); A:B = 75:25; wavelength: 214 nm; flow rate: 1.5 mL/min; column temperature: 35 °C; column pressure: 1800 psi; injection volume: 0.10 µL; cycle time: 8.0 min). Retention time RT: 2.915 min, ee = 99.8%.

¹H NMR (400 MHz, DMSO-*d₆*) δ 8.41 (s, 1H), 8.38 (s, 1H), 4.50-4.33 (m, 2H), 3.98 (t, J = 5.2 Hz, 1H), 2.32-2.09 (m, 2H), 2.08-1.956 (m, 1H), 1.86-1.75 (m, 1H).

MS (ESI) m/z: 229.0, 231.0 [M+H]⁺.

### Step 2: Synthesis of Compound INT-5B

At room temperature, N,N'-carbonyldiimidazole (210 mg, 1.31 mmol) and N,N-diisopropylethylamine (845 mg, 6.55 mmol) were added to a solution of compound **INT-5-1** (150 mg, 0.66 mmol) in anhydrous tetrahydrofuran (4 mL). The mixture was stirred at room temperature for 2 hours. After LCMS indicated disappearance of the starting material, deuterated methylamine hydrochloride (92 mg, 1.31 mmol) was added, and the mixture was stirred at room temperature for 16 hours. After the reaction was completed, the mixture was diluted with water (5 mL) and concentrated. The residue was separated and purified by reverse-phase column chromatography (0.1% formic acid-acetonitrile, 15% acetonitrile) to obtain compound **INT-5B** (120 mg).

MS (ESI) m/z: 289.1, 291.1 [M+H]⁺.

### Step 3: Synthesis of Compound INT-5C

Under nitrogen protection at 25 °C, N,N'-carbonyldiimidazole (42.16 mg, 0.26 mmol) and ethyldiisopropylamine (336.02 mg, 2.60 mmol) were added to a solution of compound **INT-5-1** (30 mg, 0.13 mmol) in tetrahydrofuran (3 mL). The reaction mixture was stirred at 25 °C under nitrogen protection for 2 hours. Deuterated methanol (3 mL) was added to the above mixture, and stirring continued at 60 C under nitrogen protection for 16 hours. The reaction mixture was quenched with saturated ammonium chloride solution (10 mL) and extracted with ethyl acetate (10 mL × 2). The combined organic phases were washed with saturated brine (10 mL × 3), dried, filtered, and concentrated under reduced pressure. The residue was purified by silica gel column chromatography to obtain compound **INT-5C** (30 mg).

MS (ESI) m/z: 289.9, 291.9 [M+H]⁺.

### Step 4: Synthesis of Compound INT-5D

Under nitrogen protection at 25 °C, N,N'-carbonyldiimidazole (42.16 mg, 0.26 mmol) and ethyldiisopropylamine (336.02 mg, 2.60 mmol) were added to a solution of **INT-5-1** (30 mg, 0.13 mmol) in tetrahydrofuran (3 mL). The reaction mixture was stirred at 25 C under nitrogen protection for 2 hours. Anhydrous methanol (2 mL) was added to the above mixture, and stirring continued at 60 C under nitrogen protection for 16 hours. The reaction mixture was quenched with saturated ammonium chloride solution (10 mL) and extracted with ethyl acetate (10 mL × 2). The combined organic phases were washed with saturated brine (10 mL × 3), dried, filtered, and concentrated under reduced pressure. The residue was purified by silica gel column chromatography to obtain **INT-5D** (25 mg).

MS (ESI) m/z: 288.8 [M+H]⁺.

### Step 5: Synthesis of Compound INT-5E

Under nitrogen protection at 25 °C, N,N'-carbonyldiimidazole (42.16 mg, 0.26 mmol) and ethyldiisopropylamine (336.02 mg, 2.60 mmol) were added to a solution of **INT-5-1** (30 mg, 0.13 mmol) in tetrahydrofuran (3 mL). The reaction mixture was stirred at 25 °C under nitrogen protection for 2 hours. Methylamine hydrochloride (180 mg, 2.60 mmol) was added to the above mixture, and stirring continued at 60 °C under nitrogen protection for 16 hours. The reaction mixture was quenched with saturated ammonium chloride solution (10 mL) and extracted with ethyl acetate (10 mL × 2). The combined organic phases were washed with saturated brine (10 mL × 3), dried, filtered, and concentrated under reduced pressure. The residue was purified by silica gel column chromatography to obtain **INT-5E** (25 mg).

MS (ESI) m/z: 285.9, 287.9 [M+H]⁺.

### Intermediate INT-6

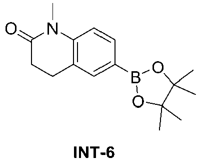

Intermediate **INT-6** is a commercially available reagent, CAS number: 1427587-32-3, obtained by purchase.

### Intermediate INT-7

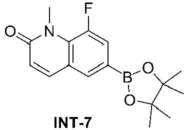

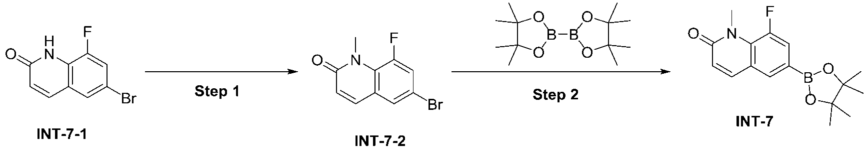

### Step 1: Synthesis of Compound INT-7-2

Compound **INT-7-1** (520 mg, 2.15 mmol) was dissolved in N,N-dimethylformamide (10 mL). At 0 °C, 60% sodium hydride (300 mg, 8.6 mmol) was added slowly. The mixture was stirred at room temperature for 30 minutes, then iodomethane (366 mg, 2.58 mmol) was added. The system was reacted at 25 °C for 5 hours. After TLC indicated completion of the reaction, water (50 mL) was added to dilute, and the mixture was extracted with ethyl acetate (20 mL) twice. The combined organic phases were dried over anhydrous sodium sulfate, filtered, and concentrated under reduced pressure. The residue was purified by silica gel column chromatography to obtain compound **INT-7-2** (350 mg).

MS (ESI) m/z: 256.0 [M+H]⁺.

### Step 2: Synthesis of Compound INT-7

Compound **INT-7-2** (350 mg, 1.37 mmol), bis(pinacolato)diboron (696 mg, 2.74 mmol), potassium acetate (404 mg, 4.11 mmol), and [1,1'-bis(diphenylphosphino)ferrocene]palladium(II) dichloride dichloromethane complex (112 mg, 0.14 mmol) were added to 1,4-dioxane (15 mL). The mixture was substituted with nitrogen three times, then reacted at 90 C for 16 hours. LCMS monitored the reaction completion. The reaction mixture was directly concentrated under reduced pressure and purified by silica gel column chromatography to obtain compound **INT-7** (256 mg).

MS (ESI) m/z: 304.1 [M+H]⁺.

### Intermediate INT-8

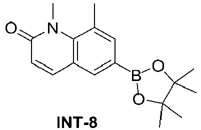

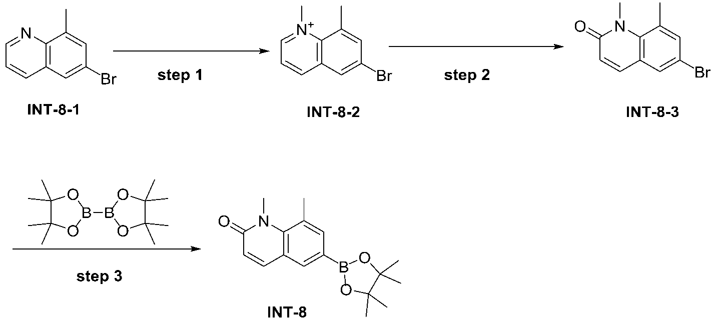

### Step 1: Synthesis of Compound INT-8-2

At room temperature, compound **INT-8-1** (1.0 g, 4.51 mmol) was dissolved in dimethyl sulfate (5 mL). The mixture was stirred at 60 °C for 16 hours, and a white solid precipitated. After LCMS indicated completion of the reaction, the reaction mixture was filtered. The white solid was eluted with tetrahydrofuran and dried to obtain compound **INT-8-2** (1.0 g).

MS (ESI) m/z: 238.0 [M+H]⁺.

### Step 2: Synthesis of Compound INT-8-3

At room temperature, potassium hydroxide (708 mg, 12.66 mmol) and potassium ferricyanide (2.78 g, 8.44 mmol) were added to a solution of compound **INT-8-2** (1.2 g) in acetonitrile and water (8/8 mL). The mixture was stirred at room temperature for 2 hours. After LCMS indicated completion, the mixture was diluted with water (20 mL) and extracted with ethyl acetate (30 mL × 3). The combined organic phases were dried over anhydrous sodium sulfate, filtered, and concentrated. The residue was separated and purified by column chromatography to obtain compound **INT-8-3** (1.0 g).

MS (ESI) m/z: 252.1 [M+H]⁺.

### Step 3: Synthesis of Compound INT-8

At room temperature, bis(pinacolato)diboron (756 mg, 2.98 mmol), potassium acetate (350 mg, 3.57 mmol), and [1,1'-bis(diphenylphosphino)ferrocene]palladium(II) dichloride dichloromethane complex (87 mg, 0.119 mmol) were added to a solution of compound **INT-8-3** (300 mg, 1.19 mmol) in dioxane (10 mL). The resulting reaction mixture was stirred at 90 °C under nitrogen protection for 16 hours. The mixture was filtered, and the mother liquor was concentrated under reduced pressure. The residue was purified by column chromatography to obtain compound INT-8 (300 mg).

MS (ESI) m/z: 300.1 [M+H]⁺.

### Intermediate INT-9

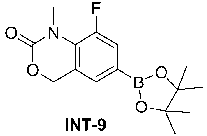

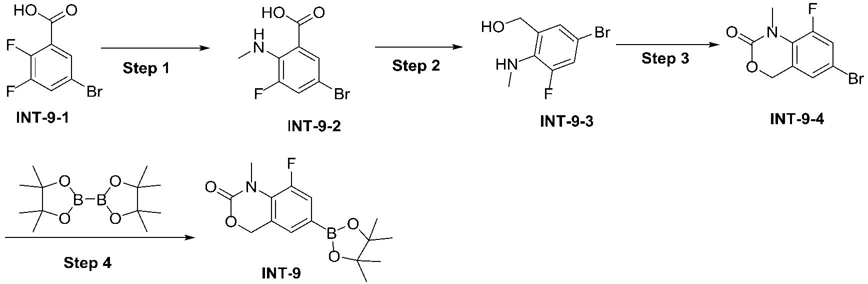

### Step 1: Synthesis of Compound INT-9-2

Compound **INT-9-1** (1 g, 4.24 mmol) was dissolved in N-methylpyrrolidone (3 mL). Methylamine tetrahydrofuran solution (2 M, 5 mL, 10 mmol) was added at room temperature, and the mixture was heated to 110 °C in a sealed tube and reacted for 16 hours. The reaction mixture was concentrated under reduced pressure, and the residue was purified by reverse-phase flash chromatography (80 g; mobile phase A: pure water; mobile phase B: acetonitrile; flow rate: 70 mL/min). When the content of mobile phase B reached 15%, compound **INT-9-2** (610 mg) was collected.

MS (ESI) m/z: 249.9 [M+H]⁺.

### Step 2: Synthesis of Compound INT-9-3

Compound **INT-9-2** (550 mg, 2.21 mmol) was dissolved in tetrahydrofuran (10 mL). Borane tetrahydrofuran solution (1 M, 13.32 mL) was added dropwise at 0 °C. The system was reacted at room temperature under nitrogen protection for 16 hours. The reaction mixture was quenched with methanol (4 mL) in an ice bath, diluted with water, and extracted twice with ethyl acetate. The combined organic phases were dried over anhydrous sodium sulfate, filtered, and concentrated under reduced pressure. The residue was purified by silica gel column chromatography to obtain compound **INT-9-3** (330 mg).

MS (ESI) m/z: 234.0 [M+H]⁺.

### Step 3: Synthesis of Compound INT-9-4

Compound **INT-9-3** (270 mg, 1.15 mmol) was dissolved in tetrahydrofuran (6 mL). Triethylamine (349.11 mg, 3.45 mmol) and triphosgene (341.26 mg, 1.15 mmol) were added at -60 °C. After addition, the temperature was raised to -40 °C and stirred for 2 hours. TLC and LCMS monitored the disappearance of the starting material. The reaction mixture was diluted by adding water (10 mL), and extracted with ethyl acetate (10 mL × 2). The organic phase was washed twice with saturated brine, dried over anhydrous sodium sulfate, filtered, and concentrated under reduced pressure. The residue was purified by silica gel column chromatography to obtain compound **INT-9-4** (116 mg).

MS (ESI) m/z: 302.9 [M+41+H]⁺.

### Step 4: Synthesis of Compound INT-9

Potassium acetate (55.94 mg, 0.57 mmol) and [1,1'-bis(diphenylphosphino)ferrocene]palladium(II) dichloride dichloromethane complex (15.52 mg, 0.019 mmol) were added to a mixture of compound **INT-9-4** (50 mg, 0.19 mmol) and bis(pinacolato)diboron (96.50 mg, 0.38 mmol) in 1,4-dioxane (5.0 mL). The resulting reaction mixture was stirred at 90 °C under nitrogen protection for 16 hours. LCMS monitoring showed disappearance of the starting material. The reaction mixture was diluted by adding water (10 mL), and extracted with ethyl acetate (10 mL × 2). The combined organic phases were dried over anhydrous sodium sulfate, filtered, and concentrated under reduced pressure. The residue was purified by silica gel column chromatography to obtain compound **INT-9** (30 mg).

MS (ESI) m/z: 308.0 [M+H]⁺.

### Example A02-P1 and A02-P2

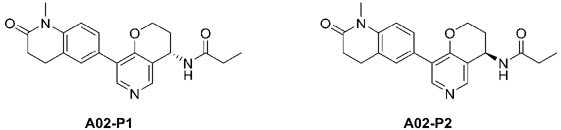

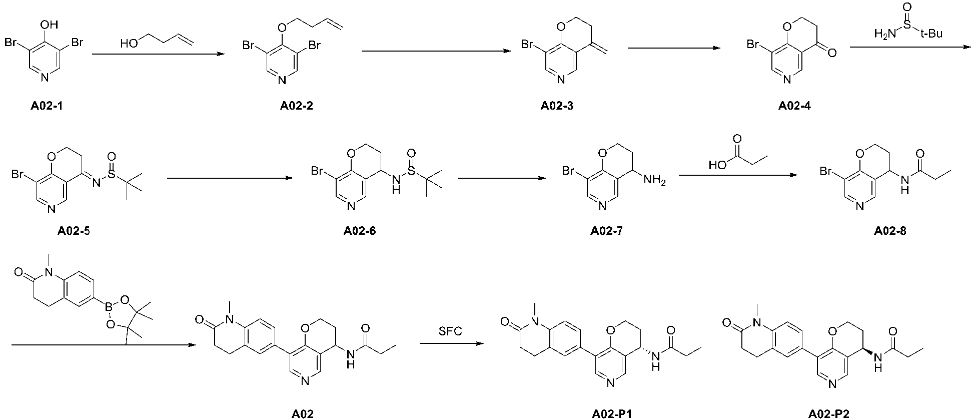

### Step 1: Synthesis of Compound A02-2

At 0 °C under nitrogen protection, diisopropyl azodicarboxylate (11.99 g, 59.35 mmol) was added to a solution of compound **A02-1** (10.0 g, 39.53 mmol), but-3-en-1-ol (3.4 g, 47.22 mmol), and triphenylphosphine (15.55 g, 59.35 mmol) in tetrahydrofuran (100 mL). The mixture was stirred at room temperature for 16 hours. After the reaction was completed, the mixture was concentrated, and the residue was separated and purified by column chromatography to obtain compound **A02-2** (7.00 g).

MS (ESI) m/z: 305.9, 307.9, 309.9 [M+H]⁺.

### Step 2: Synthesis of Compound A02-3

At room temperature under nitrogen protection, triphenylphosphine (1.28 g, 4.89 mmol), palladium acetate (0.37 g, 1.63 mmol), tetraethylammonium bromide (2.16 g, 13.03 mmol), and potassium acetate (7.99 g, 81.45 mmol) were added to a solution of compound **A02-2** (5.00 g, 16.29 mmol) in anhydrous N,N-dimethylformamide (220 mL). The mixture was stirred at 105 °C for 16 hours. After the reaction was completed, the mixture was diluted with water (100 mL) and extracted with ethyl acetate (200 mL × 2). The combined organic phases were dried over anhydrous sodium sulfate, filtered, and concentrated. The residue was separated and purified by column chromatography to obtain compound **A02-3** (2.00 g).

MS (ESI) m/z: 226.0, 228.0 [M+H]⁺.

### Step 3: Synthesis of Compound A02-4

At room temperature, sodium periodate (9.46 g, 44.25 mmol) and potassium osmate dihydrate (0.33 g, 0.89 mmol) were added to a solution of compound **A02-3** (2.00 g, 8.85 mmol) in tetrahydrofuran (15 mL)/water (15 mL). The mixture was stirred at room temperature for 16 hours. After the reaction was completed, the mixture was diluted with water (200 mL) and extracted with ethyl acetate (200 mL × 2). The combined organic phases were dried over anhydrous sodium sulfate, filtered, and concentrated. The residue was separated and purified by column chromatography to obtain compound **A02-4** (1.19 g).

MS (ESI) m/z: 228.0, 230.0 [M+H]⁺.

### Step 4: Synthesis of Compound A02-5

At room temperature, titanium(IV) ethoxide (1.00 g, 4.38 mmol) was added to a solution of compound **A02-4** (500 mg, 2.19 mmol) and tert-butylsulfinamide (400 mg, 0.328 mmol) in tetrahydrofuran (10 mL). The reaction mixture was stirred at 65 °C under nitrogen protection for 16 hours. After the reaction was completed, the mixture was concentrated under reduced pressure. The residue was purified by column chromatography to obtain compound **A02-5** (545 mg).

MS (ESI) m/z: 330.9, 332.9 [M+H]⁺.

### Step 5: Synthesis of Compound A02-6

At 0 °C under nitrogen protection, borane tetrahydrofuran solution (1 M, 7.5 mL, 7.5 mmol) was added to a solution of compound **A02-5** (500 mg, 1.51 mmol) in tetrahydrofuran (10 mL). The reaction mixture was stirred at 0 °C under nitrogen protection for 4 hours. After the reaction was completed, the reaction mixture was quenched with methanol (20 mL). The mixture was concentrated, and the residue was purified by column chromatography to obtain crude compound **A02-6** (495 mg).

MS (ESI) m/z: 333.0, 335.0 [M+H]⁺.

### Step 6: Synthesis of Compound A02-7

At room temperature, hydrochloric acid-methanol solution (4 M, 6.2 mL, 24.80 mmol) was added to a solution of compound **A02-6** (445 mg, 0.64 mmol) in dioxane (18 mL). The mixture was stirred at room temperature for 5 hours. After the reaction was completed, the reaction mixture was quenched with ammonia-methanol (200 mL). The mixture was concentrated, and the residue was purified by column chromatography to obtain compound **A02-7** (230 mg). MS (ESI) m/z: 229.0, 231.0 [M+H]⁺.

### Step 7: Synthesis of Compound A02-8

At room temperature under nitrogen protection, propionic acid (120 mg, 1.66 mmol), 2-(7-aza-1H-benzotriazol-1-yl)-1,1,3,3-tetramethyluronium hexafluorophosphate (630 mg, 1.66 mmol), and ethyldiisopropylamine (540 mg, 4.15 mmol) were added to a solution of compound **A02-7** (190 mg, 0.83 mmol) in DMF (3 mL). The mixture was stirred at room temperature under nitrogen protection for 2 hours. After the reaction was completed, the reaction mixture was poured into water (50 mL) and extracted with ethyl acetate (50 mL × 2). The combined organic phases were dried over anhydrous sodium sulfate, filtered, and concentrated. The residue was purified by reverse-phase flash chromatography (chromatography conditions: column: spherical C18, 20-40 µm, 25 g; mobile phase A: contains pure water; mobile phase B: acetonitrile; flow rate: 35 mL/min; gradient: 5% B to 95% B over 30 minutes; detector: 214 nm) to obtain compound **A02-8** (140 mg).

MS (ESI) m/z: 285.0, 287.0 [M+H]⁺.

### Step 8: Synthesis of Compound A02

At room temperature under nitrogen protection, compound 1-methyl-6-(4,4,5,5-tetramethyl-1,3,2-dioxaborolan-2-yl)-3,4-dihydroquinolin-2(1H)-one (140 mg, 0.50 mmol), anhydrous potassium carbonate (69 mg, 0.50 mmol), and tetrakis(triphenylphosphine)palladium (29 mg, 0.025 mmol) were added to a solution of compound **A02-8** (70 mg, 0.25 mmol) in anhydrous ethanol (3 mL)/water (1 mL). The reaction mixture was stirred at 85 °C under nitrogen protection for 16 hours. After the reaction was completed, the reaction mixture was filtered through a membrane and concentrated. The residue was purified by column chromatography to obtain crude **A02** as a brown solid. The crude product was purified by reverse-phase flash chromatography (chromatography conditions: column: spherical C18, 20-40 µm, 25 g; mobile phase A: contains pure water; mobile phase B: acetonitrile; flow rate: 35 mL/min; gradient: 5% B to 95% B over 30 minutes; detector: 214 nm) to obtain compound **A02** (56 mg).

### Step 9: Synthesis of Compounds A02-P1 and A02-P2

Compound **A02** (56 mg) was purified by supercritical fluid chromatography (chromatography conditions: system: Waters SFC 80; column name: DAICELCHIRALPAK^{®}AS; column dimensions: 250 × 25 mm, 10 µm; mobile phase A: supercritical CO₂; mobile phase B: methanol (+0.1% 7.0 mol/L ammonia in methanol); A:B = 70:30; wavelength: 214 nm; flow rate: 50 mL/min; column temperature: room temperature; column pressure: 100 bar; injection volume: 2.0 mL; cycle time: 4.00 min) to obtain compound **A02-P1** (peak 1, 20.51 mg) and compound **A02-P2** (peak 2, 22.34 mg).

**Compound A02-P1:** Peak 1, analyzed by supercritical fluid chromatography (chromatography conditions: system: Waters UPCC (CA 415); column name: DAICELCHIRALCEL^{®}AS; column dimensions: 100 × 3.0 mm × 3.0 µm; mobile phase A: supercritical CO₂; mobile phase B: methanol (0.1% DEA); A:B = 80:20; wavelength: 214 nm; flow rate: 1.5 mL/min; column temperature: 35 °C; column pressure: 1800 psi; injection volume: 1.0 µL; cycle time: 10.0 min). Retention time RT: 2.279 min, ee = 100%.

¹H NMR (400 MHz, DMSO-*d₆*): δ 8.36 (d, J = 8.0 Hz, 1H), 8.26 (s, 1H), 8.22 (s, 1H), 7.40 (d, J = 8.4 Hz, 2.0 Hz, 1H), 7.37 (s, 1H), 7.15 (d, J = 8.4 Hz, 1H), 5.12-5.07 (m, 1H), 4.37-4.23 (m, 2H), 3.28 (s, 3H), 2.91 (t, J = 6.4 Hz, 2H), 2.58 (t, J = 8.0 Hz, 2H), 2.21-2.04 (m, 3H), 1.97-1.90 (m, 1H), 1.05 (t, J = 7.6 Hz, 3H).

MS (ESI) m/z: 366.1 [M+H]⁺.

**A02-P2:** Peak 2, analyzed by supercritical fluid chromatography (chromatography conditions: system: Waters UPCC (CA 415); column name: DAICELCHIRALCEL^{®}AS; column dimensions: 100 × 3.0 mm × 3.0 µm; mobile phase A: supercritical CO₂; mobile phase B: methanol (0.1% DEA); A:B = 80:20; wavelength: 214 nm; flow rate: 1.5 mL/min; column temperature: 35 °C; column pressure: 1800 psi; injection volume: 1.0 µL; cycle time: 10.0 min). Retention time RT: 2.959 min, ee = 98.6%.

¹H NMR (400 MHz, DMSO-d₆): δ 8.36 (d, J = 8.0 Hz, 1H), 8.26 (s, 1H), 8.22 (s, 1H), 7.40 (d, J = 8.4 Hz, 2.0 Hz, 1H), 7.36 (s, 1H), 7.15 (d, J = 8.4 Hz, 1H), 5.12-5.07 (m, 1H), 4.37-4.23 (m, 2H), 3.28 (s, 3H), 2.91 (t, J = 6.8 Hz, 2H), 2.58 (t, J = 8.0 Hz, 2H), 2.21-2.05 (m, 3H), 1.97-1.91 (m, 1H), 1.05 (t, J = 7.6 Hz, 3H).

MS (ESI) m/z: 366.1 [M+H]⁺.

### Example A08

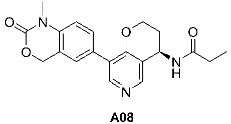

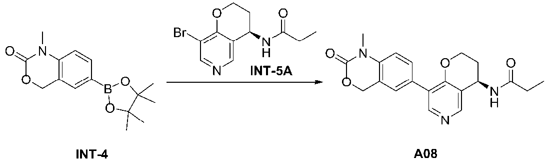

### Step 1: Synthesis of Compound A08

Compound **INT-4** (64 mg, 0.22 mmol), **INT-5A** (30 mg, 0.11 mmol), potassium carbonate (45.61 mg, 0.33 mmol), and 1,1'-bis(diphenylphosphino)ferrocene palladium(II) dichloride (8.05 mg, 0.011 mmol) were dissolved in 1,4-dioxane (5 mL) and water (0.5 mL). The mixture was substituted with nitrogen three times and reacted at 90 °C for 4 hours. The reaction mixture was directly concentrated under reduced pressure and purified by silica gel column chromatography to obtain a crude product. The crude product was purified by reverse-phase flash chromatography (chromatography conditions: column: spherical C18, 20-40 µm, 40 g; mobile phase A: pure water; mobile phase B: acetonitrile; flow rate: 40 mL/min; gradient: 5% B to 95% B over 20 minutes; detector: 214 nm) to obtain compound **A08** (32.17 mg).

**Compound A08:** Analyzed by supercritical fluid chromatography (chromatography conditions: system: Waters UPCC (CA352); column name: DAICELCHIRALCEL^{®}AD; column dimensions: 100 × 3.0 mm, 3.0 µm; mobile phase A: supercritical CO₂; mobile phase B: methanol (0.1% DEA); A:B = 70:30; wavelength: 214 nm; flow rate: 1.5 mL/min; column temperature: 35 °C; column pressure: 1800 psi; injection volume: 1.0 µL; cycle time: 8.0 min). Retention time RT: 2.404 min, ee = 100%.

¹H NMR (400 MHz, DMSO-*d₆*): δ 8.35 (d, J = 8.0 Hz, 1H), 8.26 (s, 1H), 8.23 (s, 1H), 7.51 (dd, J = 8.4 Hz, 1.6 Hz, 1H), 7.42 (d, J = 2.0 Hz, 1H), 7.16 (d, J = 8.4 Hz, 1H), 5.29 (s, 2H), 5.12-5.08 (m, 1H), 4.38-4.33 (m, 1H), 4.29-4.24 (m, 1H), 3.31 (s, 3H), 2.21-2.14 (m, 2H), 2.12-2.04 (m, 1H), 1.97-1.90 (m, 1H), 1.05 (t, J = 7.6 Hz, 3H).

MS (ESI) m/z: 368.2 [M+H]⁺.

### Example A09

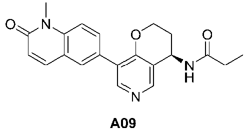

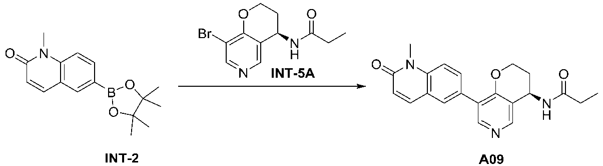

### Step 1: Synthesis of Compound A09

Compound **INT-5A** (30 mg, 0.11 mmol), **INT-2** (38 mg, 0.13 mmol), potassium carbonate (46 mg, 0.33 mmol), and 1,1'-bis(diphenylphosphino)ferrocene palladium(II) dichloride (8.05 mg, 0.011 mmol) were added to 1,4-dioxane (5 mL) and water (0.5 mL). The mixture was substituted with nitrogen three times and reacted at 90 °C for 4 hours. The reaction mixture was directly concentrated under reduced pressure and purified by silica gel column chromatography to obtain a crude product. The crude product was purified by reverse-phase flash chromatography (chromatography conditions: column: spherical C18, 20-40 µm, 40 g; mobile phase A: pure water; mobile phase B: acetonitrile; flow rate: 40 mL/min; gradient: 5% B to 95% B over 20 minutes; detector: 214 nm) to obtain compound **A09** (9.42 mg).

**Compound A09:** Analyzed by supercritical fluid chromatography (chromatography conditions: system: Waters UPCC (CA352); column name: DAICELCHIRALCEL^{®}AD; column dimensions: 100 × 3.0 mm, 3.0 µm; mobile phase A: supercritical CO₂; mobile phase B: methanol (0.1% DEA); A:B = 70:30; wavelength: 214 nm; flow rate: 1.5 mL/min; column temperature: 35 °C; column pressure: 1800 psi; injection volume: 1.0 µL; cycle time: 8.0 min). Retention time RT: 2.020 min, ee = 100%.

¹H NMR (400 MHz, CDCl₃) δ 8.37 (s, 1H), 8.32 (s, 1H), 7.69-7.64 (m, 2H), 7.61 (d, J = 2.0 Hz, 1H), 7.39 (d, J = 8.8 Hz, 1H), 6.70 (d, J = 9.2 Hz, 1H), 6.20 (d, J = 7.2 Hz, 1H), 5.32-5.28 (m, 1H), 4.42-4.37 (m, 1H), 4.32-4.26 (m, 1H), 3.72 (s, 3H), 2.36-2.30 (m, 2H), 2.27-2.23 (m, 1H), 2.20-2.13 (m, 1H), 1.24 (t, J = 7.6 Hz, 3H).

MS (ESI) m/z: 364.2 [M+H]⁺.

### Example A10-P1 and A10-P2

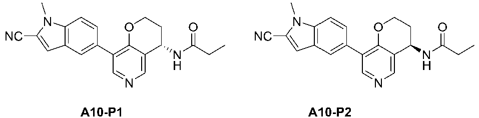

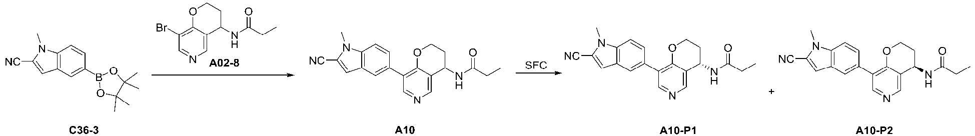

### Step 1: Synthesis of Compound A10

Compound **A02-8** (50 mg, 0.18 mmol), **C36-3** (127 mg, 0.45 mmol), potassium carbonate (75 mg, 0.54 mmol), and 1,1'-bis(diphenylphosphino)ferrocene palladium(II) dichloride (14 mg, 0.018 mmol) were added to a mixed solvent of 1,4-dioxane (5 mL) and water (0.5 mL). The mixture was substituted with nitrogen three times and reacted at 90 °C for 16 hours. The reaction mixture was directly concentrated under reduced pressure and purified by silica gel column chromatography to obtain compound **A10** (61 mg).

MS (ESI) m/z: 361.1 [M+H]⁺.

### Step 2: Synthesis of Compounds A10-P1 and A10-P2

Compound **A10** (61 mg) was purified by supercritical fluid chromatography (chromatography conditions: system: Waters SFC 150; column name: DAICEL CHIRALPAK^{®} IA; column dimensions: 250 × 25 mm, 10 µm; mobile phase A: supercritical CO₂; mobile phase B: ethanol (+0.1% 7.0 mol/L ammonia in methanol); A:B = 60:40; wavelength: 214 nm; flow rate: 120 mL/min; column temperature: room temperature; column pressure: 100 bar; injection volume: 3.0 mL; cycle time: 9.42 min) to obtain compound A10-P1 (peak 1, 12.83 mg) and compound **A10-P2** (peak 2, 13.19 mg).

**Compound A10-P1:** Peak 1, analyzed by supercritical fluid chromatography (chromatography conditions: system: Waters UPCC (CA-352); column name: DAICELCHIRALCEL^{®}MIC; column dimensions: 100 × 3.0 mm, 3.0 µm; mobile phase A: supercritical CO₂; mobile phase B: methanol (0.1% DEA); A:B = 70:30; wavelength: 214 nm; flow rate: 1.5 mL/min; column temperature: 35 °C; column pressure: 1800 psi; injection volume: 1.0 µL; cycle time: 8.0 min). Retention time RT: 3.050 min, ee = 100%.

¹H NMR (400 MHz, DMSO-*d₆*) δ 8.37 (d, J = 8.0 Hz, 1H), 8.30 (s, 1H), 8.24 (s, 1H), 7.80 (d, J = 1.2 Hz, 1H), 7.68 (d, J = 8.8 Hz, 1H), 7.55 (dd, J = 8.8 Hz, 1.6 Hz, 1H), 7.47 (d, J = 0.8 Hz, 1H), 5.14-5.09 (m, 1H), 4.38-4.24 (m, 2H), 3.93 (s, 3H), 2.20-2.07 (m, 3H), 1.98-1.91 (m, 1H), 1.06 (t, J = 7.6 Hz, 3H).

MS (ESI) m/z: 361.1 [M+H]⁺.

**Compound A10-P2:** Peak 2, analyzed by supercritical fluid chromatography (chromatography conditions: system: Waters UPCC (CA-352); column name: DAICELCHIRALCEL^{®}MIC; column dimensions: 100 × 3.0 mm, 3.0 µm; mobile phase A: supercritical CO₂; mobile phase B: methanol (0.1% DEA); A:B = 70:30; wavelength: 214 nm; flow rate: 1.5 mL/min; column temperature: 35 °C; column pressure: 1800 psi; injection volume: 1.0 µL; cycle time: 8.0 min). Retention time RT: 3.992 min, ee = 98.5%.

¹H NMR (400 MHz, DMSO-*d₆*) δ 8.38 (d, J = 7.6 Hz, 1H), 8.30 (s, 1H), 8.24 (s, 1H), 7.80 (d, J = 0.8 Hz, 1H), 7.68 (d, J = 8.8 Hz, 1H), 7.55 (dd, J = 8.8 Hz, 1.6 Hz, 1H), 7.47 (s, 1H), 5.14-5.09 (m, 1H), 4.37-4.25 (m, 2H), 3.93 (s, 3H), 2.20-2.07 (m, 3H), 1.98-1.91 (m, 1H), 1.06 (t, J = 7.6 Hz, 3H).

MS (ESI) m/z: 361.1 [M+H]⁺.

### Example A13

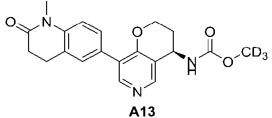

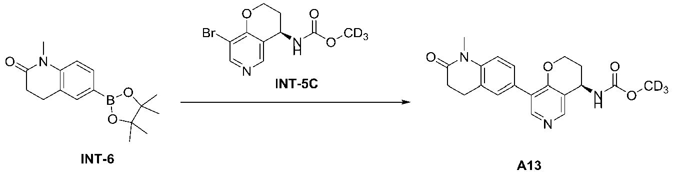

### Step 1: Synthesis of Compound A13

At room temperature, potassium carbonate (41.46 mg, 0.30 mmol) and tetrakis(triphenylphosphine)palladium (11.56 mg, 0.01 mmol) were added to a solution of compound INT-5C (30 mg, 0.10 mmol) and compound **INT-6** (43 mg, 0.15 mmol) in ethanol (2 mL) and water (0.5 mL). The mixture was stirred at 85 °C under nitrogen protection for 16 hours. The reaction mixture was concentrated under reduced pressure. The residue was purified by HPLC to obtain compound A13 (17.46 mg).

**Compound A13:** Analyzed by supercritical fluid chromatography (chromatography conditions: system: Waters UPCC (CA 415); column name: DAICEL CHIRALCEL^{®}OD; column dimensions: 100 × 3.0 mm, 3.0 µm; mobile phase A: supercritical CO₂; mobile phase B: methanol (0.1% DEA); gradient: B 5% to 40%; wavelength: 214 nm; flow rate: 1.5 mL/min; column temperature: 35 °C; column pressure: 1800 psi; injection volume: 1.00 µL; cycle time: 8.0 min). Retention time RT: 4.022 min, ee = 100%.

¹H NMR (400 MHz, DMSO-*d₆*) δ 8.26 (s, 1H), 8.25 (s, 1H), 7.79 (d, J = 8.0 Hz, 1H), 7.40 (dd, J = 8.4 Hz, 1.0 Hz, 1H), 7.36 (d, J = 1.6 Hz, 1H), 7.15 (d, J = 8.4 Hz, 1H), 4.91-4.81 (m, 1H), 4.38-4.23 (m, 2H), 3.28 (s, 3H), 2.91 (d, J = 6.8 Hz, 2H), 2.57 (d, J = 7.6 Hz, 2H), 2.15-2.04 (m, 1H), 2.02-1.91 (m, 1H).

MS (ESI) m/z: 371.1 [M+H]⁺.

### Example A15

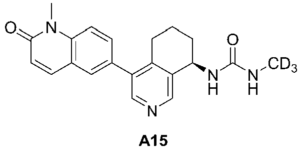

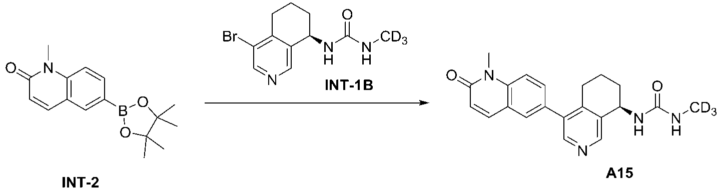

### Step 1: Synthesis of Compound A15

Under nitrogen protection, potassium carbonate (36 mg, 0.261 mmol) and [1,1'-bis(diphenylphosphino)ferrocene]palladium(II) dichloride dichloromethane complex (8 mg, 0.011 mmol) were added to a mixture of compound INT-2 (62 mg, 0.217 mmol) in dioxane and water (4/0.4 mL). Compound **INT-1B** (25 mg, 0.087 mmol) was then added. The reaction mixture was stirred at 90 °C under nitrogen protection for 16 hours. The reaction mixture was filtered and concentrated under reduced pressure. The residue was purified by column chromatography (dichloromethane/methanol = 10/1) to obtain 25 mg of crude product. The crude product was further purified by Prep-HPLC (separation conditions: preparative column: Waters sunfire C18 10µm OBD 19 × 250 mm; mobile phase: 0.1% FA aqueous solution/CH₃CN; flow rate: 20 mL/min; gradient: 13%-25%; detection wavelength: 214 nm/254 nm; cycle time: 16 min) to obtain compound **A15** (7.78 mg).

**Compound A15:** Analyzed by supercritical fluid chromatography (chromatography conditions: system: Waters UPCC (CA352); column name: DAICELCHIRALCEL^{®}OJ; column dimensions: 100 × 3.0 mm, 3.0 µm; mobile phase A: supercritical CO₂; mobile phase B: methanol (0.1% DEA); A:B = 60:40; wavelength: 214 nm; flow rate: 1.5 mL/min; column temperature: 35 °C; column pressure: 1800 psi; injection volume: 1.0 µL; cycle time: 8.0 min). Retention time RT: 2.878 min, ee = 100%.

¹H NMR (400 MHz, DMSO- *d₆*) δ 8.44 (s, 1H), 8.26 (s, 1H), 7.95 (d, J = 9.6 Hz, 1H), 7.75 (s, 1H), 7.65-7.60 (m, 2H), 6.68 (d, J = 9.2 Hz, 1H), 6.44 (d, J = 8.8 Hz, 1H), 5.65 (s, 1H), 4.94-4.90 (m, 1H), 3.66 (s, 3H), 2.63-2.59 (m, 2H), 1.92-1.86 (m, 1H), 1.76-1.66 (m, 3H).

MS (ESI) m/z: 366.1 [M+H]⁺.

### Example A16

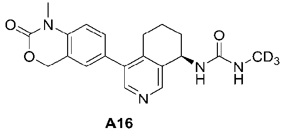

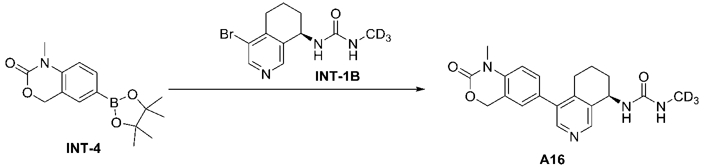

### Step 1: Synthesis of Compound A16

Under nitrogen protection, compound **INT-1B** (50 mg, 0.174 mmol), potassium carbonate (36 mg, 0.261 mmol), and [1,1'-bis(diphenylphosphino)ferrocene]palladium(II) dichloride dichloromethane complex (8 mg, 0.009 mmol) were added to a mixture of compound INT-4 (25 mg, 0.087 mmol) in dioxane and water (4/0.4 mL). The reaction mixture was stirred at 90 °C under nitrogen protection for 16 hours. The reaction mixture was filtered, and the filtrate was concentrated under reduced pressure. The residue was purified by column chromatography to obtain 25 mg of crude product. The crude product was further purified by Prep-HPLC (separation conditions: preparative column: Waters sunfire C18 10µm OBD 19 × 250 mm; mobile phase: 0.1% FA aqueous solution/CH₃CN; flow rate: 20 mL/min; gradient: 13%-20%; detection wavelength: 214 nm/254 nm; cycle time: 16 min) to obtain compound **A16** (8.13 mg).

**Compound A16:** Analyzed by supercritical fluid chromatography (chromatography conditions: system: Waters UPCC (CA352); column name: DAICELCHIRALCEL^{®}OJ; column dimensions: 100 × 3.0 mm, 3.0 µm; mobile phase A: supercritical CO₂; mobile phase B: methanol (0.1% DEA); A:B = 60:40; wavelength: 214 nm; flow rate: 1.5 mL/min; column temperature: 35 °C; column pressure: 1800 psi; injection volume: 1.0 µL; cycle time: 8.0 min). Retention time RT: 3.062 min, ee = 100%.

¹H NMR (400 MHz, DMSO-*d₆*) δ 8.41 (s, 1H), 8.19 (s, 1H), 7.36 (dd, J = 8.4 Hz, 2.0 Hz, 1H), 7.29 (d, J = 1.6 Hz, 1H), 7.18 (d, J = 8.4 Hz, 1H), 6.42 (d, J = 8.8 Hz, 1H), 5.63 (s, 1H), 5.29 (s, 2H), 4.92-4.89 (m, 1H), 3.31 (s, 3H), 2.60-2.56 (m, 2H), 1.88-1.86 (m, 1H), 1.74-1.67 (m, 3H).

MS (ESI) m/z: 370.1 [M+H]⁺.

### Example A17

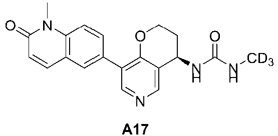

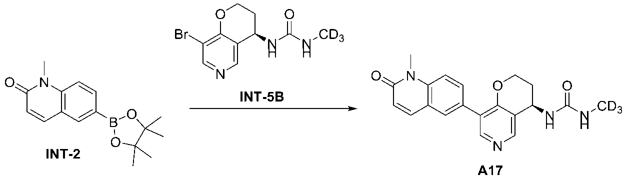

### Step 1: Synthesis of Compound A17

Under nitrogen protection, compound **INT-2** (80 mg, 0.277 mmol), potassium carbonate (57 mg, 0.415 mmol), and [1,1'-bis(diphenylphosphino)ferrocene]palladium(II) dichloride dichloromethane complex (10 mg, 0.014 mmol) were added to a solution of compound **INT-5B** (40 mg, 0.138 mmol) in dioxane and water (4/0.4 mL). The reaction mixture was stirred at 90 °C under nitrogen protection for 16 hours. The reaction mixture was filtered, and the filtrate was concentrated under reduced pressure. The residue was purified by column chromatography to obtain 25 mg of crude product. The crude product was further purified by Prep-HPLC (separation conditions: preparative column: Waters sunfire C18 10µm OBD 19 × 250 mm; mobile phase: 0.1% FA aqueous solution/CH₃CN; flow rate: 20 mL/min; gradient: 13%-25%; detection wavelength: 214 nm/254 nm; cycle time: 16 min) to obtain compound **A17** (13.14 mg).

**Compound A17:** Analyzed by supercritical fluid chromatography (chromatography conditions: system: Waters UPCC (CA352); column name: DAICELCHIRALCEL^{®}OJ; column dimensions: 100 × 3.0 mm, 3.0 µm; mobile phase A: supercritical CO₂; mobile phase B: methanol (0.1% DEA); A:B = 60:40; wavelength: 214 nm; flow rate: 1.5 mL/min; column temperature: 35 °C; column pressure: 1800 psi; injection volume: 1.0 µL; cycle time: 8.0 min). Retention time RT: 2.915 min, ee = 100%.

¹H NMR (400 MHz, DMSO-*d₆*) δ 8.33 (s, 1H), 8.31 (s, 1H), 7.96 (d, J = 9.6 Hz, 1H), 7.88 (d, J = 2.0 Hz, 1H), 7.79 (dd, J = 8.8 Hz, 2.0 Hz, 1H), 7.60 (d, J = 8.8 Hz, 1H), 6.65 (d, J = 9.6 Hz, 1H), 6.58 (d, J = 8.0 Hz, 1H), 5.71 (s, 1H), 4.96-4.91 (m, 1H), 4.39-4.34 (m, 1H), 4.29-4.23 (m, 1H), 3.65 (s, 3H), 2.11-2.04 (m, 1H), 2.00-1.95 (m, 1H).

MS (ESI) m/z: 368.1 [M+H]⁺.

### Example A18

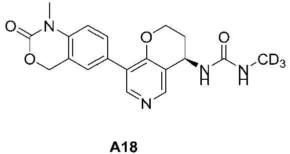

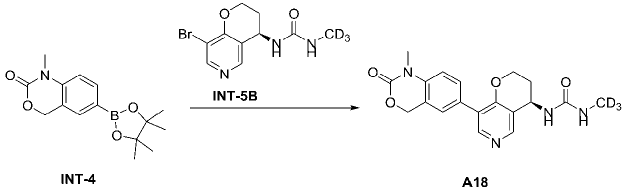

### Step 1: Synthesis of Compound A18

Under nitrogen protection, compound **INT-4** (80 mg, 0.277 mmol), potassium carbonate (57 mg, 0.415 mmol), and [1,1'-bis(diphenylphosphino)ferrocene]palladium(II) dichloride dichloromethane complex (10 mg, 0.014 mmol) were added to a mixture of compound **INT-5B** (40 mg, 0.138 mmol) in dioxane and water (4/0.4 mL). The reaction mixture was stirred at 90 °C under nitrogen protection for 16 hours. The reaction mixture was filtered, and the filtrate was concentrated under reduced pressure. The residue was purified by column chromatography to obtain 27 mg of crude product. The crude product was further purified by Prep-HPLC (separation conditions: preparative column: Waters sunfire C18 10µm OBD 19 × 250 mm; mobile phase: 0.1% FA aqueous solution/CH₃CN; flow rate: 20 mL/min; gradient: 13%-20%; detection wavelength: 214 nm/254 nm; cycle time: 16 min) to obtain compound **A18** (8.13 mg).

**Compound A18:** Analyzed by supercritical fluid chromatography (chromatography conditions: system: Waters UPCC (CA352); column name: DAICELCHIRALCEL^{®}OJ; column dimensions: 100 × 3.0 mm, 3.0 µm; mobile phase A: supercritical CO₂; mobile phase B: methanol (0.1% DEA); A:B = 60:40; wavelength: 214 nm; flow rate: 1.5 mL/min; column temperature: 35 °C; column pressure: 1800 psi; injection volume: 1.0 µL; cycle time: 8.0 min). Retention time RT: 3.140 min, ee = 100%.

¹H NMR (400 MHz, DMSO-*d₆*) δ 8.28 (s, 1H), 8.25 (s, 1H), 7.51 (dd, J = 8.4 Hz, 2.0 Hz, 1H), 7.42 (d, J = 1.6 Hz, 1H), 7.16 (d, J = 8.4 Hz, 1H), 6.59 (d, J = 7.6 Hz, 1H), 5.72 (s, 1H), 5.29 (s, 2H), 4.94-4.89 (m, 1H), 4.37-4.32 (m, 1H), 4.27-4.21 (m, 1H), 3.31 (s, 3H), 2.10-2.02 (m, 1H), 1.99-1.91 (m, 1H).

MS (ESI) m/z: 372.1 [M+H]⁺.

### Example C04

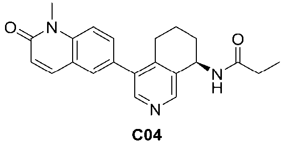

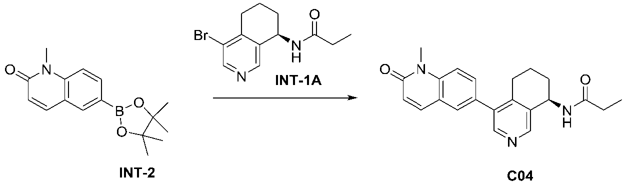

### Step 1: Synthesis of Compound C04

At room temperature, compound **INT-2** (20 mg, 0.07 mmol) and compound **INT-1A** (24 mg, 0.084 mmol) were dissolved in 1,4-dioxane (10 mL). Nitrogen was bubbled through the mixture for 5 minutes, and then tetrakis(triphenylphosphine)palladium (8.1 mg, 0.007 mmol) was added. Sodium carbonate (8.9 mg, 0.084 mmol) in water (3 mL) was added to the system. The system was thoroughly substituted with N₂ and then reacted at 85 °C for 1 hour. The reaction mixture was cooled to room temperature, diluted with water (30 mL), and extracted with ethyl acetate (30 mL × 3). The combined organic phases were dried over anhydrous sodium sulfate, filtered, and concentrated. The residue was purified by Prep-HPLC (YMC Triart 20 × 250 mm, 5 µm, water (0.01% formic acid)/acetonitrile, 10% to 100%, 20 mL/min) to obtain compound **C04** (12 mg).

**Compound C04:** ¹H NMR (400 MHz, DMSO-*d₆*) δ 8.36 (s, 1H), 8.30-8.28 (m, 2H), 7.96 (d, J = 9.5 Hz, 1H), 7.75 (s, 1H), 7.63 (s, 2H), 6.68 (d, J = 9.4 Hz, 1H), 5.11 (q, J = 6.5 Hz, 1H), 3.67 (s, 3H), 2.68-2.59 (m, 2H), 2.16 (qd, J = 7.4, 4.7 Hz, 2H), 1.92-1.65 (m, 4H), 1.06 (t, J = 7.6 Hz, 3H).

MS (ESI) M/Z: 362.2 [M+H]⁺.

### Example C06

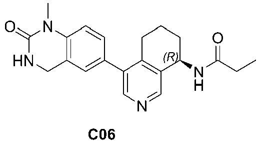

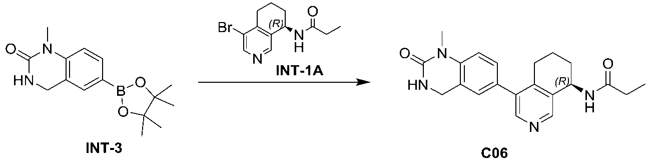

### Step 1: Synthesis of Compound C06

Compound **INT-3** (20 mg, 0.069 mmol) and compound **INT-1A** (23 mg, 0.083 mmol) were dissolved in 1,4-dioxane (10 mL). Tetrakis(triphenylphosphine)palladium (8 mg, 0.0069 mmol) was added, and then sodium carbonate (8.8 mg, 0.083 mmol) in water (3 mL) was added to the system. The system was thoroughly substituted with nitrogen and then reacted at 85 °C under a nitrogen atmosphere for 1 hour. The reaction mixture was extracted with ethyl acetate (50 mL) and water (30 mL). The organic phase was washed with saturated sodium chloride (20 mL) and dried over anhydrous sodium sulfate, filtered, and the filtrate was concentrated under reduced vacuum to obtain the crude product. The crude material was purified by Prep-HPLC (YMC Triart 20 × 250 mm, 5 µm, water (0.01% formic acid)/acetonitrile, 10% to 100%, 20 mL/min) to obtain compound **C06** (15 mg).

**Compound C06:** ¹H NMR (400 MHz, DMSO-*d₆*) δ 8.32 (s, 1H), 8.28 (d, J = 8.5 Hz, 1H), 8.20 (s, 1H), 7.24 (dd, J = 8.2, 2.1 Hz, 1H), 7.17 (d, J = 2.1 Hz, 1H), 7.13 (s, 1H), 7.02 (d, J = 8.3 Hz, 1H), 5.09 (q, J = 6.3 Hz, 1H), 4.33 (d, J = 2.0 Hz, 2H), 3.20 (s, 3H), 2.64-2.57 (m, 2H), 2.16 (qd, J = 7.4, 4.6 Hz, 2H), 1.93-1.64 (m, 4H), 1.06 (t, J = 7.6 Hz, 3H).

MS (ESI) M/Z: 365.1 [M+H]⁺.

### Example C07

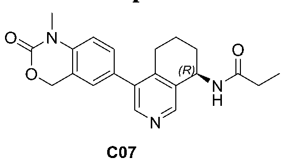

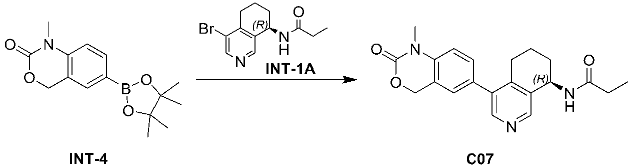

### Step 1: Synthesis of Compound C07

Compound **INT-4** (20 mg, 0.069 mmol) and compound **INT-1A** (23 mg, 0.083 mmol) were dissolved in 1,4-dioxane (5 mL). Tetrakis(triphenylphosphine)palladium (8 mg, 0.0069 mmol) was added, and then sodium carbonate (8.8 mg, 0.083 mmol) in water (3 mL) was added to the system. The system was thoroughly substituted with nitrogen and then reacted at 85 °C under a nitrogen atmosphere for 1 hour. The reaction mixture was extracted with ethyl acetate (50 mL) and water (30 mL). The organic phase was washed with saturated sodium chloride (20 mL) and dried over anhydrous sodium sulfate, filtered, and the filtrate was concentrated under reduced vacuum to obtain the crude product. The crude material was purified by Prep-HPLC (YMC Triart 20 × 250 mm, 5 µm, water (0.01% formic acid)/acetonitrile, 10% to 100%, 20 mL/min) to obtain compound **C07** (20 mg).

**Compound C07:** ¹H NMR (400 MHz, DMSO-*d₆*) δ 8.33 (s, 1H), 8.27 (d, J = 8.4 Hz, 1H), 8.21 (s, 1H), 7.36 (dd, J = 8.3, 2.1 Hz, 1H), 7.29 (d, J = 2.0 Hz, 1H), 7.19 (d, J = 8.3 Hz, 1H), 5.30 (s, 2H), 5.13-5.06 (m, 1H), 3.32 (s, 3H), 2.61-2.58 (m, 2H), 2.15 (qd, J = 7.5, 4.7 Hz, 2H), 1.93-1.64 (m, 4H), 1.05 (t, J = 7.6 Hz, 3H).

MS (ESI) M/Z: 366.2 [M+H]⁺.

### Example C36

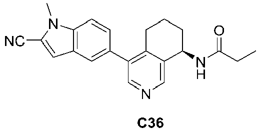

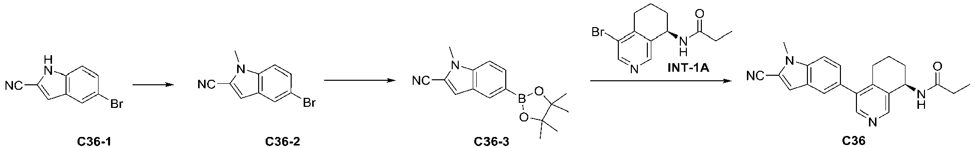

### Step 1: Synthesis of Compound C36-2

Compound **C36-1** (300 mg, 1.36 mmol) was dissolved in N,N-dimethylformamide (5 mL). 60% sodium hydride (190 mg, 5.44 mmol) was slowly added at 0 °C. After stirring the mixture at room temperature for 0.5 hours, methyl iodide (0.23 g, 1.63 mmol) was slowly added dropwise at 0 °C. The reaction mixture was then allowed to react further at 25 °C for 5 hours. Water (15 mL) was added to dilute the mixture, followed by extraction with ethyl acetate (5 mL) twice. The combined organic phases were dried over anhydrous sodium sulfate, filtered, and concentrated under reduced pressure. The residue was purified by silica gel column chromatography to obtain compound **C36-2** (306 mg).

¹H NMR (400 MHz, CDCl₃): δ 7.80 (s, 1 H), 7.48 (dd, J = 8.0 Hz, 2.0 Hz, 1 H), 7.24 (d, J = 8.8 Hz, 1 H), 7.08 (s, 1 H), 3.90 (s, 3 H).

### Step 2: Synthesis of Compound C36-3

Compound **C36-2** (400 mg, 1.70 mmol), bis(pinacolato)diboron (1079 mg, 4.25 mmol), potassium acetate (500 mg, 5.1 mmol), and [1,1'-bis(diphenylphosphino)ferrocene]palladium(II) dichloride (124 mg, 0.17 mmol) were added to 1,4-dioxane (5 mL). The mixture was reacted at 90 °C under nitrogen protection for 4 hours. The reaction mixture was directly concentrated under reduced pressure and purified by silica gel column chromatography to obtain compound **C36-3** (450 mg).

¹H NMR (400 MHz, CDCl₃): δ 8.18 (s, 1 H), 7.82 (d, J = 8.4 Hz, 1 H), 7.33 (d, J = 8.4 Hz, 1 H), 7.26 (s, 1 H), 7.18 (s, 1 H), 3.91 (s, 3 H), 1.37 (s, 12 H).

### Step 3: Synthesis of Compound C36

Compound **INT-1A** (30 mg, 0.11 mmol), **C36-3** (78 mg, 0.28 mmol), potassium carbonate (46 mg, 0.33 mmol), and [1,1'-bis(diphenylphosphino)ferrocene]palladium(II) dichloride (9 mg, 0.011 mmol) were added to 1,4-dioxane (5 mL) and water (0.5 mL). The mixture was reacted at 90 °C under nitrogen protection for 4 hours. The reaction mixture was directly concentrated under reduced pressure and purified by silica gel column chromatography to obtain the crude product. The crude product was purified by reversed-phase flash chromatography (chromatography conditions: column: spherical C18, 20-40 µm, 40 g; mobile phase A: pure water; mobile phase B: acetonitrile; flow rate: 40 mL/min; gradient: 5% B to 95% B in 20 minutes; detector: 214 nm) to obtain compound **C36** (10.77 mg).

**Compound C36:** Analyzed by supercritical fluid chromatography (chromatography conditions: system: Waters UPCC (CA-352); column name: DAICELCHIRALCEL^{®}AD; column dimensions: 100 × 3.0 mm, 3.0 µm; mobile phase A: supercritical CO₂; mobile phase B: methanol (0.1% DEA), A:B = 60:40; wavelength: 214 nm; flow rate: 1.5 mL/min; column temperature: 35°C; column pressure: 1800 psi; injection volume: 1.0 µL; cycle time: 8.0 min); retention time RT: 1.824 min, ee = 100%.

¹H NMR (400 MHz, CDCl₃) δ 8.52 (s, 1 H), 8.32 (s, 1 H), 7.55 (s, 1 H), 7.43 (d, J = 8.8 Hz, 1 H), 7.31 (d, J = 8.8 Hz, 1 H), 7.18 (s, 1 H), 5.86 (d, J = 8.0 Hz, 1 H), 5.38-5.33 (m, 1 H), 3.96 (s, 3 H), 2.68-2.56 (m, 2 H), 2.29 (q, J = 7.6 Hz, 2 H), 2.11-2.06 (m, 1 H), 1.88-1.78 (m, 3 H), 1.23 (t, J = 7.6 Hz, 3 H).

MS (ESI) M/Z: 359.2 [M+H]⁺.

### Example D01

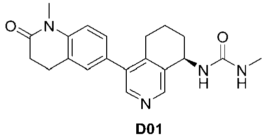

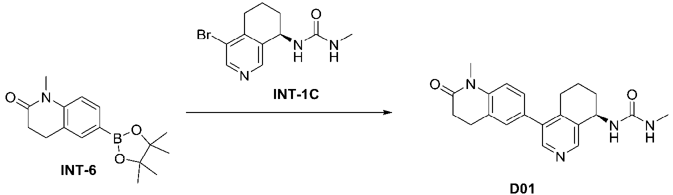

### Step 1: Synthesis of Compound D01

At room temperature, potassium carbonate (63.58 mg, 0.46 mmol) and tetrakis(triphenylphosphine)palladium (26.58 mg, 0.023 mmol) were added to a solution of compound **INT-1C** (65 mg, 0.23 mmol) and compound **INT-6** (132.09 mg, 0.46 mmol) in ethanol (5 mL) and water (1 mL). The resulting mixture was stirred and heated at 85 °C under a nitrogen atmosphere for 16 hours. The resulting reaction mixture was concentrated under reduced pressure. The residue was purified by silica gel column chromatography to obtain the crude product. The crude product was purified by Prep-HPLC (separation conditions: preparative column: Waters sunfire C18 10µm OBD 19 × 250mm; mobile phase: 0.1% FA aqueous solution/CH₃CN; flow rate: 20 mL/min; gradient: 13% to 13%; detection wavelength: 214 nm/254 nm; cycle time: 16 min) to obtain compound D01 (28.41 mg).

**Compound D01:** Analyzed by supercritical fluid chromatography (chromatography conditions: system: Waters UPCC (CA-060); column name: DAICELCHIRALCEL^{®}OJ; column dimensions: 100 × 3.0 mm, 3.0 µm; mobile phase A: supercritical CO₂; mobile phase B: methanol (0.1% DEA); gradient: 5% B to 40% B; wavelength: 214 nm; flow rate: 1.5 mL/min; column temperature: 35°C; column pressure: 1800 psi; injection volume: 1.0 µL; cycle time: 8.0 min); retention time RT: 2.744 min, ee = 94.7%.

¹H NMR (400 MHz, DMSO-*d₆*) δ 8.40 (s, 1H), 8.18 (s, 1H), 7.26-7.23 (m, 2H), 7.17 (d, J = 8.0 Hz, 1H), 6.44 (d, J = 8.8 Hz, 1H), 5.71-5.68 (m, 1H), 4.93-4.88 (m, 1H), 3.29 (s, 3H), 2.92 (t, J = 7.2 Hz, 2H), 2.65-2.55 (m, 7H), 1.90-1.84 (m, 1H), 1.74-1.69 (m, 3H).

MS (ESI) M/Z: 365.1 [M+H]⁺.

### Example D05

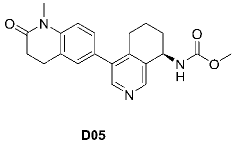

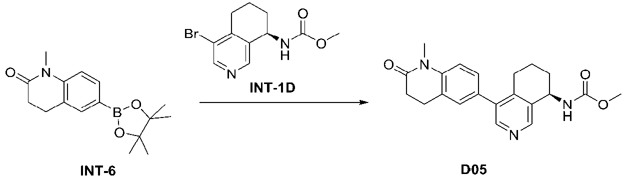

### Step 2: Synthesis of Compound D05

Compound **INT-1D** (30 mg, 0.11 mmol), **INT-6** (61 mg, 0.21 mmol), and anhydrous potassium carbonate (44 mg, 0.32 mmol) were dissolved in ethanol/water (5 mL / 1 mL). Tetrakis(triphenylphosphine)palladium (13 mg, 0.01 mmol) was added at room temperature. After the addition was complete, the mixture was substituted with nitrogen, and the reaction was stirred in a closed system at 85 °C for 12 hours. The reaction mixture was filtered and concentrated under reduced pressure. Saturated brine (50 mL) was added, and the mixture was extracted with ethyl acetate (20 mL) three times. The combined organic phases were dried over anhydrous sodium sulfate and concentrated under reduced pressure. The residue was purified by silica gel column chromatography (petroleum ether:ethyl acetate = 1:1) to obtain the crude product. The crude product was further purified by Prep-HPLC (separation conditions: preparative column: Waters sunfire C18 10µm OBD 19 × 250mm; mobile phase: 0.1% NH₄HCO₃ aqueous solution/CH₃CN; flow rate: 20 mL/min; gradient: 34% to 38%; detection wavelength: 214 nm/254 nm; cycle time: 16 min.) to obtain compound **D05** (20.3 mg).

**Compound D05:** Analyzed by supercritical fluid chromatography (chromatography conditions: system: Waters UPCC (CA352); column name: DAICEL CHIRALCEL^{®}AS; column dimensions: 100 × 3.0 mm, 3.0 µm; mobile phase A: supercritical CO₂; mobile phase B: methanol (0.1% DEA), A:B = 60:40; wavelength: 214 nm; flow rate: 1.5 mL/min; column temperature: 35°C; column pressure: 1800 psi; injection volume: 1.0 µL; cycle time: 8.0 min); retention time RT: 4.549 min, ee = 99.3%.

¹H NMR (400 MHz, CDCl₃) δ 8.57 (s, 1H), 8.29 (s, 1H), 7.19-7.16 (m, 1H), 7.09 (s, 1H), 7.05 (d, J = 8.4 Hz, 1H), 5.03-4.97 (m, 1H), 3.74 (s, 3H), 3.40 (s, 3H), 2.95 (t, J = 6.8 Hz, 2H), 2.71 (t, J = 8 Hz, 2H), 2.67-2.55 (m, 2H), 2.09-2.05 (m, 1H), 1.93-1.84 (m, 1H), 1.82-1.76 (m, 2H).

MS (ESI) M/Z: 366.2 [M+H]⁺.

### Example D08

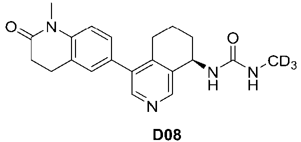

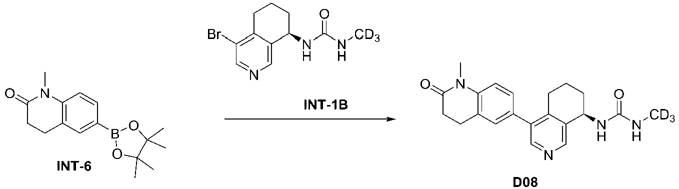

### Step 1: Synthesis of Compound D08

Compound **INT-1B** (40 mg, 0.14 mmol), **INT-6** (80 mg, 0.28 mmol), and anhydrous potassium carbonate (58 mg, 0.42 mmol) were dissolved in ethanol (6 mL) / water (1 mL). Tetrakis(triphenylphosphine)palladium (16 mg, 0.01 mmol) was added. After the reaction was completed of the addition, the mixture was substituted with N₂, and the reaction was stirred at 85 C for 5 hours. The reaction mixture was filtered and concentrated under reduced pressure. Water (100 mL) was added to dilute the mixture, which was then extracted with ethyl acetate (15 mL) three times. The combined organic phases were dried over anhydrous sodium sulfate and concentrated under reduced pressure. The residue was purified by silica gel column chromatography to obtain the crude product. The crude product was further purified by reversed-phase flash chromatography (chromatography conditions: column: spherical C18, 20-40 µm, 40 g; mobile phase A: pure water; mobile phase B: acetonitrile; flow rate: 40 mL/min; gradient: 5% B to 95% B in 20 minutes; detector: 214 nm) to obtain compound **D08** (33.91 mg).

**Compound D08:** Analyzed by supercritical fluid chromatography (chromatography conditions: system: Waters UPCC (CA352); column name: DAICELCHIRALCEL^{®}OD; column dimensions: 100 × 3.0 mm, 3.0 µm; mobile phase A: supercritical CO₂; mobile phase B: methanol (0.1% DEA), A:B = 60:40; wavelength: 214 nm; flow rate: 1.5 mL/min; column temperature: 35°C; column pressure: 1800 psi; injection volume: 0.3 µL; cycle time: 8.0 min); retention time RT: 4.390 min, ee = 98.5%.

¹H NMR (400 MHz, DMSO-*d₆*) δ 8.40 (s, 1 H), 8.18 (s, 1 H), 7.24 (d, J = 8.0 Hz, 1 H), 7.23 (s, 1 H), 7.17 (d, J = 8.0 Hz, 1 H), 6.41 (d, J = 8.4 Hz, 1 H), 5.63 (s, 1 H), 4.93-4.88 (m, 1 H), 3.29 (s, 3 H), 2.92 (t, J = 7.2 Hz, 2 H), 2.62-2.57 (m, 4 H), 1.88-1.86 (m, 1 H), 1.74-1.66 (m, 3 H).

MS (ESI) M/Z: 368.2 [M+H]⁺.

### Example D11

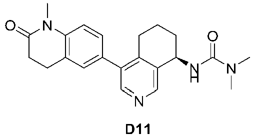

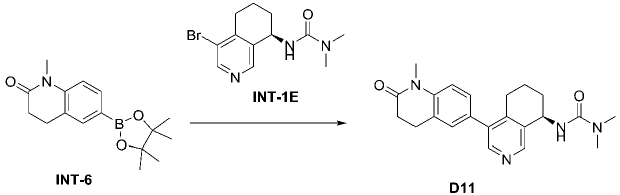

### Step 1: Synthesis of Compound D11

At room temperature, potassium carbonate (49.76 mg, 0.36 mmol) and tetrakis(triphenylphosphine)palladium (20.80 mg, 0.018 mmol) were added to a solution of compound INT-1E (55 mg, 0.18 mmol) and compound **INT-6** (103.38 mg, 0.36 mmol) in ethanol (5 mL) and water (1 mL). The resulting mixture was stirred and heated at 85 C under a nitrogen atmosphere for 16 hours. The resulting reaction mixture was concentrated under reduced pressure. The residue was purified by silica gel column chromatography to obtain the crude product. The crude product was purified by Prep-HPLC (separation conditions: preparative column: Waters sunfire C18 10µm OBD 19 × 250mm; mobile phase: 0.1% FA aqueous solution/CH₃CN; flow rate: 20 mL/min; gradient: 13% to 20%; detection wavelength: 214 nm/254 nm; cycle time: 16 min) to obtain compound **D11** (14.83 mg).

**Compound D11:** Analyzed by supercritical fluid chromatography (chromatography conditions: system: Waters UPCC (CA-352); column name: DAICEL CHIRALCEL^{®}AS; column dimensions: 100 × 3.0 mm, 3.0 µm; mobile phase A: supercritical CO₂; mobile phase B: methanol (0.1% DEA); gradient: 5% B to 40% B; wavelength: 214 nm; flow rate: 1.5 mL/min; column temperature: 35°C; column pressure: 1800 psi; injection volume: 1.0 µL; cycle time: 8.0 min); retention time RT: 4.029 min, ee = 100%.

¹H NMR (400 MHz, DMSO-*d₆*) δ 8.38 (s, 1 H), 8.18 (s, 1 H), 7.25-7.23 (m, 2 H), 7.17 (d, J = 8.4 Hz, 1 H), 6.66 (d, J = 8.4 Hz, 1 H), 5.00-4.95 (m, 1 H), 3.29 (s, 3 H), 2.92 (t, J = 6.8 Hz, 2 H), 2.84 (s, 6 H), 2.66-2.55 (m, 4 H), 1.90-1.72 (m, 3 H), 1.66-1.61 (m, 1 H).

MS (ESI) M/Z: 379.2 [M+H]⁺.

### Example D15

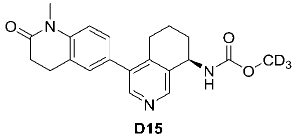

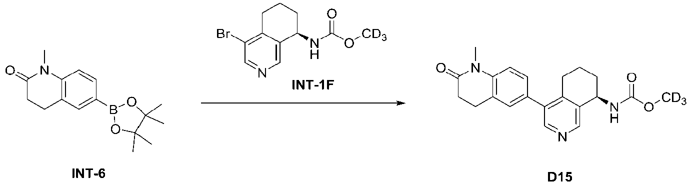

### Step 1: Synthesis of Compound D15

At room temperature, potassium carbonate (26.00 mg, 0.19 mmol) and tetrakis(triphenylphosphine)palladium (7.2 mg, 0.006 mmol) were added to a solution of compound **INT-1F** (26 mg, 0.062 mmol) and compound **INT-6** (27 mg, 0.093 mmol) in ethanol (2 mL) and water (0.5 mL). The resulting mixture was stirred and heated at 85 C under a nitrogen atmosphere for 16 hours. The resulting reaction mixture was concentrated under reduced pressure. The residue was purified by HPLC to obtain compound **D15** (2.67 mg).

**Compound D15:** Analyzed by supercritical fluid chromatography (chromatography conditions: system: Waters UPCC (CA060); column name: DAICEL CHIRALCEL^{®}OJ; column dimensions: 100 × 3.0 mm, 3.0 µm; mobile phase A: supercritical CO₂; mobile phase B: methanol (0.1% DEA); gradient: 5% B to 40% B; wavelength: 214 nm; flow rate: 1.5 mL/min; column temperature: 35°C; column pressure: 1800 psi; injection volume: 1.00 µL; cycle time: 8.0 min); retention time RT: 2.630 min, ee = 100%.

¹H NMR (400 MHz, DMSO-*d₆*) δ 8.39 (s, 1 H), 8.22 (s, 1 H), 7.70 (d, J = 8.4 Hz, 1 H), 7.25 (d, J = 8.4 Hz, 1 H), 7.24 (s, 1 H), 7.17 (d, J = 8.0 Hz, 1 H), 4.87-4.77 (m, 1 H), 3.29 (s, 3 H), 2.92 (t, J = 6.8 Hz, 2 H), 2.69-2.55 (m, 4 H), 1.98-1.87 (m, 1 H), 1.85-1.59 (m, 3 H).

MS (ESI) M/Z: 369.1 [M+H]⁺.

### Example A50

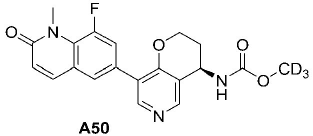

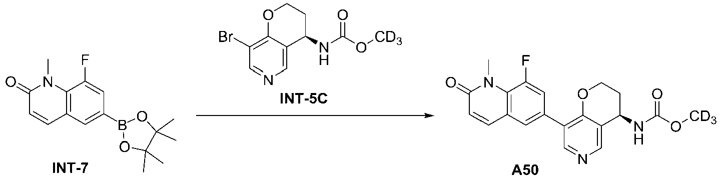

Under a nitrogen atmosphere, compound **INT-7** (63 mg, 0.21 mmol), potassium carbonate (57 mg, 0.41 mmol), and tetrakis(triphenylphosphine)palladium (16 mg, 0.014 mmol) were added to a mixture of compound **INT-5C** (40 mg, 0.14 mmol) in ethanol and water (5 mL/1 mL). The resulting reaction mixture was stirred at 85 °C under nitrogen protection for 8 hours. The resulting reaction mixture was filtered, and the filtrate was concentrated under reduced pressure. The residue was purified by column chromatography to obtain 40 mg of the crude product. The crude product was purified by Prep-HPLC (separation conditions: preparative column: Waters sunfire C18 10µm OBD 19 × 250mm; mobile phase: 0.1% FA aqueous solution/CH₃CN; flow rate: 20 mL/min; gradient: 13% to 25%; detection wavelength: 214 nm/254 nm; retention time: 8.0 to 9.6 min of 16 min) to obtain compound **A50** (16.19 mg).

MS (ESI) M/Z: 387.1 [M+H]+.

**Compound A50:** Analyzed by supercritical fluid chromatography (chromatography conditions: system: Waters UPCC (CA-352); column name: DAICEL CHIRALCEL^{®}AS; column dimensions: 100 × 3.0 mm, 3.0 µm; mobile phase A: supercritical CO₂; mobile phase B: methanol (0.1% DEA); gradient: 5% B to 40% B; wavelength: 214 nm; flow rate: 1.5 mL/min; column temperature: 35 °C; column pressure: 1800 psi; cycle time: 8.0 min); retention time RT: 4.062 min.

¹H NMR (400 MHz, DMSO-*d₆*) δ 8.39 (s, 1 H), 8.32 (s, 1 H), 7.97 (dd, J = 9.6 Hz, 1.6 Hz, 1 H), 7.80 (d, J = 8.4 Hz, 1 H), 7.75 (d, J = 2.0 Hz, 1 H), 7.67 (dd, J = 16.0 Hz, 2.0 Hz, 1 H), 6.71 (d, J = 9.6 Hz, 1 H), 4.90-4.85 (m, 1 H), 4.41-4.31 (m, 2 H), 3.81 (d, J = 8.4 Hz, 3 H), 2.14-2.09 (m, 1 H), 2.02-1.97 (m, 1 H).

¹⁹F NMR (376 MHz, DMSO-*d₆*) δ -121.58.

### Example C41

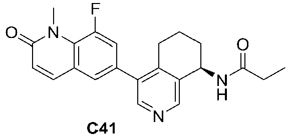

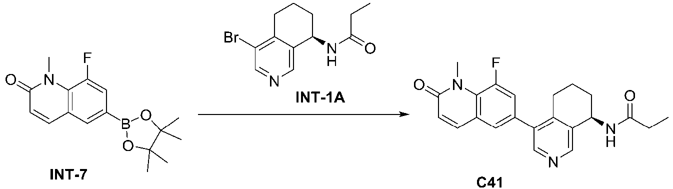

Compound **INT-1A** (33 mg, 0.12 mmol) was dissolved in 1,4-dioxane (5 mL) and water (0.5 mL). Compound **INT-7** (73 mg, 0.24 mmol), potassium carbonate (50 mg, 0.36 mmol), and [1,1'-bis(diphenylphosphino)ferrocene]palladium(II) dichloride (9 mg, 0.012 mmol) were added sequentially. The mixture was substituted with nitrogen three times and then reacted at 85 °C for 16 hours. Reaction completion was monitored by LCMS. The reaction mixture was directly concentrated under reduced pressure and purified by silica gel column chromatography to obtain the crude product. The crude product was purified by reversed-phase flash chromatography (chromatography conditions: column: spherical C18, 20-40 µm, 40 g; mobile phase A: pure water; mobile phase B: acetonitrile; flow rate: 40 mL/min; gradient: 5% B to 95% B in 20 minutes; detector: 214 nm). When the content of mobile phase B reached 25%, compound **C41** (32.17 mg) was collected.

MS (ESI) M/Z: 380.2 [M+H]⁺.

¹H NMR (400 MHz, DMSO-*d₆*) δ 8.38 (s, 1 H), 8.29 (s, 1 H), 8.29 (d, J = 7.2 Hz, 1 H), 7.97 (dd, J = 9.6 Hz, 1.6 Hz, 1 H), 7.60-7.55 (m, 2 H), 6.73 (d, J = 9.6 Hz, 1 H), 5.13-5.08 (m, 1 H), 3.82 (d, J = 8.4 Hz, 3 H), 2.66 (t, J = 10.0 Hz, 1 H), 2.20-2.13 (m, 2 H), 1.91-1.87 (m, 1 H), 1.82-1.70 (m, 3 H), 1.06 (t, J = 7.6 Hz, 3 H).

### Example C43

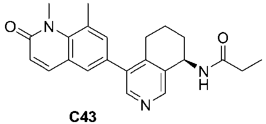

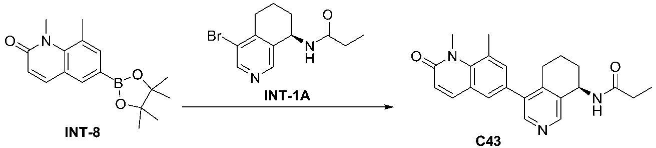

Under a nitrogen atmosphere, compound **INT-1A** (30 mg, 0.11 mmol), potassium carbonate (44 mg, 0.32 mmol), and [1,1'-bis(diphenylphosphino)ferrocene]palladium(II) dichloride dichloromethane complex (8 mg, 0.011 mmol) were added to a mixture of compound **INT-8** (64 mg, 0.22 mmol) in dioxane and water (5/0.5 mL). The resulting reaction mixture was stirred at 90 °C under nitrogen protection for 16 hours. The resulting reaction mixture was filtered and concentrated under reduced pressure. The residue was purified by column chromatography to obtain 39 mg of the crude product. The crude product was purified by reversed-phase flash chromatography (chromatography conditions: column: spherical C18, 20-40 µm, 25 g; mobile phase A: containing 0.1% formic acid; mobile phase B: acetonitrile; flow rate: 35 mL/min; gradient: 5% B to 95% B in 30 minutes; detector: 214 nm). When the content of mobile phase B reached 30%, the product was collected and concentrated under reduced pressure to obtain compound **C43** (17.52 mg).

MS (ESI) M/Z: 376.1 [M+H]⁺.

¹H NMR (400 MHz, DMSO-*d₆*) δ 8.35 (s, 1 H), 8.28 (d, J = 8.4 Hz, 1 H), 8.27 (s, 2 H), 7.90 (d, J = 9.2 Hz, 1 H), 7.56 (d, J = 2.0 Hz, 1 H), 7.42 (d, J = 2.0 Hz, 1 H), 6.64 (d, J = 9.6 Hz, 1 H), 5.13-5.08 (m, 1 H), 3.79 (s, 3 H), 2.77 (s, 3 H), 2.65-2.62 (m, 2 H), 2.18-2.10 (m, 2 H), 1.92-1.87 (m, 1 H), 1.79-1.68 (m, 3 H), 1.06 (t, J = 7.6 Hz, 3 H).

### Example C44

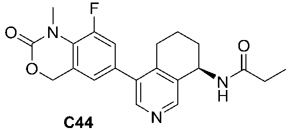

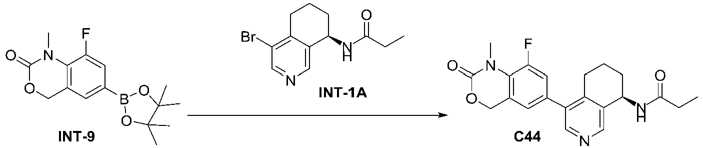

Potassium carbonate (40.63 mg, 0.29 mmol) and [1,1'-bis(diphenylphosphino)ferrocene]palladium(II) dichloride dichloromethane complex (8.00 mg, 0.0098 mmol) were added to a solution of compound **INT-9** (30 mg, 0.098 mmol) and compound **INT-1A** (24.97 mg, 0.088 mmol) in 1,4-dioxane (5.0 mL) and water (0.5 mL). The resulting mixture was stirred and heated at 85 °C under a nitrogen atmosphere for 5 hours. LCMS monitoring indicated the disappearance of the starting material. The residue was purified by Prep-HPLC to obtain compound **C44** (3.16 mg).

MS (ESI) M/Z: 384.1 [M+H]⁺.

¹H NMR (400 MHz, DMSO-*d₆*) δ 8.35 (s, 1 H), 8.27 (d, J = 8.4 Hz, 1 H), 8.23 (s, 1 H), 7.33 (dd, J = 13.6 Hz, 1.6 Hz, 1 H), 7.17 (d, J = 1.2 Hz, 1 H), 5.28 (s, 2 H), 5.14-5.05 (m, 1 H), 3.43 (d, J = 6.0 Hz, 3 H), 2.68-2.59 (m, 2 H), 2.22-2.09 (m, 2 H), 1.94-1.83 (m, 1 H), 1.80-1.63 (m, 3 H), 1.05 (t, J = 7.6 Hz, 3 H).

### Example A14

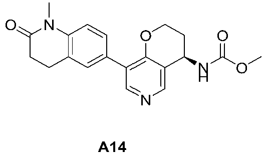

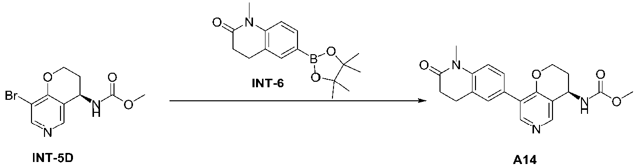

At room temperature, potassium carbonate (36.07 mg, 0.26 mmol) and tetrakis(triphenylphosphine)palladium (10.05 mg, 0.0087 mmol) were added to a solution of **INT-SD** (25 mg, 0.087 mmol) and **INT-6** (37.47 mg, 0.13 mmol) in ethanol (2 mL) and water (0.5 mL). The resulting mixture was stirred and heated at 85 °C under a nitrogen atmosphere for 16 hours. The resulting reaction mixture was concentrated under reduced pressure. The residue was purified by HPLC to obtain compound **A14** (22.02 mg).

MS (ESI) M/Z: 368.1 [M+H]⁺.

¹H NMR (400 MHz, DMSO-*d₆*) δ 8.26 (s, 1 H), 8.25 (s, 1 H), 7.80 (d, J = 8.0 Hz, 1 H), 7.39 (d, J = 8.4 Hz, 1 H), 7.36 (s, 1 H), 7.15 (d, J = 8.4 Hz, 1 H), 4.90-4.81 (m, 1 H), 4.38-4.23 (m, 2 H), 3.61 (s, 3 H), 3.28 (s, 3 H), 2.91 (d, J = 6.8 Hz, 2 H), 2.57 (d, J = 8.0 Hz, 2 H), 2.15-2.04 (m, 1 H), 2.02-1.91 (m, 1 H).

### Example A37

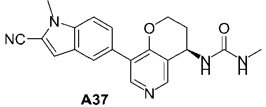

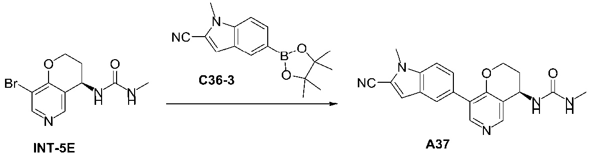

At room temperature, potassium carbonate (36.07 mg, 0.26 mmol) and tetrakis(triphenylphosphine)palladium (10.05 mg, 0.0087 mmol) were added to a solution of **INT-5E** (25 mg, 0.087 mmol) and **C36-3** (36.82 mg, 0.13 mmol) in ethanol (2 mL) and water (0.5 mL). The resulting mixture was stirred and heated at 85 °C under a nitrogen atmosphere for 16 hours. The resulting reaction mixture was concentrated under reduced pressure. The residue was purified by HPLC to obtain compound **A37** (5.23 mg).

Analyzed by supercritical fluid chromatography (chromatography conditions: system: Waters UPCC (CA060); column name: DAICEL- CHIRALCEL^{®}OD; column dimensions: 100 × 3.0 mm, 3.0 µm; mobile phase A: supercritical CO₂; mobile phase B: methanol (0.1% DEA), A:B = 60%:40%; wavelength: 214 nm; flow rate: 1.5 mL/min; column temperature: 35 °C; column pressure: 1800 psi; injection volume: 1.00 µL; cycle time: 8.0 min); retention time RT: 1.778 min.

MS (ESI) M/Z: 362.1 [M+H]⁺.

¹H NMR (400 MHz, DMSO-*d₆*) δ 8.29 (s, 1 H), 8.28 (s, 1 H), 7.80 (d, J = 0.8 Hz, 1 H), 7.67 (d, J = 8.8 Hz, 1 H), 7.54 (dd, J = 8.8 Hz, 1.6 Hz, 1 H), 7.47 (s, 1 H), 6.58 (d, J = 7.6 Hz, 1 H), 5.73 (q, J = 4.8 Hz, 1 H), 4.98-4.89 (m, 1 H), 4.39-4.30 (m, 1 H), 4.29-4.19 (m, 1 H), 3.93 (s, 3 H), 2.61 (d, J = 4.8 Hz, 3 H), 2.13-2.02 (m, 1 H), 2.01-1.90 (m, 1 H).

### Biological Evaluation

The following test examples further describe and explain the present disclosure, but these examples are not intended to limit the scope of the present disclosure.

### Test Example 1: Inhibitory Effect of the Compounds of the Present Disclosure on CYP11B2

G402 cells (ATCC, CRL 1440) are epithelioid cells isolated from the kidney of a white female patient with renal failure and are a type of leiomyoblastoma. For the screening of highly specific CYP11B2 inhibitors, we specifically used G402 cell lines stably expressing human CYP11B2 enzyme or human CYP11B1 enzyme for experimental development.

Construction of Stably Transfected Cell Lines: Expression plasmids containing suitable promoters, suitable drug resistance markers, and the ORF of human CYP11B1 or CYP11B2 were transfected into G402 cells using standard techniques. The cells were then screened using the specific puromycin resistance marker. Monoclonal cells stably expressing human CYP11B2 were selected for expansion, and 11-deoxycorticosterone (11-DOC) was used as the specific substrate to evaluate enzyme activity based on aldosterone production. Monoclonal cells stably expressing human CYP11B1 were selected for expansion, and 11-deoxycortisol (11-DC) was used as the specific substrate, with cortisol production as the detection target for specific enzyme activity evaluation. The G402 cell lines stably expressing CYP11B1/CYP11B2 were cultured at 37 °C, 5% CO₂, 95% air in complete medium (McCoy's 5A + 10% FBS + 1% PS + 0.5 µg/mL puromycin).

Enzyme Inhibition Activity Assay: CYP11B1 G402/CYP11B2 G402 cells were spread out flatly in cell culture plates. After 24 hours, the medium was changed to an enzyme testing system (DMEM/F12 + 2.5% charcoal-stripped FCS). Small molecule inhibitors at various concentrations were added. After 1 hour, appropriate concentrations of specific substrates (1 µM 11-DOC / 1 µM 11-DC) were added and co-incubated with the cells for 16 hours. Cells were then collected, and specific products were detected using LC/MS methods with an AB SCIEX TRIPLE QUAD 5500+, CTO-40S instrument (CYP11B2 enzyme detecting aldosterone; CYP11B1 enzyme detecting cortisol).

The IC₅₀ values of the compounds were determined based on the G402 testing system. The dose-dependent inhibition of enzyme activity was calculated by plotting the added inhibitor concentration (x-axis) against the percentage of product inhibitory activity relative to the control group (y-axis) based on product formation at various compound concentrations. The following data were then fitted using the least squares method with XLfit 5.5.0 for a four-parameter logistic curve fit: 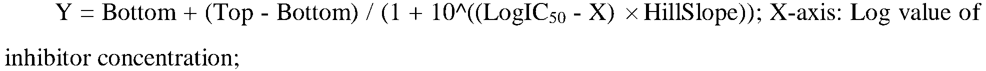 Y = Bottom + (Top - Bottom) / (1 + 10^((LogIC50 - X) × HillSlope)); X-axis: Log value of inhibitor concentration;
Y-axis: Percentage of product inhibitory activity relative to the control group;
logIC₅₀: IC₅₀ was determined using XLFit.

The maximum enzyme activity corresponds to the amount of control product steroid/aldosterone in the absence of inhibitor, set as 100%. The minimum enzyme activity corresponds to the amount of reference control product steroid/aldosterone in the presence of inhibitor. The experimental results are shown in Table 3.

**Table 3 Inhibitory Activity of the Compounds of the Present Disclosure on CYP11B2 and CYP11B1**

| Example Compound | CYP11B2 Inhibition IC₅₀ (nM) | CYP11B1 Inhibition IC₅₀ (nM) |
|---|---|---|
| A01 | 41.7 | |
| C04 | 19.2 | 2501.7 |
| C06 | 31.1 | 2395.1 |
| C07 | 13.2 | 1153.2 |
| C09 | 31.0 | 3853.3 |
| C15 | 24.5 | 2552 |
| C16 | 43.3 | |
| C21 | 26.5 | |
| C24 | 24.2 | |
| D01 | 5.9 | |
| D03 | 8.1 | |
| D05 | 3.4 | |
| A02-P2 | 12.3 | 3677 |
| A04-P1 | 5.6 | 244 |
| A09 | 8.2 | 3384 |
| A09 | 13.8 | 18789 |
| A10-P2 | 1.8 | 316 |
| All | 7.3 | 1258 |
| A12 | 12.6 | 3122 |
| A13 | 4.5 | 381 |
| A14 | 2.3 | 202 |
| A15 | 9.9 | |
| A16 | 9.5 | |
| A17 | 23.1 | |
| A18 | 7.5 | 517 |
| C33 | 32.7 | |
| C36 | 1.4 | 266 |
| C37 | 11.4 | 1717 |
| C41 | 11.1 | 4904 |
| C42 | 11.1 | 4035 |
| C43 | 16.7 | 7218 |
| C44 | 187 | |
| D06 | 8.2 | 288 |
| D07-P 1 | 33.9 | |
| D07-P2 | 11.8 | 1626 |
| D08 | 11 | 803 |
| D09 | 28.2 | |
| D10 | 4.7 | 275 |
| D11 | 7.4 | 1056 |
| D12 | 7.3 | 362 |
| D15 | 2.5 | 128 |
| D16 | 19.5 | 1234 |
| D17 | 33.3 | |
| D14 | 13 | |
| C02 | 16.9 | |
| C45 | 33.0 | |
| A19 | 35.5 | |
| A20 | 29.1 | |
| A21 | 9.4 | 739 |
| A22 | 7.6 | 1193 |
| A23 | 12.5 | 1924 |
| A24 | 11.7 | 1552 |
| A25 | 14.9 | 2149 |
| A26 | 19.0 | |
| A27 | 5.0 | 213 |
| A28 | 3.6 | 111 |
| A29 | 8.8 | 479 |
| A32 | 34.7 | |
| A33 | 9.7 | 312 |
| A34 | 7.5 | 294 |
| A35 | 1.4 | 184 |
| A36 | 1.3 | 136 |
| A37 | 2.5 | 311 |
| A38 | 2.3 | 283 |
| A39 | 10.1 | 1447 |
| A40 | 6.1 | 487 |
| A41 | 28.9 | |
| A42 | 12.9 | 1697 |
| A43 | 6.6 | 572 |
| A44 | 21.9 | |
| A47 | 6.9 | 720 |
| A48 | 5.9 | 712 |
| A49 | 14.9 | 2210 |
| A50 | 11.1 | 2115 |
| A51 | 4.4 | 625 |
| A52 | 5.9 | 797 |
| A53 | 13.1 | 2007 |
| A54 | 13.6 | 1664 |
| A55 | 4.5 | |
| A56 | 11.6 | |

The results indicate that the compounds of the present disclosure exhibit significant inhibitory effects on CYP11B2 and possess distinct selective inhibitory activity.

### Test Example 2: Pharmacokinetic Determination of the Compounds of the Present Disclosure in Mice and Rats

Mice and rats were used as test animals to study the pharmacokinetic behavior of the compounds of the present disclosure in plasma following gavage administration (P.O.). Plasma samples were collected at specific time points, compound concentrations in plasma were detected by LC-MS/MS, and pharmacokinetic parameters were calculated to evaluate the pharmacokinetics.

### 1. Experimental Protocol

### 1.1 Test Compounds:

A part of the compounds of the present disclosure.

### 1.2 Test Animals:

Mice, CD-1, male, Zhejiang Vital River Laboratory Animal Technology Co., Ltd.
Rats, Sprague-Dawley, male, Zhejiang Vital River Laboratory Animal Technology Co., Ltd., Shanghai Jihui Experimental Animal Breeding Co., Ltd.

### 1.3 Administration

### 1.3.1 Administration to Mice:

Route: P.O.; Information: 3 mice, dose: 10 mg/kg, administration volume: 10 mL/kg, vehicle: 5% DMSO/10% Solutol HS15/85% water (5 vol% dimethyl sulfoxide + 10 vol% solubilizer HS15 + 85 vol% water);
Administered compounds: Example Compound C07, Example Compound A16, Example Compound A50, Example Compound C41, Example Compound C44, Control Compound;

### 1.3.2 Administration to Rats:

Route: P.O.; Information: 3 rats, dose: 10 mg/kg, administration volume: 10 mL/kg, vehicle: 5% DMSO/10% Solutol HS15/85% water.
Administered compounds: Example Compound A02-P2, Example Compound A09, Example Compound C43, Control Compound.

### 1.4 Experimental Equipment

Centrifuge from Eppendorf Company, pipettes from Eppendorf Company, vortex mixer from IKA.

### 1.5 Sample Collection

After administration to mice and rats, venous blood samples of 0.03 mL and 0.2 mL, respectively, were collected at 0.0833 (IV), 0.25, 0.5, 1, 2, 4, 8, and 24 hours and placed in EDTA-K₂ test tubes. Plasma was separated by centrifugation at 4 °C, 4000 g for 5 minutes and stored at -80 °C.

### 1.6 Sample Processing

### Mice Plasma Sample Processing:

1) 10 µL of plasma sample was precipitated by adding 200 µL of acetonitrile, vortex mixed, and centrifuged for 15 minutes.
2) The processed supernatant was diluted with water and analyzed by LC/MS/MS to determine the concentration of the test compound.

### Rats Plasma Sample Processing:

1) 50 µL of plasma sample was precipitated by adding 200 µL of acetonitrile, vortex mixed, and centrifuged for 15 minutes.
2) The processed supernatant was diluted with water and analyzed by LC/MS/MS to determine the concentration of the test compound.

### 2. Experimental Results

Pharmacokinetic parameters were calculated using WinNonlin 8.3. The pharmacokinetic parameters for mice P.O. are shown in Table 4, and for rats P.O. in Table 5. Cₘₐₓ represents the maximum plasma concentration, T_{1/2} represents the terminal elimination half-life, MRT_{Inf} represents the mean residence time, and AUC represents the area under the plasma concentration-time curve.

**Table 4 Pharmacokinetic Parameters of the Compounds of the Present Disclosure in Mice**

| Compound | Dose | Cₘₐₓ | T_{1/2} | MRT_{Inf} | AUC₀₋₂₄ |
|---|---|---|---|---|---|
| | (mg/kg) | (nM) | (hr) | (hr) | (hr×nM) |
| C07 | 10 | 30193 | 2.54 | 4.36 | 223279 |
| A16 | 10 | 51700 | 1.07 | 2.37 | 183998 |
| A50 | 10 | 9067 | / | / | 136296 |
| C41 | 10 | 24984 | 2.83 | 5.07 | 209331 |
| C44 | 10 | 53119 | 2.28 | 4.78 | 420726 |
| Control Compound | 10 | 12207 | 1.86 | 3.59 | 57331 |

| | | | | | |
|---|---|---|---|---|---|
| Note: "/" indicates not determined. | | | | | |

Conclusion: As can be seen from Table 4, the compounds of the present disclosure exhibit favorable pharmacokinetic properties in mice.

**Table 5 Pharmacokinetic Parameters of the Compounds of the Present Disclosure in Rats**

| Compound | **Dose** | Cₘₐₓ | T_{1/2} | MRT_{Inf} | AUC₀₋₂₄ |
|---|---|---|---|---|---|
| | (mg/kg) | (nM) | (hr) | (hr) | (hr×nM) |
| A02-P2 | 10 | 22693 | 3.47 | 5.30 | 189686 |
| A09 | 10 | 52098 | 5.91 | 8.19 | 521831 |
| C43 | 10 | 47896 | 4.77 | 6.24 | 344535 |
| Control Compound | 10 | 11372 | 4.31 | 6.41 | 108679 |

Conclusion: As can be seen from Table 5, the compounds of the present disclosure exhibit favorable pharmacokinetic properties in rats.

### Test Example 3: Pharmacokinetic Determination of the Compounds of the Present Disclosure in Monkeys

Cynomolgus monkeys (*Macaca fascicularis*) were used as test animals to study the pharmacokinetic behavior of the compounds of the present disclosure in plasma following gavage administration (P.O.). Plasma samples were collected at specific time points, compound concentrations in plasma were detected by LC-MS/MS, and pharmacokinetic parameters were calculated to reflect the pharmacokinetics in cynomolgus monkeys.

### 1. Experimental Protocol

### 1.1 Test Compounds:

A part of the compounds of the present disclosure.

### 1.2 Test Animals:

Cynomolgus monkeys, male, Ankaiyibo (Zhanjiang) Biotechnology Co., Ltd.

### 1.3 Administration

Route: P.O.; dose: 1 mg/kg; administration volume: 5 mL/kg; vehicle: 5% DMSO/10% Solutol HS15/85% water;
Administered compounds: Example Compound A02-P2, Example Compound A10-P2, Example Compound A50, Example Compound C43, and Control Compound.

### 1.4 Experimental Equipment

Centrifuge from Eppendorf Company, pipettes from Eppendorf Company, vortex mixer from IKA.

### 1.5 Sample Collection

After administration to cynomolgus monkeys, venous blood samples of 0.3 mL were collected at 0.25, 0.5, 1, 2, 4, 8, and 24 hours and placed in EDTA-K₂ test tubes. Plasma was separated by centrifugation at 4 °C, 4000 g for 5 minutes and stored at -80 °C.

### 1.6 Sample Processing

### Cynomolgus Monkey Plasma Sample Processing:

1) 50 µL of plasma sample was precipitated by adding 200 µL of acetonitrile, vortex mixed, and centrifuged for 15 minutes.
2) The processed supernatant was diluted with water and analyzed by LC/MS/MS to determine the concentration of the test compound.

### 2. Experimental Results

Pharmacokinetic parameters were calculated using WinNonlin 8.3. The pharmacokinetic parameters for gavage administration in cynomolgus monkeys are shown in Table 6. Cₘₐₓ represents the maximum plasma concentration, T_{1/2} represents the terminal elimination half-life, MRT_{Inf} represents the mean residence time, and AUC represents the area under the plasma concentration-time curve.

**Table 6 Pharmacokinetic Parameters of the Compounds of the Present Disclosure in Monkeys**

| Compound | Route of Administration | Dose | Cₘₐₓ | T_{1/2} | MRT_{Inf} | AUC₀₋₂₄ |
|---|---|---|---|---|---|---|
| | | (mg/kg) | (nM) | (hr) | (hr) | (hr×nM) |
| A02-P2 | P.O. | 1 | 1603 | 7.44 | 8.77 | 12813 |
| A10-P2 | P.O. | 1 | 3283 | 8.48 | 9.14 | 29108 |
| A50 | P.O. | 1 | 1142 | 10.5 | 13.0 | 13519 |
| Control Compound | P.O. | 1 | 834 | / | / | 6000 |

| | | | | | | |
|---|---|---|---|---|---|---|
| Note: "/" indicates not determined. | | | | | | |

Conclusion: As can be seen from Table 6, the compounds of the present disclosure exhibit favorable pharmacokinetic properties in cynomolgus monkeys.

### Test Example 4: Pharmacodynamic Determination of the Compounds of the Present Disclosure in Monkeys

Cynomolgus monkeys were used as test animals to evaluate the pharmacodynamics of multiple compounds of the present disclosure on plasma hormone levels following gavage administration (P.O.) at various doses such as 0.1 mpk (and others, e.g., 0.03 mpk, 0.1 mpk, and 0.3 mpk).

### 1. Experimental Protocol

### 1.1 Test Compounds:

A part of the compounds of the present disclosure: Example Compound A02-P2, Example Compound A09, Example Compound C43, Example Compound C44, Example Compound A50.

### 1.2 Test Animals:

Male cynomolgus monkeys, Suzhou Xishan Zhongke Laboratory Animal Co., Ltd. Animal production license number: SCXK (Su) 2023-0001, animal use license number: SYXK (Su) 2020-0017.

### 1.3 Administration Protocol:

Male cynomolgus monkeys were performed gavage administration (P.O.) at doses set as: 0 mpk (vehicle group, i.e., Veh group), 0.03 mpk, 0.1 mpk, and 0.3 mpk. In each group, 15 µg/kg ACTH was injected intramuscularly 1 hour after dosing. Continuous venous blood samples were collected from each group at 1 hour before administration and at multiple time points after administration. Blood samples were collected, and plasma aldosterone and its precursors, as well as plasma corticosterone and its precursors, at multiple time points were analyzed using LC/MS methods.

### 1.4 Experimental Equipment:

**Table 7 Experimental Equipment**

| Name | Model | Manufacturer |
|---|---|---|
| Low Speed Refrigerated Centrifuge | KDC-2044 | Anhui Ustc Zonkia Scientific Instruments Co., Ltd |
| Ultra-Low Temperature Refrigerator | DW-HL398G | Zhongke Meiling Cryogenic Tech Co., Ltd |
| Clean Bench | SW-CJ-1FD | Suzhou Shangtian Clean Tech Co., Ltd |
| Electronic Platform Scale | IW2P1-300IG-R | Minebea Intec Industrial Weighing Equipment (Beijing) Co., Ltd |
| Liquid Chromatograph | ACQUITY UPLC I-Class PLUS | Waters |
| Mass Spectrometer | ATriple Quad^{™} 7500 | SCIEX |

### 1.5 Sample Collection

Venous blood samples were collected at 1-2 hours before gavage administration and at multiple time points after intramuscular injection of ACTH (adrenocorticotropic hormone). The samples were placed in EDTA-K2 tubes, centrifuged at 4 °C, 2600 g for 10 minutes to separate plasma, which was then stored at -80 °C.

### 1.6 Liquid Chromatography Analysis

HPLC: Waters ACQUITY UPLC I-Class PLUS;
Mass spectrometry conditions: Triple Quad^{™} 7500 (Serial No. FA224362212) mass spectrometer;
Chromatographic column: phenomenex kinetex 2.6µm C8 100A 50 × 4.60 mm;
Mobile phase: mobile phase A was 0.5 mM ammonium fluoride solution containing 0.1% formic acid; mobile phase B was MeOH.

### 2. Experimental Results and Analysis

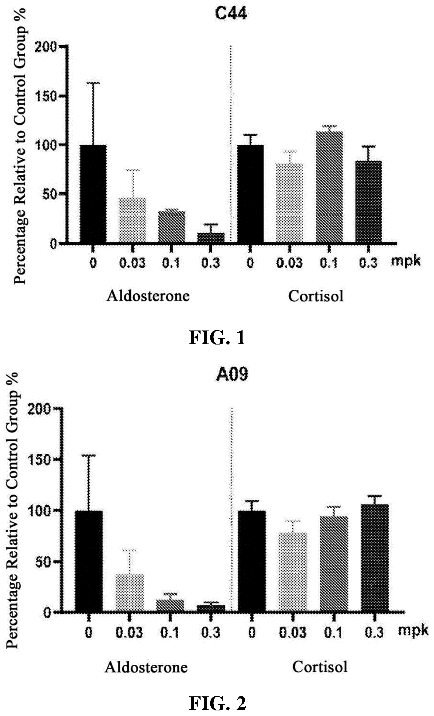

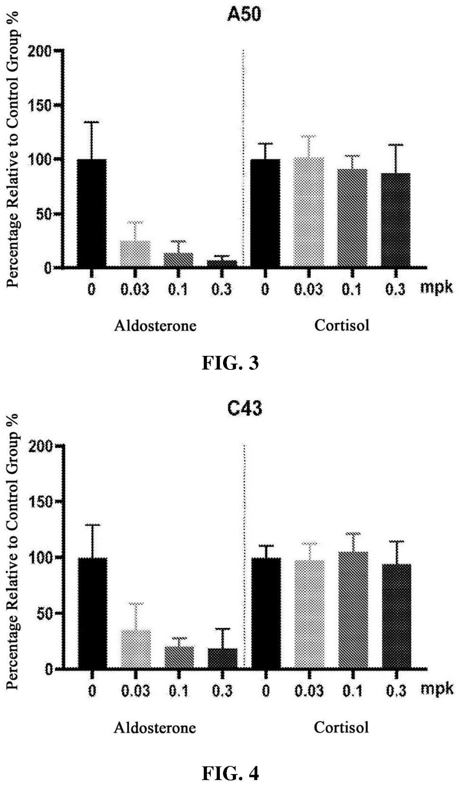

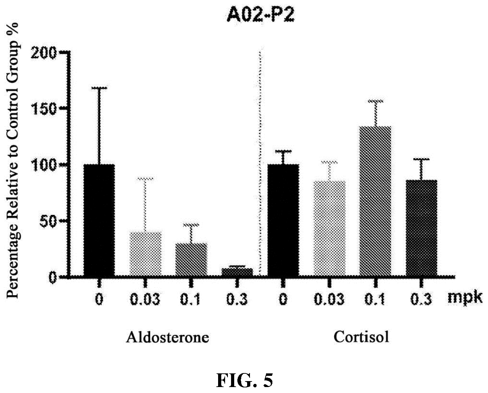

The pharmacodynamic results are shown in Tables 8, 9, 10, 11, 12, and Figs. 1, 2, 3, 4, and 5. Among them, Fig. 1 shows the pharmacodynamic results of Example Compound C44 in monkeys; Fig. 2 shows the pharmacodynamic results of Example Compound A09 in monkeys; Fig. 3 shows the pharmacodynamic results of Example Compound A50 in monkeys; Fig. 4 shows the pharmacodynamic results of Example Compound C43 in monkeys; Fig. 5 shows the pharmacodynamic results of Example Compound A02-P2 in monkeys.

The data in Figs. 1, 2, 3, 4, and 5 are analyzed based on PD changes at 30 minutes after ACTH stimulation. The percentage of PD relative to the control group (Veh group) at 30 minutes after ACTH stimulation serves as the main basis for pharmacodynamic evaluation, calculated according to the following formula: 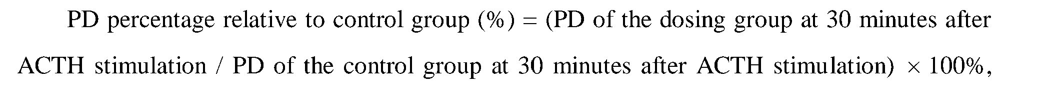 PD percentage relative to control group (%) = (PD of the dosing group at 30 minutes after ACTH stimulation / PD of the control group at 30 minutes after ACTH stimulation) × 100%,where PD refers to aldosterone or cortisol.

**Table 8 Pharmacodynamic Evaluation of Example Compound C44 in Monkeys**

| | Dose | Aldosterone (ng/mL) | | Cortisol (ng/mL) | |
|---|---|---|---|---|---|
| | mpk | 0h | 30min | 0h | 30min |
| Veh | 0 | 0.24 | 0.61 | 343.3 | 474.3 |
| C44 | 0.03 | 0.07 | 0.28 | 279.5 | 384.0 |
| | 0.1 | 0.24 | 0.20 | 455.0 | 538.5 |
| | 0.3 | 0.07 | 0.07 | 265.5 | 396.0 |

**Table 9 Pharmacodynamic Evaluation of Example Compound A09 in Monkeys**

| | Dose | Aldosterone (ng/mL) | | Cortisol (ng/mL) | |
|---|---|---|---|---|---|
| | mpk | 0h | 30min | 0h | 30min |
| Veh | 0 | 0.32 | 0.92 | 272.3 | 365.3 |
| A09 | 0.03 | 0.20 | 0.34 | 214.0 | 285.3 |
| | 0.1 | 0.24 | 012 | 256.7 | 345.0 |
| | 0.3 | 0.08 | 0.07 | 286.7 | 388.3 |

**Table 10 Pharmacodynamic Evaluation of Example Compound A50 in Monkeys**

| | Dose | Aldosterone (ng/mL) | | Cortisol (ng/mL) | |
|---|---|---|---|---|---|
| | mpk | 0h | 30min | 0h | 30min |
| Veh | 0 | 0.34 | 0.86 | 358.0 | 423.0 |
| A50 | 0.03 | 0.12 | 0.22 | 358.3 | 430.0 |
| | 0.1 | 0.49 | 0.12 | 282.0 | 386.0 |
| | 0.3 | 0.12 | 0.06 | 304.7 | 369.7 |

**Table 11 Pharmacodynamic Evaluation of Example Compound C43 in Monkeys**

| | Dose | Aldosterone (ng/mL) | | Cortisol (ng/ml) | |
|---|---|---|---|---|---|
| | mpk | 0h | 30min | 0h | 30min |
| Veh | 0 | 0.25 | 0.75 | 293.8 | 368.3 |
| C43 | 0.03 | 0.16 | 0.26 | 268.3 | 358.7 |
| | 0.1 | 0.16 | 0.15 | 282.0 | 386.0 |
| | 0.3 | 0.20 | 0.14 | 276.0 | 346.7 |

**Table 12 Pharmacodynamic Evaluation of Example Compound A02-P2 in Monkeys**

| | Dose | Aldosterone (ng/ml) | | Cortisol (ng/mL) | |
|---|---|---|---|---|---|
| | mpk | 0h | 30min | 0h | 30min |
| Veh | 0 | 0.26 | 0.67 | 345.3 | 448.3 |
| A02-P2 | 0.03 | 0.13 | 0.27 | 269.0 | 384.0 |
| | 0.1 | 0.40 | 0.20 | 436.0 | 599.0 |
| | 03 | 0.13 | 0.05 | 283.0 | 387.0 |

Conclusion: As can be seen from Tables 8, 9, 10, 11, 12, and Figs. 1-5, the compounds of the present disclosure can effectively inhibit ACTH-stimulated aldosterone release while not causing significant changes in hormones such as cortisol.

The above description presents only preferred embodiments of the present application and is not intended to limit the present application. Any modifications, equivalent substitutions, improvements, etc., made within the spirit and principles of the present application shall be included within the scope of protection of the present application.

## Claims

1. A compound of formula (IA), a stereoisomer thereof, an isotopic derivative thereof, a prodrug thereof, an N-oxide thereof, a solvate thereof, or a pharmaceutically acceptable salt thereof; 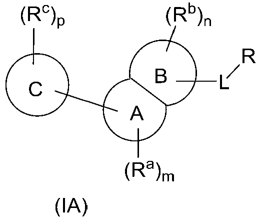 wherein:
ring A is 5- to 6-membered heteroaryl;
ring B is selected from the group consisting of 3- to 8-membered heterocyclyl, 3- to 8-membered monocyclic cycloalkyl, 3- to 8-membered monocyclic cycloalkenyl, 5- to 10-membered fused ring group, 5- to 10-membered spiro ring group, and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -(CR^{d}R^{e})ᵣ-, -(CR^{d}=CR^{e})ᵣ-, -O-(CR^{d}R^{e})ᵣ-, -(CR^{d}R^{e})ᵣ-O-, -NRⁿ¹-(CR^{d}R^{e})ᵣ-, -(CR^{d}R^{e})r-NRⁿ¹-, -(CR^{d}R^{e})ᵣ-C(O)-, -C(O)-(CR^{d}R^{e})ᵣ-, -O-C(O)-, -C(O)-O-, -O-C(O)-NRⁿ¹-, -NRⁿ¹-C(O)-O-, -NRⁿ¹-C(O)-NRⁿ¹-, -(CR^{d}R^{e})ᵣ-NRⁿ¹-C(O)-, -C(O)-NRⁿ¹-(CR^{d}R^{e})ᵣ-, -NRⁿ¹-C(O)-(CR^{d}R^{e})ᵣ-, -NRⁿ¹-S(O)₂-, and -S(O)₂-NRⁿ¹-;
R is selected from the group consisting of hydrogen atom, C₁₋₆ alkyl, C₂₋₆ alkenyl, C₂₋₆ alkynyl, halogen, cyano, -OR^{o}, -NRⁿ²Rⁿ³, 3- to 8-membered heterocyclyl, 3- to 8-membered cycloalkyl, 3- to 8-membered cycloalkenyl, 6- to 10-membered aryl, and 5- to 10-membered heteroaryl, wherein the C₁₋₆ alkyl, C₂₋₆ alkenyl, C₂₋₆ alkynyl, 3- to 8-membered heterocyclyl, 3-to 8-membered cycloalkyl, 3- to 8-membered cycloalkenyl, 6- to 10-membered aryl, and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR°, -C(O)NRⁿ⁴OR^{o}, hydroxy, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or Rⁿ¹ and R together with the atom to which they are attached form 3- to 8-membered heterocyclyl or 5- to 10-membered heteroaryl; wherein the 3- to 8-membered heterocyclyl or 5-to 10-membered heteroaryl is each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
each R^{a}, R^{b}, and R^{c} is identical or different, and is independently selected from the group consisting of hydrogen atom, deuterium atom, C₁₋₆ alkyl, -OR^{o}, -NRⁿ²Rⁿ³, halogen, cyano, oxo, -C(O)NRⁿ²Rⁿ³, -C(O)R^{o}, -C(O)OR^{o}, -C(O)NRⁿ¹OR^{o}, 3- to 8-membered heterocyclyl, 3- to 8-membered cycloalkyl, 3- to 8-membered cycloalkenyl, 6- to 10-membered aryl, and 5- to 10-membered heteroaryl, wherein the C₁₋₆ alkyl, 3- to 8-membered heterocyclyl, 3- to 8-membered cycloalkyl, 3- to 8-membered cycloalkenyl, 6- to 10-membered aryl, and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR°, -C(O)NRⁿ⁴OR^{o}, hydroxy, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or two R^{b} together with the atom to which they are attached form 3- to 8-membered heterocyclyl or 3- to 8-membered cycloalkyl, wherein the 3- to 8-membered heterocyclyl and 3-to 8-membered cycloalkyl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or two R^{c} together with the atom to which they are attached form 3- to 8-membered heterocyclyl or 3- to 8-membered cycloalkyl, wherein the 3- to 8-membered heterocyclyl and 3-to 8-membered cycloalkyl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl, or 5- to 10-membered heteroaryl; wherein the 3- to 8-membered heterocyclyl, 3- to 8-membered cycloalkyl, 3- to 8-membered cycloalkenyl, 6- to 10-membered aryl, and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
R^{d} and R^{e} are identical or different at each occurrence and are each independently selected from the group consisting of hydrogen atom, deuterium atom, C₁₋₆ alkyl, halogen, deuterated C₁₋₆ alkyl, C₁₋₆ haloalkyl, C₁₋₆ hydroxyalkyl, C₂₋₆ alkenyl, C₂₋₆ alkynyl, C₂₋₆ haloalkenyl, C₂₋₆ haloalkynyl, -OR^{o}, and C₁₋₆ haloalkoxy;
R^{o} is identical or different at each occurrence and is each independently selected from the group consisting of hydrogen atom, C₁₋₆ alkyl, C₁₋₆ haloalkyl, C₁₋₆ hydroxyalkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl, and 5- to 10-membered heteroaryl; wherein the C₁₋₆ alkyl, C₂₋₆ alkenyl, C₂₋₆ alkynyl, 3- to 8-membered heterocyclyl, 3- to 8-membered cycloalkyl, 3- to 8-membered cycloalkenyl, 6- to 10-membered aryl, and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
Rⁿ¹, Rⁿ², Rⁿ³, Rⁿ⁴, and Rⁿ⁵ are identical or different at each occurrence and are each independently selected from the group consisting of hydrogen atom, C₁₋₆ alkyl, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl; wherein the C₁₋₆ alkyl, 3- to 8-membered heterocyclyl, 3- to 8-membered cycloalkyl, 3- to 8-membered cycloalkenyl, 6-to 10-membered aryl, and 5- to 10-membered heteroaryl are each independently optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or Rⁿ² and Rⁿ³, Rⁿ⁴ and Rⁿ⁵, together with the nitrogen atom to which they are attached, respectively, form 3- to 8-membered heterocyclyl; wherein the 3- to 8-membered heterocyclyl is optionally substituted with one or more substituents selected from the group consisting of deuterium atom, halogen, oxo, C₁₋₆ alkyl, deuterated C₁₋₆ alkyl, C₁₋₆ haloalkyl, C₁₋₆ alkoxy, C₁₋₆ haloalkoxy, cyano, -NRⁿ⁴Rⁿ⁵, -C(O)NRⁿ⁴Rⁿ⁵, -C(O)R^{o}, -C(O)OR°, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
n is 0, 1, 2, 3, 4, 5, 6, 7, 8, 9, 10, 11 or 12;
m is 0, 1, 2, 3, 4, 5 or 6;
p is 0, 1, 2, 3, 4, 5, 6, 7, 8, 9, 10, 11 or 12; and
r is 0, 1, 2, 3 or 4.

2. The compound according to claim 1, the stereoisomer thereof, the isotopic derivative thereof, the prodrug thereof, the N-oxide thereof, the solvate thereof, or the pharmaceutically acceptable salt thereof, wherein ring A is 5- to 6-membered heteroaryl containing 1 to 3 nitrogen atoms;
preferably, ring A is selected from the group consisting of pyridinyl, pyrimidinyl, pyridazinyl, and a 5-membered heteroaryl containing 1 to 3 nitrogen atoms.

3. The compound according to claim 1 or 2, the stereoisomer thereof, the isotopic derivative thereof, the prodrug thereof, the N-oxide thereof, the solvate thereof, or the pharmaceutically acceptable salt thereof, which is a compound of formula (I), a stereoisomer thereof, an isotopic derivative thereof, a prodrug thereof, an N-oxide thereof, a solvate thereof, or a pharmaceutically acceptable salt thereof; 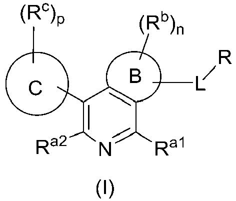 wherein:
R^{a1} and R^{a2} are identical or different, and are each independently as defined for R^{a} in claim 1;
ring B is selected from the group consisting of 3- to 8-membered heterocyclyl, 5- to 6-membered cycloalkenyl, 7- to 8-membered monocyclic cycloalkyl, 5- to 10-membered fused ring group, 5- to 10-membered spiro ring group, and 5- to 10-membered bridged ring group;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -NRⁿ¹-C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl, or 5- to 10-membered heteroaryl; wherein the 3- to 8-membered heterocyclyl, 3- to 8-membered cycloalkyl, 3- to 8-membered cycloalkenyl, 6- to 10-membered aryl, and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴0R^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
provided that the compound of formula (I) is not the compound with CAS number 1428652-88-3;
R, R^{a}, R^{b}, R^{c}, Rⁿ¹, Rⁿ⁴, Rⁿ⁵, R^{o}, R^{d}, R^{e}, n, and p are as defined in claim 1.

4. The compound according to claim 3, the stereoisomer thereof, the isotopic derivative thereof, the prodrug thereof, the N-oxide thereof, the solvate thereof, or the pharmaceutically acceptable salt thereof, wherein in the structural moiety 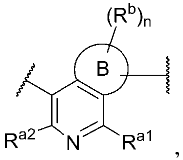 ring B is selected from the group consisting of 5- to 6-membered heterocyclyl, 5- to 6-membered cycloalkenyl, 6-to 10-membered fused ring group, and 6- to 10-membered bridged ring group; R^{a1} and R^{a2} are identical or different, and are each independently as defined for R^{a} in claim 1; n and R^{b} are as defined in claim 1;
preferably, in the structural moiety 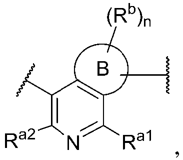 ring B is selected from the group consisting of 5- to 6-membered heterocyclyl, 5- to 6-membered cycloalkenyl, 6- to 10-membered fused ring group, and 6- to 10-membered bridged ring group; each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3, or 4; R^{a1} and R^{a2} are identical or different, and are each independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
more preferably, 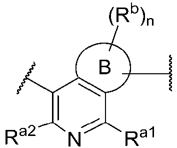 is selected from the group consisting of 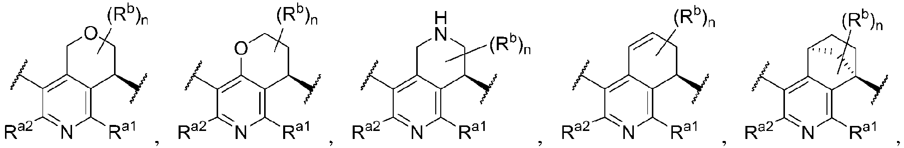 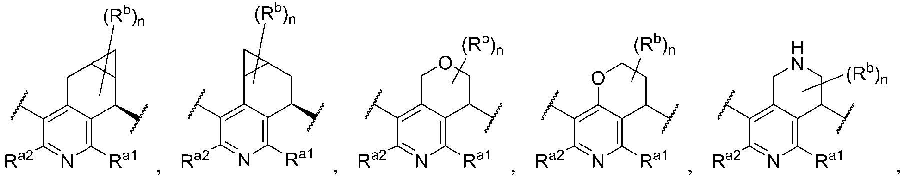 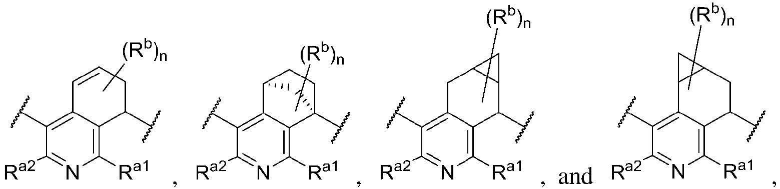 wherein 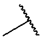 and 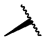 are attached to L, each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3, or 4; R^{a1} and R^{a2} are identical or different, and are each independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
further preferably, 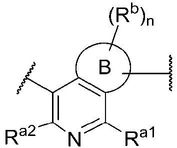 is selected from the group consisting of 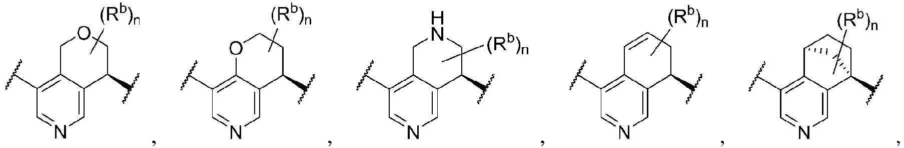 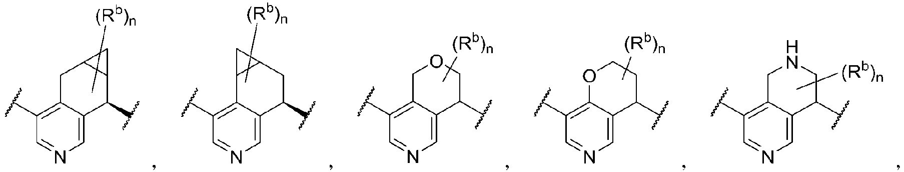 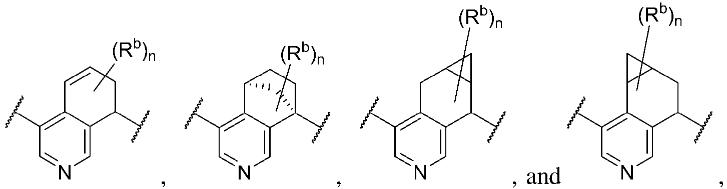 wherein 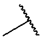 and 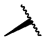 are attached to L, each R^{b} is identical or different, and is independently selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; n is 0, 1, 2, 3, or 4;
even more preferably, 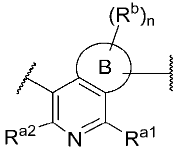 is selected from the group consisting of 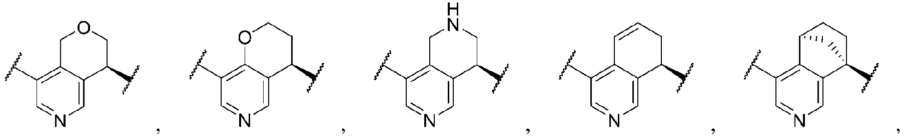 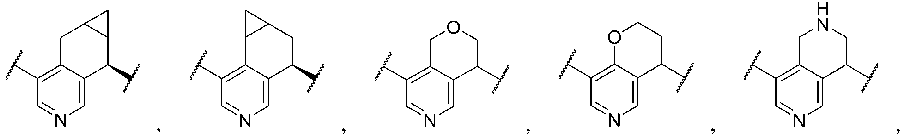 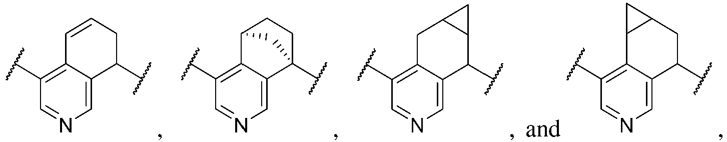 wherein 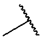 and 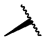 are attached to L.

5. The compound according to any one of claims 1 to 4, the stereoisomer thereof, the isotopic derivative thereof, the prodrug thereof, the N-oxide thereof, the solvate thereof, or the pharmaceutically acceptable salt thereof, wherein ring B is selected from the group consisting of 5- to 6-membered heterocyclyl, 5- to 6-membered cycloalkenyl, 6- to 10-membered fused ring group, and 6- to 10-membered bridged ring group.

6. The compound according to any one of claims 1 to 5, the stereoisomer thereof, the isotopic derivative thereof, the prodrug thereof, the N-oxide thereof, the solvate thereof, or the pharmaceutically acceptable salt thereof, wherein ring B is selected from the group consisting of 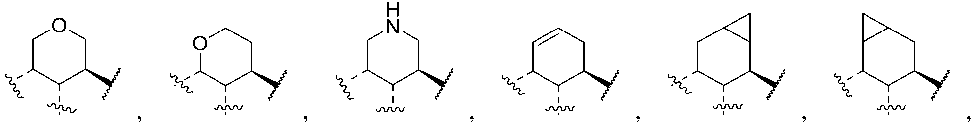 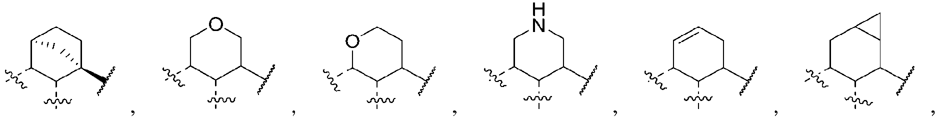 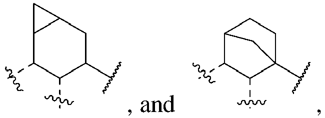 wherein 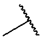 and 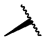 are attached to L.

7. The compound according to claim 1 or 2, the stereoisomer thereof, the isotopic derivative thereof, the prodrug thereof, the N-oxide thereof, the solvate thereof, or the pharmaceutically acceptable salt thereof, which is a compound of formula (IV) or formula (IV-1), a stereoisomer thereof, an isotopic derivative thereof, a prodrug thereof, an N-oxide thereof, a solvate thereof, or a pharmaceutically acceptable salt thereof; 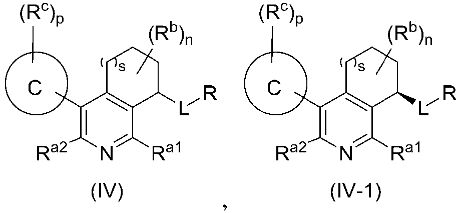 wherein:
R^{a1} and R^{a2} are as defined for R^{a} in claim 1;
s is 0, 1, 2, 3, or 4;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group; and 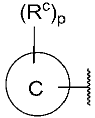 is not 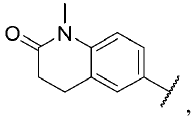 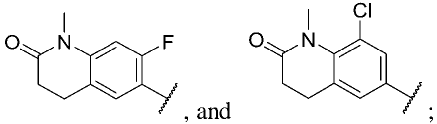
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl, or 5- to 10-membered heteroaryl; wherein the 3- to 8-membered heterocyclyl, 3- to 8-membered cycloalkyl, 3- to 8-membered cycloalkenyl, 6- to 10-membered aryl, and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴0R^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
R, R^{a}, R^{b}, R^{c}, Rⁿ¹, Rⁿ⁴, Rⁿ⁵, R^{o}, R^{d}, R^{e}, n, and p are as defined in claim 1.

8. The compound according to claim 1 or 2, the stereoisomer thereof, the isotopic derivative thereof, the prodrug thereof, the N-oxide thereof, the solvate thereof, or the pharmaceutically acceptable salt thereof, which is a compound of formula (V) or formula (V-1), a stereoisomer thereof, an isotopic derivative thereof, a prodrug thereof, an N-oxide thereof, a solvate thereof, or a pharmaceutically acceptable salt thereof; 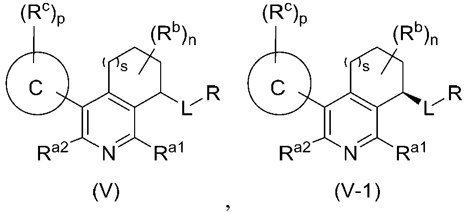 wherein:
R^{a1} and R^{a2} are as defined for R^{a} in claim 1;
s is 0, 1, 2, 3, or 4;
ring C is selected from the group consisting of phenyl, 5- to 6-membered heteroaryl, 7- to 14-membered fused aryl, 6- to 14-membered fused heterocyclyl, 6- to 14-membered fused heteroaryl, and 6- to 14-membered fused ring group;
L is selected from the group consisting of -NRⁿ¹-, -NRⁿ¹C(O)-, -NRⁿ¹-C(O)-NRⁿ¹-, and -CR^{d}=CR^{e}-; or Rⁿ¹ and R together with the atom to which they are attached form 3- to 8-membered heterocyclyl or 5- to 10-membered heteroaryl; wherein the 3- to 8-membered heterocyclyl or 5- to 10-membered heteroaryl is each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR^{o}, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl;
or R^{b} and R together with the atom to which they are attached form 3- to 8-membered heterocyclyl, 3- to 8-membered cycloalkyl, 3- to 8-membered cycloalkenyl, 6- to 10-membered aryl, or 5- to 10-membered heteroaryl; wherein the 3- to 8-membered heterocyclyl, 3- to 8-membered cycloalkyl, 3- to 8-membered cycloalkenyl, 6- to 10-membered aryl, and 5- to 10-membered heteroaryl are each independently optionally substituted with one or more substituents selected from the group consisting of halogen, oxo, C₁₋₆ alkyl, C₁₋₆ haloalkyl, C₁₋₆ haloalkoxy, cyano, -NRⁿ⁴Rⁿ⁵, -C(O)NRⁿ⁴Rⁿ⁵, -C(O)R^{o}, -C(O)OR°, -C(O)NRⁿ⁴OR^{o}, -OR^{o}, C₁₋₆ hydroxyalkyl, 3- to 8-membered heterocyclyl, 3- to 8-membered cycloalkyl, 3- to 8-membered cycloalkenyl, 6- to 10-membered aryl, and 5- to 10-membered heteroaryl; and
when L is -NHC(O)-, R is not methyl, ethyl, cyclopropyl, CH₃CH(OH)-, and 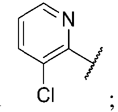
R, R^{a}, R^{b}, R^{c}, Rⁿ¹, Rⁿ⁴, Rⁿ⁵, R^{o}, R^{d}, R^{e}, n, and p are as defined in claim 1.

9. The compound according to any one of claims 1 to 8, the stereoisomer thereof, the isotopic derivative thereof, the prodrug thereof, the N-oxide thereof, the solvate thereof, or the pharmaceutically acceptable salt thereof, which is a compound of formula (I-1) or formula (I-2), a stereoisomer thereof, an isotopic derivative thereof, a prodrug thereof, an N-oxide thereof, a solvate thereof, or a pharmaceutically acceptable salt thereof; 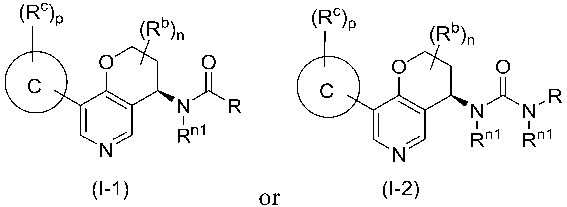 wherein:
ring C, R, Rⁿ¹, R^{b}, R^{c}, n, and p are as defined in any one of claims 1 to 8.

10. The compound according to any one of claims 1 to 9, the stereoisomer thereof, the isotopic derivative thereof, the prodrug thereof, the N-oxide thereof, the solvate thereof, or the pharmaceutically acceptable salt thereof, wherein in the structural moiety 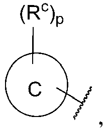 ring C is selected from the group consisting of phenyl, 5- to 6-membered heteroaryl, 7- to 14-membered fused aryl, 6- to 14-membered fused heterocyclyl, and 6- to 14-membered fused heteroaryl, each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, deuterated C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3, or 4;
preferably, in the structural moiety 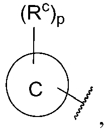 ring C is selected from the group consisting of phenyl, 5- to 6-membered heteroaryl, 5- to 8-membered monocyclic heterocyclyl-fused phenyl, 7- to 10-membered fused heterocyclyl-fused phenyl, 7- to 10-membered spiro heterocyclyl-fused phenyl, 5- to 8-membered monocyclic heterocyclyl-fused 5- to 6-membered heteroaryl, 7- to 10-membered fused heterocyclyl-fused 5- to 6-membered heteroaryl, and 5- to 6-membered heteroaryl-fused phenyl; each R^{c} is identical or different, and is independently selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, deuterated C₁₋₆ alkyl, C₁₋₆ alkoxy, 3- to 8-membered cycloalkyl, 3- to 8-membered cycloalkenyl, 3- to 8-membered heterocyclyl, and 5- to 6-membered heteroaryl, wherein the 3- to 8-membered cycloalkyl, 3- to 8-membered cycloalkenyl, 3- to 8-membered heterocyclyl, and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3, or 4;
more preferably, the structural moiety 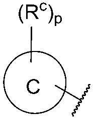 is selected from the group consisting of 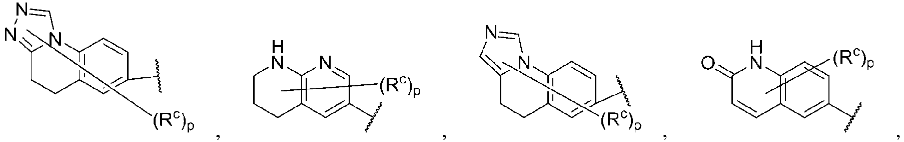 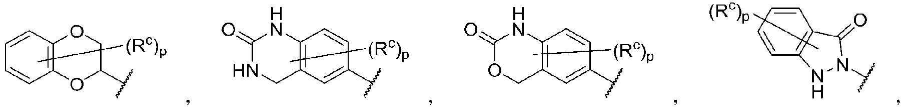 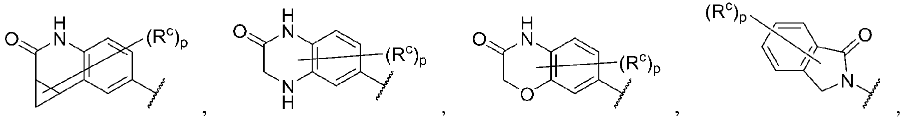 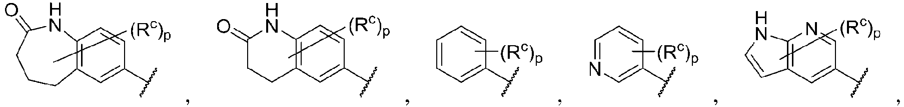 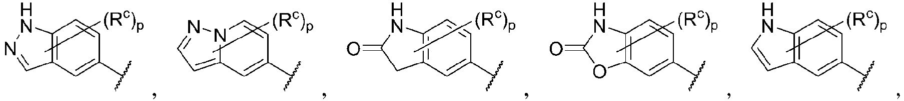 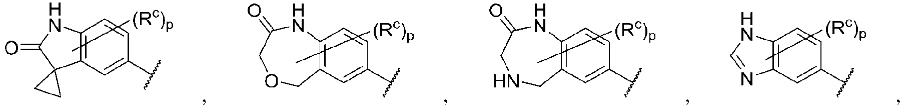 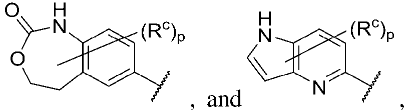 each R^{c} is identical or different, and is independently selected from the group consisting of hydrogen atom, halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, deuterated C₁₋₆ alkyl, C₁₋₆ alkoxy, 3- to 6-membered cycloalkyl, and 5- to 6-membered heteroaryl, wherein the 3- to 6-membered cycloalkyl and 5- to 6-membered heteroaryl are optionally substituted with one or more substituents selected from the group consisting of halogen, hydroxy, oxo, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; p is 0, 1, 2, 3, or 4;
even more preferably, the structural moiety 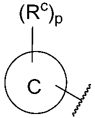 is selected from the group consisting of 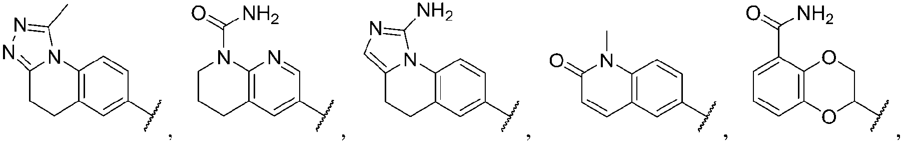 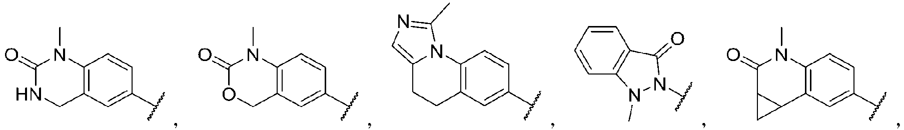 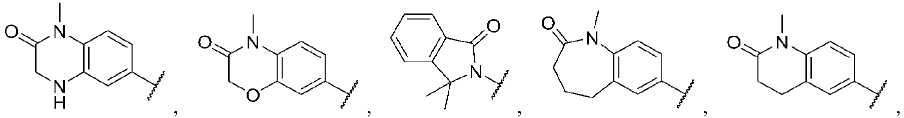 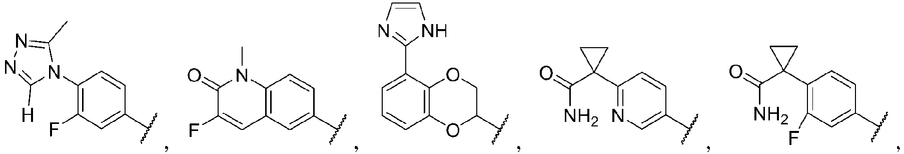 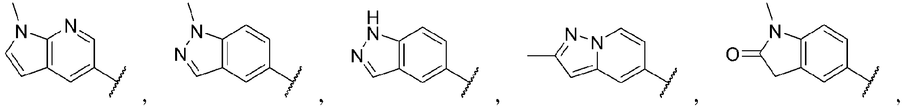 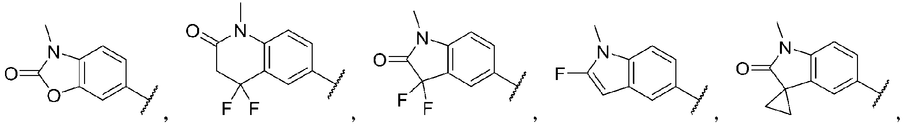 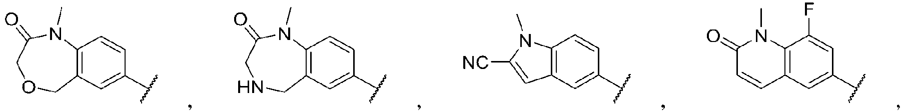 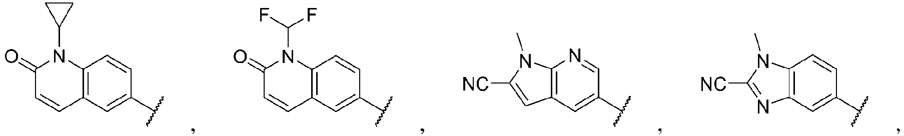 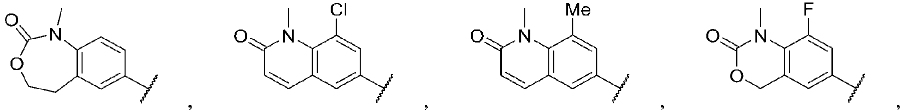 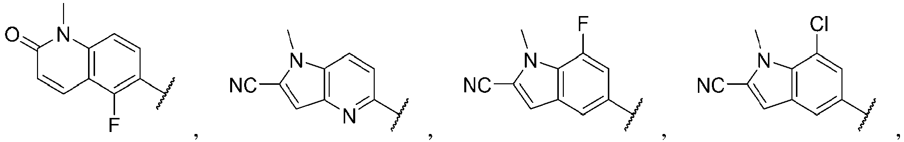 and

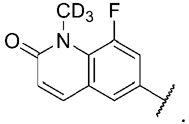

11. The compound according to any one of claims 1 to 10, the stereoisomer thereof, the isotopic derivative thereof, the prodrug thereof, the N-oxide thereof, the solvate thereof, or the pharmaceutically acceptable salt thereof, wherein ring C is selected from the group consisting of phenyl, 5- to 6-membered heteroaryl, 5- to 8-membered monocyclic heterocyclyl-fused phenyl, 7- to 10-membered fused heterocyclyl-fused phenyl, 5- to 8-membered monocyclic heterocyclyl-fused 5- to 6-membered heteroaryl, 7- to 10-membered fused heterocyclyl-fused 5-to 6-membered heteroaryl, 5- to 6-membered heteroaryl-fused phenyl, and 5- to 6-membered heteroaryl-fused 5- to 6-membered heteroaryl.

12. The compound according to any one of claims 1 to 11 the stereoisomer thereof, the isotopic derivative thereof, the prodrug thereof, the N-oxide thereof, the solvate thereof, or the pharmaceutically acceptable salt thereof, wherein ring C is selected from the group consisting of

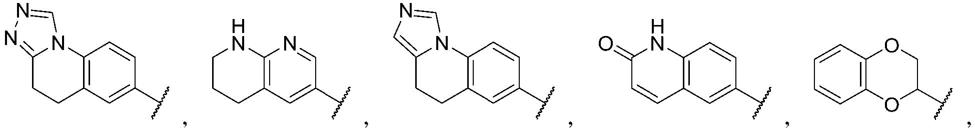

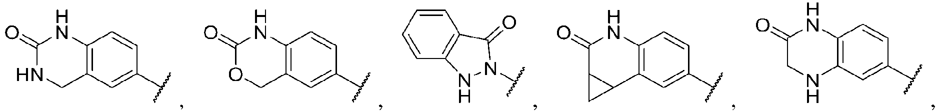

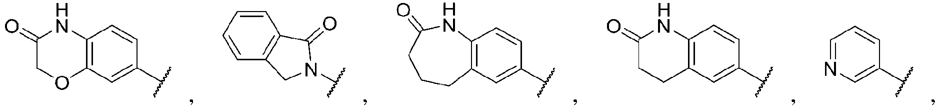

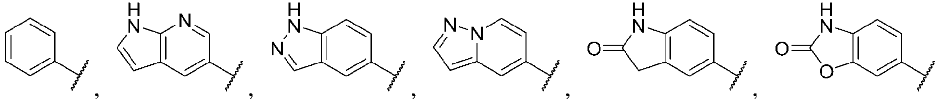

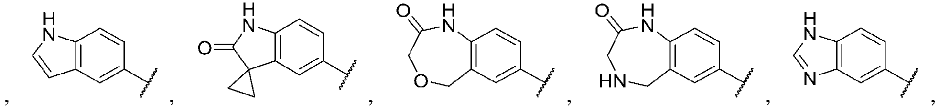

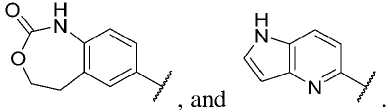

13. The compound according to any one of claims 1 to 12, the stereoisomer thereof, the isotopic derivative thereof, the prodrug thereof, the N-oxide thereof, the solvate thereof, or the pharmaceutically acceptable salt thereof, wherein each R^{c} is identical or different, and is independently selected from the group consisting of hydrogen atom, halogen, oxo, C₁₋₆ haloalkyl, cyano, -CONH₂, amino, C₁₋₆ alkyl, deuterated C₁₋₆ alkyl, an optionally -CONH₂ substituted 3- to 8-membered cycloalkyl, and an optionally C₁₋₆ alkyl substituted 5- to 6-membered heteroaryl; or two R^{c} attached to the same carbon atom or to adjacent carbon atoms together with the carbon atom to which they are attached form 3- to 6-membered cycloalkyl.

14. The compound according to any one of claims 1 to 13, the stereoisomer thereof, the isotopic derivative thereof, the prodrug thereof, the N-oxide thereof, the solvate thereof, or the pharmaceutically acceptable salt thereof, wherein each R^{c} is identical or different, and is independently selected from the group consisting of hydrogen atom, F, methyl, amino, -CONH₂, 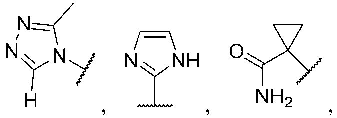 cyano, -CHF₂, cyclopropyl, -CD₃, and Cl.

15. The compound according to any one of claims 1 to 14, the stereoisomer thereof, the isotopic derivative thereof, the prodrug thereof, the N-oxide thereof, the solvate thereof, or the pharmaceutically acceptable salt thereof, wherein L is selected from the group consisting of -NRⁿ¹-, -NRⁿ¹C(O)-, -NRⁿ¹-C(O)-NRⁿ¹-, and -CR^{d}=CR^{e}-; Rⁿ¹, R^{d}, and R^{e} are as defined in claim 1; R is selected from the group consisting of C₁₋₆ alkyl, C₁₋₆ alkoxy, C₁₋₆ hydroxyalkyl, cyclopropyl, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 3- to 8-membered heterocyclyl or 5- to 10-membered heteroaryl; or R^{b} and R together with the atom to which they are attached form 3- to 6-membered heterocyclyl;
wherein the 3- to 8-membered heterocyclyl, 3- to 6-membered heterocyclyl, and 5- to 10-membered heteroaryl are each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom, C₁₋₆ alkyl, and deuterated C₁₋₆ alkyl; R^{d} and R^{e} are identical or different, and are each independently selected from the group consisting of hydrogen atom, C₁₋₆ alkyl, and halogen; R is selected from the group consisting of C₁₋₆ alkyl, C₁₋₆ alkoxy, C₁₋₆ hydroxyalkyl, cyclopropyl, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 5- to 8-membered heterocyclyl; or R^{b} and R together with the atom to which they are attached form 5- to 6-membered heterocyclyl; wherein the 5- to 8-membered heterocyclyl, and 5- to 6-membered heterocyclyl are each independently optionally substituted with one or more substituents selected from the group consisting of halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy;
more preferably, L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom, C₁₋₆ alkyl, and deuterated C₁₋₆ alkyl; R^{d} and R^{e} are identical or different, and are each independently selected from the group consisting of hydrogen atom, C₁₋₆ alkyl, and halogen; R is selected from the group consisting of C₁₋₆ alkyl, C₁₋₆ alkoxy, C₁₋₆ hydroxyalkyl, cyclopropyl, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 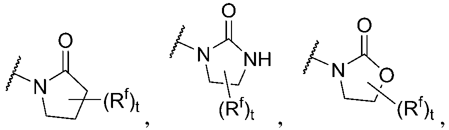 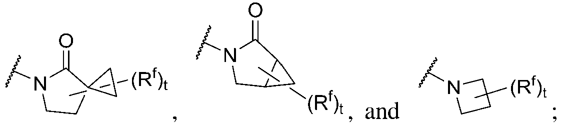 or R^{b} and R together with the atom to which they are attached form 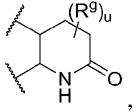 each R^{f} and R^{g} is identical or different, and is independently selected from the group consisting of hydrogen atom, halogen, hydroxy, C₁₋₆ hydroxyalkyl, C₁₋₆ haloalkyl, C₁₋₆ haloalkoxy, cyano, amino, -CONH₂, NH(C₁₋₆ alkyl), N(C₁₋₆ alkyl)₂, C₁₋₆ alkyl, and C₁₋₆ alkoxy; t is 0, 1, 2, 3, or 4; u is 0, 1, 2, 3, 4, or 5;
more preferably, L is selected from the group consisting of -NHC(O)-, -NH-C(O)-NH-, -NH-C(O)-N(CH₃)-, -NH-C(O)-N(CD₃)-, and -CH=CF-; R is selected from the group consisting of C₁₋₆ alkyl, C₁₋₆ alkoxy, C₁₋₆ haloalkyl, deuterated C₁₋₆ alkyl, deuterated C₁₋₆ alkoxy, and C₁₋₆ alkoxy C₁₋₆ alkyl; or Rⁿ¹ and R together with the atom to which they are attached form 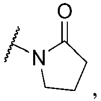 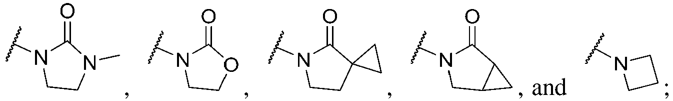 or R^{b} and R together with the atom to which they are attached form

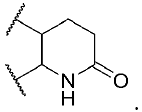

16. The compound according to any one of claims 1 to 15, the stereoisomer thereof, the isotopic derivative thereof, the prodrug thereof, the N-oxide thereof, the solvate thereof, or the pharmaceutically acceptable salt thereof, wherein L is selected from the group consisting of -NRⁿ¹C(O)-, -NRⁿ¹-C(O)-NRⁿ¹-, and -CR^{d}=CR^{e}-; Rⁿ¹ is selected from the group consisting of hydrogen atom, C₁₋₆ alkyl, and deuterated C₁₋₆ alkyl; R^{d} and R^{e} are identical or different, and are each independently selected from the group consisting of hydrogen atom, C₁₋₆ alkyl, and halogen.

17. The compound according to any one of claims 1 to 16, the stereoisomer thereof, the isotopic derivative thereof, the prodrug thereof, the N-oxide thereof, the solvate thereof, or the pharmaceutically acceptable salt thereof, which is a compound of formula (I-a), a stereoisomer thereof, an isotopic derivative thereof, a prodrug thereof, an N-oxide thereof, a solvate thereof, or a pharmaceutically acceptable salt thereof; 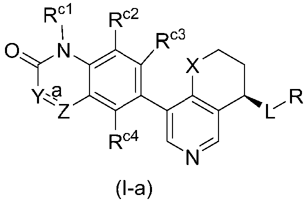 wherein:
X is selected from the group consisting of -CH₂-, -O-, -NH-, and -S-;
L is selected from the group consisting of -NRⁿ¹C(O)- and -NRⁿ¹-C(O)-NRⁿ¹-;
bond a is a single bond or a double bond;
when bond a is a single bond, Y and Z are identical or different, and are each independently selected from the group consisting of -O-, -NH-, and -CR^{c5}R^{c6}-;
when bond a is a double bond, Y and Z are identical and are both selected from CH;
R^{c1} is selected from the group consisting of hydrogen atom, C₁₋₆ alkyl, deuterated C₁₋₆ alkyl, C₁₋₆ haloalkyl, 3- to 6-membered heterocyclyl, and 3- to 6-membered cycloalkyl;
R^{c2}, R^{c3}, R^{c4}, R^{c5}, R^{c6} are identical or different, and are each independently selected from the group consisting of hydrogen atom, halogen, C₁₋₆ alkyl, and C₁₋₆ haloalkyl;
Rⁿ¹ and R are as defined in formula (IA);
provided that: when X is -CH₂-, 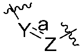 is not -CH₂-CH₂- at the same time, and -L-R is not -NHC(O)-CH₂CH₃ at the same time.

18. The compound according to any one of claims 1 to 17, the stereoisomer thereof, the isotopic derivative thereof, the prodrug thereof, the N-oxide thereof, the solvate thereof, or the pharmaceutically acceptable salt thereof, wherein X is selected from the group consisting of -CH₂- and -O-.

19. The compound according to any one of claims 1 to 18, the stereoisomer thereof, the isotopic derivative thereof, the prodrug thereof, the N-oxide thereof, the solvate thereof, or the pharmaceutically acceptable salt thereof, wherein when bond a is a single bond, one of Y and Z is -O- and the other is -CR^{c5}R^{c6}-; R^{c5} and R^{c6} are as defined in claim 17.

20. The compound according to any one of claims 1 to 19, the stereoisomer thereof, the isotopic derivative thereof, the prodrug thereof, the N-oxide thereof, the solvate thereof, or the pharmaceutically acceptable salt thereof, wherein R^{c2}, R^{c3}, R^{c4}, R^{c5}, R^{c6} are identical or different, and are each independently selected from the group consisting of hydrogen atom, F, Cl, -CHF₂, and methyl.

21. The compound according to any one of claims 1 to 20, the stereoisomer thereof, the isotopic derivative thereof, the prodrug thereof, the N-oxide thereof, the solvate thereof, or the pharmaceutically acceptable salt thereof, wherein R^{c1} is selected from the group consisting of hydrogen atom, cyclopropyl, methyl, and deuterated methyl; preferably, R^{c1} is selected from the group consisting of methyl and deuterated methyl; more preferably, R^{c1} is selected from the group consisting of methyl and -CD₃.

22. The compound according to any one of claims 1 to 20, the stereoisomer thereof, the isotopic derivative thereof, the prodrug thereof, the N-oxide thereof, the solvate thereof, or the pharmaceutically acceptable salt thereof, wherein L is selected from the group consisting of -NHC(O)-, -NH-C(O)-NH-, -NH-C(O)-N(CH₃)-, -NH-C(O)-N(CD₃)-, and -CH=CF-; preferably, L is selected from the group consisting of -NHC(O)- and -NH-C(O)-NH-.

23. The compound according to any one of claims 1 to 22, the stereoisomer thereof, the isotopic derivative thereof, the prodrug thereof, the N-oxide thereof, the solvate thereof, or the pharmaceutically acceptable salt thereof, wherein R is selected from the group consisting of C₁₋₆ haloalkyl, deuterated C₁₋₆ alkyl, C₁₋₆ alkoxy C₁₋₆ alkyl, C₁₋₆ alkyl, C₁₋₆ alkoxy, and deuterated C₁₋₆ alkoxy; and/or Rⁿ¹ is selected from the group consisting of hydrogen atom, C₁₋₆ alkyl, and deuterated C₁₋₆ alkyl.

24. The compound according to any one of claims 1 to 23, the stereoisomer thereof, the isotopic derivative thereof, the prodrug thereof, the N-oxide thereof, the solvate thereof, or the pharmaceutically acceptable salt thereof, wherein R is selected from the group consisting of 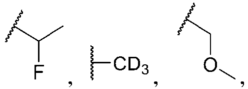 ethyl, methyl, methoxy, and

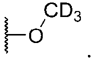

25. The compound according to any one of claims 1 to 24, the stereoisomer thereof, the isotopic derivative thereof, the prodrug thereof, the N-oxide thereof, the solvate thereof, or the pharmaceutically acceptable salt thereof, wherein the structural moiety 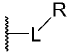 is selected from the group consisting of 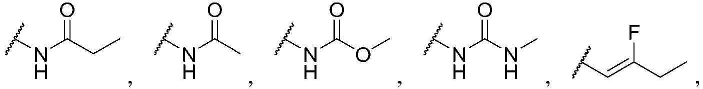 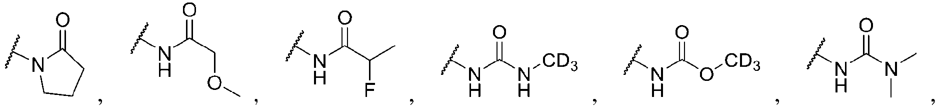 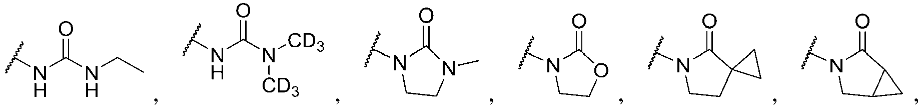 and

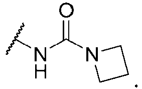

26. The compound according to any one of claims 1 to 25, the stereoisomer thereof, the isotopic derivative thereof, the prodrug thereof, the N-oxide thereof, the solvate thereof, or the pharmaceutically acceptable salt thereof, wherein the compound is selected from the group consisting of:

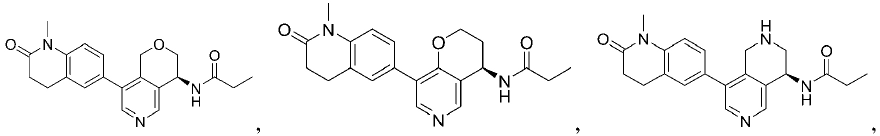

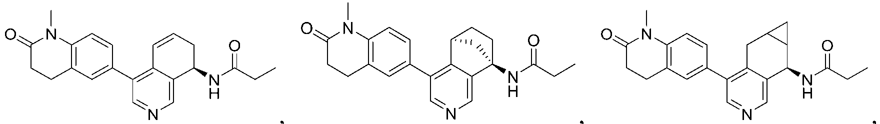

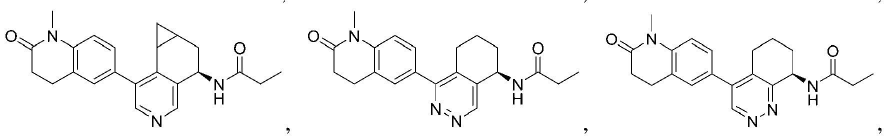

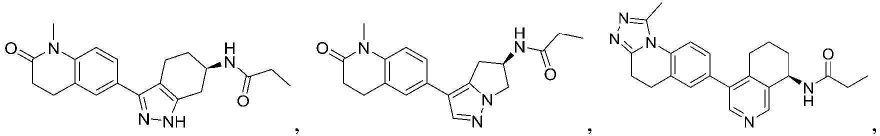

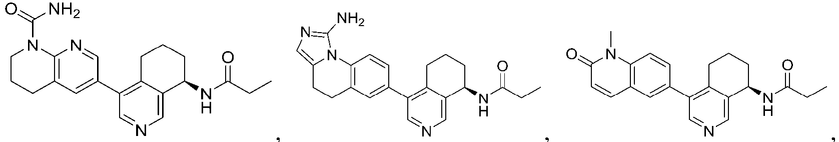

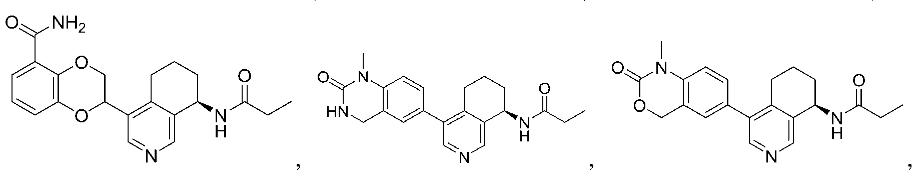

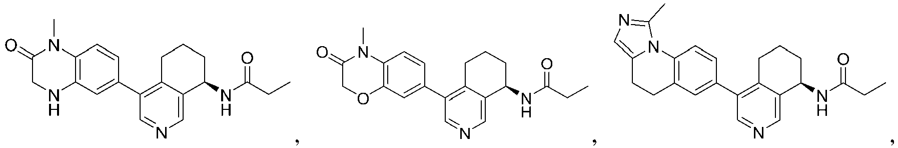

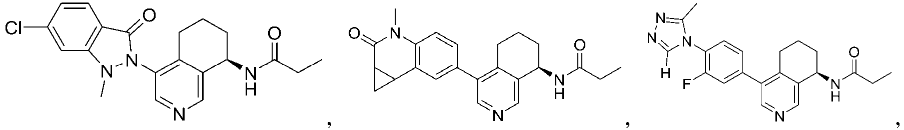

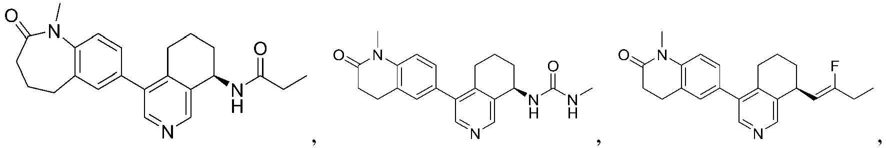

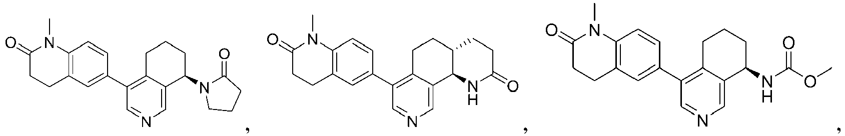

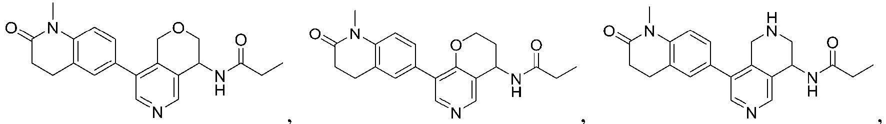

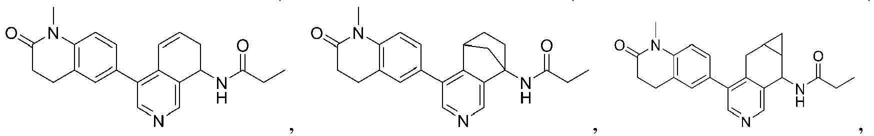

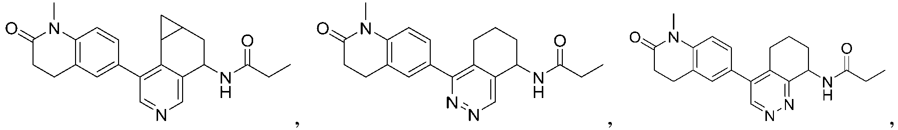

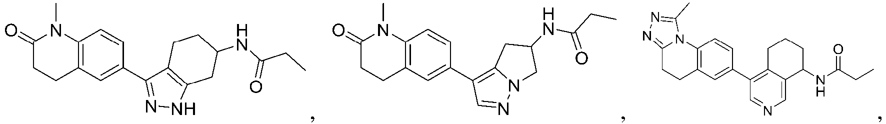

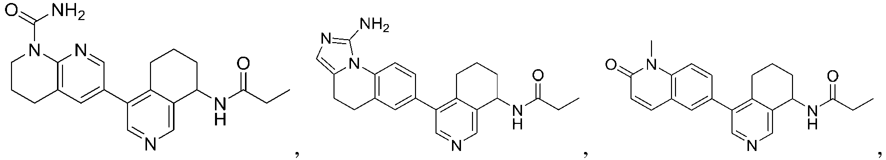

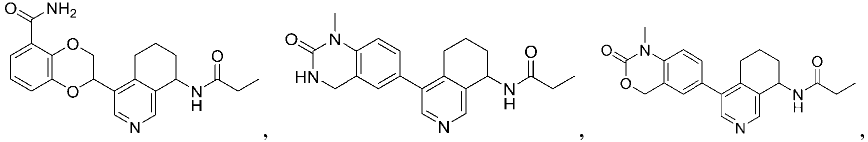

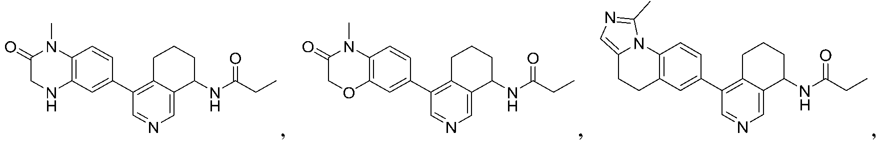

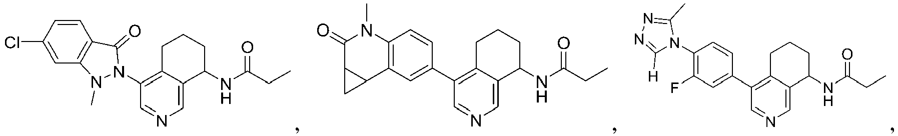

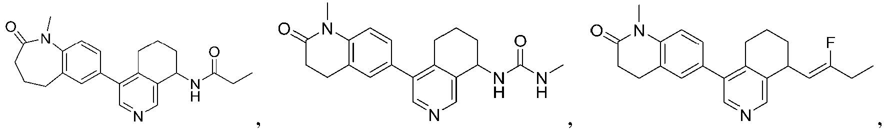

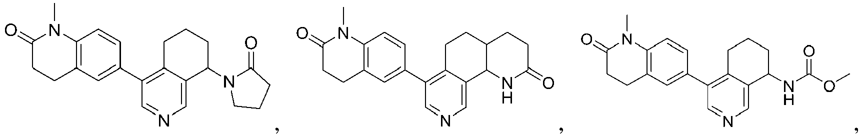

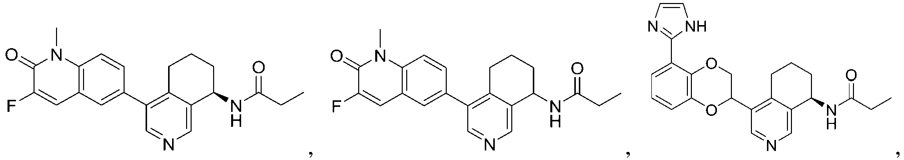

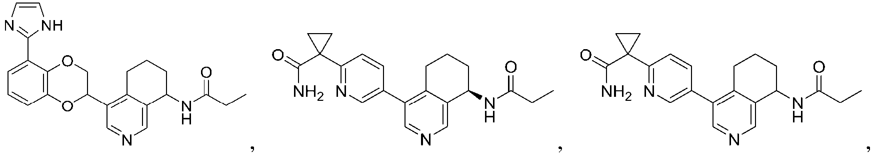

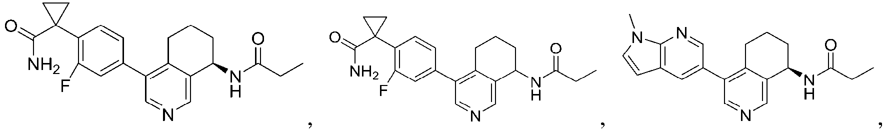

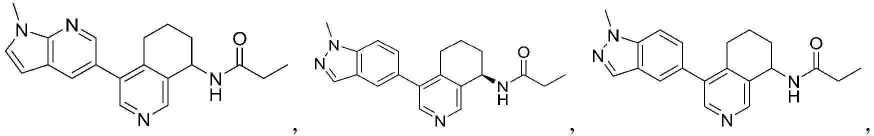

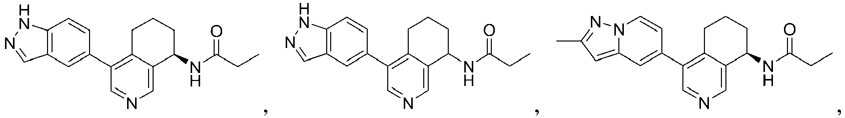

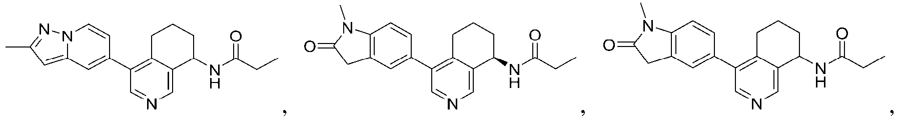

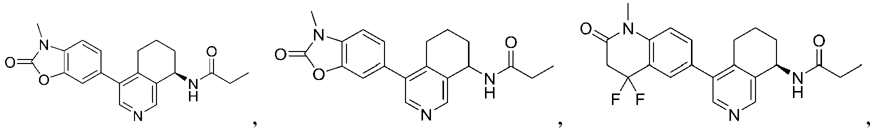

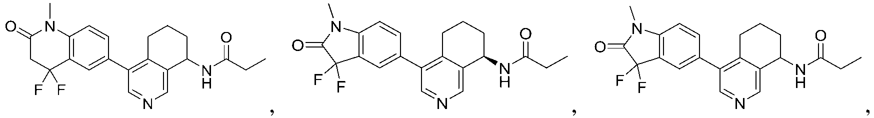

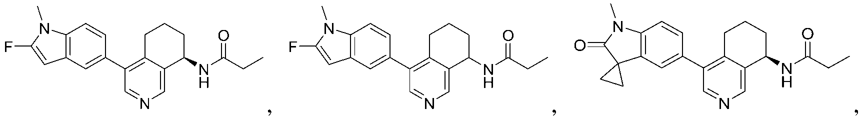

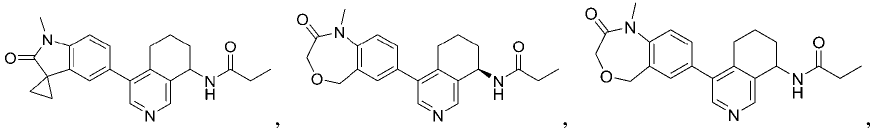

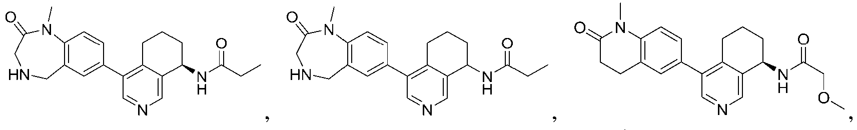

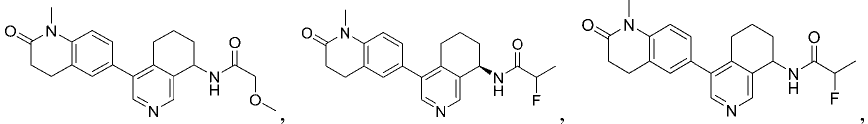

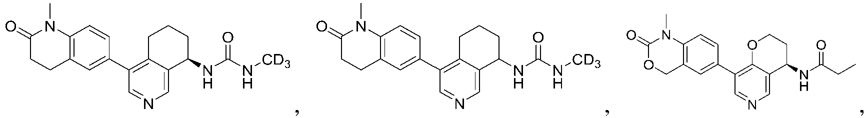

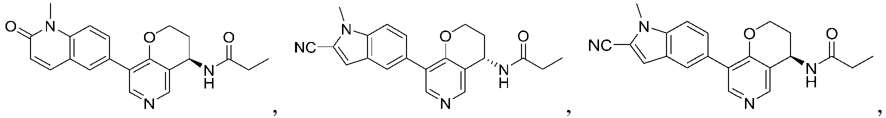

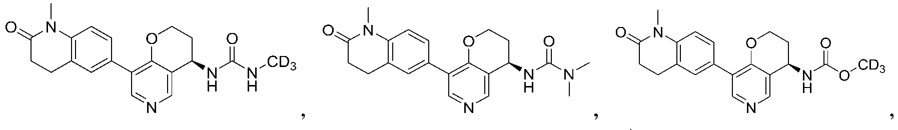

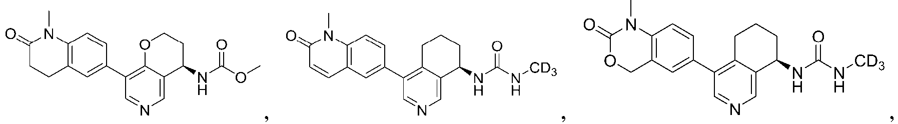

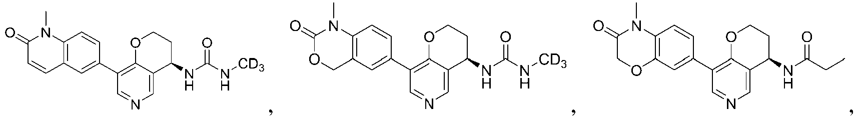

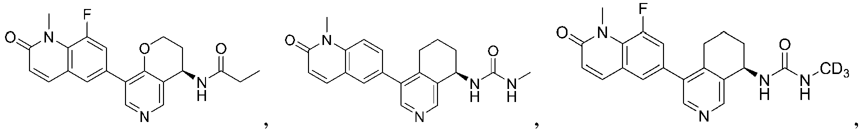

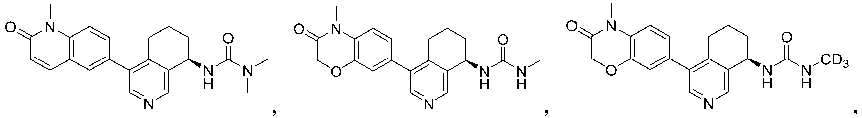

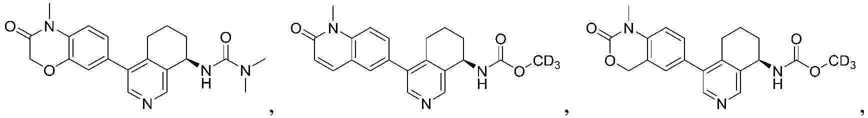

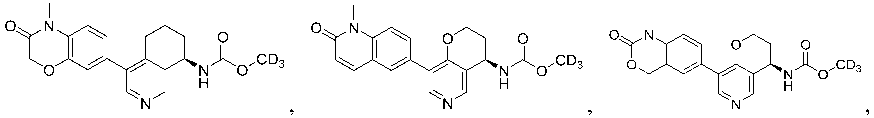

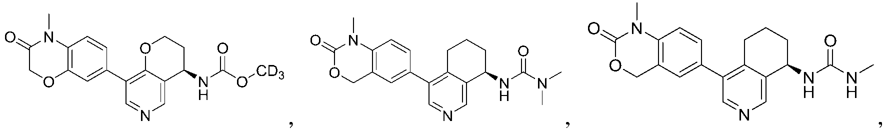

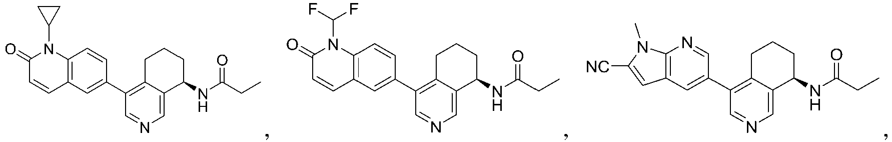

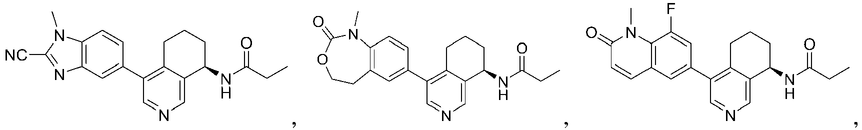

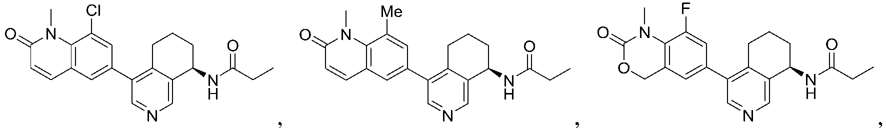

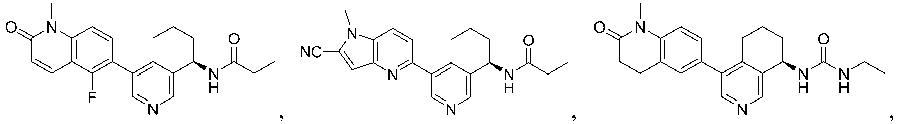

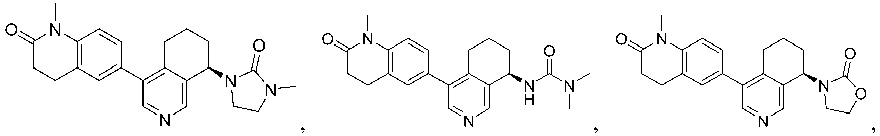

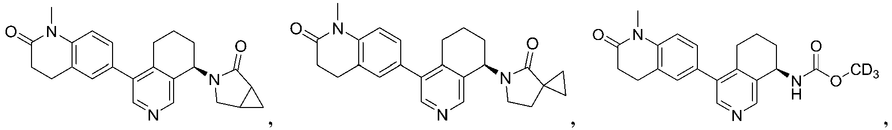

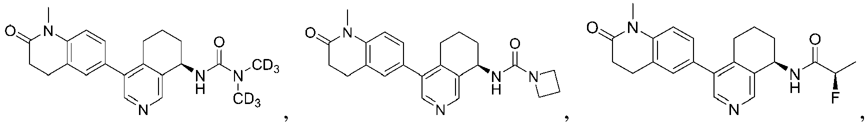

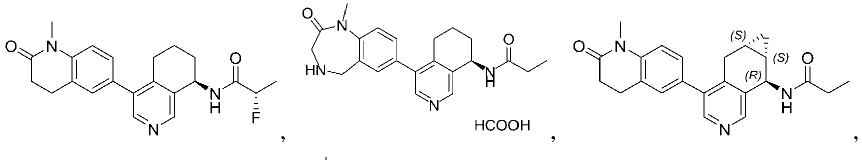

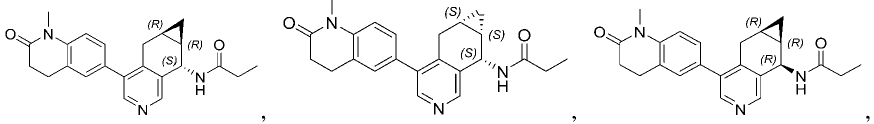

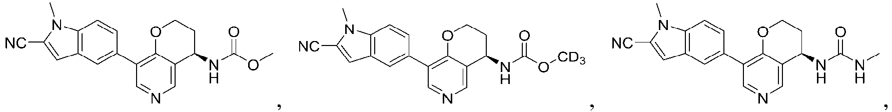

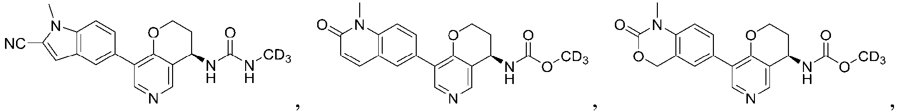

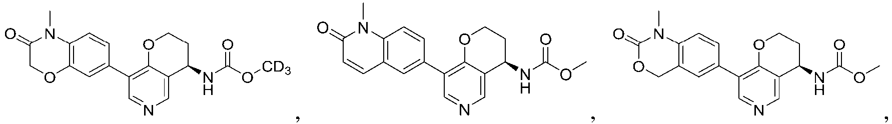

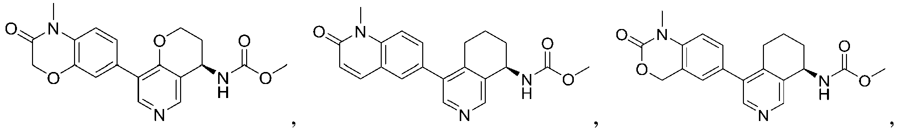

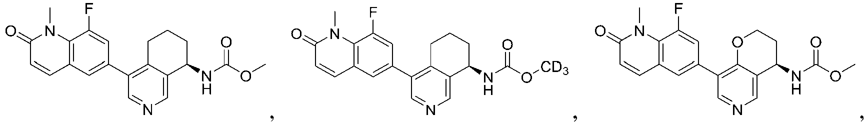

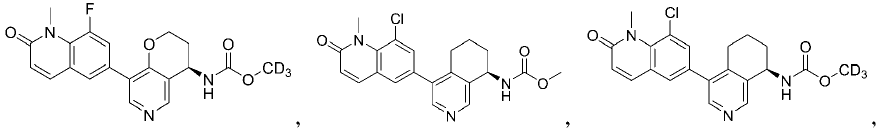

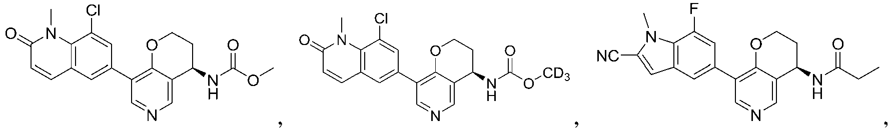

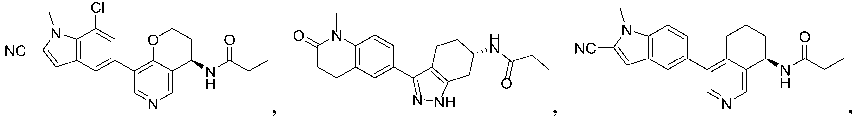

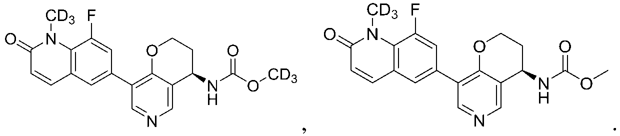

27. A pharmaceutical composition comprising the compound according to any one of claims 1 to 26, the stereoisomer thereof, the isotopic derivative thereof, the prodrug thereof, the N-oxide thereof, the solvate thereof, or the pharmaceutically acceptable salt thereof, and one or more pharmaceutically acceptable carriers.

28. Use of the compound according to any one of claims 1 to 26, the stereoisomer thereof, the isotopic derivative thereof, the prodrug thereof, the N-oxide thereof, the solvate thereof, or the pharmaceutically acceptable salt thereof, or the pharmaceutical composition according to claim 27, in the manufacture of a medicament for inhibiting CYP11B2, preferably in the manufacture of a medicament for selectively inhibiting CYP11B2.

29. Use of the compound according to any one of claims 1 to 26, the stereoisomer thereof, the isotopic derivative thereof, the prodrug thereof, the N-oxide thereof, the solvate thereof, or the pharmaceutically acceptable salt thereof, or the pharmaceutical composition according to claim 27, in the manufacture of a medicament for treating and/or preventing a CYP11B2-mediated disease.

30. The use according to claim 29, wherein the disease is selected from the group consisting of hypertension, chronic kidney disease, diabetic nephropathy, primary aldosteronism, chronic kidney disease, and diabetic nephropathy; the hypertension is preferably selected from the group consisting of resistant hypertension, uncontrolled hypertension, low-renin hypertension, and primary hypertension.

31. Use of the compound according to any one of claims 1 to 26, the stereoisomer thereof, the isotopic derivative thereof, the prodrug thereof, the N-oxide thereof, the solvate thereof, or the pharmaceutically acceptable salt thereof, or the pharmaceutical composition according to claim 27, in the manufacture of a medicament for reducing aldosterone levels in a subject.
